# EUROPEAN PATENT APPLICATION

(11) **EP 4 684 807 A1**
(43) Date of publication of application: **28.01.2026**
(21) Application number: 24774256.2
(22) Date of filing: 22.03.2024
(51) Int. Cl.: A61K 47/68, A61K 31/551, A61K 31/4375, C07K 5/062, C07K 5/027, C07K 7/56, C07D 519/00, A61P 35/00

(54) **LINKER AND USE THEREOF IN LIGAND DRUG CONJUGATE**

(30) Priority: 22.03.2023 CN 202310294300
(71) Applicant: Duality Biologics (Shanghai) Co., Ltd., Shanghai 201204 (CN)
(72) Inventor: ZHANG, Yu, Suzhou, Jiangsu 215000 (CN); YI, Lei, Suzhou, Jiangsu 215000 (CN); LI, Bing, Suzhou, Jiangsu 215000 (CN); YANG, Junjie, Suzhou, Jiangsu 215000 (CN); HUA, Haiqing, Suzhou, Jiangsu 215000 (CN); ZHU, Zhongyuan, Suzhou, Jiangsu 215000 (CN)
(74) Representative: Greaves Brewster LLP
(86) International application number: PCT/CN2024/083287
(87) International publication number: WO 2024/193692

(57) **Abstract**

A linker and a use thereof in a ligand drug conjugate. Provided is a compound as represented by formula (III), or a mesomer, a racemate, an enantiomer, a diastereomer or a mixture form thereof, or a pharmaceutically acceptable salt thereof, or a solvate thereof, or a pharmaceutically acceptable salt of a solvate thereof. The linker can be selectively coupled with thiols in biomacromolecules, has extremely high coupling efficiency and high drug loading capacity; and also shows high chemical stability.

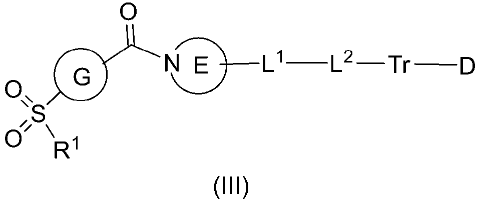

## Description

The present application claims the right of the priority of Chinese patent application 2023102943004 filed on March 22, 2023. The contents of the above Chinese patent application are incorporated herein by reference in its entirety.

### TECHNICAL FIELD

The present disclosure relates to a heteroaromatic ring linker, a ligand-drug conjugate prepared from the linker, and a use of the ligand-drug conjugate in treating tumors or other diseases.

### BACKGROUND

Antibody-drug conjugates (ADCs) link monoclonal antibodies or antibody fragments to biologically active cytotoxic drugs via stable chemical linker compounds, making full use of the specificity of antibodies in binding antigens on the surfaces of normal and tumor cells, as well as the high efficacy of cytotoxic drugs, while avoiding the shortcomings of the former's relatively low efficacy and the latter's excessive toxic side effects. This implies that, compared to traditional chemotherapeutic drugs, antibody-drug conjugates can accurately bind to tumor cells and reduce the impact on normal cells (Mullard A, (2013) Nature Reviews Drug Discovery, 12:329-332; DiJoseph JF, Armellino DC, (2004) Blood, 103:1807-1814).

An antibody-drug conjugate generally consists of several components including an antibody, a linker, a payload, and a conjugation method. Most ADCs currently under investigation contain maleimide-based linkers. In these ADCs, antibody conjugation is generally achieved by reducing the interchain cysteine disulfide bonds of the antibody to provide multiple free thiols as conjugation sites, followed by a Michael addition reaction with maleimide, such as Adcetris and Enhertu. However, according to literature reports, ADCs obtained via thiol-Michael addition may undergo retro-Michael addition during systemic circulation, and the resulting maleimide can further undergo Michael addition reactions with various thiol-containing proteins or small molecules (e.g., plasma albumin) *in vivo,* not only reducing the efficacy of the ADC but also causing potential toxic reactions (Peter D. Senter, Bioconjugate Chem. 2008, 19, 3, 759-765) (Formula A).

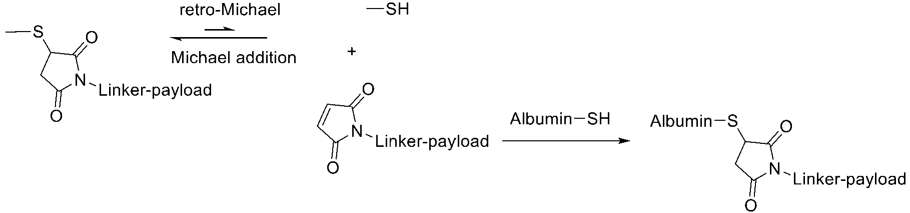

In view of the defects in preparing antibody-drug conjugates in the prior art, there is a need to develop a new linker technology that has high reactivity and selectivity with antibody cysteine and overcomes the instability associated with maleimide linker components.

### SUMMARY

The technical problem to be solved by the present disclosure is the poor stability of antibody-drug conjugates in the prior art. To address this, the present disclosure provides a linker and a use thereof in a ligand-drug conjugate. The linker provided by the present disclosure can selectively conjugate with thiols in biomacromolecules (e.g., antibodies, polypeptides, and proteins), exhibiting high conjugation efficiency and high drug loading capacity. Meanwhile, the bioconjugate generated by the linker maintains nearly unchanged drug-to-antibody ratio (DAR) after incubation with GSH under physiological conditions for over one week or in plasma for over three weeks, showing high chemical stability. In most cases, such high chemical stability will enhance the therapeutic index and/or other advantageous properties of the ADC.

In one aspect, the present disclosure provides a compound of formula (I), or a mesomer, racemate, enantiomer, diastereomer, or any mixture thereof, or a pharmaceutically acceptable salt thereof, or a solvate thereof, or a pharmaceutically acceptable salt of the solvate thereof: 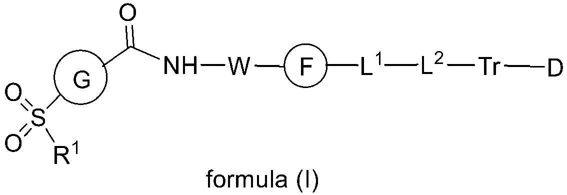 wherein
R¹ is -C₁₋₆ alkyl, -C₃₋₈ cycloalkyl, -C₆₋₁₀ aryl, or 5- to 10-membered heteroaryl, wherein the alkyl, cycloalkyl, aryl, and heteroaryl are each optionally substituted by one or more substituents selected from hydrogen, deuterium, halogen, hydroxyl, cyano, nitro, -OC₁₋₆ alkyl, -C₂₋₆ alkenyl, and -C₂₋₆ alkynyl;
ring G is 5- to 10-membered heteroaryl, wherein the heteroaryl is optionally substituted by one or more substituents selected from hydrogen, deuterium, halogen, hydroxyl, amino, cyano, nitro, -C₁₋₆ alkyl, -OC₁₋₆ alkyl, -NHC₁₋₆ alkyl, and -N(C₁₋₆ alkyl)₂;
W is -(C(R^{w}₂)ₜ-, wherein t is a natural number,
any -C(R^{w})₂- moiety in W is independently and optionally replaced by the following structural moieties: -O-, -S-, -SO₂-, -C(O)-, -NH-, -NC₁₋₆ alkyl-, -N(S(O)₂C₁₋₆ alkyl)-, - N(C(O)C₁₋₆ alkyl)-, -N(C(O)N(C₁₋₆ alkyl)₂)-, 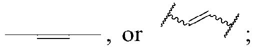 wherein the alkyl is independently and optionally substituted by one or more substituents selected from hydrogen, deuterium, halogen, -CN, -OH, -NH₂, -N(Me)₂, -CO₂H, -S(O)₂Me, -S(O)₂OH, -C(O)NH₂, - SO₂NH₂, and -C₁₋₆ alkyl;
each R^{w} is independently hydrogen, deuterium, halogen, -NO₂, -CN, -OH, -NH₂, - N(C₁₋₆ alkyl)₂, or -C₁₋₆ alkyl, wherein the alkyl is independently and optionally substituted by one or more substituents selected from hydrogen, deuterium, halogen, -CN, -OH, -NH₂, - N(Me)₂, -N⁺(Me)₃, -CO₂H, -S(O)₂Me, -S(O)₂OH, -C(O)NH₂, and -SO₂NH₂;
ring F is 3- to 8-membered cycloalkylene or 4- to 10-membered heterocycloalkylene; wherein the cycloalkylene and heterocycloalkylene are each independently and optionally substituted by one or more substituents selected from hydrogen, deuterium, halogen, hydroxyl, amino, cyano, nitro, -C₁₋₆ alkyl, -C₁₋₆ alkylene-OH, -halo-C₁₋₆ alkyl, -OC₁₋₆ alkyl, -NHC₁₋₆ alkyl, and -N(C₁₋₆ alkyl)₂;
L¹ is -(C(R^{L11})₂)ₙ- (L¹ is a linking moiety);
wherein n is a natural number,
any -C(R^{L11})₂- moiety in L¹ is independently and optionally replaced by the following structural moieties: -Cy-, -NR^{L12}-, -C(O)-, -O-, -S-, -SO-, -SO₂-, -P(R^{L12})-, -P(-O)(R^{L12})-, - C(=S)-, -C(=NR^{L12})-, -N=N-, -C=N-, -N=C-, 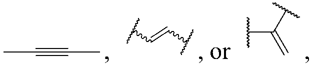
-Cy- is phenylene, 5- to 8-membered heteroarylene, 3- to 10-membered heterocycloalkylene, or 3- to 10-membered cycloalkylene, wherein each -Cy- is independently unsubstituted or substituted by one or more R^{cx},
R^{L11}, R^{L12}, and R^{cx} are each independently hydrogen, deuterium, halogen, -NO₂, -CN, -OR^{L1a}, -SR^{L1a}, -N(R^{L1a})₂, -N⁺(R^{L1a})₃, -C(O)R^{L1a}, -CO₂R^{L1a}, -C(O)C(O)R^{L1a},-C(O)CH₂C(O)R^{L1a}, -S(O)R^{L1a}, -S(O)₂R^{L1a}, -C(O)N(R^{L1a})₂, -SO₂N(R^{L1a})2, -OC(O)R^{L1a}, - N(R^{L1a})SO₂R^{L1b}, -N(R^{L1a})COR^{L1b}, -(CH₂)_{y}-CO-(N(Me)CH₂C(O))ₘ-OR^{L1a}, -(CH₂)_{y}-CO-(N(Me)CH₂C(O))ₘ-NHR^{L1a}, -(CH₂)_{y}-CO-(N(Me)CH₂C(O))ₘ-N⁺(R^{L1a})₃, -(CH₂)_{y}-NHCOCH₂(OCH₂CH₂)OR^{L1a}, -(CH₂)_{y}-NH(COCH₂(N(Me))ₘ-R^{L1a}, -(CH₂)_{y}-CONH-(CH₂CH₂O)ₘ-R^{L1a}, -(CH₂)_{y}-NHCO-(CH₂CH₂O)ₘ-R^{L1a}, -(CH₂CH₂O)ₘ-R^{L1a}, - (COCH₂N(Me))ₘ-R^{L1a}, -COCH₂(OCH₂CH2)ₘ-OR^{L1a}, -CO-(CH₂CH₂O)ₘ-R^{L1a}, -CO-(CH₂)_{y}-CONH-(CH₂CH₂O)ₘ-R^{L1a}, -CO-(CH₂)_{y}-NHCO-(CH₂CH₂O)ₘ-R^{L1a}, -C₁₋₆ alkyl, -C₂₋₆ alkenyl, - C₂₋₆ alkynyl, 3- to 8-membered cycloalkyl, 4- to 10-membered heterocycloalkyl, 6- to 10-membered aryl, or 5- to 10-membered heteroaryl, wherein the -C₁₋₆ alkyl, -C₂₋₆ alkenyl, -C₂₋₆ alkynyl, 3- to 8-membered cycloalkyl, 4- to 10-membered heterocycloalkyl, 6- to 10-membered aryl, and 5- to 10-membered heteroaryl are each independently and optionally substituted by one or more R^{L1a};
m and y are each independently a natural number,
R^{L1a} and R^{L1b} are each independently hydrogen, deuterium, halogen, -NO₂, -CN, -OH, -SH, -NH₂, -N(Me)₂, -CO₂H, -S(O)₂Me, -S(O)₂OH, -C(O)NH₂, -SO₂NH₂, -C₁₋₆ alkyl, -C₂₋₆ alkenyl, -C₂₋₆ alkynyl, 3- to 8-membered cycloalkyl, 4- to 10-membered heterocycloalkyl, 6-to 10-membered aryl, or 5- to 10-membered heteroaryl;
L² is a chemical bond or selected from one or more of an amino acid residue, a short peptide composed of 2 to 10 amino acid residues, 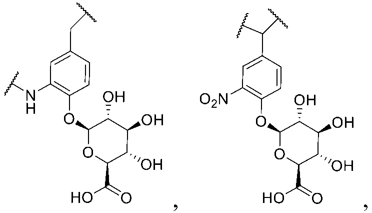 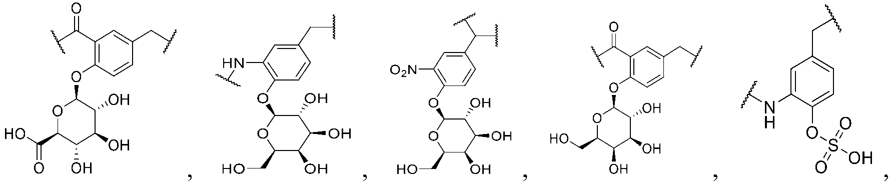 and 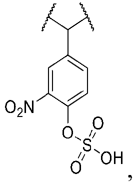 wherein the amino acid residue is a natural amino acid residue or an unnatural amino acid residue;
Tr is a chemical bond or selected from one or more of 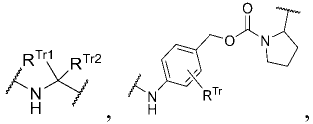 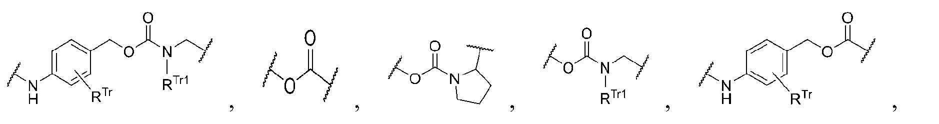 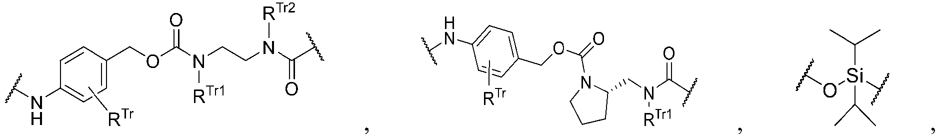 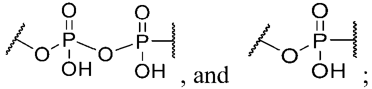
R^{Tr}, R^{Tr1}, and R^{Tr2} are each independently hydrogen, deuterium, halogen, -NO₂, -CN, -OH, -SH, -NH₂, -CO₂H, -S(O)₂OH, -C(O)NH₂, -SO₂NH₂, -OC(O)NH₂, -CH₂CO-(N(Me)CH₂C(O))_{z}-OR^{Tra}, -CH₂CO-(N(Me)CH₂C(O))_{z}-NHR^{Tra}, -(CH₂CH₂O)_{z}-R^{Tra}, -CONH-(CH₂CH₂O)_{z}-R^{Tra}, -C₁₋₆ alkyl, -C₂₋₆ alkenyl, -C₂₋₆ alkynyl, 3- to 8-membered cycloalkyl, 4- to 10-membered heterocycloalkyl, 6- to 10-membered aryl, or 3- to 10-membered heteroaryl, wherein the -C₁₋₆ alkyl, -C₂₋₆ alkenyl, -C₂₋₆ alkynyl, 3- to 8-membered cycloalkyl, 4- to 10-membered heterocycloalkyl, 6- to 10-membered aryl, and 3- to 10-membered heteroaryl are each independently and optionally substituted by one or more R^{Tra};
each R^{Tra} is independently hydrogen, deuterium, halogen, -NO₂, -CN, -OH, -SH, -NH₂, -N(Me)₂, -S(O)₂Me, -CO₂H, -S(O)₂OH, -C(O)NH₂, -SO₂NH₂, -C₁₋₆ alkyl, -C₂₋₆ alkenyl, -C₂₋₆ alkynyl, 3- to 8-membered cycloalkyl, 4- to 10-membered heterocycloalkyl, 6- to 10-membered aryl, or 5- to 10-membered heteroaryl;
z is a natural number;
D is a drug active molecule (e.g., D is a small molecule, nuclide, polypeptide, or nucleic acid);
heteroatoms in the 5- to 10-membered heteroaryl, 4- to 10-membered heterocycloalkylene, 5- to 8-membered heteroarylene, 3- to 10-membered heterocycloalkylene, and 4- to 10-membered heterocycloalkyl are each independently selected from one or more types of N, O, and S, and the number of heteroatoms is independently 1, 2, 3, or 4.

In a preferred embodiment of the present disclosure, in the compound of formula (I), R¹ is -C₁₋₆ alkyl or -C₃₋₈ cycloalkyl, wherein the alkyl and cycloalkyl are each independently and optionally substituted by one or more substituents selected from hydrogen, deuterium, and halogen; preferably, R¹ is -Me, -Et, -isopropyl, or -cyclopropyl; more preferably, R¹ is -Me.

In a preferred embodiment of the present disclosure, in the compound of formula (I), R¹ is -C₁₋₆ alkyl.

In a preferred embodiment of the present disclosure, in the compound of formula (I), ring G is pyridine, pyrimidine, pyridazine, pyrazine, oxazole, isoxazole, thiazole, isoxazole, oxadiazole, thiadiazole, benzothiazole, or benzoxazole; preferably, ring G is 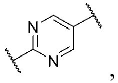 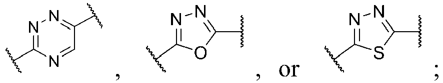 further preferably, ring G is 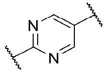 or 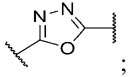 more preferably, ring G is

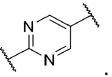

In a preferred embodiment of the present disclosure, in the compound of formula (I), in ring G, ring G may be 5- to 6-membered heteroaryl.

In a preferred embodiment of the present disclosure, in the compound of formula (I), in ring G, the heteroatom of the 5- to 10-membered heteroaryl may be N and/or O, and the number of heteroatoms may be 1, 2, or 3.

In a preferred embodiment of the present disclosure, in the compound of formula (I), ring G is 5- to 6-membered heteroaryl, wherein the heteroatom of the 5- to 6-membered heteroaryl may be N and/or O, and the number of heteroatoms is 1, 2, or 3.

In a preferred embodiment of the present disclosure, in the compound of formula (I), W is -(CH₂)ₜ-; and t is a natural number from 0 to 30;
any -CH₂- moiety in W is independently and optionally replaced by the following structural moieties: -O-, -SO₂-, -C(O)-, or -NH-.

In a preferred embodiment of the present disclosure, in the compound of formula (I), W is -(CH₂)ₜ₁-(O)ₜ₂-(CH₂CH₂O)ₜ₃-(CH₂)ₜ₄-(CO)ₜ₅- or -(CH₂)ₜ₁-(NH)ₜ₂-(CH₂CH₂O)ₜ₃-(CH₂)ₜ₄-(CO)ts-;
t1, t3, and t4 are each independently a natural number from 0 to 6;
t2 and t5 are each independently 0 or 1;

In a preferred embodiment of the present disclosure, in the compound of formula (I), W is a chemical bond, -O-, -NH-, -NHCO-, -(CH2)ₜ₆-, -(CH₂CH₂O)ₜ₆-, -(CH₂CH₂O)ₜ₆-CH₂-CO-, or -(CH2)ₜ₆-(CO)-;
each t6 is independently a natural number from 1 to 6.

In a preferred embodiment of the present disclosure, in the compound of formula (I), t in W is a natural number from 0 to 30; preferably, t is a natural number from 0 to 10; for example, t is 0, 1, 2, or 3.

In a preferred embodiment of the present disclosure, in the compound of formula (I), W is-(CH₂)ₜ-.

In a preferred embodiment of the present disclosure, in the compound of formula (I), any -CH₂- moiety in W is independently and optionally replaced by -O- or -C(O)-.

In a preferred embodiment of the present disclosure, in the compound of formula (I), W is -(CH₂)ₜ-, wherein t is a natural number from 0 to 30; any -CH₂- moiety in W is independently and optionally replaced by the following structural moieties: -O- or -C(O)-; preferably, W is -(CH₂)ₜ₁-(O)ₜ₂-(CH₂CH₂O)ₜ₃-(CH₂)ₜ₄-(CO)ₜ₅-;
t1, t3, and t4 are each independently 0, 1, 2, 3, 4, 5, or 6;
t2 and t5 are each independently 0 or 1;
further preferably, W is -(CH₂CH₂O)ₜ₆- or -(CH2)ₜ₆-(CO)-; each t6 is independently 0, 1, 2, 3, 4, 5, or 6; for example, W is a chemical bond, -CH₂CH₂O-, or -(CH₂)-(CO)-.

In a preferred embodiment of the present disclosure, in the compound of formula (I), ring F is 3- to 6-membered cycloalkylene or 4- to 6-membered heterocycloalkylene; wherein the cycloalkylene and heterocycloalkylene are each independently and optionally substituted by one or more substituents selected from hydrogen, deuterium, halogen, hydroxyl, amino, cyano, and nitro;
preferably, ring F is 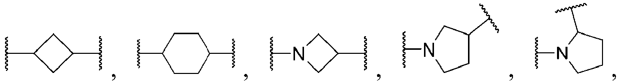 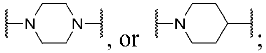
more preferably, ring F is

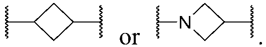

In a preferred embodiment of the present disclosure, in the compound of formula (I), in F, 3- to 8-membered cycloalkylene may be 3- to 6-membered cycloalkylene, for example,

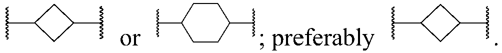

In a preferred embodiment of the present disclosure, in the compound of formula (I), in F, 4- to 10-membered heterocycloalkylene may be 4- to 6-membered heterocycloalkylene.

In a preferred embodiment of the present disclosure, in the compound of formula (I), in F, the heteroatom of the 4- to 10-membered heterocycloalkylene may be N, and the number of heteroatoms may be 1 or 2.

In a preferred embodiment of the present disclosure, in the compound of formula (I), in F, the 4- to 10-membered heterocycloalkylene is

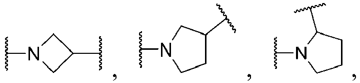

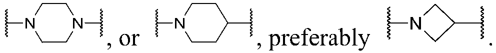

In a preferred embodiment of the present disclosure, in the compound of formula (I), ring F is 3- to 6-membered cycloalkylene or 4- to 6-membered heterocycloalkylene; wherein the heteroatom of the 4- to 6-membered heterocycloalkylene is N, and the number of heteroatoms is 1 or 2.

In a preferred embodiment of the present disclosure, in the compound of formula (I), L¹ is -(CHR^{L11})ₙ-;
wherein n is a natural number from 0 to 50;
any -CHR^{L11}- moiety in L¹ is independently and optionally replaced by the following structural moieties: -Cy-, -NR^{L12}-, -C(O)-, -S-, -O-, 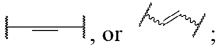
-Cy- is phenylene, 5- to 6-membered heteroarylene, 4- to 10-membered heterocyclylene, or 3- to 6-membered cycloalkylene, wherein each -Cy- is independently unsubstituted or substituted by 1 to 3 R^{cx};
R^{L11}, R^{L12}, and R^{cx} are each independently hydrogen, halogen, -OR^{L1a}, -N(R^{L1a})₂, - C(O)R^{L1a}, -S(O)₂R^{L1a}, -C(O)N(R^{L1a})₂, -SO₂N(R^{L1a})2, -N(R^{L1a})SO₂R^{L1b}, -N(R^{L1a})COR^{L1b}, - (CH₂)_{y}-CO-(N(Me)CH₂C(O))ₘ-OR^{L1a}, -(CH₂)_{y}-CO-(N(Me)CH₂C(O))ₘ-NHR^{L1a}, -(CH₂)_{y}-CONH-(CH₂CH₂O)ₘ-R^{L1a}, -(CH₂)_{y}-NHCO-(CH₂CH₂O)ₘ-R^{L1a}, -(CH₂)_{y}-NHCOCH₂(OCH₂CH₂)OR^{L1a}, -(CH₂)_{y}-NH(COCH₂(N(Me))ₘ-R^{L1a}, -(CH₂)_{y}-NHCO-(CH₂CH₂O)ₘ-R^{L1a}, -(CH₂CH₂O)ₘ-R^{L1a}, -(COCH₂N(Me))ₘ-R^{L1a}, -COCH₂(OCH₂CH₂)ₘ-OR^{L1a}, -CO-(CH₂CH₂O)ₘ-R^{L1a}, -C₁₋₆ alkyl, -C₂₋₆ alkenyl, -C₂₋₆ alkynyl, 3- to 8-membered cycloalkyl, 4- to 10-membered heterocycloalkyl, 6- to 10-membered aryl, or 5- to 10-membered heteroaryl; wherein the -C₁₋₆ alkyl, -C₂₋₆ alkenyl, -C₂₋₆ alkynyl, 3- to 8-membered cycloalkyl, 4- to 10-membered heterocycloalkyl, 6- to 10-membered aryl, and 5- to 10-membered heteroaryl are each independently and optionally substituted by one or more R^{L1a};
m is a natural number from 0 to 8;
y is 0, 1, 2, 3, or 4;
R^{L1a} and R^{L1b} are each independently hydrogen, halogen, -CN, -OH, -NH₂, -N(Me)₂, -CO₂H, -C(O)NH₂, or -C₁₋₆ alkyl.

In a preferred embodiment of the present disclosure, in the compound of formula (I), L¹ is -(CH₂)ₙ-;
wherein n is a natural number from 0 to 50;
any -CH₂- moiety in L¹ is independently and optionally replaced by the following structural moieties: phenylene, 5- to 6-membered heteroarylene, 4- to 6-membered heterocyclylene, 3- to 6-membered cycloalkylene, -NR^{L12}-, -C(O)-, -S-, or -O-;
each R^{L12} is independently hydrogen, -OR^{L1a}, -C(O)R^{L1a}, -S(O)₂R^{L1a}, -C(O)N(R^{L1a})₂, -SO₂N(R^{L1a})₂, -(CH₂)_{y}-CO-(N(Me)CH₂C(O))ₘ-OR^{L1a}, -(CH₂)_{y}-CO-(N(Me)CH₂C(O))ₘ-NHR^{L1a}, -(CH₂)_{y}-CONH-(CH₂CH₂O)ₘ-R^{L1a}, -(CH₂)_{y}-NHCOCH₂(OCH₂CH₂)OR^{L1a}, - (CH₂CH₂O)ₘ-R^{L1a}, -(COCH₂N(Me))m-R^{L1a}, -COCH₂(OCH₂CH₂)ₘ-OR^{L1a}, -CO-(CH₂CH₂O)ₘ-R^{L1a}, or -C₁₋₆ alkyl optionally substituted by R^{L1a};
m is a natural number from 0 to 8;
y is 0, 1, 2, 3, or 4;
each R^{L1a} is independently hydrogen, halogen, -CN, -OH, -NH₂, -N(Me)₂, -CO₂H, - C(O)NH₂, or -C₁₋₆ alkyl.

In a preferred embodiment of the present disclosure, in the compound of formula (I), L¹ is -(CH₂)ₙ-;
wherein n is a natural number from 0 to 50;
any -CH₂- moiety in L¹ is independently and optionally replaced by the following structural moieties: -NH-, -NMe, -C(O)-, -S-, or -O-.

In a preferred embodiment of the present disclosure, in the compound of formula (I), L¹ is: 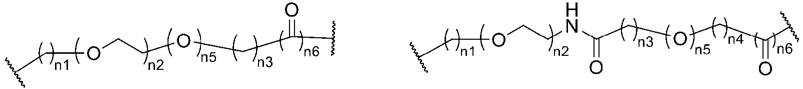 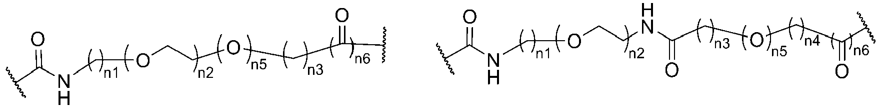 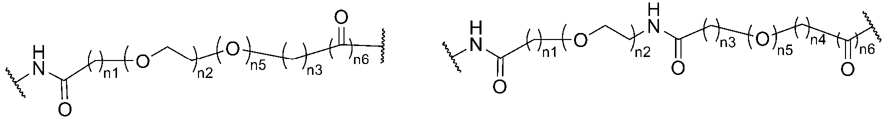 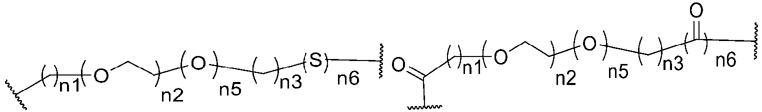 wherein
n1, n2, n3, and n4 are each independently a natural number from 0 to 8;
n5 and n6 are each independently 0 or 1.

In a preferred embodiment of the present disclosure, in the compound of formula (I), in L¹, n may be a natural number from 0 to 50; preferably, n is a natural number from 1 to 10, for example, 1, 2, 3, 4, or 5.

In a preferred embodiment of the present disclosure, in the compound of formula (I), R^{L11} and R^{L12} in L¹ may each be hydrogen.

In a preferred embodiment of the present disclosure, in the compound of formula (I), in L¹, any -CHR^{L11}- moiety in L¹ is independently and optionally replaced by the following structural moieties: -C(O)-, -O-, or -S-.

In a preferred embodiment of the present disclosure, in the compound of formula (I), L¹ is -(CH₂)ₙ-.

In a preferred embodiment of the present disclosure, in the compound of formula (I), L¹ is -(CH₂)ₙ-, wherein n is a natural number from 0 to 50, and any -CH₂- moiety in L¹ is independently and optionally replaced by the following structural moieties: -C(O)-, -O-, or -S-; preferably, L¹ is 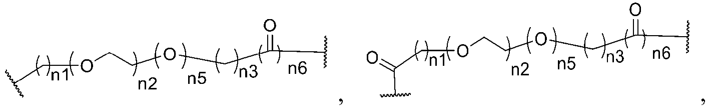 or 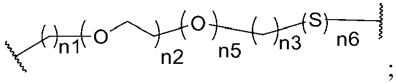 n1, n2, and n3 are each independently 0, 1, 2, 3, 4, 5, 6, 7, or 8;

n5 and n6 are each independently 0 or 1;
for example, L¹ is

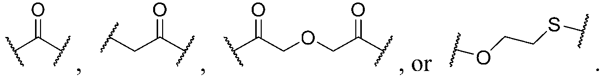

In a preferred embodiment of the present disclosure, in the compound of formula (I), L² is a chemical bond or an amino acid residue, a short peptide composed of 2 to 7 amino acid residues, 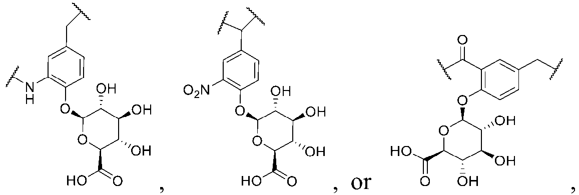 wherein the amino acid residue is Val, D-Val, Nva, Phe, Lys, Leu, Ile, Gly, Ala, D-Ala, Cit, Asp, Asn, Glu, Gln, Pro, Arg, Lys(Ac), Lys(Me), Lys(Et), Lys(nPr), Nva, or AA;
AA is

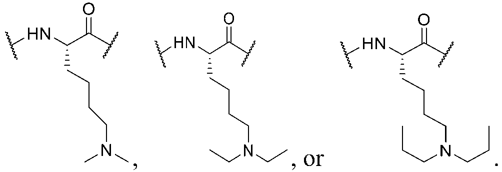

In a preferred embodiment of the present disclosure, in the compound of formula (I), L² is a chemical bond, Val, D-Val, Phe, Lys, Leu, Ile, Gly, Ala, D-Ala, Cit, Asp, Asn, Glu, Gln, Val-Cit, Val-Ala, Val-Lys, Val-Lys(Ac), Phe-Lys, Phe-Lys(Ac), Leu-Lys, Leu-Lys(Ac), Ala-Ala, Ala-Lys, D-Ala-Ala, Gly-Glu, Gly-Asp, Gly-Asn, Val-Glu, Val-Asp, Asp-Asp, Asn-Asn, Glu-Val-Cit, Ala-Ala-Ala, Ala-(D-Ala)-Ala, Ala-Ala-Asn, Ala-(D-Ala)-Asn, Ala-Ala-Asp, Val-Lys-Gly, D-Val-Leu-Lys, Gly-Gly-Arg, Gly-Gly-Gly, Lys-Ala-Asn, Gly-Phe-Gly, Gly-Gly-Phe, Asn-Pro-Val, Ala-Lys-Gly, Gly-Lys-Gly, Gly-Glu-Gly, Gly-Gly-Gly-Gly, Gly-Gly-Phe-Gly, Gly-Gly-Glu-Gly, Lys-Ala-Ala-Asn, Lys-Ala-Ala-Asp, Ala-Ala-Pro-Val, Ala-Ala-Pro-Nva,

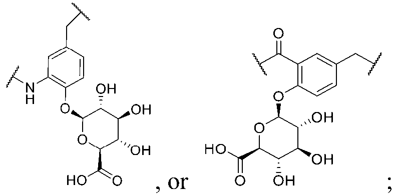

In a preferred embodiment of the present disclosure, in the compound of formula (I), L² is a chemical bond, Lys, Cit, Asp, Asn, Glu, Gln, Val-Cit, Val-Ala, Asp-Asp, Ala-Ala, Gly-Glu, Gly-Asp, Gly-Asn, Asn-Asn, Gly-Glu-Gly, Gly-Gly-Glu-Gly, Gly-Gly-Phe-Gly, or Gly-Gly-Gly-Gly.

In a preferred embodiment of the present disclosure, in the compound of formula (I), L² is a chemical bond, Val-Cit, Val-Ala, Gly-Glu, Gly-Asp, Gly-Glu-Gly, Gly-Gly-Phe-Gly, or

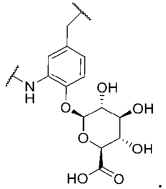

In a preferred embodiment of the present disclosure, in the compound of formula (I), 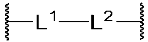 is: 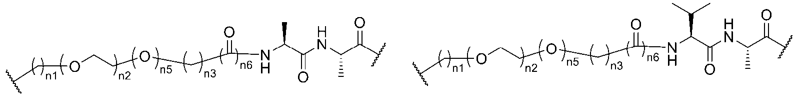 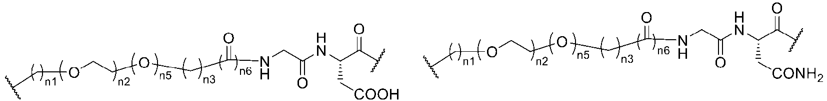 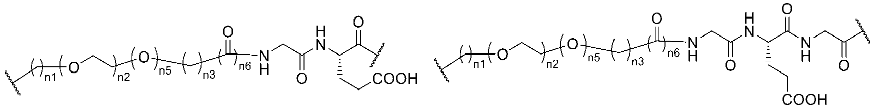 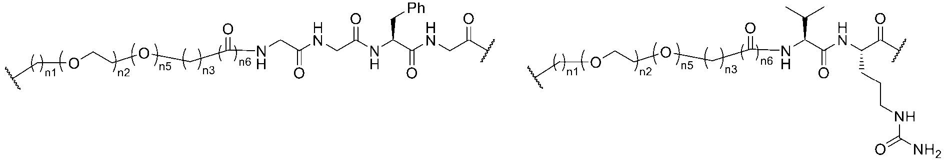 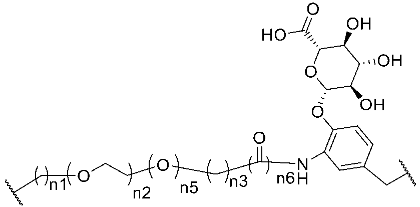 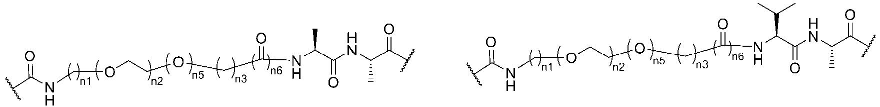 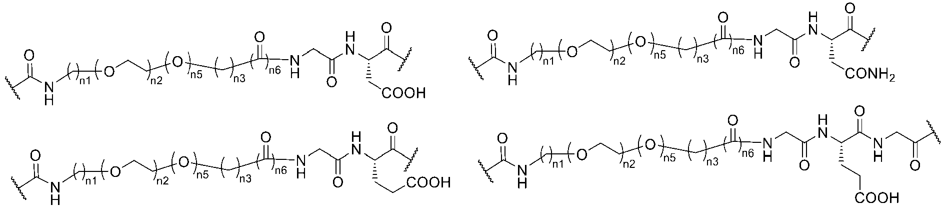 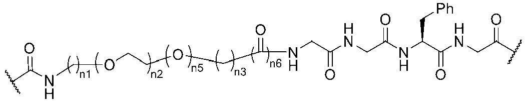 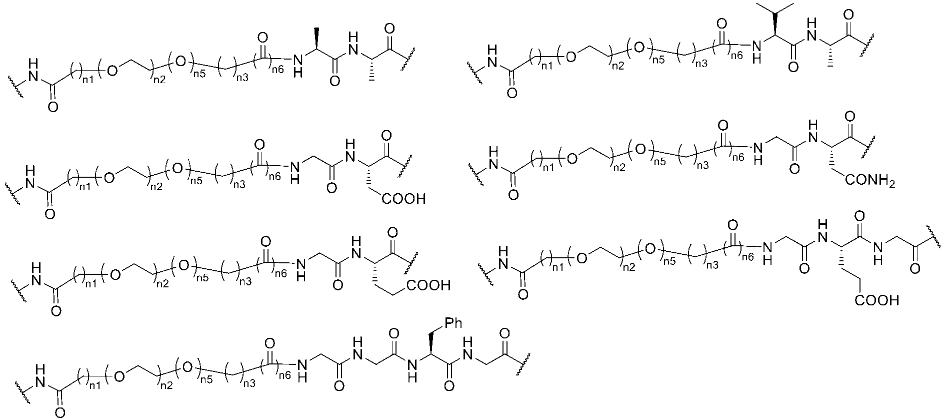 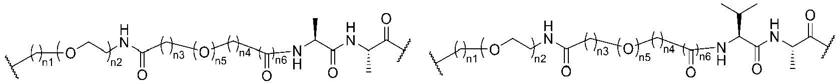 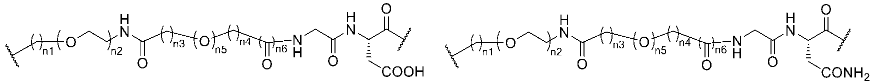 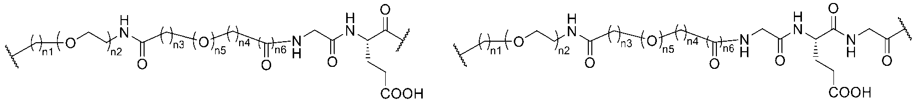 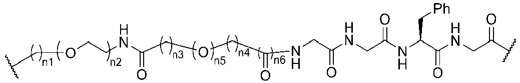 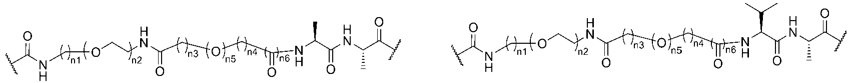 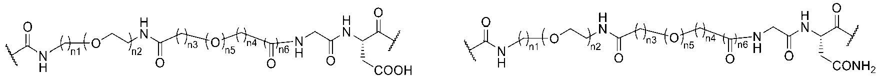 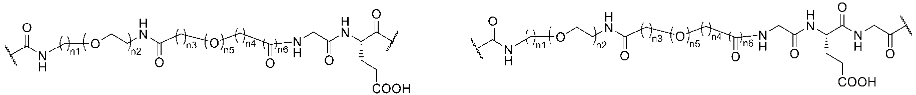 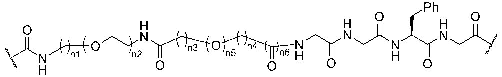 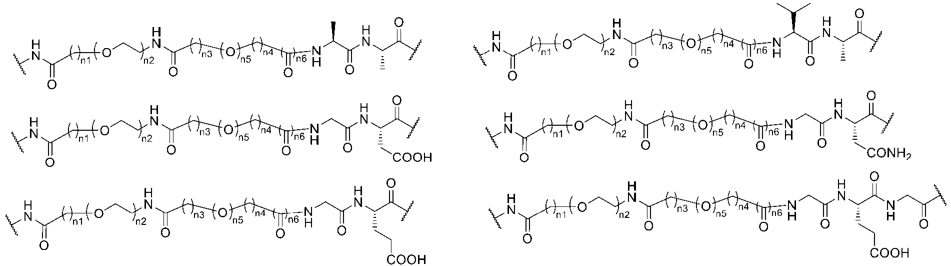 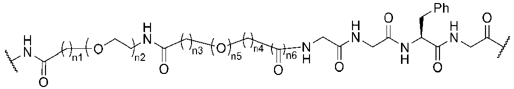 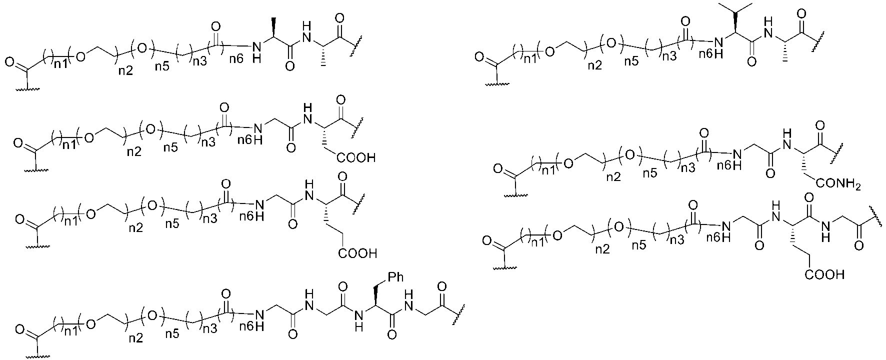 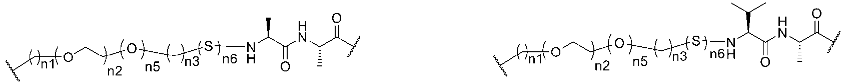 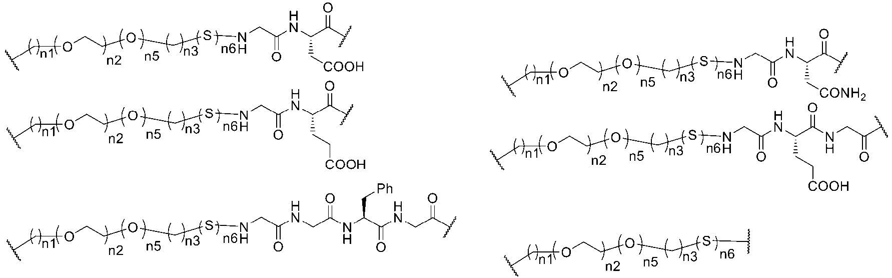
wherein n1, n2, n3, and n4 are each independently a natural number from 0 to 8;
n5 and n6 are each independently 0 or 1.

In a preferred embodiment of the present disclosure, in the compound of formula (I), 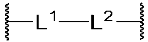 is: 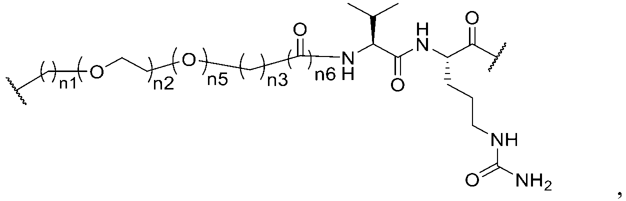 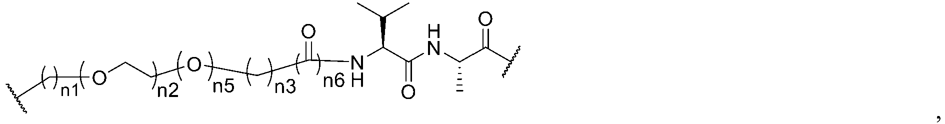 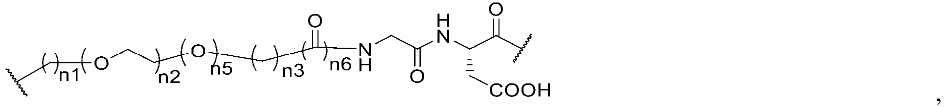 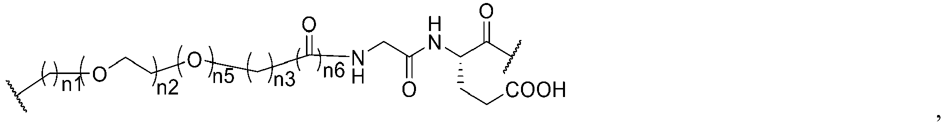 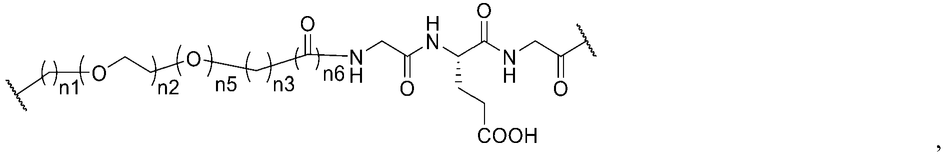 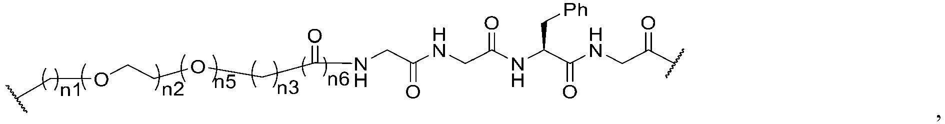 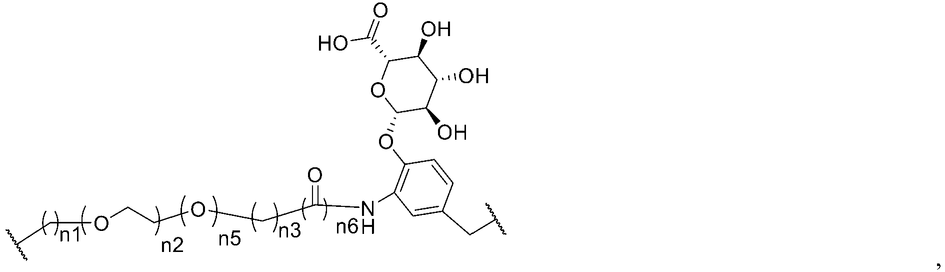 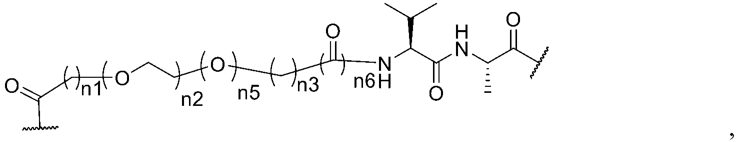 or 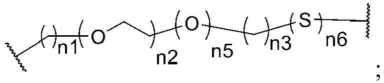
n1, n2, and n3 are each independently 0, 1, 2, 3, 4, 5, 6, 7, or 8;
n5 and n6 are each independently 0 or 1;
for example, 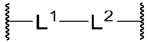 is

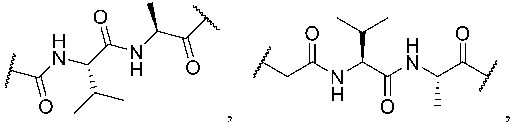

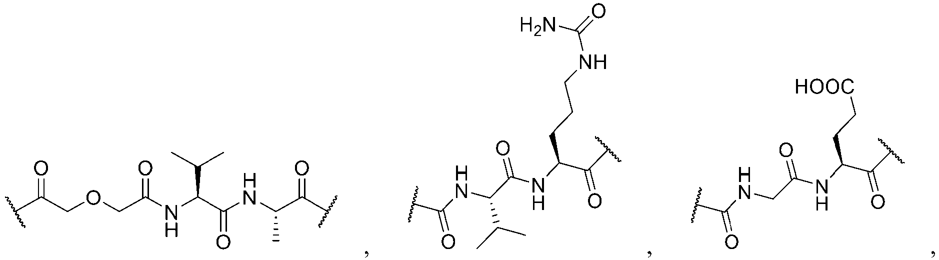

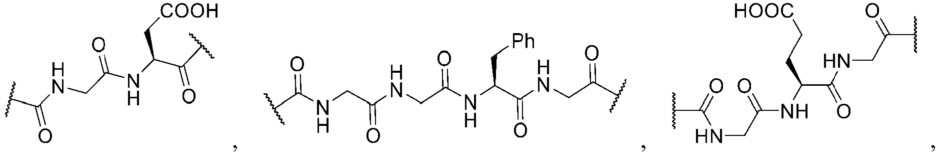

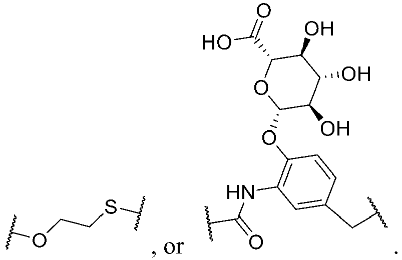

In a preferred embodiment of the present disclosure, in the compound of formula (I), Tr is a chemical bond or selected from one or more of 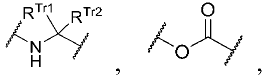 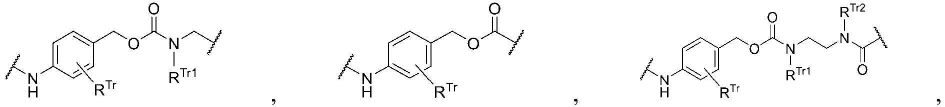 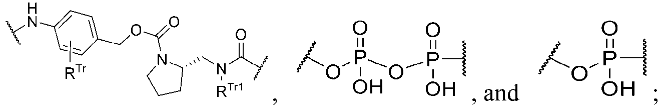
R^{Tr}, R^{Tr1}, and R^{Tr2} are each independently hydrogen, halogen, -NO₂, -CN, -OH, -NH₂, -CO₂H, -S(O)₂H, -C(O)NH₂, -SO₂NH₂, -OC(O)NH₂, -CH₂CO-(N(Me)CH₂C(O))_{z}-OR^{Tra}, - CH₂CO-(N(Me)CH₂C(O))_{z}-NHR^{Tra}, -(CH₂CH₂O)_{z}-R^{Tra}, -CONH-(CH₂CH₂O)_{z}-R^{Tra}, or -C₁₋₆ alkyl optionally substituted by R^{Tra};
R^{Tra} is hydrogen, deuterium, halogen, -NO₂, -CN, -OH, -NH₂, -N(Me)₂, -S(O)₂Me, - CO₂H, -S(O)₂OH, -C(O)NH₂, -SO₂NH₂, or -C₁₋₆ alkyl;
z is a natural number.

In a preferred embodiment of the present disclosure, in the compound of formula (I), Tr is a chemical bond or selected from one or more of

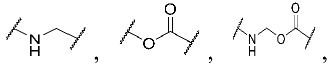

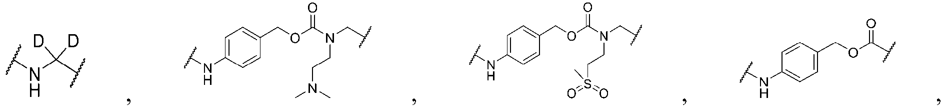

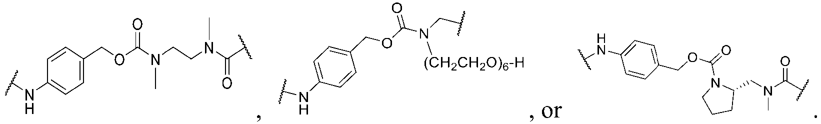

In a preferred embodiment of the present disclosure, in the compound of formula (I), Tr is a chemical bond,

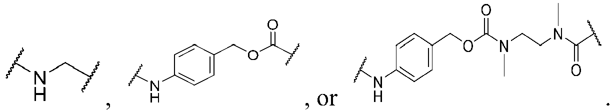

In a preferred embodiment of the present disclosure, in the compound of formula (I), R^{Tr}, R^{Tr1}, and R^{Tr2} in Tr are each independently hydrogen or methyl.

In a preferred embodiment of the present disclosure, in the compound of formula (I), Tr is a chemical bond or selected from one or more of 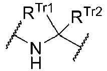 (e.g., 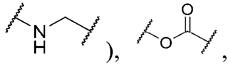 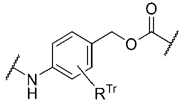 (e.g., 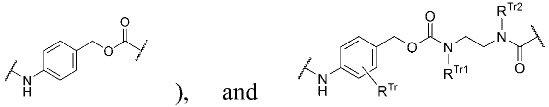 (e.g.,

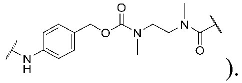

In a preferred embodiment of the present disclosure, in the compound of formula (I), Tr is a chemical bond, 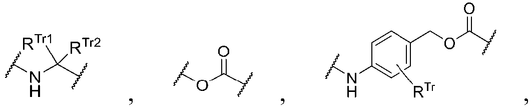 or 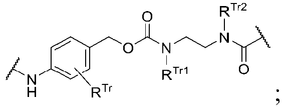 R^{Tr}, R^{Tr1}, and R^{Tr2} are each independently hydrogen or -C₁₋₆ alkyl, for example, Tr is a chemical bond, 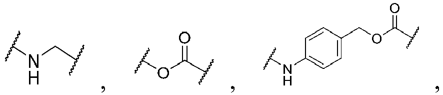 or

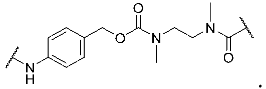

In a preferred embodiment of the present disclosure, in the compound of formula (I), D may be a tubulin inhibitor, a topoisomerase inhibitor, a DNA damaging agent, an RNA polymerase inhibitor, an immunomodulator, or an immunostimulant.

In a preferred embodiment of the present disclosure, the tubulin inhibitor may be Auristatins (e.g., MMAE, MMAF, or MMAD), Maytansinoids (e.g., DM1 or DM4), Vinblastines, Taxols, Eribulins, Tubulysins, Colchicines, or Hemiasterlin; or a stereoisomer, isostere, analog, or derivative thereof; for example, 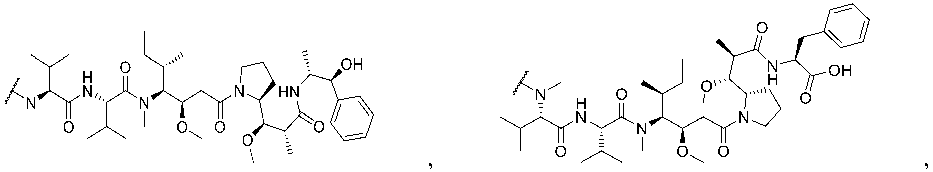 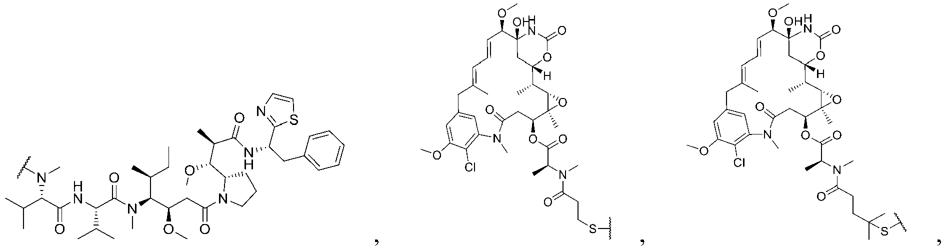 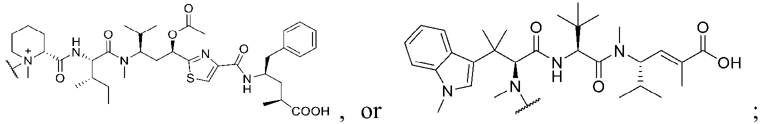 more preferably

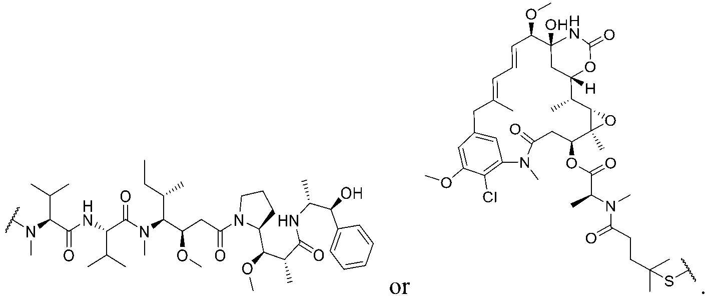

In a preferred embodiment of the present disclosure, the topoisomerase inhibitor may be camptothecin (e.g., SN38 or DX8951F); for example, 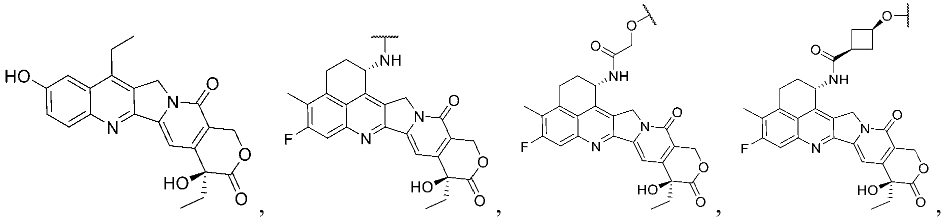 or

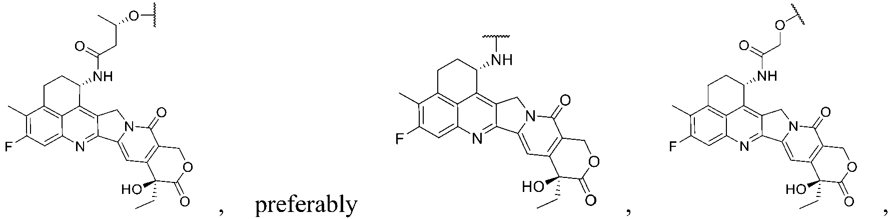

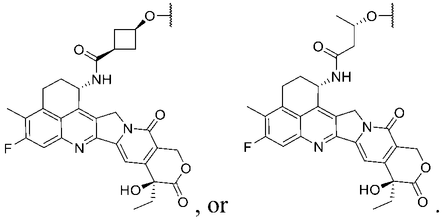

In a preferred embodiment of the present disclosure, the DNA damaging agent may be Calicheamicins, Mitomycins, anthracyclines (e.g., doxorubicin), methotrexates, Duocarmycins, a CBI dimer, a CPI dimer, a CTI dimer, a PBD dimer, an IGN dimer, a PDD dimer, or a Duocarmycin-PBD dimer; for example, 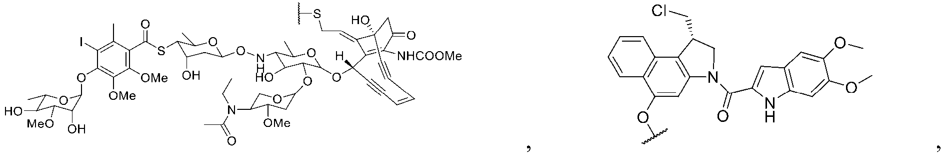 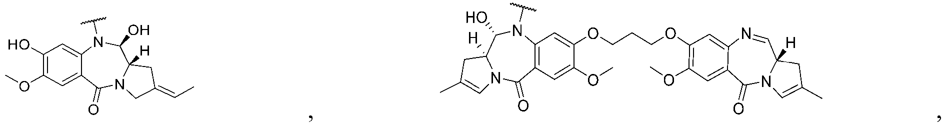 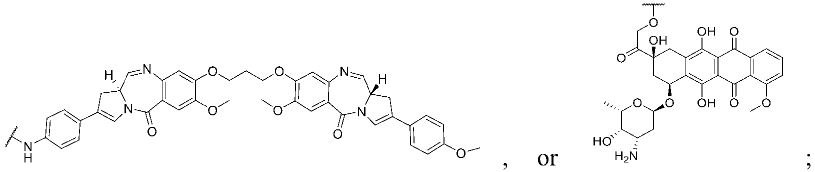 preferably

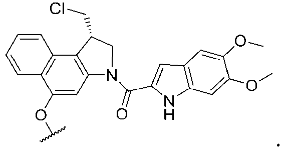

In a preferred embodiment of the present disclosure, the RNA polymerase inhibitor may be Amatoxins or a spliceosome inhibitor (e.g., Thailanstatin A); for example,

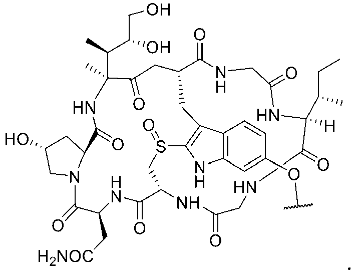

In a preferred embodiment of the present disclosure, the immunomodulator may be a glucocorticoid; for example,

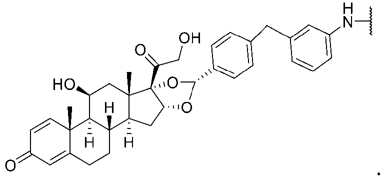

In a preferred embodiment of the present disclosure, the immunostimulant may be (e.g., Sting or TLR7/8 agonist), a protein toxin (e.g., interleukin, interferon, or tumor necrosis factor TNF-α), a selective or non-selective kinase inhibitor, a Bcl-x1 inhibitor, and a Bcl-2/Bcl-X1 inhibitor; for example,

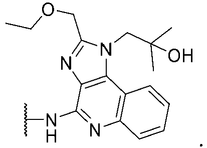

In a preferred embodiment of the present disclosure, in the compound of formula (I), D is a tubulin inhibitor, a topoisomerase inhibitor, a DNA damaging agent, an RNA polymerase inhibitor, an immunomodulator, or an immunostimulant, for example, D is

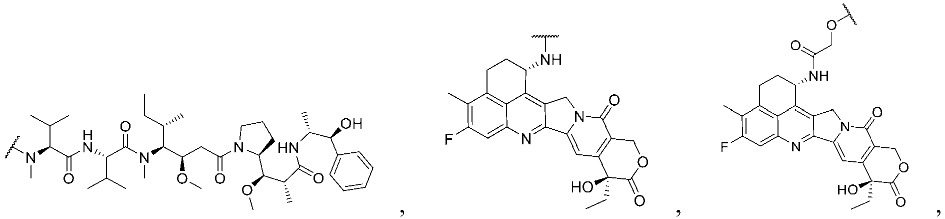

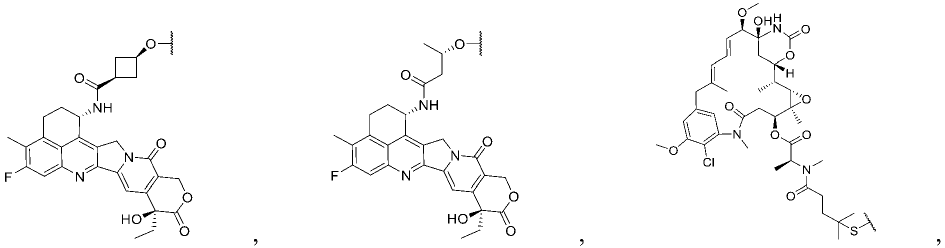

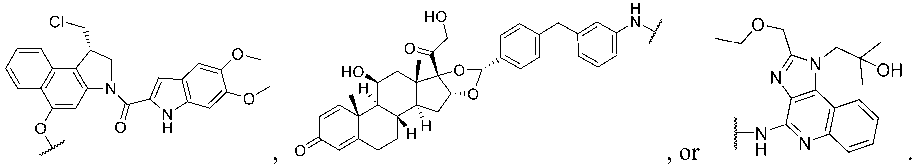

In a preferred embodiment of the present disclosure, in the compound of formula (I),
R¹ is -C₁₋₆ alkyl;
ring G is 5- to 6-membered heteroaryl, wherein the heteroatom of the 5- to 6-membered heteroaryl may be N and/or O, and the number of heteroatoms is 1, 2, or 3; for example, ring G is 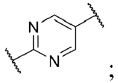
W is -(CH₂)ₜ-, wherein t is a natural number from 0 to 30;
any -CH₂- moiety in W is independently and optionally replaced by the following structural moieties: -O- or -C(O)-;
ring F is 3- to 6-membered cycloalkylene or 4- to 6-membered heterocycloalkylene; wherein the heteroatom of the 4- to 6-membered heterocycloalkylene is N, and the number of heteroatoms is independently 1 or 2;
L¹ is -(CH₂)ₙ-; wherein n is a natural number from 0 to 50;
any -CH₂- moiety in L¹ is independently and optionally replaced by the following structural moieties: -C(O)-, -NH-, -O-, or -S-;
L² is a chemical bond, Val-Cit, Val-Ala, Gly-Glu, Gly-Asp, Gly-Glu-Gly, Gly-Gly-Phe-Gly, or 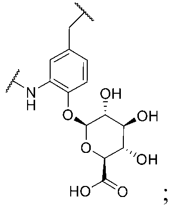
Tr is a chemical bond, 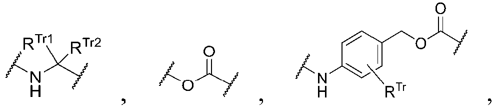 or 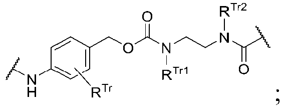 R^{Tr}, R^{Tr1}, and R^{Tr2} are each independently hydrogen or -C₁₋₆ alkyl;
D is a tubulin inhibitor, a topoisomerase inhibitor, a DNA damaging agent, an RNA polymerase inhibitor, an immunomodulator, or an immunostimulant, for example, D is

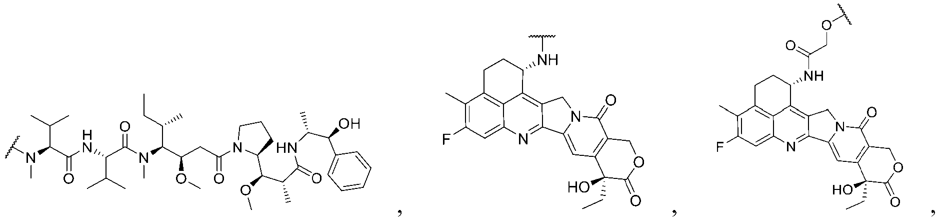

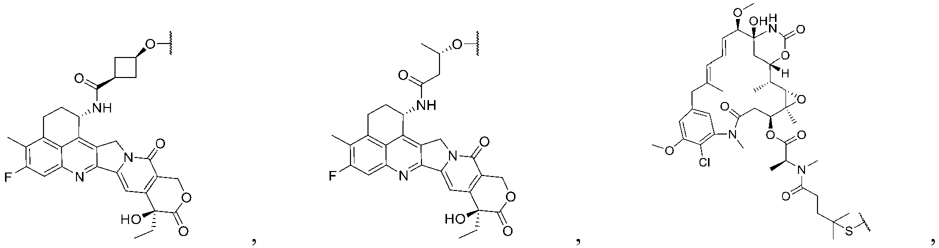

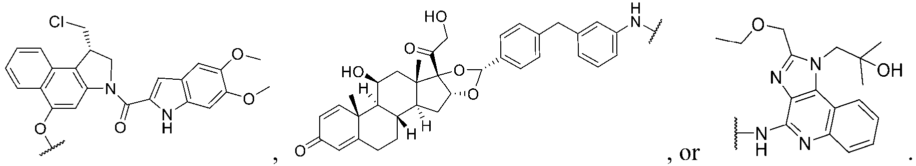

In a preferred embodiment of the present disclosure, in the compound of formula (I), or the mesomer, racemate, enantiomer, diastereomer, or any mixture thereof, or the pharmaceutically acceptable salt thereof, or the solvate thereof, or the pharmaceutically acceptable salt of the solvate thereof, the compound of formula (I) is a compound of formula (I-a): 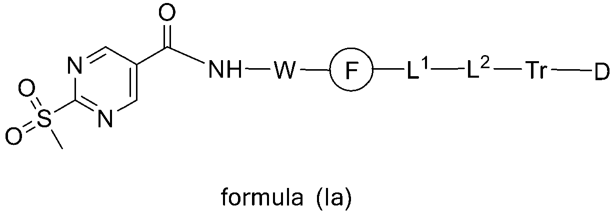 W, ring F, L¹, L², Tr, and D are as defined in any one of the above embodiments of general formula (I).

In a preferred embodiment of the present disclosure, the compound of formula (I) or formula (Ia), or the mesomer, racemate, enantiomer, diastereomer, or any mixture thereof, or the pharmaceutically acceptable salt thereof, or the solvate thereof, or the pharmaceutically acceptable salt of the solvate thereof, is selected from:

| | |
|---|---|
| A1 | 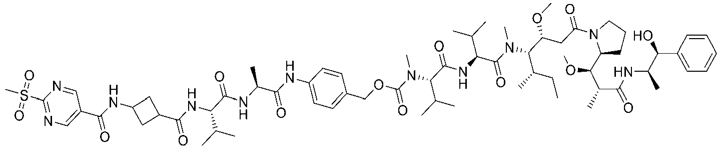 |
| A2 | 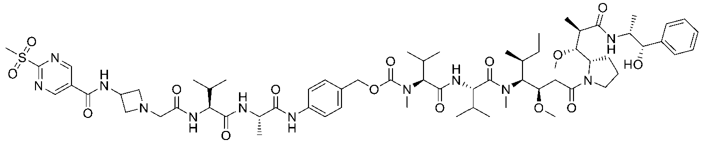 |
| A3 | 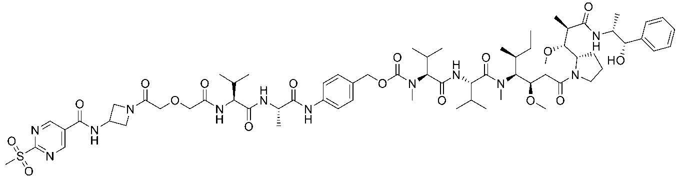 |
| A4 | 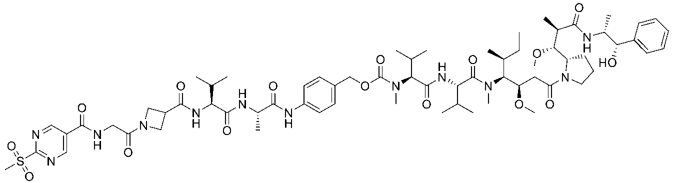 |
| A5 | 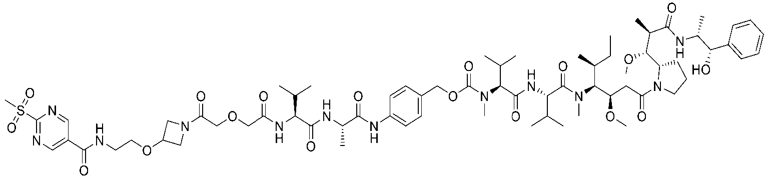 |
| A6 | 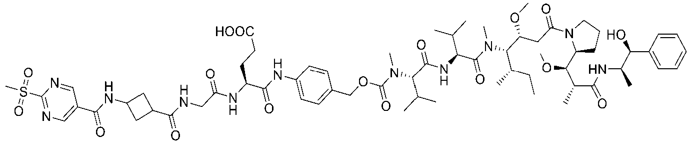 |
| A7 | 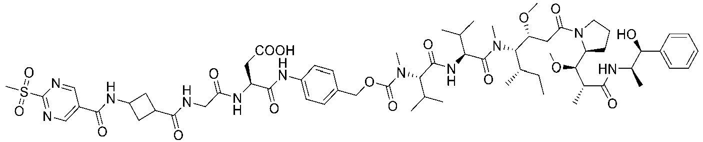 |
| A8 | 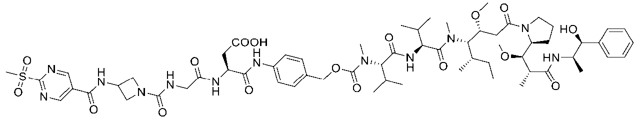 |
| A9 | 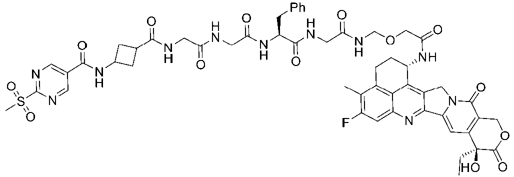 |
| A10 | 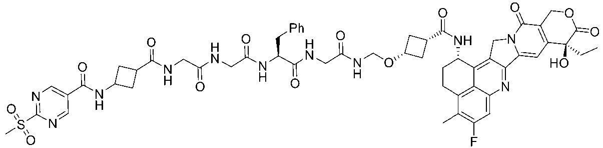 |
| A11 | 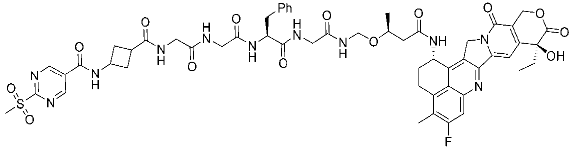 |
| A12 | 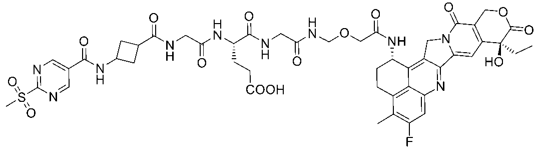 |
| A13 | 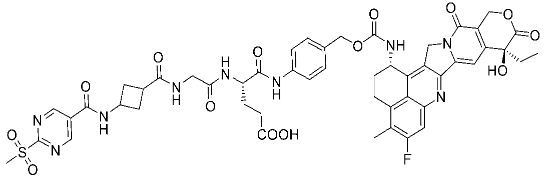 |
| A14 | 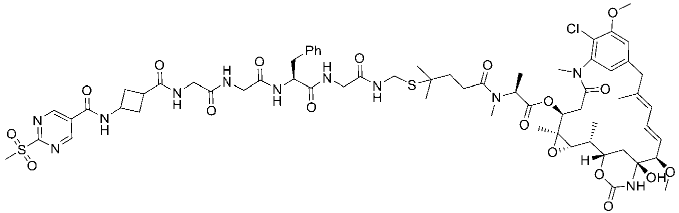 |
| A15 | 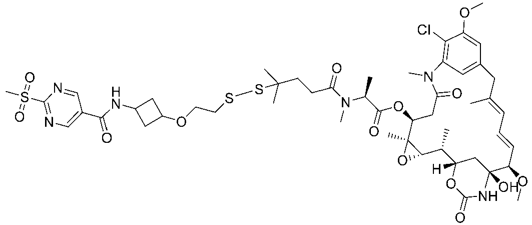 |
| A16 | 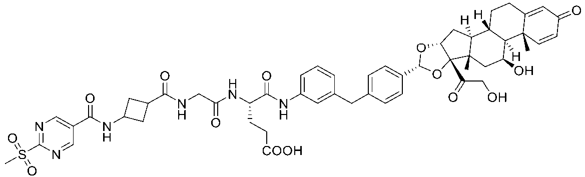 |
| A17 | 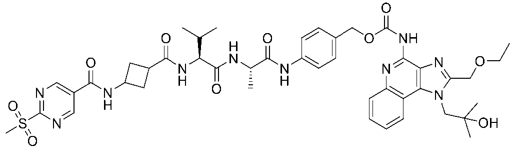 |
| A18 | 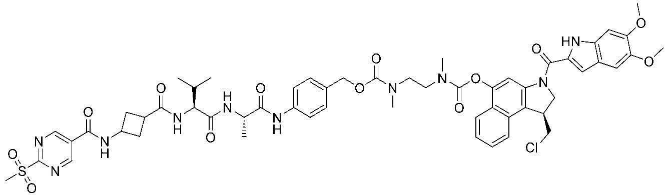 |
| A19 | 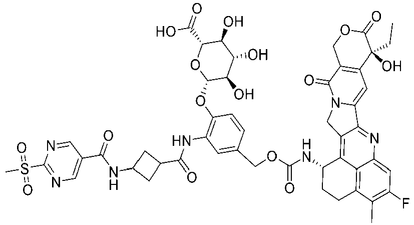 |
| A20 | 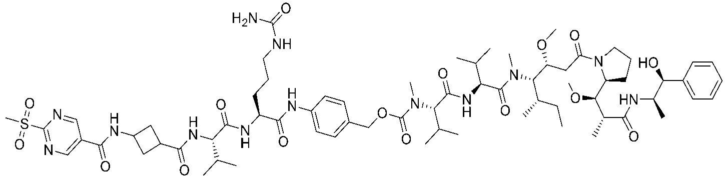 |
| A21 | 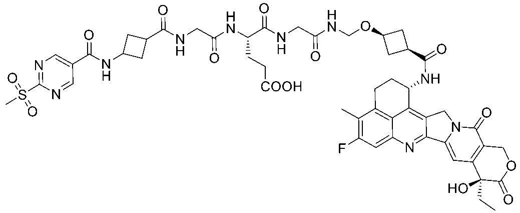 |
| A22 | 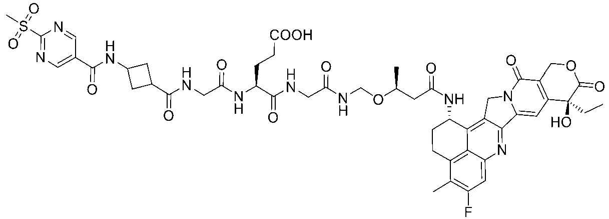 |
| A23 | 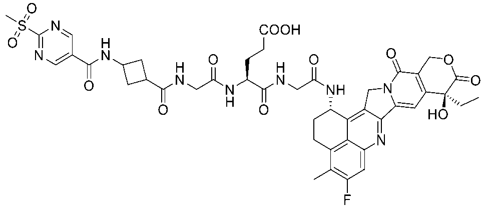 |

In another aspect, the present disclosure provides a compound of formula (II), or a mesomer, racemate, enantiomer, diastereomer, or any mixture thereof, or a pharmaceutically acceptable salt thereof, or a solvate thereof, or a pharmaceutically acceptable salt of the solvate thereof, or a solvate thereof, or a pharmaceutically acceptable salt of the solvate thereof: 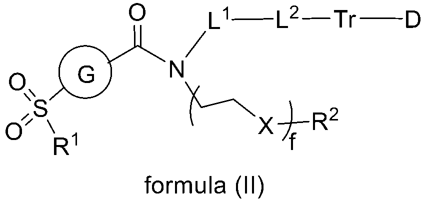 wherein
f is a natural number from 1 to 30;
X is -O- or -NH-;
R² is hydrogen, deuterium, -C₁₋₆ alkyl, -N(R^{2a})₂, -S(O)₂R^{2a}, -SO₂N(R^{2a})₂, -C(O)R^{2a}, - C(O)N(R^{2a})₂, -P(O)(OR^{2a})₂, -NHC(O)R^{2a}, or -N(R^{2a})SO₂R^{2a};
R^{2a} is hydrogen, deuterium, halogen, or -C₁₋₆ alkyl;
R¹, ring G, L¹, L², Tr, and D are as defined in any one of the embodiments of general formula (I), respectively.

In a preferred embodiment of the present disclosure, in the compound of formula (II), R¹ is -C₁₋₆ alkyl or -C₃₋₈ cycloalkyl, wherein the alkyl and cycloalkyl are each independently and optionally substituted by one or more substituents selected from hydrogen, deuterium, and halogen; preferably, R¹ is -Me, -Et, -isopropyl, or -cyclopropyl; more preferably, R¹ is -Me.

In a preferred embodiment of the present disclosure, in the compound of formula (II), R¹ is -C₁₋₆ alkyl.

In a preferred embodiment of the present disclosure, in the compound of formula (II), ring G is pyridine, pyrimidine, pyridazine, pyrazine, oxazole, isoxazole, thiazole, isoxazole, oxadiazole, thiadiazole, benzothiazole, or benzoxazole; preferably, ring G is 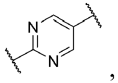 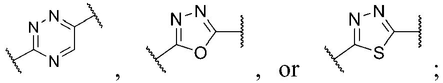 further preferably, ring G is 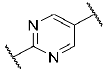 or 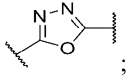 more preferably, ring G is

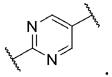

In a preferred embodiment of the present disclosure, in the compound of formula (II), in ring G, ring G may be 5- to 6-membered heteroaryl.

In a preferred embodiment of the present disclosure, in the compound of formula (II), in ring G, the heteroatom of the 5- to 10-membered heteroaryl may be N and/or O, and the number of heteroatoms may be 1, 2, or 3.

In a preferred embodiment of the present disclosure, in the compound of formula (II), ring G is 5- to 6-membered heteroaryl, wherein the heteroatom of the 5- to 6-membered heteroaryl may be N and/or O, and the number of heteroatoms is 1, 2, or 3.

In a preferred embodiment of the present disclosure, in the compound of formula (II), X is -O-.

In a preferred embodiment of the present disclosure, in the compound of formula (II), X is -NH-.

In a preferred embodiment of the present disclosure, in the compound of formula (II), R² is hydrogen, -C₁₋₆ alkyl, -S(O)₂R^{2a}, or -P(O)(OR^{2a})₂; more preferably hydrogen or -C₁₋₆ alkyl, for example, hydrogen or methyl.

In a preferred embodiment of the present disclosure, in the compound of formula (II), R^{2a} is hydrogen or -C₁₋₆ alkyl.

In a preferred embodiment of the present disclosure, f is a natural number from 1 to 20; preferably, f is a natural number from 1 to 8, for example, f is 1, 2, 3, 4, 5, 6, 7, or 8; for another example, 1, 6, or 8.

In a preferred embodiment of the present disclosure, in 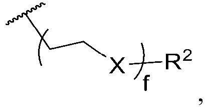 X is -O-; R² is hydrogen or -C₁₋₆ alkyl; f is a natural number from 1 to 20; for example,

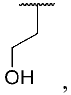

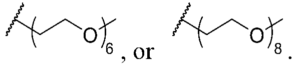

In a preferred embodiment of the present disclosure, in the compound of formula (II), L¹ is -(CHR^{L11})ₙ-;
wherein n is a natural number from 0 to 50;
any -CHR^{L11}- moiety in L¹ is independently and optionally replaced by the following structural moieties: -Cy-, -NR^{L12}-, -C(O)-, -S-, -O-, 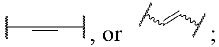
-Cy- is phenylene, 5- to 6-membered heteroarylene, 4- to 10-membered heterocyclylene, or 3- to 6-membered cycloalkylene, wherein each -Cy- is independently unsubstituted or substituted by 1 to 3 R^{cx};
R^{L11}, R^{L12}, and R^{cx} are each independently hydrogen, halogen, -OR^{L1a}, -N(R^{L1a})₂, - C(O)R^{L1a}, -S(O)₂R^{L1a}, -C(O)N(R^{L1a})₂, -SO₂N(R^{L1a})₂, -N(R^{L1a})SO₂R^{L1b}, -N(R^{L1a})COR^{L1b}, - (CH₂)_{y}-CO-(N(Me)CH₂C(O))ₘ-OR^{L1a}, -(CH₂)_{y}-CO-(N(Me)CH₂C(O))ₘ-NHR^{L1a}, -(CH₂)_{y}-CONH-(CH₂CH₂O)ₘ-R^{L1a}, -(CH₂)_{y}-NHCO-(CH₂CH₂O)ₘ-R^{L1a}, -(CH₂)_{y}-NHCOCH₂(OCH₂CH₂)OR^{L1a}, -(CH₂)_{y}-NH(COCH₂(N(Me))ₘ-R^{L1a}, -(CH₂)_{y}-NHCO-(CH₂CH₂O)ₘ-R^{L1a}, -(CH₂CH₂O)ₘ-R^{L1a}, -(COCH₂N(Me))ₘ-R^{L1a}, -COCH₂(OCH₂CH₂)ₘ-OR^{L1a}, -CO-(CH₂CH₂O)ₘ-R^{L1a}, -C₁₋₆ alkyl, -C₂₋₆ alkenyl, -C₂₋₆ alkynyl, 3- to 8-membered cycloalkyl, 4- to 10-membered heterocycloalkyl, 6- to 10-membered aryl, or 5- to 10-membered heteroaryl; wherein the -C₁₋₆ alkyl, -C₂₋₆ alkenyl, -C₂₋₆ alkynyl, 3- to 8-membered cycloalkyl, 4- to 10-membered heterocycloalkyl, 6- to 10-membered aryl, and 5- to 10-membered heteroaryl are each independently and optionally substituted by one or more R^{L1a};
m is a natural number from 0 to 8;
y is 0, 1, 2, 3, or 4;
R^{L1a} and R^{L1b} are each independently hydrogen, halogen, -CN, -OH, -NH₂, -N(Me)₂, -CO₂H, -C(O)NH₂, or -C₁₋₆ alkyl.

In a preferred embodiment of the present disclosure, in the compound of formula (II), L¹ is -(CH₂)ₙ-;
wherein n is a natural number from 0 to 50;
any -CH₂- moiety in L¹ is independently and optionally replaced by the following structural moieties: phenylene, 5- to 6-membered heteroarylene, 4- to 6-membered heterocyclylene, 3- to 6-membered cycloalkylene, -NR^{L12}-, -C(O)-, -S-, or -O-;
each R^{L12} is independently hydrogen, -OR^{L1a}, -C(O)R^{L1a}, -S(O)₂R^{L1a}, -C(O)N(R^{L1a})₂, -SO₂N(R^{L1a})₂, -(CH₂)_{y}-CO-(N(Me)CH₂C(O))ₘ-OR^{L1a}, -(CH₂)_{y}-CO-(N(Me)CH₂C(O))ₘ-NHR^{L1a}, -(CH₂)_{y}-CONH-(CH₂CH₂O)ₘ-R^{L1a}, -(CH₂)_{y}-NHCOCH₂(OCH₂CH₂)OR^{L1a}, - (CH₂CH₂O)ₘ-R^{L1a}, -(COCH₂N(Me))ₘ-R^{L1a}, -COCH₂(OCH₂CH₂)ₘ-OR^{L1a}, -CO-(CH₂CH₂O)ₘ-R^{L1a}, or -C₁₋₆ alkyl optionally substituted by R^{L1a};
m is a natural number from 0 to 8;
y is 0, 1, 2, 3, or 4;
each R^{L1a} is independently hydrogen, halogen, -CN, -OH, -NH₂, -N(Me)₂, -CO₂H, - C(O)NH₂, or -C₁₋₆ alkyl.

In a preferred embodiment of the present disclosure, in the compound of formula (II), L¹ is -(CH₂)ₙ-;
wherein n is a natural number from 0 to 50;
any -CH₂- moiety in L¹ is independently and optionally replaced by the following structural moieties: -NH-, -NMe, -C(O)-, -S-, or -O-.

In a preferred embodiment of the present disclosure, in the compound of formula (II), L¹ is: 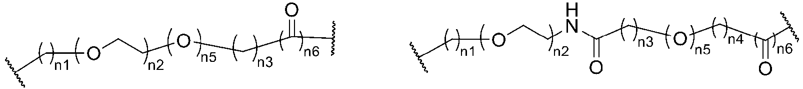 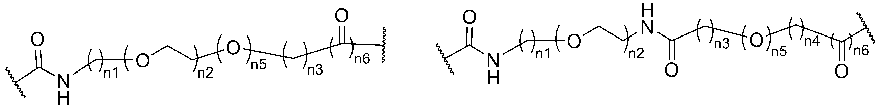 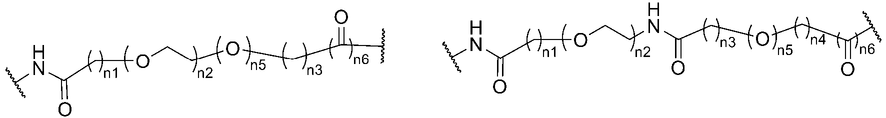 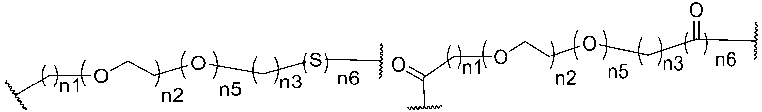 wherein
n1, n2, n3, and n4 are each independently a natural number from 0 to 8;
n5 and n6 are each independently 0 or 1.

In a preferred embodiment of the present disclosure, in the compound of formula (II), in L¹, n may be a natural number from 0 to 50; preferably, n is a natural number from 1 to 10, for example, 2 or 6.

In a preferred embodiment of the present disclosure, in the compound of formula (II), R^{L11} and R^{L12} in L¹ may each be hydrogen.

In a preferred embodiment of the present disclosure, in the compound of formula (II), any -C(R^{L11})₂- moiety in L¹ is independently and optionally replaced by the following structural moieties: -NH-, -O-, -C(O)-, or -S-.

In a preferred embodiment of the present disclosure, in the compound of formula (II), L¹ is -(CH₂)ₙ-; wherein n is a natural number from 0 to 50; any -CH₂- moiety in L¹ is independently and optionally replaced by the following structural moieties: -NH-, -O-, -C(O)-, or -S-;
preferably, L¹ is 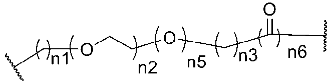 or 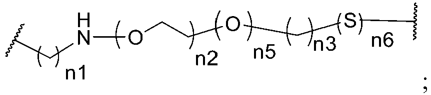 wherein
n1, n2, and n3 are each independently 0, 1, 2, 3, 4, 5, 6, 7, or 8;
n5 and n6 are each independently 0 or 1;
for example, L¹ is

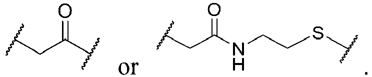

In a preferred embodiment of the present disclosure, in the compound of formula (II), L² is a chemical bond or an amino acid residue, a short peptide composed of 2 to 7 amino acid residues, 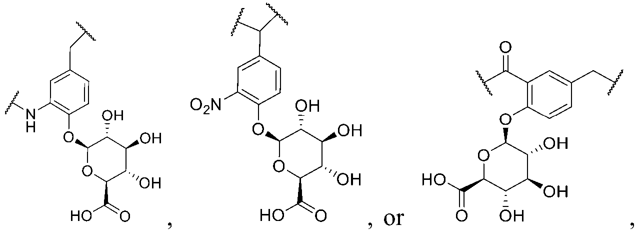 wherein the amino acid residue is Val, D-Val, Nva, Phe, Lys, Leu, Ile, Gly, Ala, D-Ala, Cit, Asp, Asn, Glu, Gln, Pro, Arg, Lys(Ac), Lys(Me), Lys(Et), Lys(nPr), Nva, or AA;
AA is

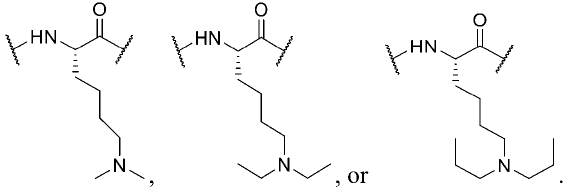

In a preferred embodiment of the present disclosure, in the compound of formula (I), L² is a chemical bond, Val, D-Val, Phe, Lys, Leu, Ile, Gly, Ala, D-Ala, Cit, Asp, Asn, Glu, Gln, Val-Cit, Val-Ala, Val-Lys, Val-Lys(Ac), Phe-Lys, Phe-Lys(Ac), Leu-Lys, Leu-Lys(Ac), Ala-Ala, Ala-Lys, D-Ala-Ala, Gly-Glu, Gly-Asp, Gly-Asn, Val-Glu, Val-Asp, Asp-Asp, Asn-Asn, Glu-Val-Cit, Ala-Ala-Ala, Ala-(D-Ala)-Ala, Ala-Ala-Asn, Ala-(D-Ala)-Asn, Ala-Ala-Asp, Val-Lys-Gly, D-Val-Leu-Lys, Gly-Gly-Arg, Gly-Gly-Gly, Lys-Ala-Asn, Gly-Phe-Gly, Gly-Gly-Phe, Asn-Pro-Val, Ala-Lys-Gly, Gly-Lys-Gly, Gly-Glu-Gly, Gly-Gly-Gly-Gly, Gly-Gly-Phe-Gly, Gly-Gly-Glu-Gly, Lys-Ala-Ala-Asn, Lys-Ala-Ala-Asp, Ala-Ala-Pro-Val, Ala-Ala-Pro-Nva,

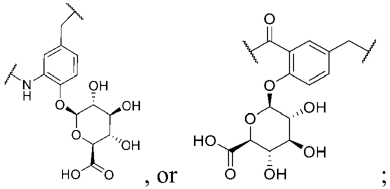

In a preferred embodiment of the present disclosure, in the compound of formula (II), L² is a chemical bond, Lys, Cit, Asp, Asn, Glu, Gln, Val-Cit, Val-Ala, Asp-Asp, Ala-Ala, Gly-Glu, Gly-Asp, Gly-Asn, Asn-Asn, Gly-Glu-Gly, Gly-Gly-Glu-Gly, Gly-Gly-Phe-Gly, or Gly-Gly-Gly-Gly.

In a preferred embodiment of the present disclosure, in the compound of formula (II), L² is a chemical bond, Val-Cit, Val-Ala, Gly-Glu, Gly-Asp, Gly-Glu-Gly, Gly-Gly-Phe-Gly, or

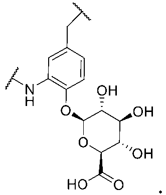

In a preferred embodiment of the present disclosure, in the compound of formula (II), 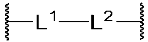 is: 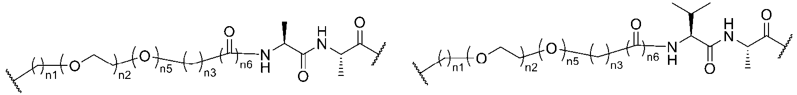 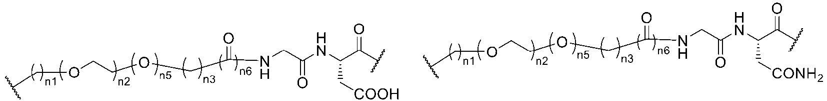 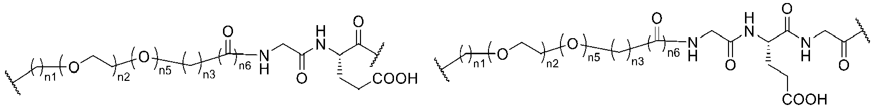 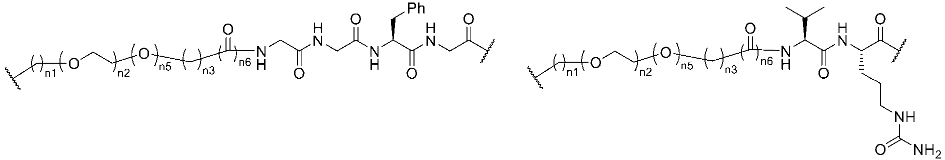 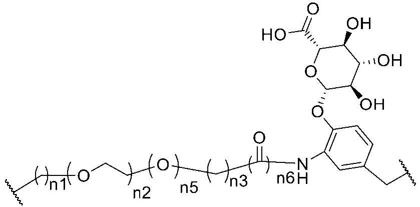 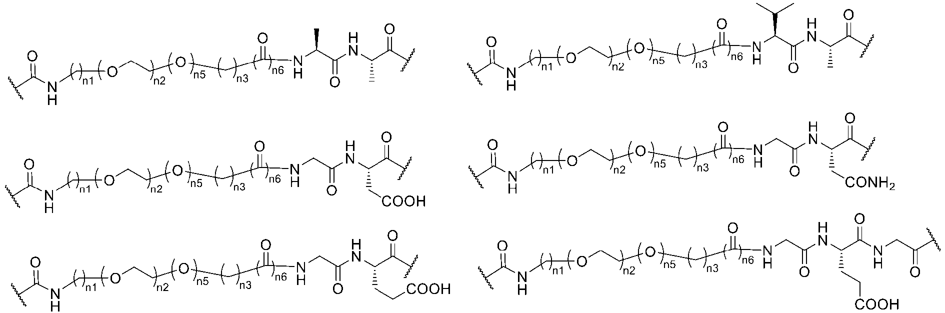 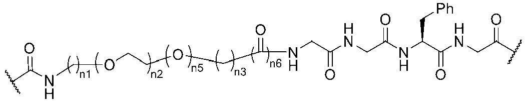 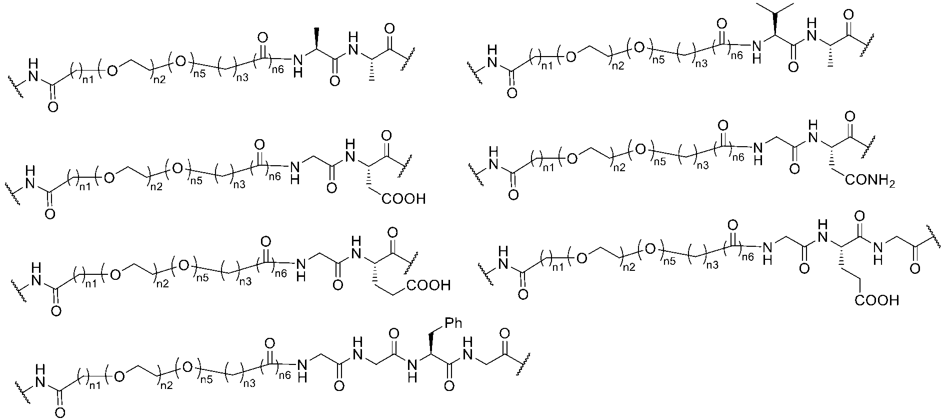 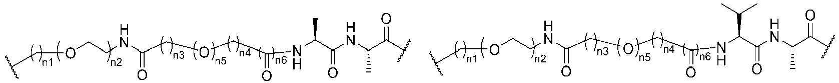 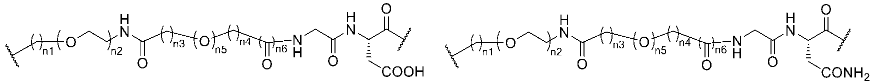 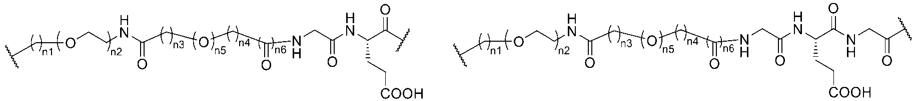 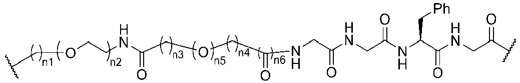 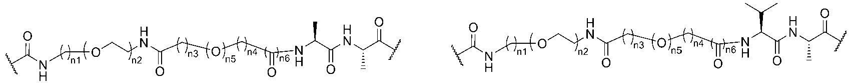 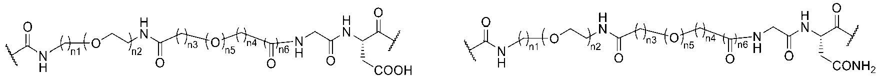 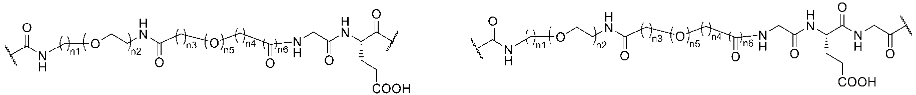 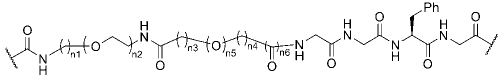 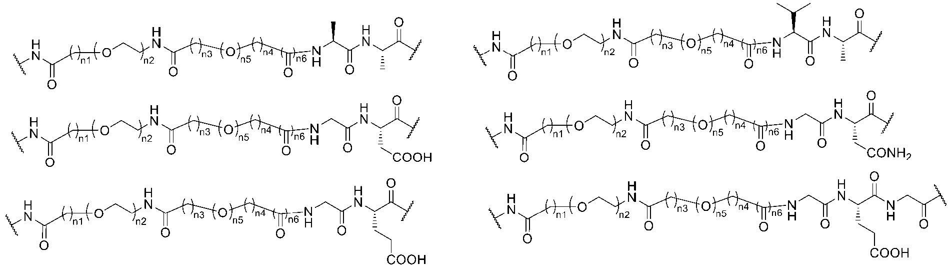 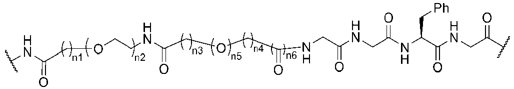 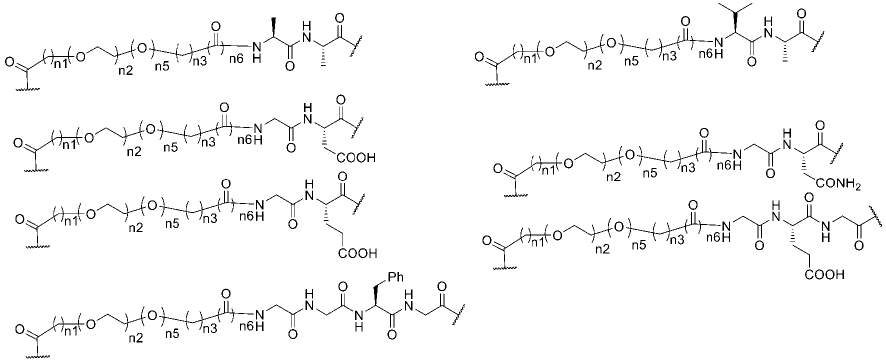 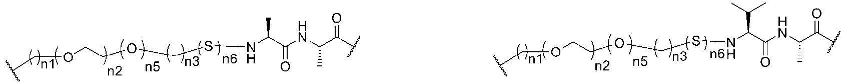 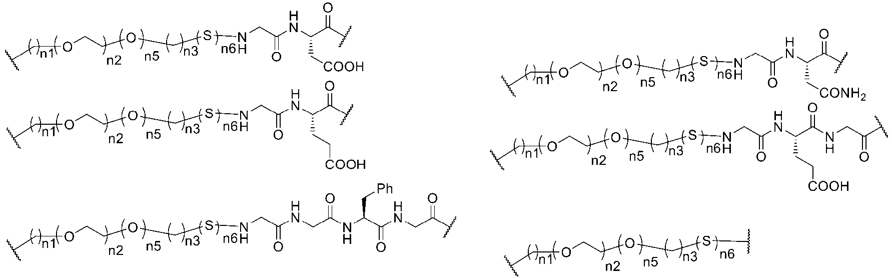 wherein
n1, n2, n3, and n4 are each independently a natural number from 0 to 8;
n5 and n6 are each independently 0 or 1.

In a preferred embodiment of the present disclosure, in the compound of formula (II), L² is a chemical bond, Val-Ala, Val-Cit, Gly-Glu, Gly-Glu-Gly, or Gly-Gly-Phe-Gly.

In a preferred embodiment of the present disclosure, in the compound of formula (II), 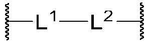 is: 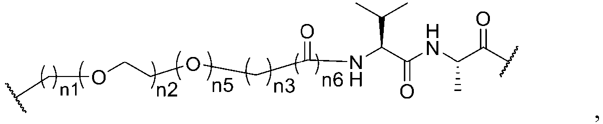 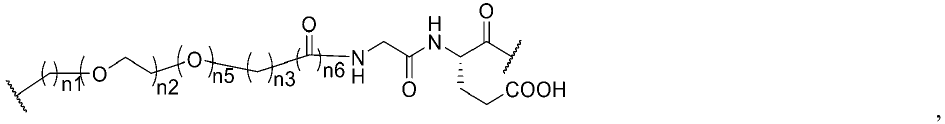 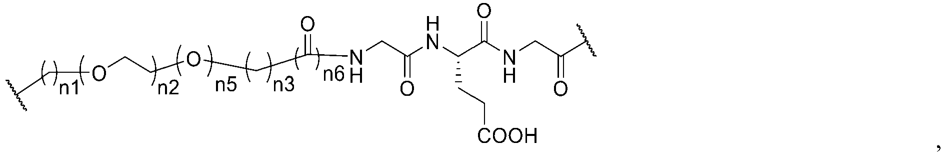 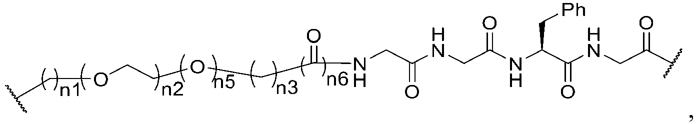 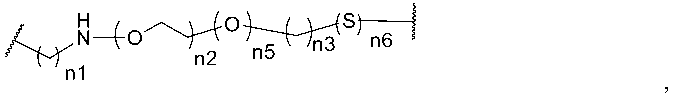 or 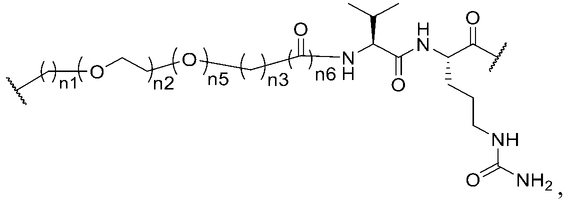
n1, n2, and n3 are each independently 0, 1, 2, 3, 4, 5, 6, 7, or 8;
n5 and n6 are each independently 0 or 1;
for example, 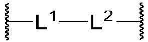 is

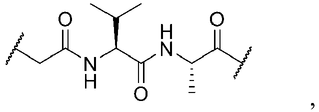

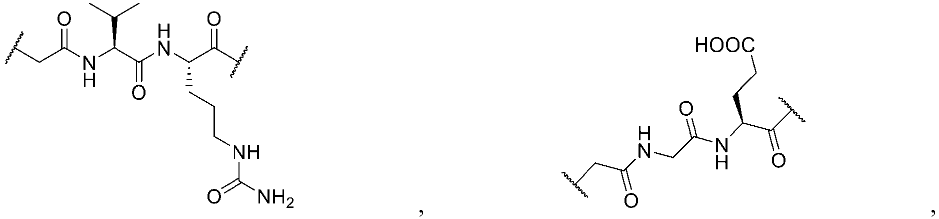

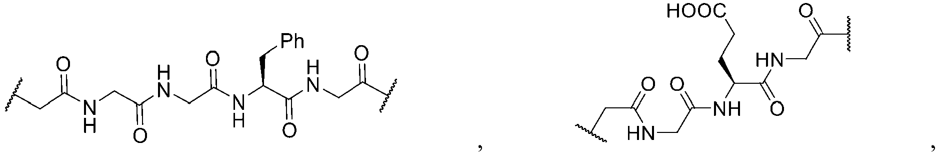

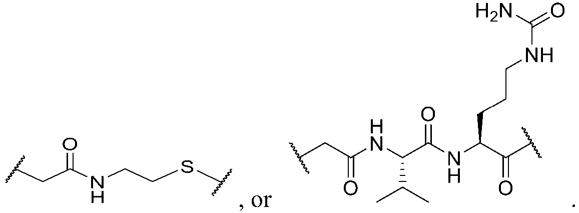

In a preferred embodiment of the present disclosure, in the compound of formula (II), Tr is a chemical bond or selected from one or more of 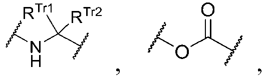 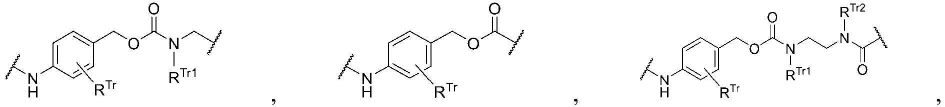 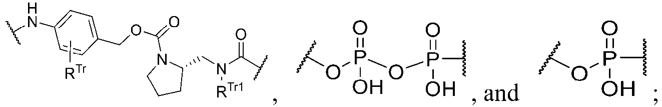
R^{Tr}, R^{Tr1}, and R^{Tr2} are each independently hydrogen, halogen, -NO₂, -CN, -OH, -NH₂, -CO₂H, -S(O)₂H, -C(O)NH₂, -SO₂NH₂, -OC(O)NH₂, -CH₂CO-(N(Me)CH₂C(O))_{z}-OR^{Tra}, - CH₂CO-(N(Me)CH₂C(O))_{z}-NHR^{Tra}, -(CH₂CH₂O)_{z}-R^{Tra}, -CONH-(CH₂CH₂O)_{z}-R^{Tra}, or -C₁₋₆ alkyl optionally substituted by R^{Tra};
R^{Tra} is hydrogen, deuterium, halogen, -NO₂, -CN, -OH, -NH₂, -N(Me)₂, -S(O)₂Me, - CO₂H, -S(O)₂OH, -C(O)NH₂, -SO₂NH₂, or -C₁₋₆ alkyl;
z is a natural number.

In a preferred embodiment of the present disclosure, in the compound of formula (II), Tr is a chemical bond or selected from one or more of

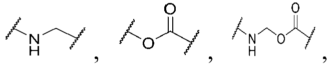

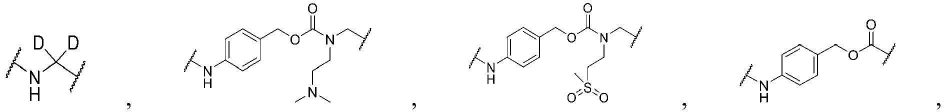

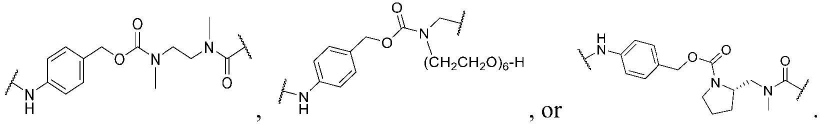

In a preferred embodiment of the present disclosure, in the compound of formula (II), Tr is a chemical bond,

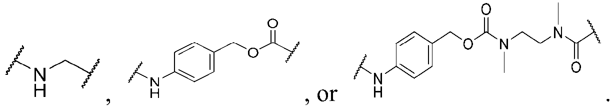

In a preferred embodiment of the present disclosure, in the compound of formula (II), R^{Tr}, R^{Tr1}, and R^{Tr2} in Tr are each independently hydrogen or methyl.

In a preferred embodiment of the present disclosure, in the compound of formula (II), R^{Tr}, R^{Tr1}, and R^{Tr2} are each independently hydrogen.

In a preferred embodiment of the present disclosure, in the compound of formula (II), Tr is a chemical bond or selected from one or more of 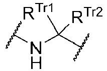 (e.g., 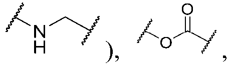 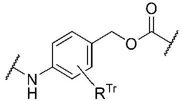 (e.g., 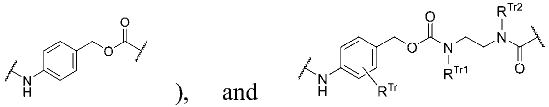 (e.g.,

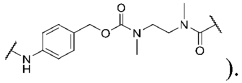

In a preferred embodiment of the present disclosure, in the compound of formula (II), Tr is a chemical bond or selected from one or more of 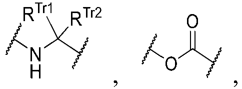 and 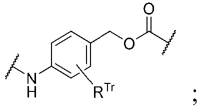 preferably, Tr is a chemical bond, 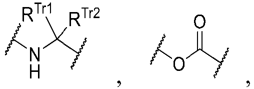 or

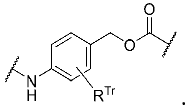

In a preferred embodiment of the present disclosure, in the compound of formula (II), Tr is a chemical bond,

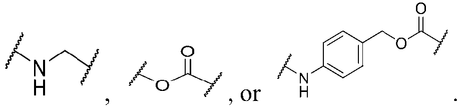

In a preferred embodiment of the present disclosure, in the compound of formula (II), D may be a tubulin inhibitor, a topoisomerase inhibitor, a DNA damaging agent, an RNA polymerase inhibitor, an immunomodulator, or an immunostimulant.

In a preferred embodiment of the present disclosure, the tubulin inhibitor may be Auristatins (e.g., MMAE, MMAF, or MMAD), Maytansinoids (e.g., DM1 or DM4), Vinblastines, Taxols, Eribulins, Tubulysins, Colchicines, or Hemiasterlin; or a stereoisomer, isostere, analog, or derivative thereof; for example, 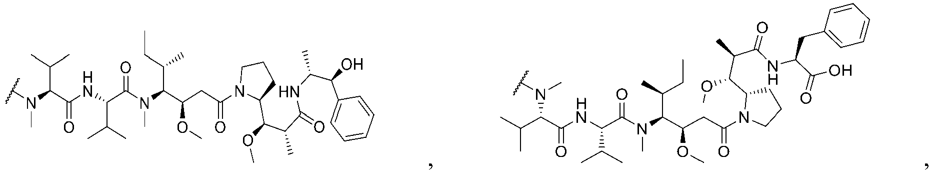 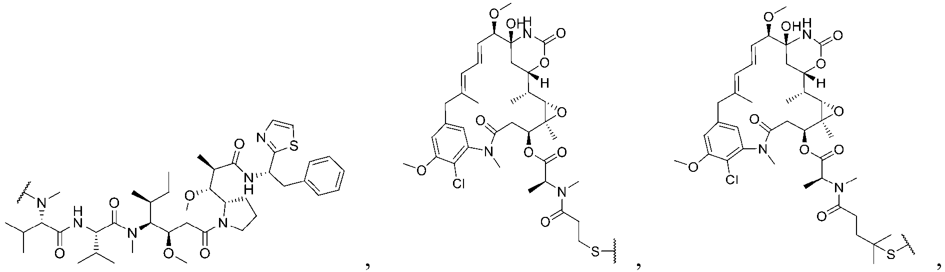 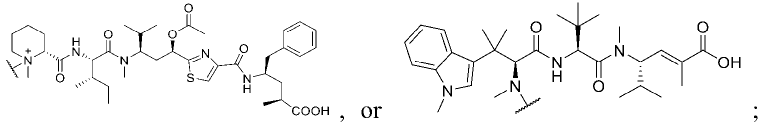 more preferably

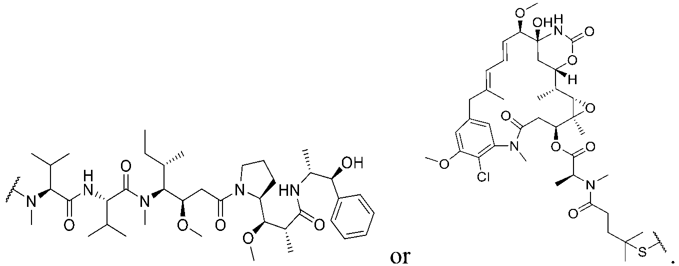

In a preferred embodiment of the present disclosure, the topoisomerase inhibitor may be camptothecin (e.g., SN38 or DX8951F); for example, 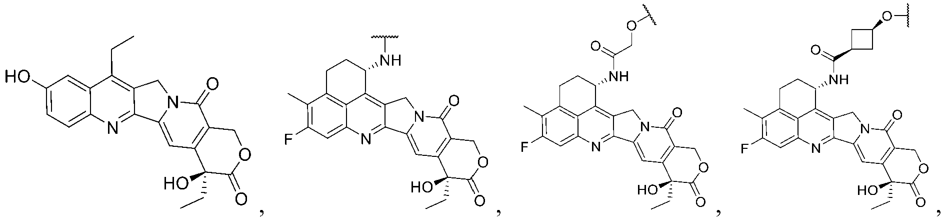 or 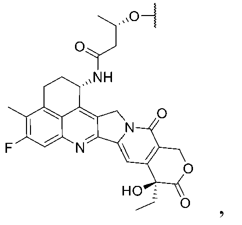 preferably

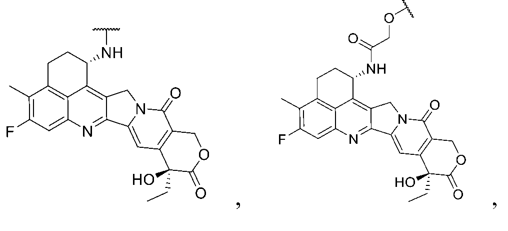

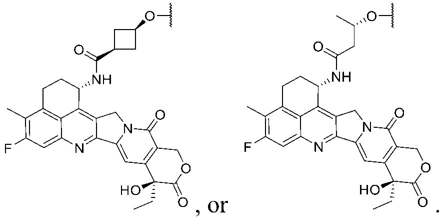

In a preferred embodiment of the present disclosure, the DNA damaging agent may be Calicheamicins, Mitomycins, anthracyclines (e.g., doxorubicin), methotrexates, Duocarmycins, a CBI dimer, a CPI dimer, a CTI dimer, a PBD dimer, an IGN dimer, a PDD dimer, or a Duocarmycin-PBD dimer; for example, 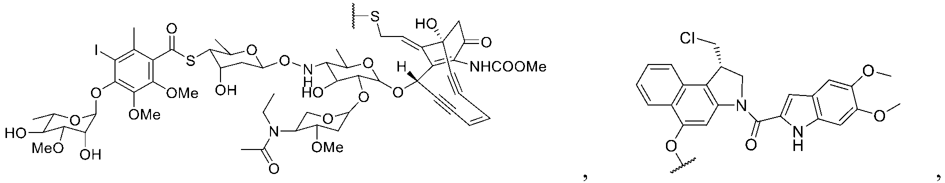 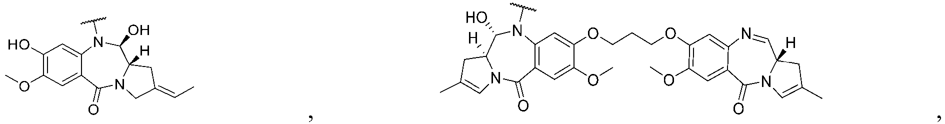 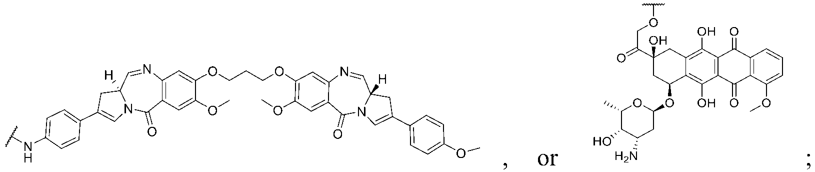 preferably

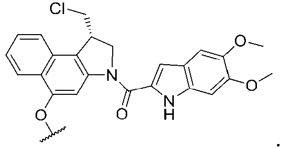

In a preferred embodiment of the present disclosure, the RNA polymerase inhibitor may be Amatoxins or a spliceosome inhibitor (e.g., Thailanstatin A); for example,

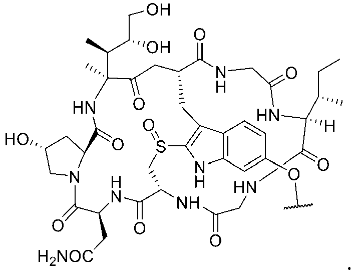

In a preferred embodiment of the present disclosure, the immunomodulator may be a glucocorticoid; for example,

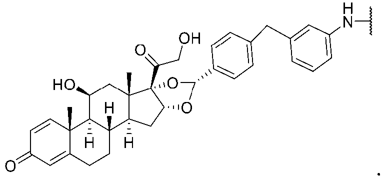

In a preferred embodiment of the present disclosure, the immunostimulant may be (e.g., Sting or TLR7/8 agonist), a protein toxin (e.g., interleukin, interferon, or tumor necrosis factor TNF-α), a selective or non-selective kinase inhibitor, a Bcl-x1 inhibitor, and a Bcl-2/Bcl-X1 inhibitor; for example,

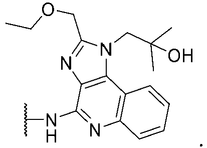

In a preferred embodiment of the present disclosure, in the compound of formula (II), D is a tubulin inhibitor, a topoisomerase inhibitor, a DNA damaging agent, an RNA polymerase inhibitor, an immunomodulator, or an immunostimulant, for example, D is

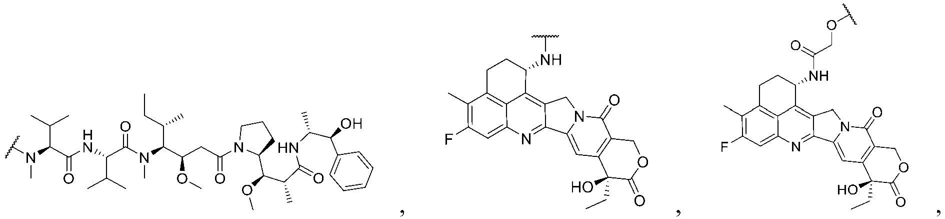

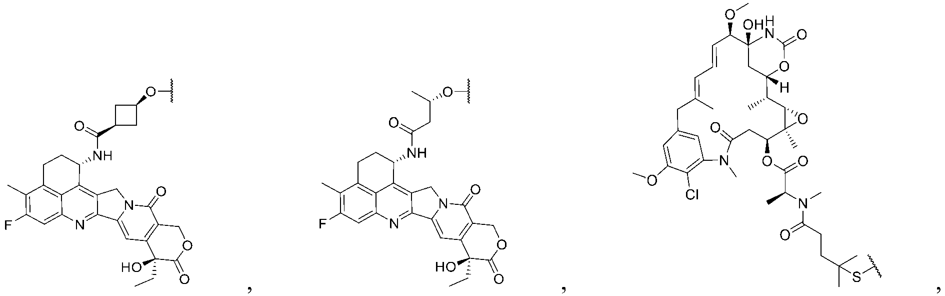

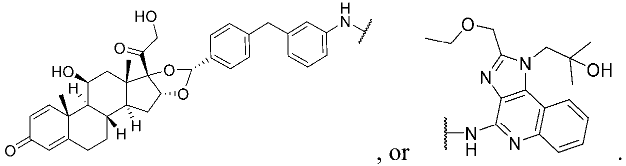

In a preferred embodiment of the present disclosure, in the compound of formula (II),
R¹ is -C₁₋₆ alkyl;
ring G is 5- to 6-membered heteroaryl, wherein the heteroatom of the 5- to 6-membered heteroaryl may be N and/or O, and the number of heteroatoms is 1, 2, or 3; for example, ring G is 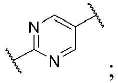
f is a natural number from 1 to 20;
X is -O-;
R² is hydrogen or -C₁₋₆ alkyl;
L¹ is -(CH₂)ₙ-; wherein n is a natural number from 0 to 50; any -CH₂- moiety in L¹ is independently and optionally replaced by the following structural moieties: -NH-, -O-, -C(O)-, or -S-;
L² is a chemical bond, Val-Cit, Val-Ala, Gly-Glu, Gly-Glu-Gly, or Gly-Gly-Phe-Gly;
Tr is a chemical bond, 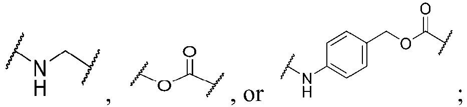
D is a tubulin inhibitor, a topoisomerase inhibitor, a DNA damaging agent, an RNA polymerase inhibitor, an immunomodulator, or an immunostimulant, for example, D is

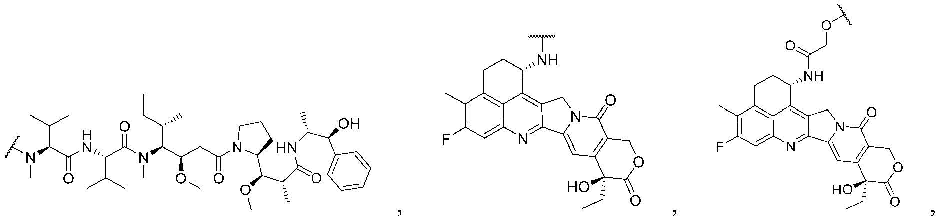

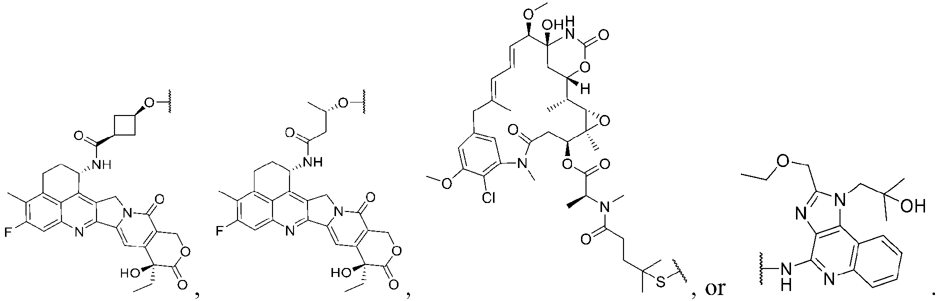

In a preferred embodiment of the present disclosure, in the compound of formula (II), or the mesomer, racemate, enantiomer, diastereomer, or any mixture thereof, or the pharmaceutically acceptable salt thereof, or the solvate thereof, or the pharmaceutically acceptable salt of the solvate thereof, the compound of formula (II) is a compound of formula (II-a): 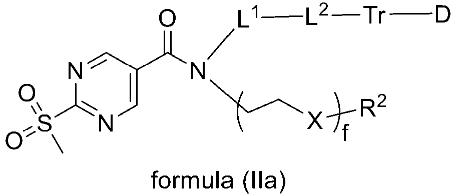 wherein
f, R², L¹, L², Tr, X, and D are as defined in any one of the above embodiments of general formula (II), respectively.

In a preferred embodiment of the present disclosure, in the compound of formula (IIa),
f is a natural number from 1 to 8, for example, f is 1, 2, 3, 4, 5, 6, 7, or 8;
X is -O-;
R² is hydrogen or -C₁₋₆ alkyl, for example, R² is hydrogen or -Me.
L¹ is -(CH₂)ₙ-; wherein n is a natural number from 0 to 50; any -CH₂- moiety in L¹ is independently and optionally replaced by the following structural moieties: -NH-, -O-, -C(O)-, or -S-;
L² is a chemical bond, Val-Cit, Val-Ala, Gly-Glu, Gly-Glu-Gly, or Gly-Gly-Phe-Gly;
Tr is a chemical bond, 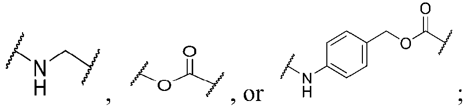
D is a tubulin inhibitor, a topoisomerase inhibitor, a DNA damaging agent, an RNA polymerase inhibitor, an immunomodulator, or an immunostimulant, for example, D is

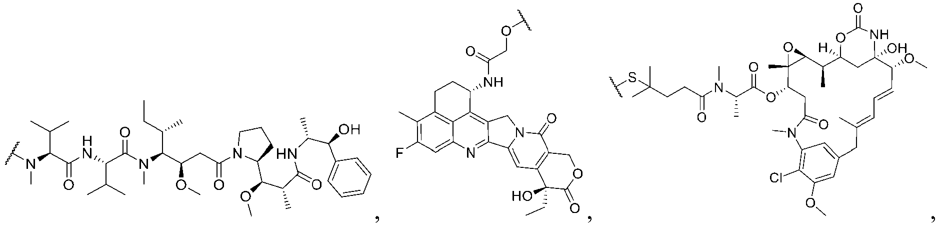

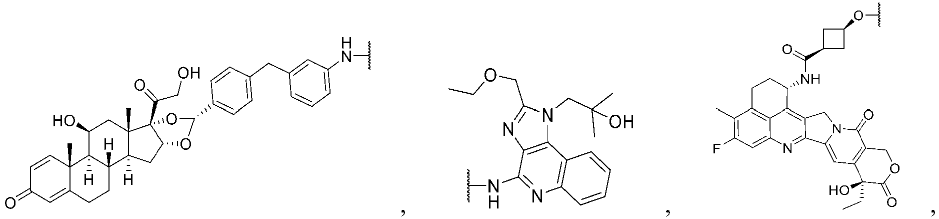

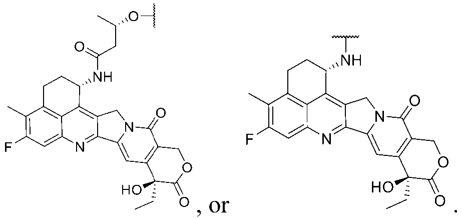

In a preferred embodiment of the present disclosure, the compound of formula (II) or formula (IIa), or the mesomer, racemate, enantiomer, diastereomer, or any mixture thereof, or the pharmaceutically acceptable salt thereof, or the solvate thereof, or the pharmaceutically acceptable salt of the solvate thereof, is selected from:

| | |
|---|---|
| B1 | 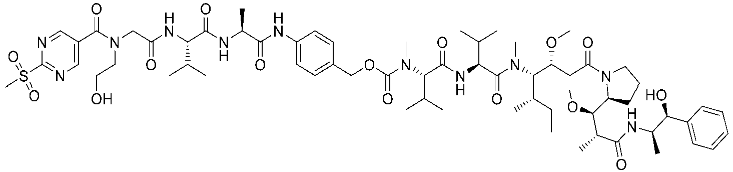 |
| B2 | 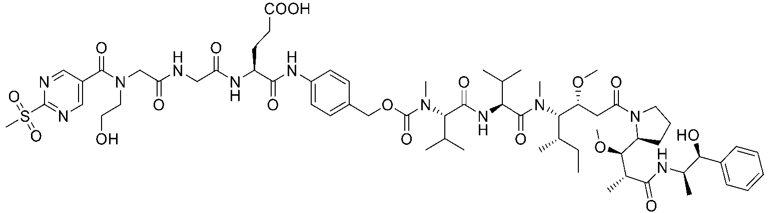 |
| B3 | 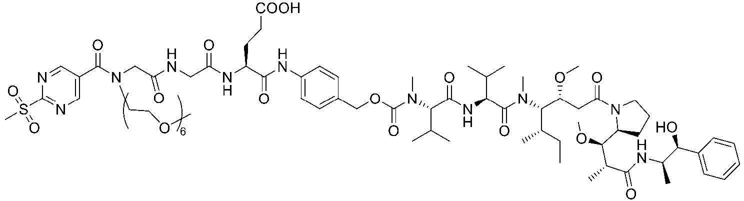 |
| B4 | 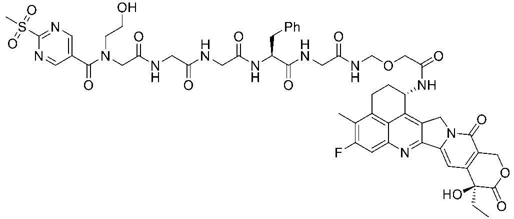 |
| B5 | 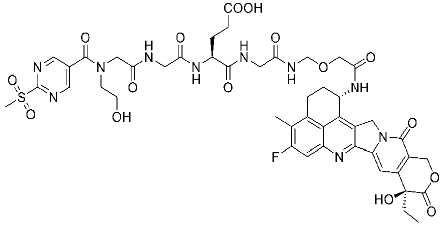 |
| B6 | 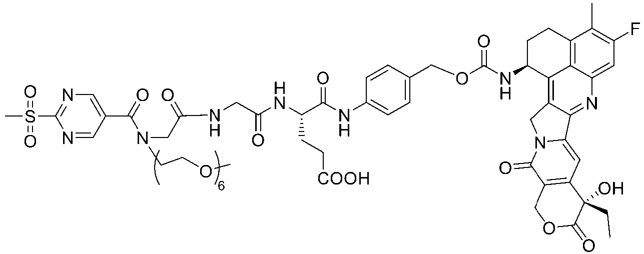 |
| B7 | 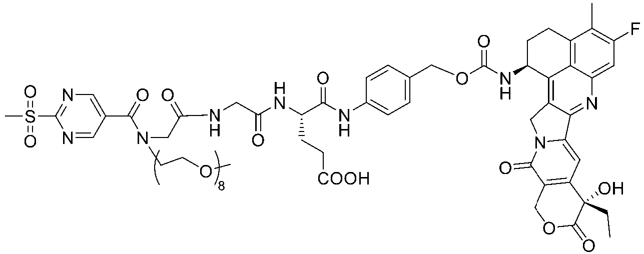 |
| B8 | 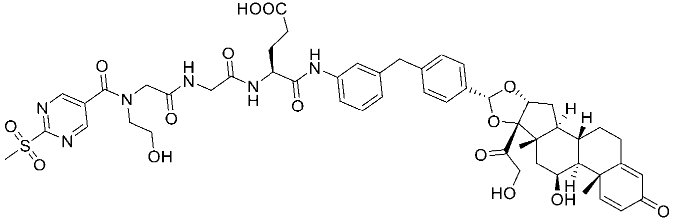 |
| B9 | 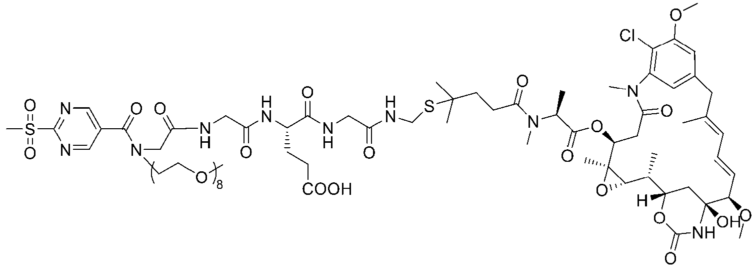 |
| B10 | 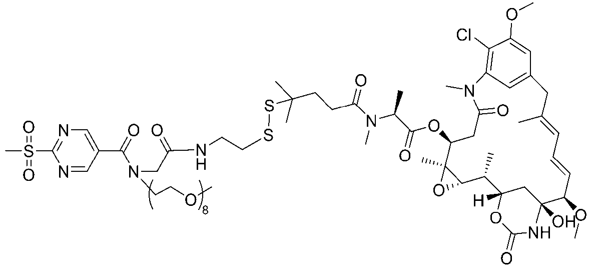 |
| B11 | 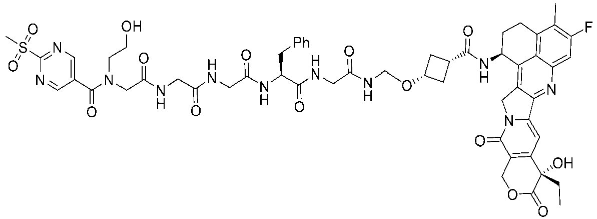 |
| B12 | 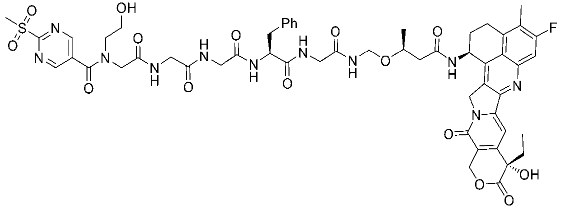 |
| B13 | 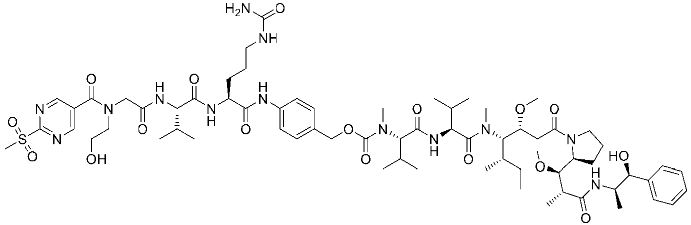 |
| B14 | 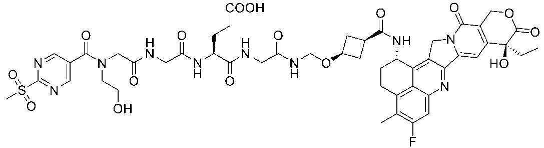 |
| B15 | 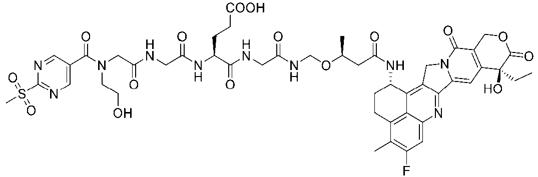 |
| B16 | 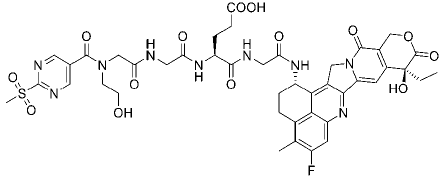 |
| B17 | 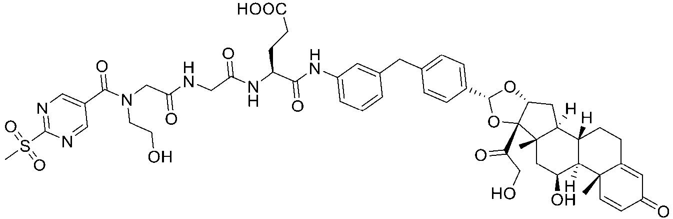 |

In another aspect, the present disclosure provides a compound of formula (III), or a mesomer, racemate, enantiomer, diastereomer, or any mixture thereof, or a pharmaceutically acceptable salt thereof, or a solvate thereof, or a pharmaceutically acceptable salt of the solvate thereof: 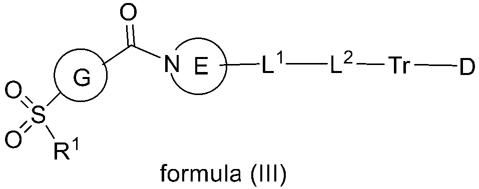 wherein
ring E is 4- to 12-membered heterocycloalkylene; wherein the 4- to 12-membered heterocycloalkylene is optionally substituted by one or more substituents selected from hydrogen, deuterium, halogen, hydroxyl, amino, cyano, nitro, -NHC₁₋₆ alkyl, -N(C₁₋₆ alkyl)₂, and -C(O)NH-(CH₂CH₂O)_{y1}CH₃; y1 is a natural number;
the heteroatom of the 4- to 12-membered heterocycloalkylene is selected from one or more types of N, O, and S, and the number of heteroatoms is 1, 2, 3, or 4.
R¹, ring G, L¹, L², Tr, and D are as defined in any one of the embodiments of general formula (I), respectively.

In a preferred embodiment of the present disclosure, in the compound of formula (III), R¹ is -C₁₋₆ alkyl or -C₃₋₈ cycloalkyl, wherein the alkyl and cycloalkyl are each independently and optionally substituted by one or more substituents selected from hydrogen, deuterium, and halogen; preferably, R¹ is -Me, -Et, -isopropyl, or -cyclopropyl; more preferably, R¹ is -Me.

In a preferred embodiment of the present disclosure, in the compound of formula (III), R¹ is -C₁₋₆ alkyl.

In a preferred embodiment of the present disclosure, in the compound of formula (III), ring G is pyridine, pyrimidine, pyridazine, pyrazine, oxazole, isoxazole, thiazole, isoxazole, oxadiazole, thiadiazole, benzothiazole, or benzoxazole; preferably, ring G is 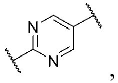 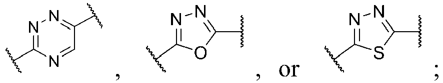 further preferably, ring G is 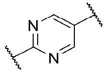 or 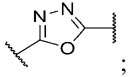 more preferably, ring G is

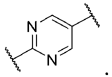

In a preferred embodiment of the present disclosure, in the compound of formula (III), in ring G, ring G may be 5- to 6-membered heteroaryl.

In a preferred embodiment of the present disclosure, in the compound of formula (III), in ring G, the heteroatom of the 5- to 10-membered heteroaryl may be N and/or O, and the number of heteroatoms may be 1, 2, or 3.

In a preferred embodiment of the present disclosure, in the compound of formula (III), in ring G, the heteroatom of the 5- to 10-membered heteroaryl is N, and the number of heteroatoms is 1, 2, or 3, for example, 2.

In a preferred embodiment of the present disclosure, in the compound of formula (III), ring G is 5- to 6-membered heteroaryl, wherein the heteroatom of the 5- to 6-membered heteroaryl may be N and/or O, and the number of heteroatoms is 1, 2, or 3.

In a preferred embodiment of the present disclosure, in the compound of formula (III), ring G is 5- to 6-membered heteroaryl, wherein the heteroatom of the 5- to 6-membered heteroaryl is N, and the number of heteroatoms is 1, 2, or 3, for example, 2.

In a preferred embodiment of the present disclosure, in the compound of formula (III), ring E is 4- to 6-membered heterocycloalkylene, wherein the heterocycloalkylene is optionally substituted by one or more substituents selected from hydrogen, deuterium, halogen, hydroxyl, amino, cyano, nitro, and -C(O)NH-(CH₂CH₂O)_{y1}CH₃; preferably, ring E is 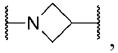 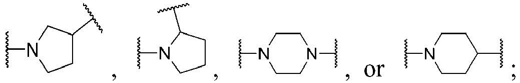 more preferably, ring E is

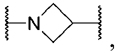

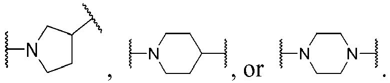

In a preferred embodiment of the present disclosure, in the compound of formula (III), 4- to 12-membered heterocycloalkylene in ring E may be 4- to 6-membered heterocycloalkylene.

In a preferred embodiment of the present disclosure, in the compound of formula (III), the heteroatom of the 4- to 12-membered heterocycloalkylene in ring E may be N, and the number of heteroatoms may be 1 or 2.

In a preferred embodiment of the present disclosure, in the compound of formula (III), the 4- to 12-membered heterocycloalkylene in ring E is optionally substituted by one or more -C(O)NH-(CH₂CH₂O)_{y1}CH₃ substituents.

In a preferred embodiment of the present disclosure, in the compound of formula (III), y1 is a natural number from 0 to 30; preferably a natural number from 0 to 10, for example, 6 or 7.

In a preferred embodiment of the present disclosure, in the compound of formula (III), -C(O)NH-(CH₂CH₂O)_{y1}CH₃ is

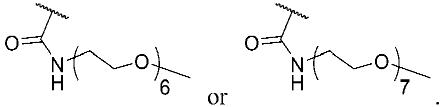

In a preferred embodiment of the present disclosure, in the compound of formula (III), ring E is 4- to 6-membered heterocycloalkylene, wherein the heteroatom of the 4- to 6-membered heterocycloalkylene is N, and the number of heteroatoms is 1 or 2;
preferably, ring E is

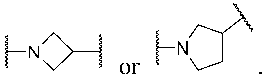

In a preferred embodiment of the present disclosure, in the compound of formula (III), ring E is 4- to 6-membered heterocycloalkylene, wherein the heteroatom of the 4- to 6-membered heterocycloalkylene is N, and the number of heteroatoms is 1.

In a preferred embodiment of the present disclosure, in the compound of formula (III), the 4- to 6-membered heterocycloalkylene in ring E is optionally substituted by one or more - C(O)NH-(CH₂CH₂O)_{y1}CH₃ substituents, and y1 is a natural number from 0 to 30.

In a preferred embodiment of the present disclosure, in the compound of formula (III), L¹ is -(CHR^{L11})ₙ-;
wherein n is a natural number from 0 to 50;
any -CHR^{L11}- moiety in L¹ is independently and optionally replaced by the following structural moieties: -Cy-, -NR^{L12}-, -C(O)-, -S-, -O-, 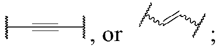
-Cy- is phenylene, 5- to 6-membered heteroarylene, 4- to 10-membered heterocyclylene, or 3- to 6-membered cycloalkylene, wherein each -Cy- is independently unsubstituted or substituted by 1 to 3 R^{cx};
R^{L11} R^{L12} and R^{cx} are each independently hydrogen, halogen, -OR^{L1a}, -N(R^{L1a})₂, - C(O)R^{L1a}, -S(O)₂R^{L1a}, -C(O)N(R^{L1a})₂, -SO₂N(R^{L1a})₂, -N(R^{L1a})SO₂R^{L1b}, -N(R^{L1a})COR^{L1b}, - (CH₂)_{y}-CO-(N(Me)CH₂C(O))ₘ-OR^{L1a}, -(CH₂)_{y}-CO-(N(Me)CH₂C(O))ₘ-NHR^{L1a}, -(CH₂)_{y}-CONH-(CH₂CH₂O)ₘ-R^{L1a}, -(CH₂)_{y}-NHCO-(CH₂CH₂O)ₘ-R^{L1a}, -(CH₂)_{y}-NHCOCH₂(OCH₂CH₂)OR^{L1a}, -(CH₂)_{y}-NH(COCH₂(N(Me))ₘ-R^{L1a}, -(CH₂)_{y}-NHCO-(CH₂CH₂O)ₘ-R^{L1a}, -(CH₂CH₂O)ₘ-R^{L1a}, -(COCH₂N(Me))ₘ-R^{L1a}, -COCH₂(OCH₂CH₂)ₘ-OR^{L1a}, -CO-(CH₂CH₂O)ₘ-R^{L1a}, -C₁₋₆ alkyl, -C₂₋₆ alkenyl, -C₂₋₆ alkynyl, 3- to 8-membered cycloalkyl, 4- to 10-membered heterocycloalkyl, 6- to 10-membered aryl, or 5- to 10-membered heteroaryl; wherein the -C₁₋₆ alkyl, -C₂₋₆ alkenyl, -C₂₋₆ alkynyl, 3- to 8-membered cycloalkyl, 4- to 10-membered heterocycloalkyl, 6- to 10-membered aryl, and 5- to 10-membered heteroaryl are each independently and optionally substituted by one or more R^{L1a};
m is a natural number from 0 to 8;
y is 0, 1, 2, 3, or 4;
R^{L1a} and R^{L1b} are each independently hydrogen, halogen, -CN, -OH, -NH₂, -N(Me)₂, -CO₂H, -C(O)NH₂, or -C₁₋₆ alkyl.

In a preferred embodiment of the present disclosure, in the compound of formula (III), L¹ is -(CH₂)ₙ-;
wherein n is a natural number from 0 to 50;
any -CH₂- moiety in L¹ is independently and optionally replaced by the following structural moieties: phenylene, 5- to 6-membered heteroarylene, 4- to 6-membered heterocyclylene, 3- to 6-membered cycloalkylene, -NR^{L12}-, -C(O)-, -S-, or -O-;
each R^{L12} is independently hydrogen, -OR^{L1a}, -C(O)R^{L1a}, -S(O)₂R^{L1a}, -C(O)N(R^{L1a})₂, -SO₂N(R^{L1a})₂, -(CH₂)_{y}-CO-(N(Me)CH₂C(O))ₘ-OR^{L1a}, -(CH₂)_{y}-CO-(N(Me)CH₂C(O))ₘ-NHR^{L1a}, -(CH₂)_{y}-CONH-(CH₂CH₂O)ₘ-R^{L1a}, -(CH₂)_{y}-NHCOCH₂(OCH₂CH₂)OR^{L1a}, - (CH₂CH₂O)ₘ-R^{L1a}, -(COCH₂N(Me))ₘ-R^{L1a}, -COCH₂(OCH₂CH₂)ₘ-OR^{L1a}, -CO-(CH₂CH₂O)ₘ-R^{L1a}, or -C₁₋₆ alkyl optionally substituted by R^{L1a};
m is a natural number from 0 to 8;
y is 0, 1, 2, 3, or 4;
each R^{L1a} is independently hydrogen, halogen, -CN, -OH, -NH₂, -N(Me)₂, -CO₂H, - C(O)NH₂, or -C₁₋₆ alkyl.

In a preferred embodiment of the present disclosure, in the compound of formula (III), L¹ is -(CH₂)ₙ-;
wherein n is a natural number from 0 to 50;
any -CH₂- moiety in L¹ is independently and optionally replaced by the following structural moieties: -NH-, -NMe, -C(O)-, -S-, or -O-.

In a preferred embodiment of the present disclosure, in the compound of formula (III), L¹ is: 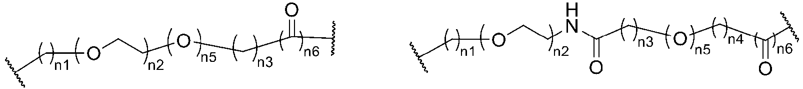 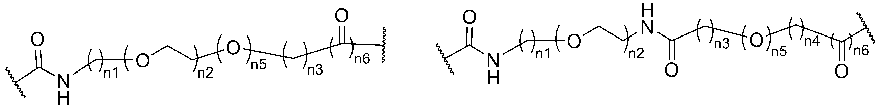 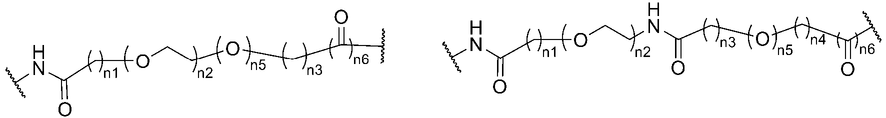 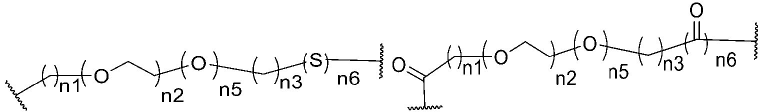 wherein
n1, n2, n3, and n4 are each independently a natural number from 0 to 8;
n5 and n6 are each independently 0 or 1.

In a preferred embodiment of the present disclosure, in the compound of formula (III), in L¹, n may be a natural number from 0 to 50; preferably, n is a natural number from 1 to 35, for example, 1, 3, 4 or 30.

In a preferred embodiment of the present disclosure, in the compound of formula (III), R^{L11} and R^{L12} in L¹ may each be hydrogen.

In a preferred embodiment of the present disclosure, in the compound of formula (III), L¹ is -(CH₂)ₙ-.

In a preferred embodiment of the present disclosure, in the compound of formula (III), any methylene moiety in L¹ is independently and optionally replaced by the following structural moieties: -NH-, -O-, -C(O)-, or -S-.

In a preferred embodiment of the present disclosure, in the compound of formula (III), L¹ is -(CH₂)ₙ-; wherein n is a natural number from 0 to 50; any -CH₂- moiety in L¹ is independently and optionally replaced by the following structural moieties: -NH-, -O-, -C(O)-, or -S-;
preferably, L¹ is 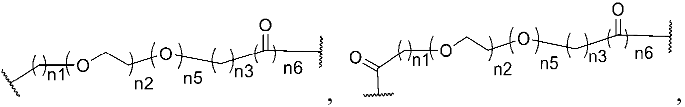 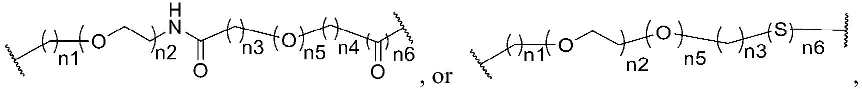
wherein n1, n2, n3, and n4 are each independently 0, 1, 2, 3, 4, 5, 6, 7, or 8;
n5 and n6 are each independently 0 or 1;
for example, L¹ is 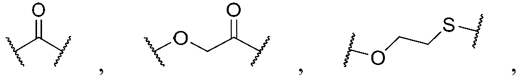 or

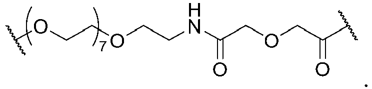

In a preferred embodiment of the present disclosure, in the compound of formula (III), L¹ is 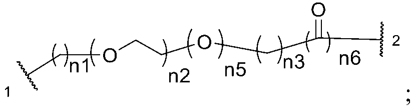
wherein position "1" is connected to ring E, and position "2" is connected to L²;
n1 and n2 are 0; n5 is 0 or 1; n3 is 0, 1, 2, 3, or 4; n6 is 1; preferably, n5 is 1.

In a preferred embodiment of the present disclosure, L¹ is 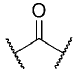 or 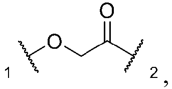 wherein position "1" is connected to ring E, and position "2" is connected to L²; preferably 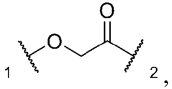 wherein position "1" is connected to ring E, and position "2" is connected to L².

In a preferred embodiment of the present disclosure, in the compound of formula (III), L² is a chemical bond, Val-Ala, Val-Cit, Ala-Ala, Gly-Glu, Gly-Asp, Gly-Glu-Gly, or Gly-Gly-Phe-Gly.

In a preferred embodiment of the present disclosure, in the compound of formula (III), L² is a chemical bond or an amino acid residue, a short peptide composed of 2 to 7 amino acid residues, 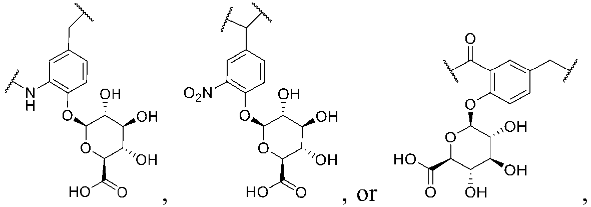 wherein the amino acid residue is Val, D-Val, Nva, Phe, Lys, Leu, Ile, Gly, Ala, D-Ala, Cit, Asp, Asn, Glu, Gln, Pro, Arg, Lys(Ac), Lys(Me), Lys(Et), Lys(nPr), Nva, or AA;
AA is

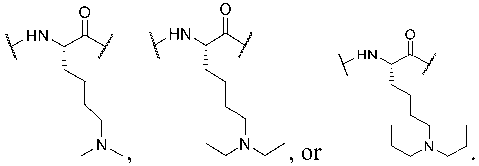

In a preferred embodiment of the present disclosure, in the compound of formula (III), L² is a chemical bond, Val, D-Val, Phe, Lys, Leu, Ile, Gly, Ala, D-Ala, Cit, Asp, Asn, Glu, Gln, Val-Cit, Val-Ala, Val-Lys, Val-Lys(Ac), Phe-Lys, Phe-Lys(Ac), Leu-Lys, Leu-Lys(Ac), Ala-Ala, Ala-Lys, D-Ala-Ala, Gly-Glu, Gly-Asp, Gly-Asn, Val-Glu, Val-Asp, Asp-Asp, Asn-Asn, Glu-Val-Cit, Ala-Ala-Ala, Ala-(D-Ala)-Ala, Ala-Ala-Asn, Ala-(D-Ala)-Asn, Ala-Ala-Asp, Val-Lys-Gly, D-Val-Leu-Lys, Gly-Gly-Arg, Gly-Gly-Gly, Lys-Ala-Asn, Gly-Phe-Gly, Gly-Gly-Phe, Asn-Pro-Val, Ala-Lys-Gly, Gly-Lys-Gly, Gly-Glu-Gly, Gly-Gly-Gly-Gly, Gly-Gly-Phe-Gly, Gly-Gly-Glu-Gly, Lys-Ala-Ala-Asn, Lys-Ala-Ala-Asp, Ala-Ala-Pro-Val, Ala-Ala-Pro-Nva,

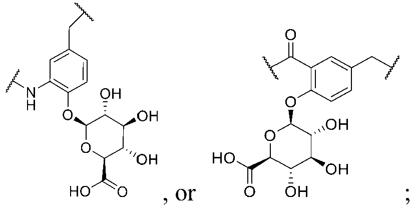

In a preferred embodiment of the present disclosure, in the compound of formula (III), L² is a chemical bond, Lys, Cit, Asp, Asn, Glu, Gln, Val-Cit, Val-Ala, Asp-Asp, Ala-Ala, Gly-Glu, Gly-Asp, Gly-Asn, Asn-Asn, Gly-Glu-Gly, Gly-Gly-Glu-Gly, Gly-Gly-Phe-Gly, or Gly-Gly-Gly-Gly.

In a preferred embodiment of the present disclosure, in the compound of formula (III), L² is a chemical bond, Val-Cit, Val-Ala, Gly-Glu, Gly-Asp, Gly-Glu-Gly, Gly-Gly-Phe-Gly, or

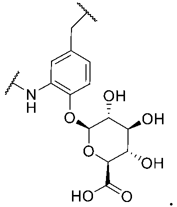

In a preferred embodiment of the present disclosure, in the compound of formula (III), L² is Val, Ala, Gly, Glu, Cit, Asp, Phe, Val-Ala, Ala-Ala, Gly-Glu, Gly-Asp, Gly-Glu-Gly, Gly-Gly-Phe-Gly, or Val-Cit; wherein the N-terminus is connected to ring E, and the C-terminus is connected to L²; preferably, L² is Val-Ala, Ala-Ala, Gly-Glu, Gly-Asp, Gly-Glu-Gly, Gly-Gly-Phe-Gly, or Val-Cit, wherein the N-terminus is connected to ring E, and the C-terminus is connected to L²; more preferably, L² is Val-Ala, Ala-Ala, Gly-Glu, Gly-Gly-Phe-Gly, or Val-Cit, wherein the N-terminus is connected to ring E, and the C-terminus is connected to L²; most preferably, L² is Gly-Gly-Phe-Gly or Val-Cit, wherein the N-terminus is connected to ring E, and the C-terminus is connected to L².

In a preferred embodiment of the present disclosure, in the compound of formula (III), 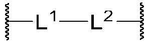 is: 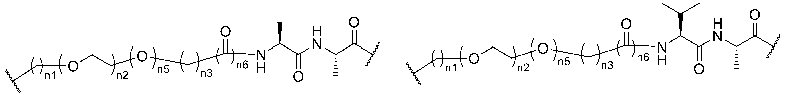 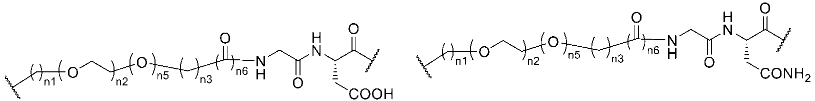 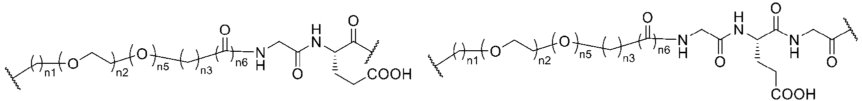 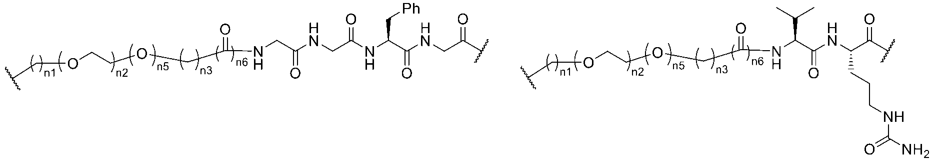 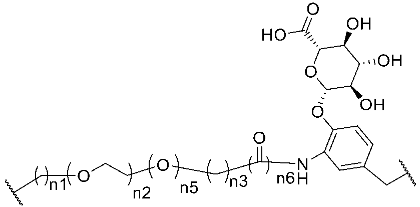 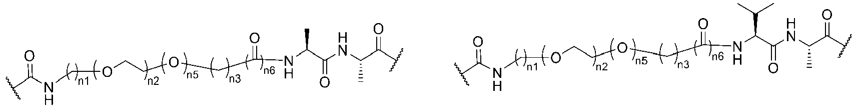 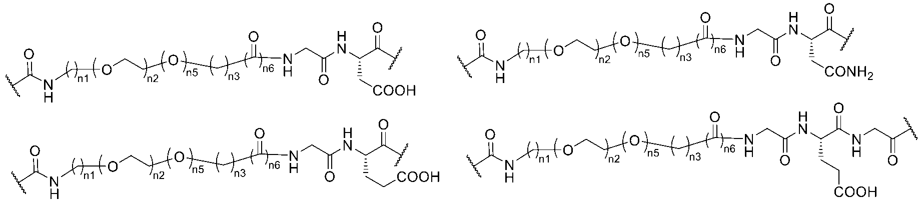 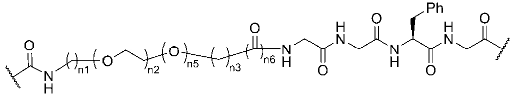 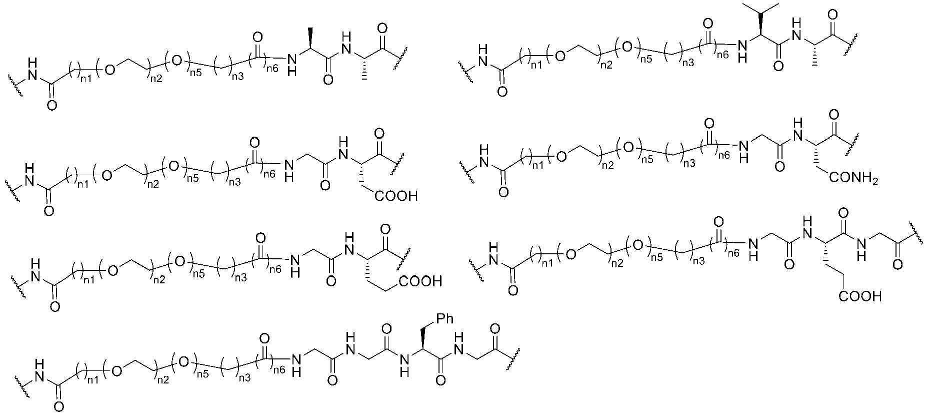 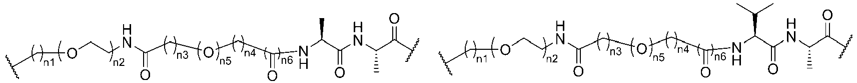 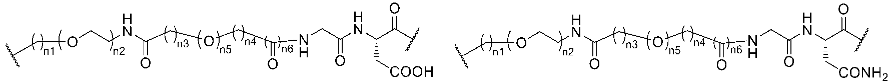 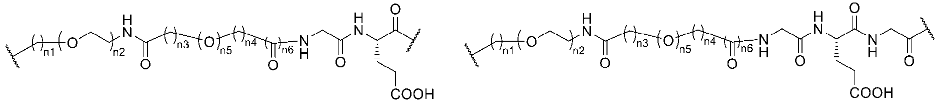 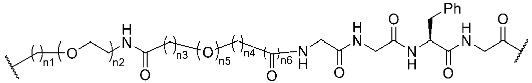 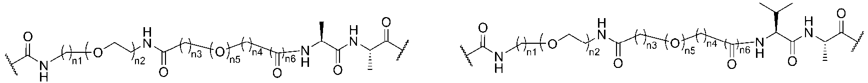 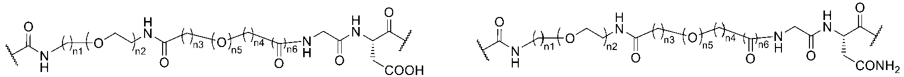 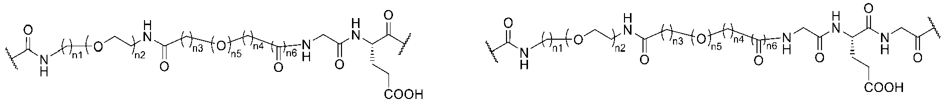 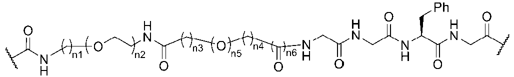 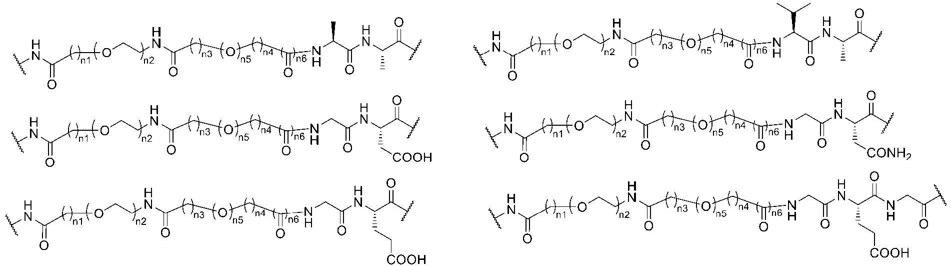 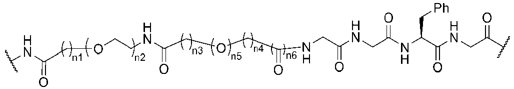 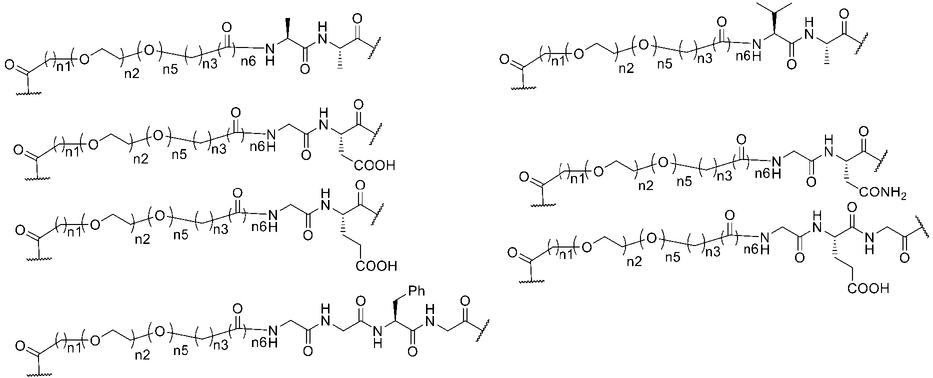 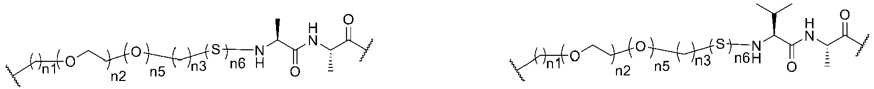 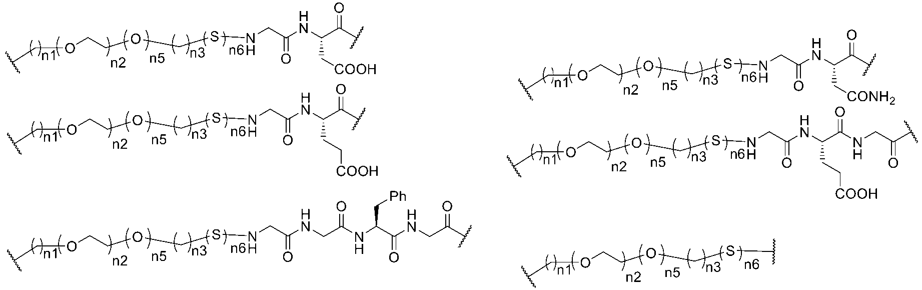
wherein n1, n2, n3, and n4 are each independently a natural number from 0 to 8;
n5 and n6 are each independently 0 or 1.

In a preferred embodiment of the present disclosure, in the compound of formula (III), 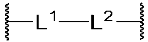 is: 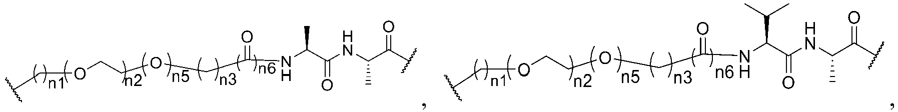 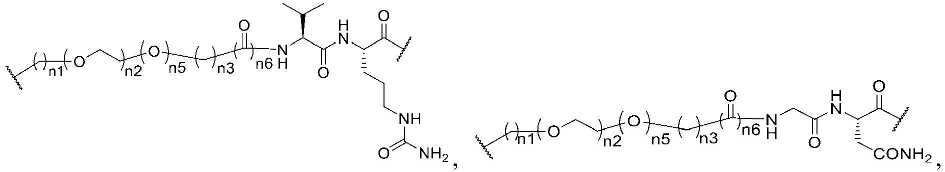 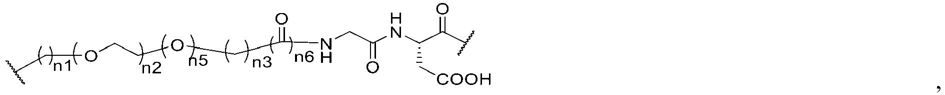 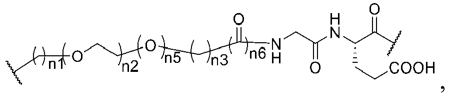 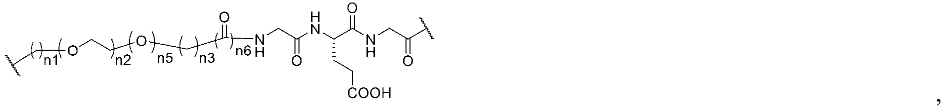 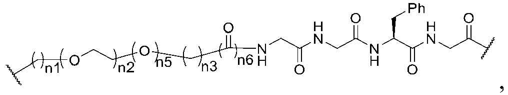 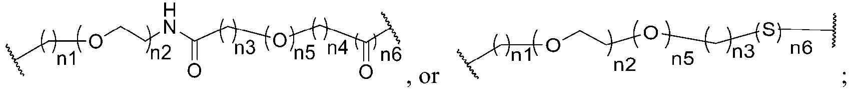
n1, n2, n3, and n4 are each independently 0, 1, 2, 3, 4, 5, 6, 7, or 8;
n5 and n6 are each independently 0 or 1;
for example, 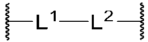 is:

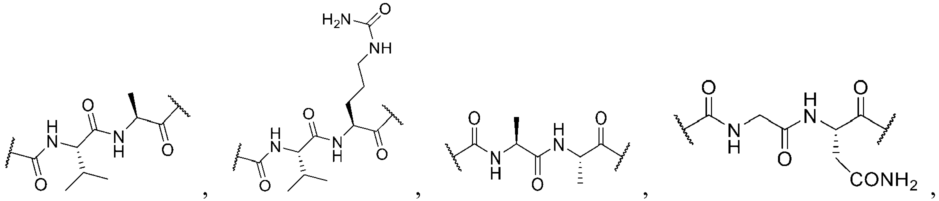

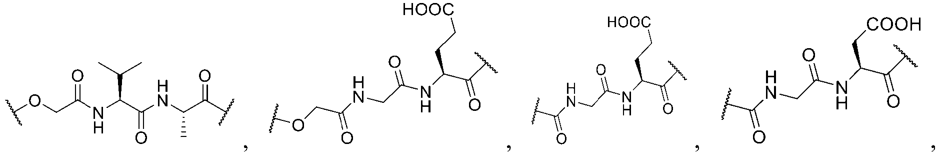

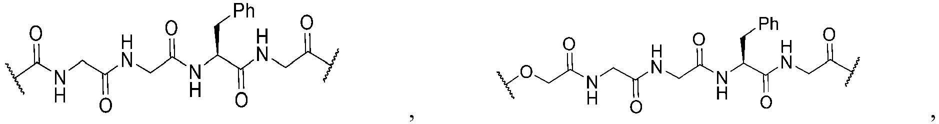

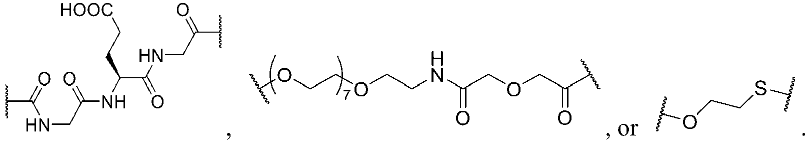

In a preferred embodiment of the present disclosure, in the compound of formula (III), 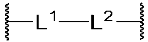 is: 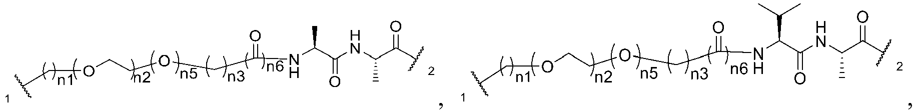 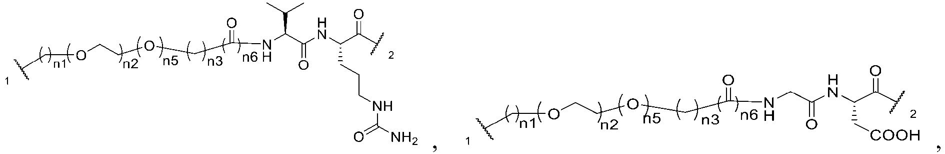 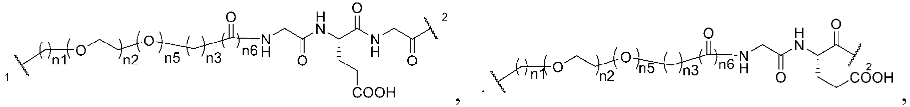 or 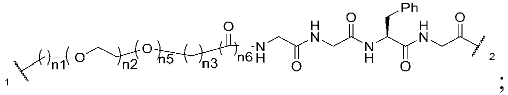
wherein position "1" is connected to ring E, and position "2" is connected to Tr;
each of n1 and n2 is 0; n3 is 0, 1, 2, 3, or 4; n5 is 0 or 1; n6 is 1;
preferably, 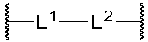 is: 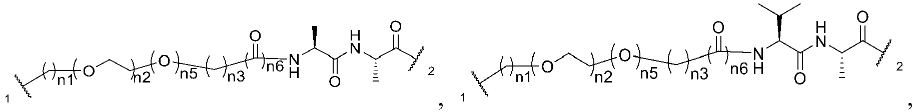 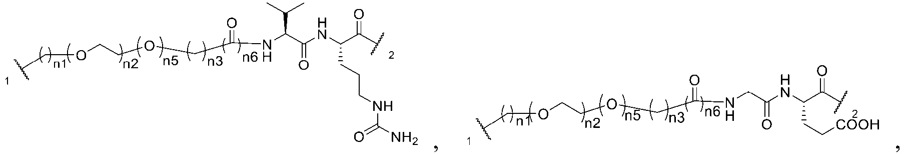 or 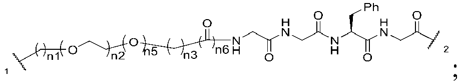
   wherein position "1" is connected to ring E, and position "2" is connected to Tr;
   each of n1 and n2 is 0; n3 is 0, 1, 2, 3, or 4; n5 is 0 or 1; n6 is 1;
   more preferably, 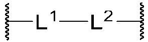 is: 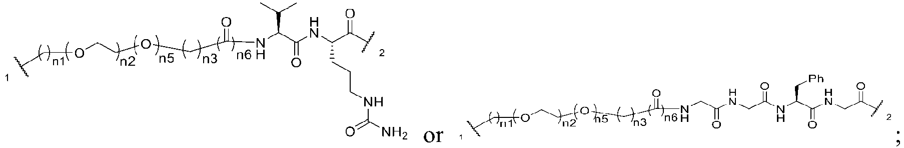
      wherein position "1" is connected to ring E, and position "2" is connected to Tr;
      each of n1 and n2 is 0; n3 is 0, 1, 2, 3, or 4; n5 is 0 or 1; n6 is 1.

In a preferred embodiment of the present disclosure, 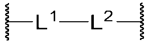 is: 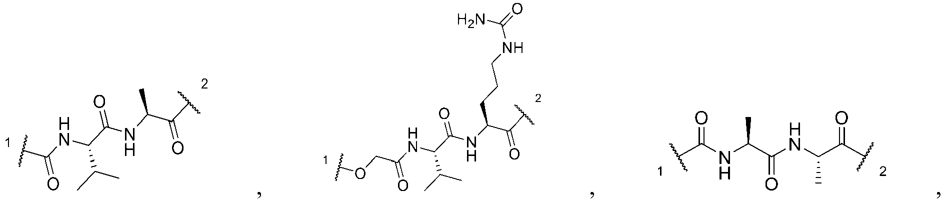 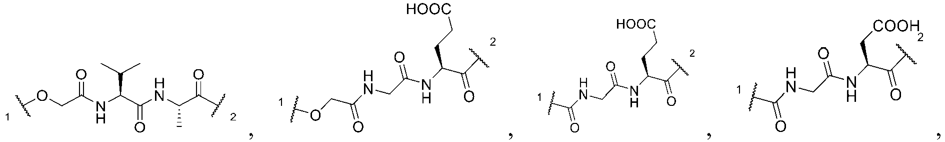 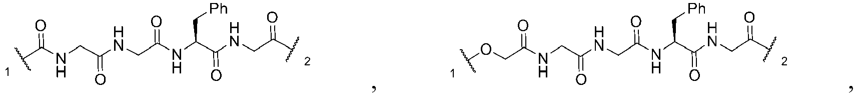 or 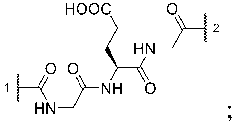 wherein position "1" is connected to ring E, and position "2" is connected to Tr;

Preferably, 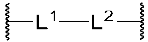 is: 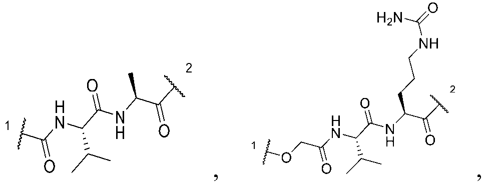 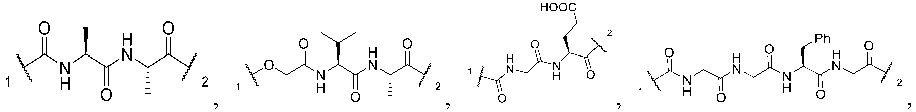 or 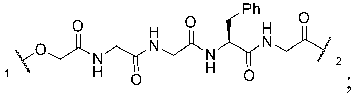 wherein position "1" is connected to ring E, and position "2" is connected to Tr;
more preferably, 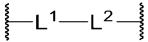 is 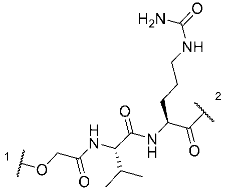 or 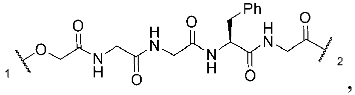 wherein position "1" is connected to ring E, and position "2" is connected to Tr.

In a preferred embodiment of the present disclosure, in the compound of formula (III), Tr is a chemical bond or selected from one or more of 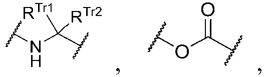 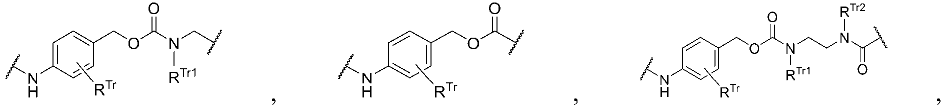 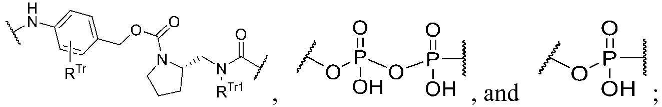
R^{Tr}, R^{Tr1}, and R^{Tr2} are each independently hydrogen, halogen, -NO₂, -CN, -OH, -NH₂, -CO₂H, -S(O)₂H, -C(O)NH₂, -SO₂NH₂, -OC(O)NH₂, -CH₂CO-(N(Me)CH₂C(O))_{z}-OR^{Tra}, - CH₂CO-(N(Me)CH₂C(O))_{z}-NHR^{Tra}, -(CH₂CH₂O)_{z}-R^{Tra}, -CONH-(CH₂CH₂O)_{z}-R^{Tra}, or -C₁₋₆ alkyl optionally substituted by R^{Tra};
R^{Tra} is hydrogen, deuterium, halogen, -NO₂, -CN, -OH, -NH₂, -N(Me)₂, -S(O)₂Me, - CO₂H, -S(O)₂OH, -C(O)NH₂, -SO₂NH₂, or -C₁₋₆ alkyl;
z is a natural number.

In a preferred embodiment of the present disclosure, in the compound of formula (III), Tr is a chemical bond or selected from one or more of

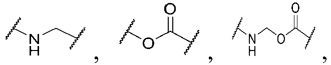

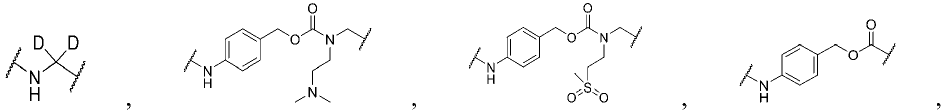

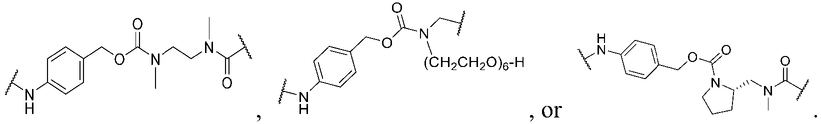

In a preferred embodiment of the present disclosure, in the compound of formula (III), Tr is a chemical bond,

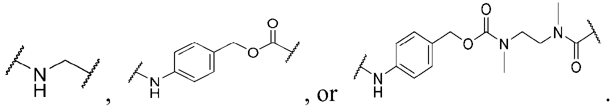

In a preferred embodiment of the present disclosure, in the compound of formula (III), R^{Tr}, R^{Tr1}, and R^{Tr2} in Tr are each independently hydrogen or methyl.

In a preferred embodiment of the present disclosure, in the compound of formula (III), Tr is a chemical bond or selected from one or more of 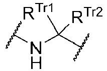 (e.g., 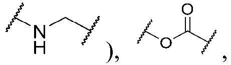 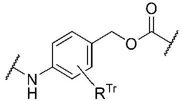 (e.g., 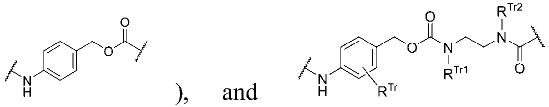 (e.g.,

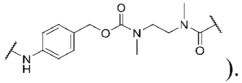

In a preferred embodiment of the present disclosure, in the compound of formula (III), Tr is a chemical bond, 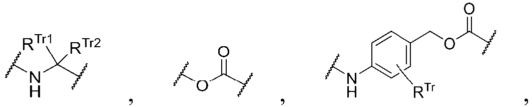 or 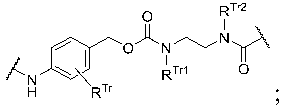 R^{Tr}, R^{Tr1}, and R^{Tr2} are each independently hydrogen or -C₁₋₆ alkyl, for example, Tr is a chemical bond, 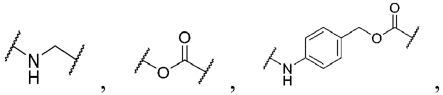 or

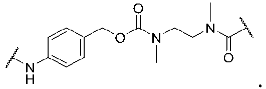

In a preferred embodiment of the present disclosure, in the compound of formula (III), Tr is a chemical bond, 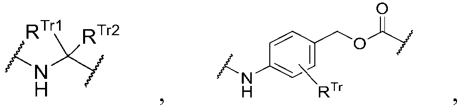 or

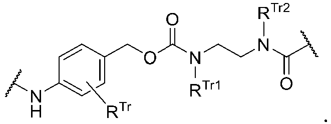

In a preferred embodiment of the present disclosure, in the compound of formula (III), Tr is a chemical bond, 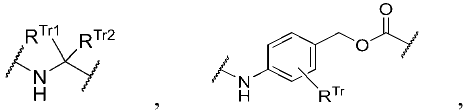 or 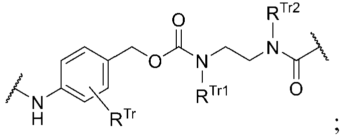 R^{Tr}, R^{Tr1}, and R^{Tr2} are each independently hydrogen or -C₁₋₆ alkyl, for example, Tr is a chemical bond, 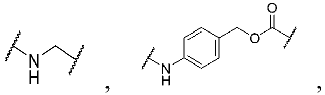 or

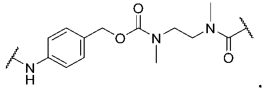

In a preferred embodiment of the present disclosure, in the compound of formula (III), Tr is 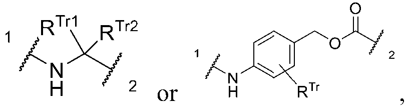 wherein position "1" is connected to L², and position "2" is connected to D; R^{Tr}, R^{Tr1}, and R^{Tr2} are each independently hydrogen or -C₁₋₆ alkyl, for example, Tr is 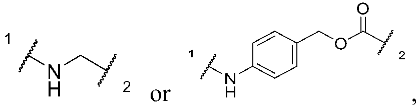 wherein position "1" is connected to L², and position "2" is connected to D.

In a preferred embodiment of the present disclosure, in the compound of formula (III), D may be a tubulin inhibitor, a topoisomerase inhibitor, a DNA damaging agent, an RNA polymerase inhibitor, an immunomodulator, or an immunostimulant.

In a preferred embodiment of the present disclosure, the tubulin inhibitor may be Auristatins (e.g., MMAE, MMAF, or MMAD), Maytansinoids (e.g., DM1 or DM4), Vinblastines, Taxols, Eribulins, Tubulysins, Colchicines, or Hemiasterlin; or a stereoisomer, isostere, analog, or derivative thereof; for example, 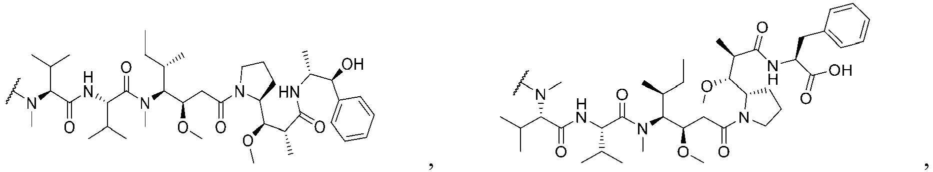 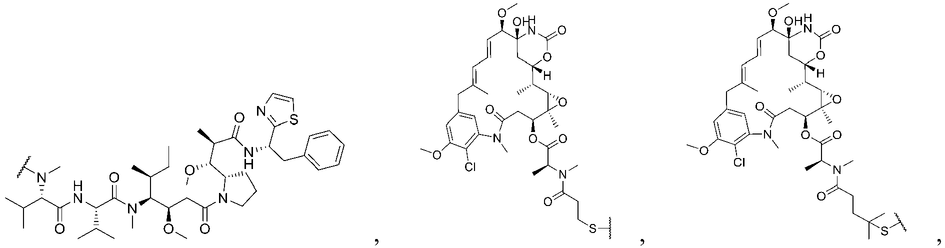 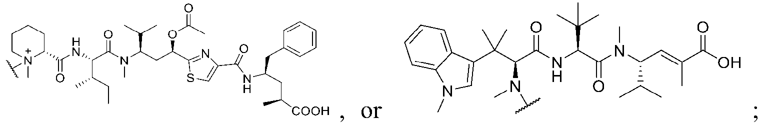 more preferably

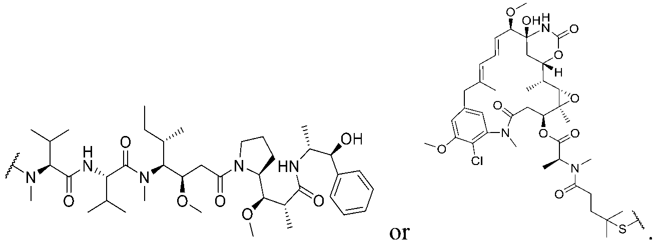

In a preferred embodiment of the present disclosure, the topoisomerase inhibitor may be camptothecin (e.g., SN38 or DX8951F); for example, 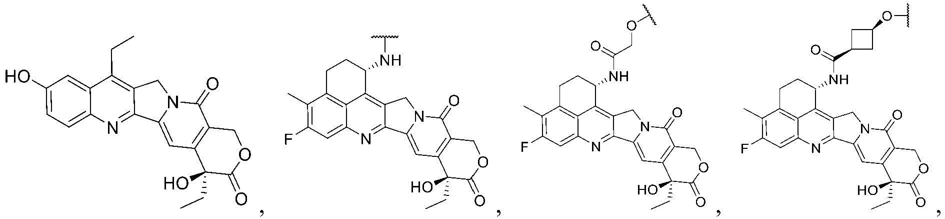 or

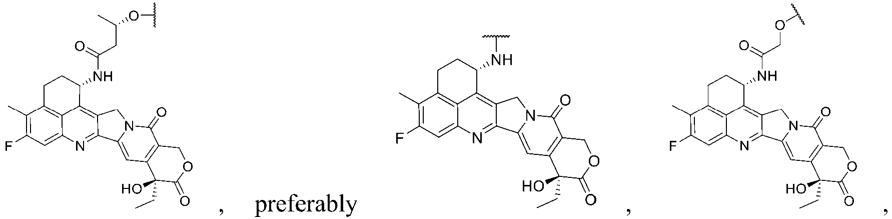

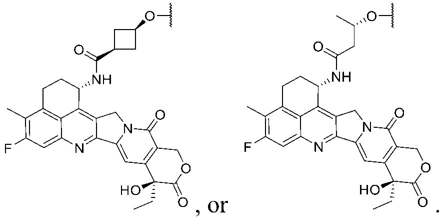

In a preferred embodiment of the present disclosure, the DNA damaging agent may be Calicheamicins, Mitomycins, anthracyclines (e.g., doxorubicin), methotrexates, Duocarmycins, a CBI dimer, a CPI dimer, a CTI dimer, a PBD dimer, an IGN dimer, a PDD dimer, or a Duocarmycin-PBD dimer; for example, 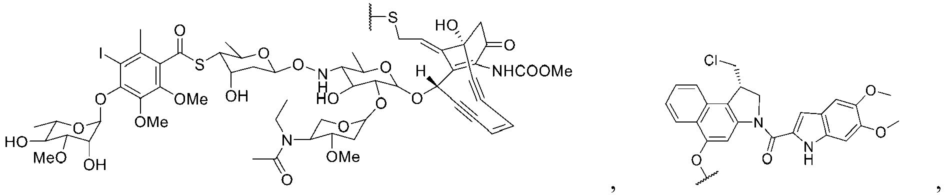 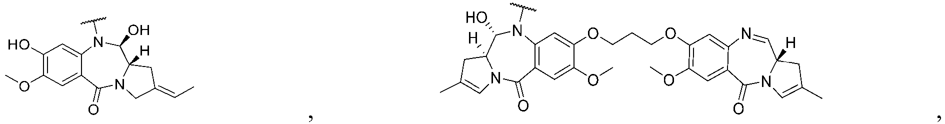 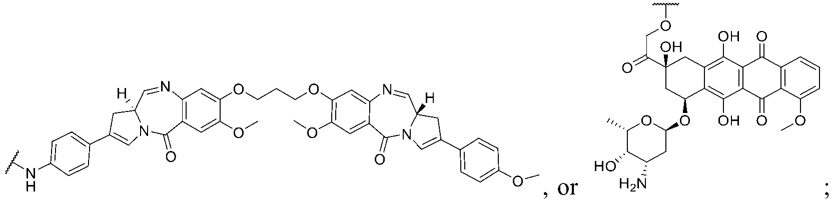 preferably

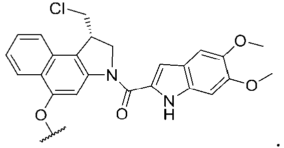

In a preferred embodiment of the present disclosure, the RNA polymerase inhibitor may be Amatoxins or a spliceosome inhibitor (e.g., Thailanstatin A); for example,

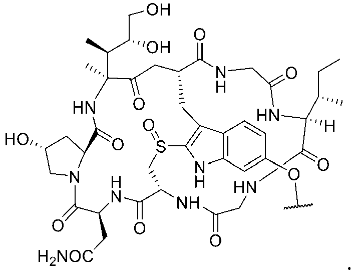

In a preferred embodiment of the present disclosure, the immunomodulator may be a glucocorticoid; for example,

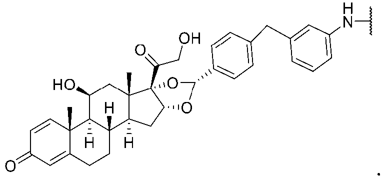

In a preferred embodiment of the present disclosure, the immunostimulant may be (e.g., Sting or TLR7/8 agonist), a protein toxin (e.g., interleukin, interferon, or tumor necrosis factor TNF-α), a selective or non-selective kinase inhibitor, a Bcl-x1 inhibitor, and a Bcl-2/Bcl-X1 inhibitor; for example,

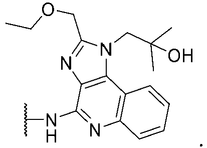

In a preferred embodiment of the present disclosure, in the compound of formula (III), D is a tubulin inhibitor, a topoisomerase inhibitor, a DNA damaging agent, an RNA polymerase inhibitor, an immunomodulator, or an immunostimulant, for example, D is 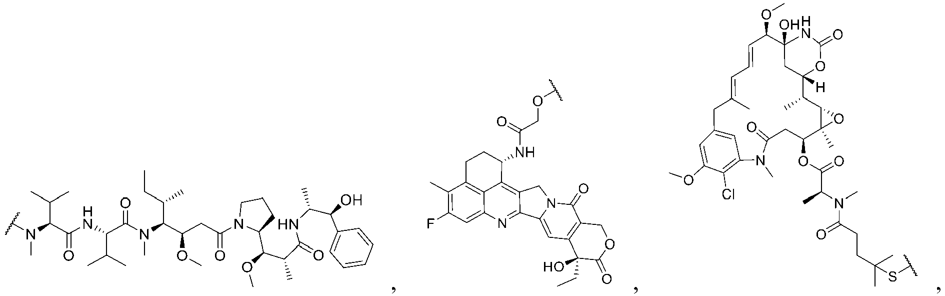 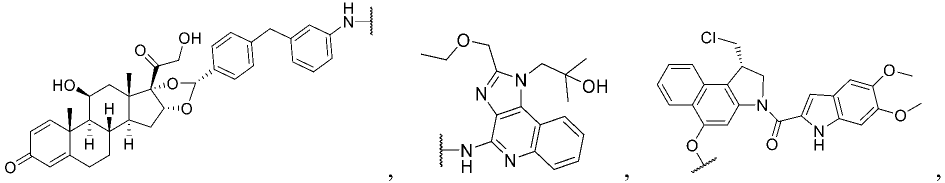 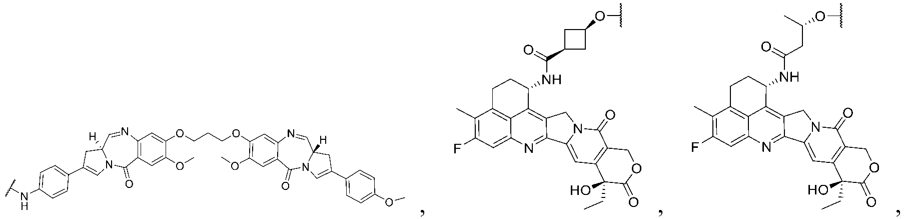 or

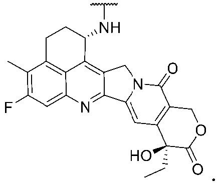

In a preferred embodiment of the present disclosure, in the compound of formula (III), D is 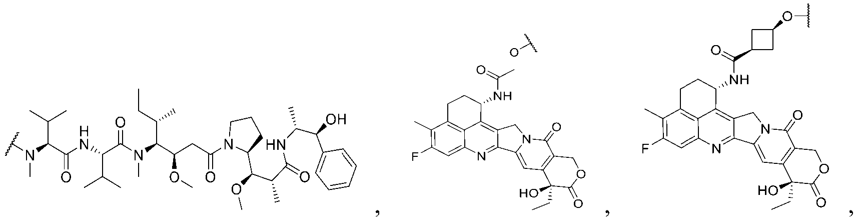 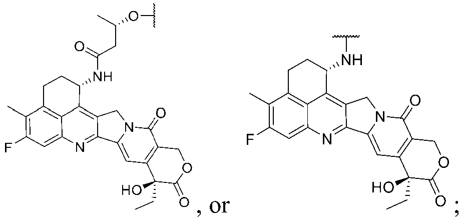 preferably, D is 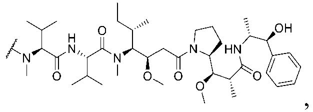 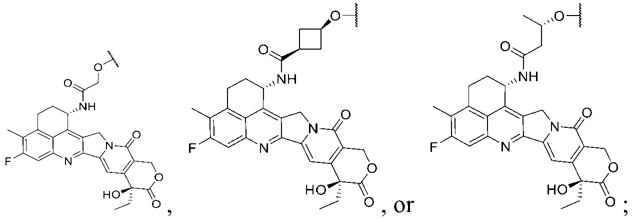 more preferably, D is

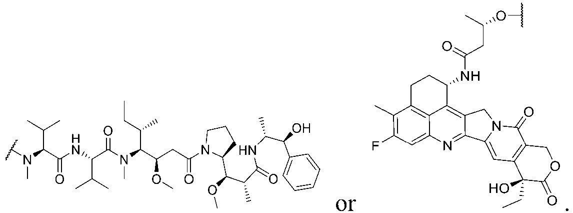

In a preferred embodiment of the present disclosure, in the compound of formula (III),
R¹ is -C₁₋₆ alkyl;
ring G is 5- to 6-membered heteroaryl, wherein the heteroatom of the 5- to 6-membered heteroaryl may be N and/or O, and the number of heteroatoms is 1, 2, or 3; for example, ring G is 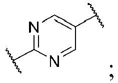
ring E is 4- to 6-membered heterocycloalkylene, wherein the heteroatom of the 4- to 6-membered heterocycloalkylene is N, and the number of heteroatoms is 1 or 2;
the 4- to 6-membered heterocycloalkylene is optionally substituted by one or more - C(O)NH-(CH₂CH₂O)_{y1}CH₃ substituents;
y1 is a natural number from 0 to 30;
L¹ is -(CH₂)ₙ-; wherein n is a natural number from 0 to 50; any -CH₂- moiety in L¹ is independently and optionally replaced by the following structural moieties: -NH-, -C(O)-, -O-, or -S-;
L² is a chemical bond, Val-Ala, Ala-Ala, Gly-Glu, Gly-Asp, Gly-Glu-Gly, or Gly-Gly-Phe-Gly;
Tr is a chemical bond, 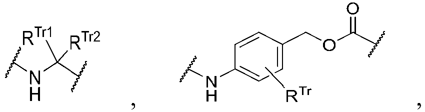 or 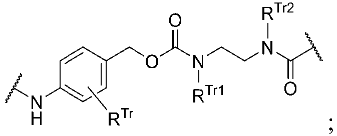 R^{Tr}, R^{Tr1}, and R^{Tr2} are each independently hydrogen or -C₁₋₆ alkyl;
D is a tubulin inhibitor, a topoisomerase inhibitor, a DNA damaging agent, an RNA polymerase inhibitor, an immunomodulator, or an immunostimulant, for example, D is 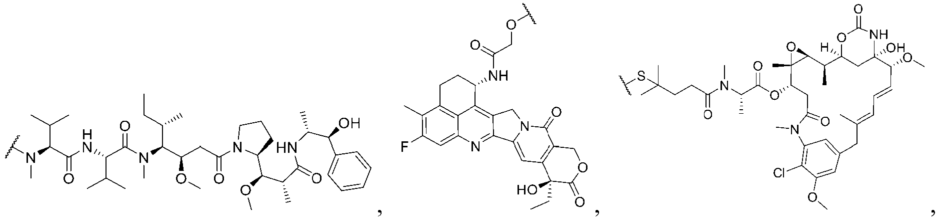 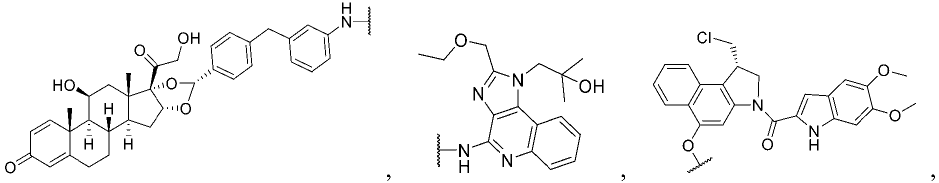 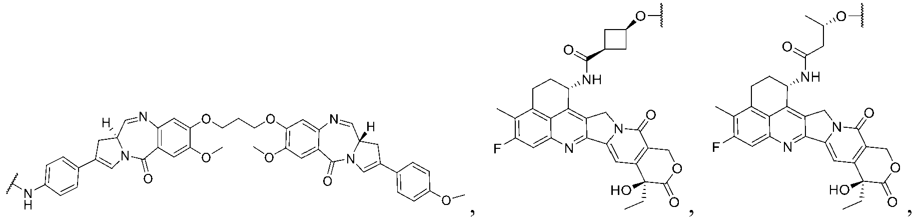 or

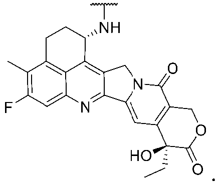

In a preferred embodiment of the present disclosure, in the compound of formula (III),
R¹ is -C₁₋₆ alkyl;
ring G is 5- to 6-membered heteroaryl, wherein the heteroatom of the 5- to 6-membered heteroaryl is N, and the number of heteroatoms is 1, 2, or 3;
ring E is 4- to 6-membered heterocycloalkylene, wherein the heteroatom of the 4- to 6-membered heterocycloalkylene is N, and the number of heteroatoms is 1 or 2;
L¹ is 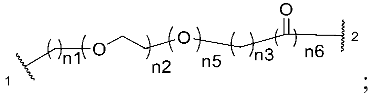 wherein position "1" is connected to ring E, and position "2" is connected to L²;
each of n1 and n2 is 0;
n3 is 0, 1, 2, 3, or 4;
n5 is 0 or 1;
n6 is 1;
L² is Val-Ala, Ala-Ala, Gly-Glu, Gly-Asp, Gly-Glu-Gly, Gly-Gly-Phe-Gly, or Val-Cit; wherein the N-terminus is connected to ring E, and the C-terminus is connected to L²;
Tr is 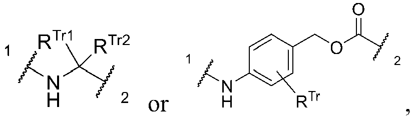 wherein position "1" is connected to L², and position "2" is connected to D; R^{Tr}, R^{Tr1}, and R^{Tr2} are each independently hydrogen or -C₁₋₆ alkyl;
D is

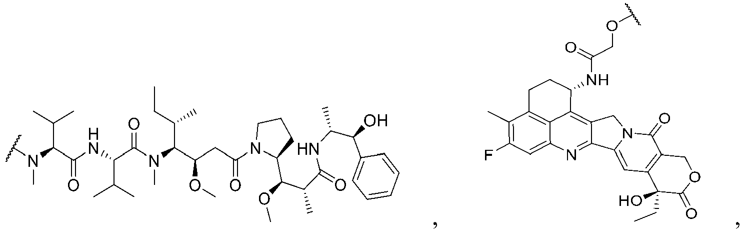

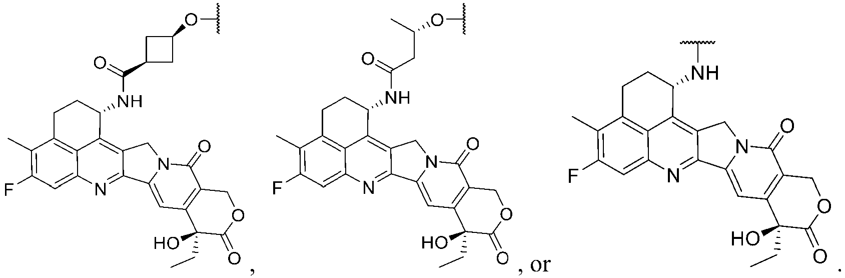

In a preferred embodiment of the present disclosure, in the compound of formula (III),
R¹ is -C₁₋₆ alkyl;
ring G is 5- to 6-membered heteroaryl, wherein the heteroatom of the 5- to 6-membered heteroaryl is N, and the number of heteroatoms is 1, 2, or 3;
ring E is 4- to 6-membered heterocycloalkylene, wherein the heteroatom of the 4- to 6-membered heterocycloalkylene is N, and the number of heteroatoms is 1 or 2;
L¹ is 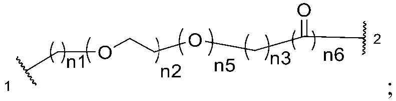 wherein position "1" is connected to ring E, and position "2" is connected to L²;
each of n1 and n2 is 0; n3 is 0, 1, 2, 3, or 4; n5 is 0 or 1; n6 is 1;
L² is Val-Ala, Ala-Ala, Gly-Glu, Gly-Gly-Phe-Gly, or Val-Cit, wherein the N-terminus is connected to ring E, and the C-terminus is connected to L²;
Tr is 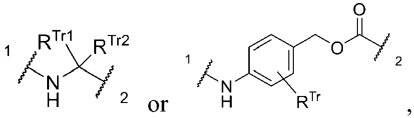 wherein position "1" is connected to L², and position "2" is connected to D; R^{Tr}, R^{Tr1}, and R^{Tr2} are each independently hydrogen or -C₁₋₆ alkyl;
D is

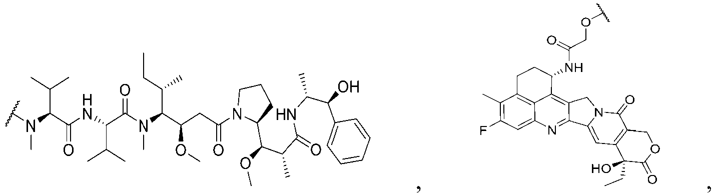

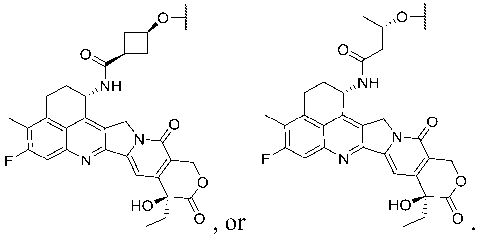

In a preferred embodiment of the present disclosure, in the compound of formula (III),
R¹ is -C₁₋₆ alkyl;
ring G is 5- to 6-membered heteroaryl, wherein the heteroatom of the 5- to 6-membered heteroaryl is N, and the number of heteroatoms is 1, 2, or 3;
ring E is 4- to 6-membered heterocycloalkylene, wherein the heteroatom of the 4- to 6-membered heterocycloalkylene is N, and the number of heteroatoms is 1 or 2;
L¹ is 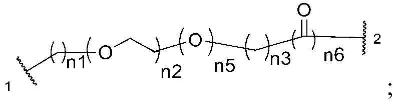 wherein position "1" is connected to ring E, and position "2" is connected to L²;
each of n1 and n2 is 0; n3 is 1, 2, 3, or 4; n5 is 1; n6 is 1;
L² is Gly-Gly-Phe-Gly or Val-Cit; wherein the N-terminus is connected to ring E, and the C-terminus is connected to L²;
Tr is 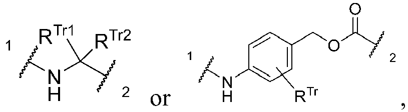 wherein position "1" is connected to L², and position "2" is connected to D; R^{Tr}, R^{Tr1}, and R^{Tr2} are each independently hydrogen or -C₁₋₆ alkyl;
D is

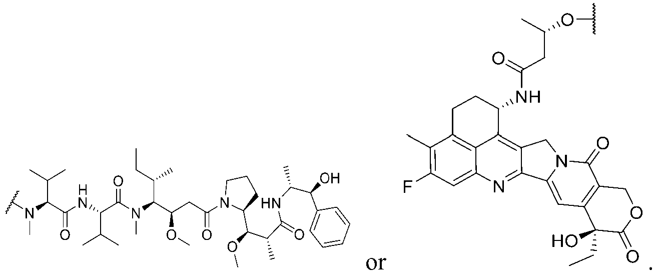

In a preferred embodiment of the present disclosure, in the compound of formula (III), or the mesomer, racemate, enantiomer, diastereomer, or any mixture thereof, or the pharmaceutically acceptable salt thereof, or the solvate thereof, or the pharmaceutically acceptable salt of the solvate thereof, the compound of formula (III) is a compound of formula (III-a): 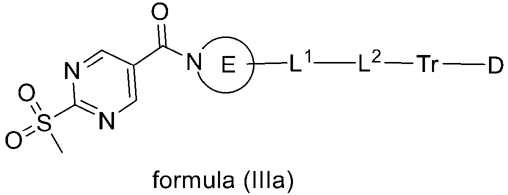 wherein ring E is as defined in general formula (III); L¹, L², Tr, and D are as defined in any one of the above embodiments of general formula (I).

In a preferred embodiment of the present disclosure, in the compound of formula (III-a), ring E, L¹, L², Tr, and D are as defined in any one of the embodiments of general formula (III).

In a preferred embodiment of the present disclosure, in the compound of formula (IIIa),
ring E is 4- to 6-membered heterocycloalkylene, wherein the heteroatom of the 4- to 6-membered heterocycloalkylene is N, and the number of heteroatoms is 1 or 2;
the 4- to 6-membered heterocycloalkylene is optionally substituted by one or more - C(O)NH-(CH₂CH₂O)_{yl}CH₃ substituents;
y1 is a natural number from 0 to 30;
L¹ is -(CH₂)ₙ-; wherein n is a natural number from 0 to 50; any -CH₂- moiety in L¹ is independently and optionally replaced by the following structural moieties: -NH-, -C(O)-, -O-, or -S-;
L² is a chemical bond, Val-Ala, Ala-Ala, Gly-Glu, Gly-Asp, Gly-Glu-Gly, or Gly-Gly-Phe-Gly;
Tr is a chemical bond, 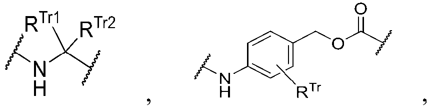 or 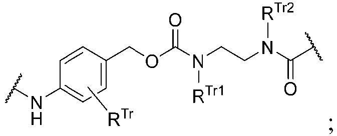 R^{Tr}, R^{Tr1}, and R^{Tr2} are each independently hydrogen or -C₁₋₆ alkyl;
D is a tubulin inhibitor, a topoisomerase inhibitor, a DNA damaging agent, an RNA polymerase inhibitor, an immunomodulator, or an immunostimulant, for example, D is 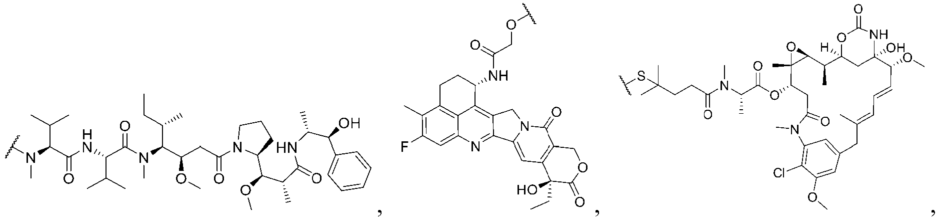 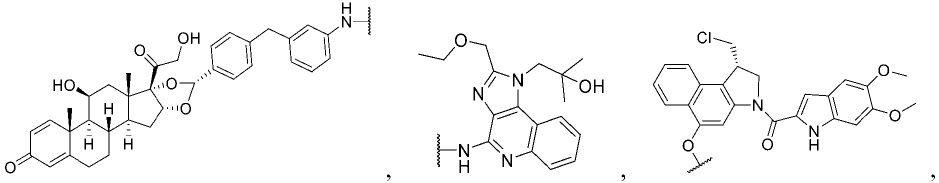 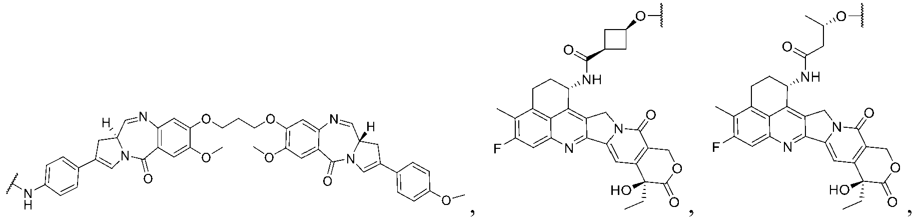 or

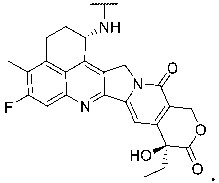

In a preferred embodiment of the present disclosure, the compound of formula (III) is a compound of the following formula: 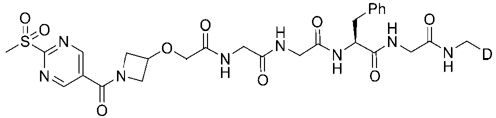 or 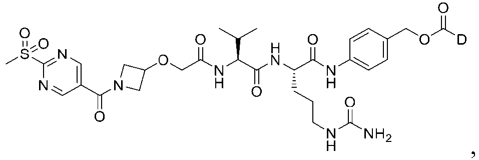 wherein D is as defined in any one of the embodiments of formula (III).

In a preferred embodiment of the present disclosure, the compound of formula (III) or formula (IIIa), or the mesomer, racemate, enantiomer, diastereomer, or any mixture thereof, or the pharmaceutically acceptable salt thereof, or the solvate thereof, or the pharmaceutically acceptable salt of the solvate thereof, is selected from:

| | |
|---|---|
| C1 | 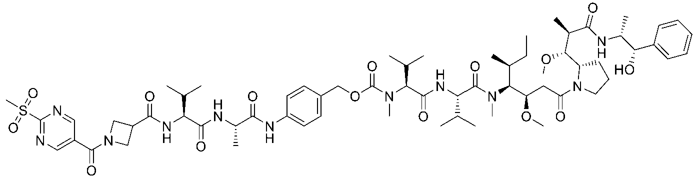 |
| C2 | 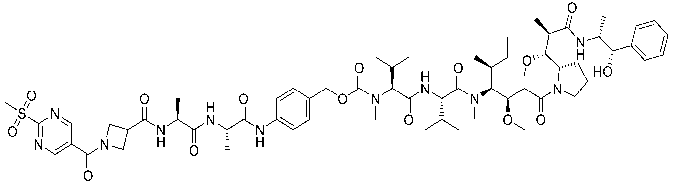 |
| C3 | 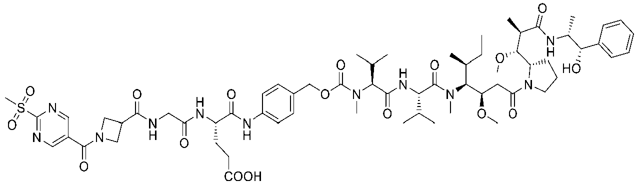 |
| C4 | 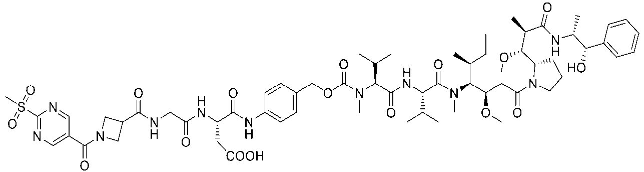 |
| C5 | 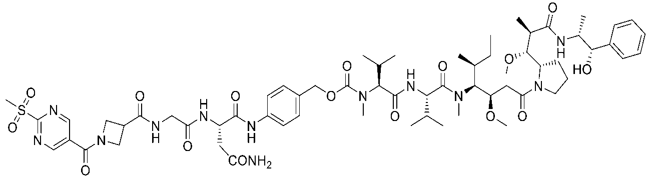 |
| C6 | 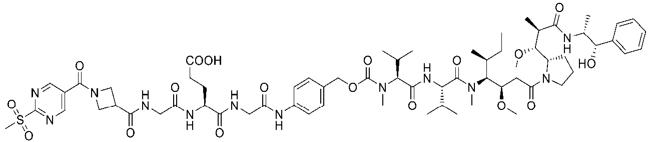 |
| C7 | 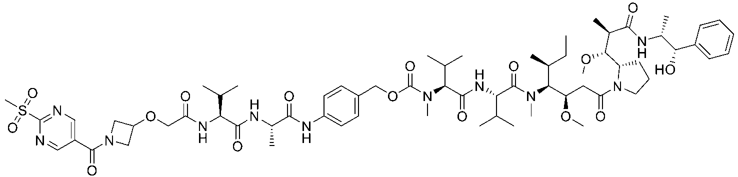 |
| C8 | 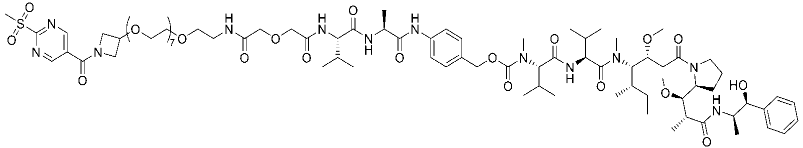 |
| C9 | 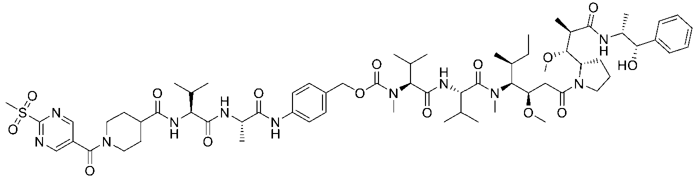 |
| C10 | 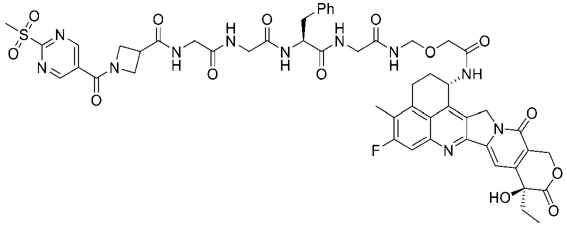 |
| C11 | 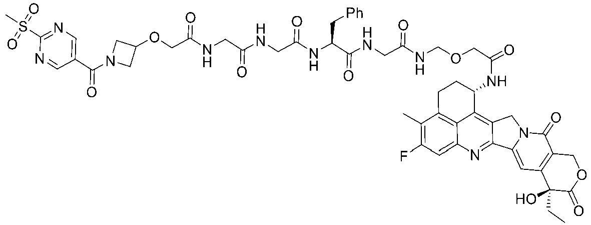 |
| C12 | 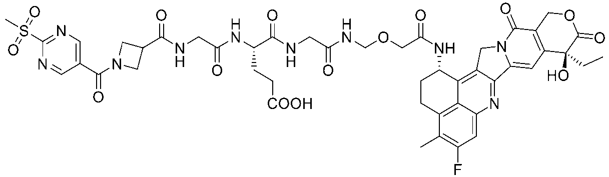 |
| C13 | 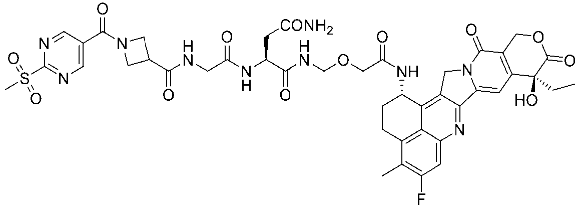 |
| C14 | 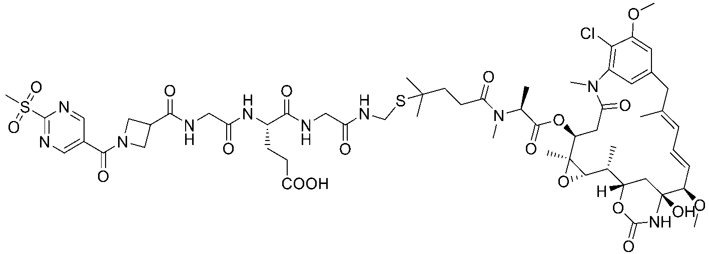 |
| C15 | 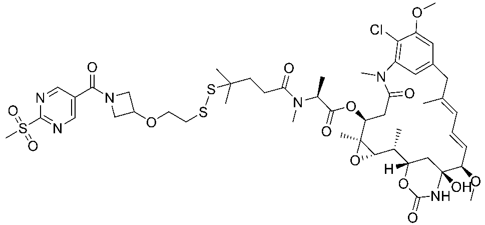 |
| C16 | 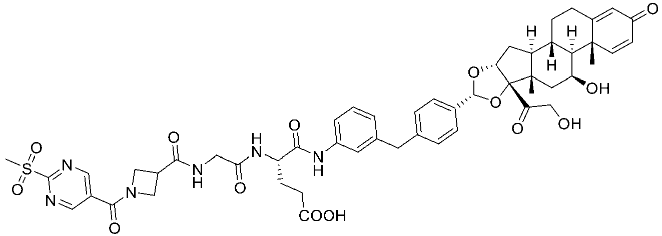 |
| C17 | 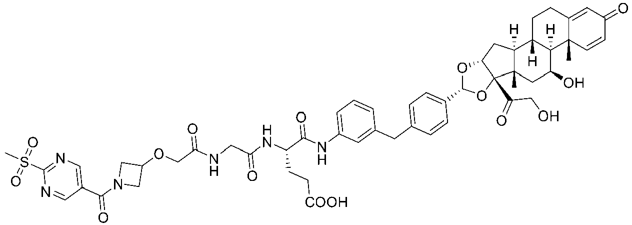 |
| C18 | 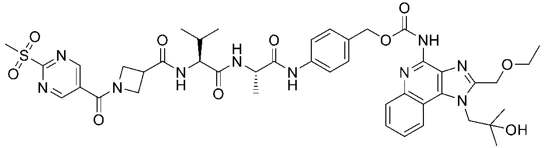 |
| C19 | 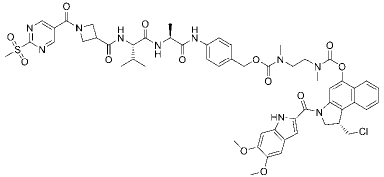 |
| C20 | 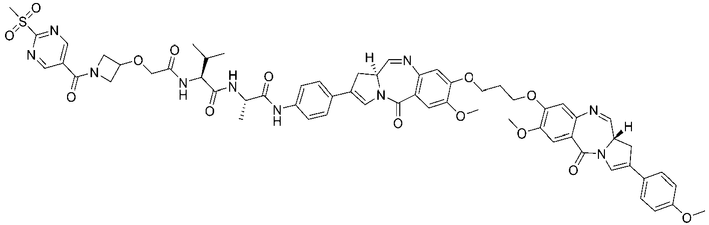 |
| C21 | 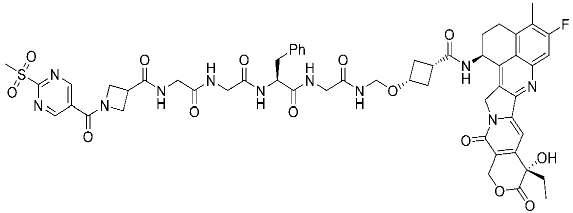 |
| C22 | 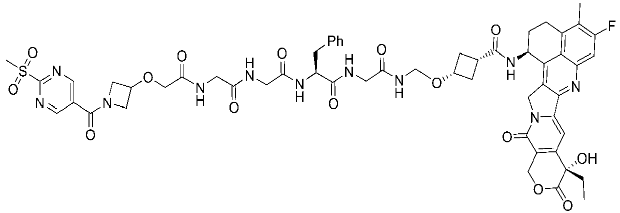 |
| C23 | 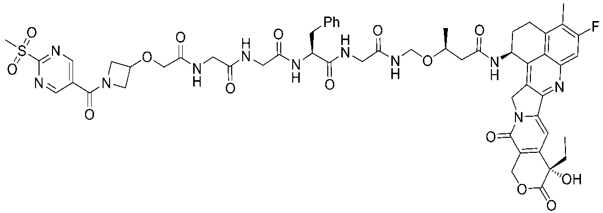 |
| C24 | 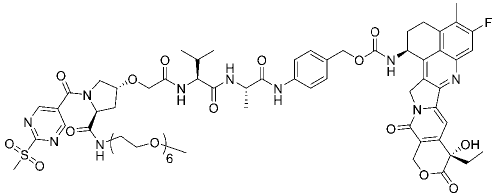 |
| C25 | 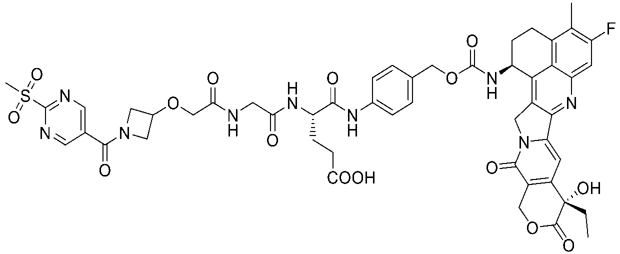 |
| C26 | 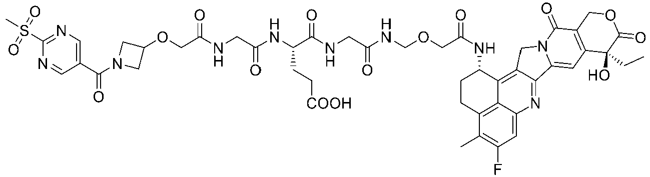 |
| C27 | 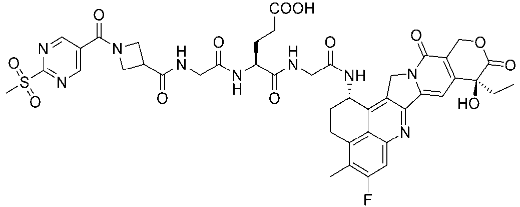 |
| C28 | 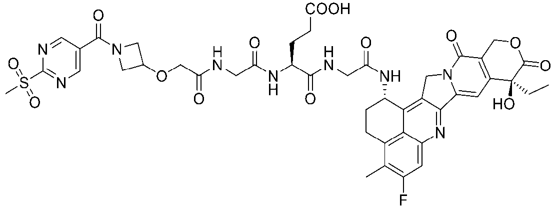 |
| C29 | 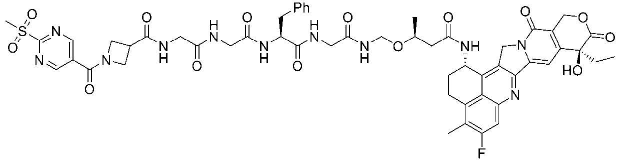 |
| C30 | 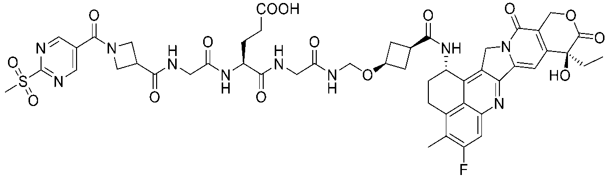 |
| C31 | 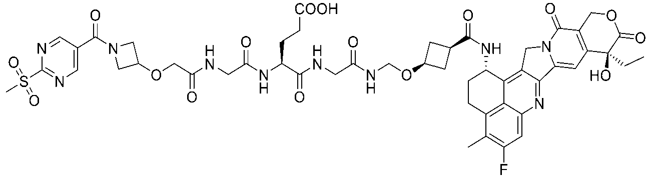 |
| C32 | 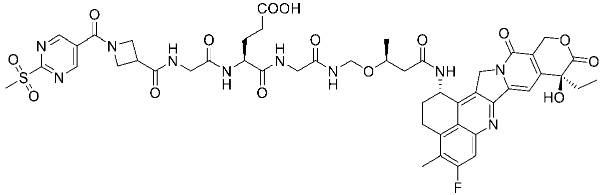 |
| C33 | 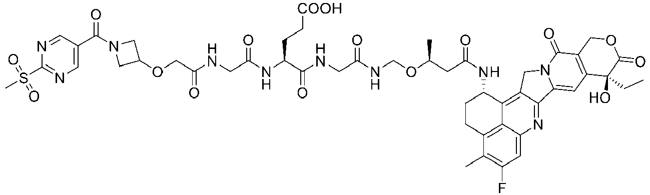 |
| C34 | 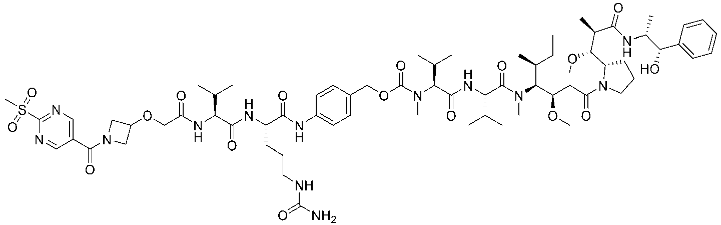 |

In another aspect, the present disclosure provides a compound of formula (1-1), or a mesomer, racemate, enantiomer, diastereomer, or any mixture thereof, or a pharmaceutically acceptable salt thereof, or a solvate thereof, or a pharmaceutically acceptable salt of the solvate thereof: 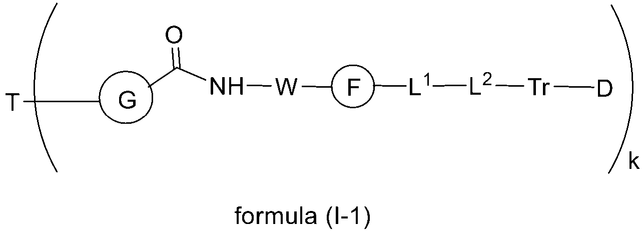 wherein
T is a ligand binding to a target, wherein the T forms a covalent linkage with ring G via a thiol or amino group thereon;
k is a natural number or a decimal from 1 to 16 (k represents drug-to-antibody ratio),
W, ring G, ring F, L¹, L², Tr, and D are as defined in any one of the embodiments of general formula (I), respectively.
In a preferred embodiment of the present disclosure, in the compound of formula (I-1),
R¹ is -C₁₋₆ alkyl;
ring G is 5- to 6-membered heteroaryl, wherein the heteroatom of the 5- to 6-membered heteroaryl may be N and/or O, and the number of heteroatoms is 1, 2, or 3; for example, ring G is 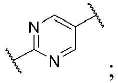
W is -(CH₂)ₜ-, wherein t is a natural number from 0 to 30;
any -CH₂- moiety in W is independently and optionally replaced by the following structural moieties: -O- or -C(O)-;
ring F is 3- to 6-membered cycloalkylene or 4- to 6-membered heterocycloalkylene; wherein the heteroatom of the 4- to 6-membered heterocycloalkylene is N, and the number of heteroatoms is independently 1 or 2;
L¹ is -(CH₂)ₙ-; wherein n is a natural number from 0 to 50; any -CH₂- moiety in L¹ is independently and optionally replaced by the following structural moieties: -C(O)-, -NH-, -O-, or -S-;
L² is a chemical bond, Val-Cit, Val-Ala, Gly-Glu, Gly-Asp, Gly-Glu-Gly, Gly-Gly-Phe-Gly, or 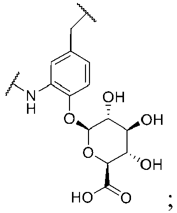
Tr is a chemical bond, 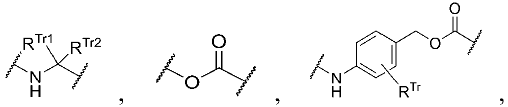 or 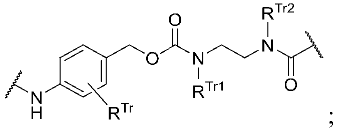 R^{Tr}, R^{Tr1}, and R^{Tr2} are each independently hydrogen or -C₁₋₆ alkyl;
D is a tubulin inhibitor, a topoisomerase inhibitor, a DNA damaging agent, an RNA polymerase inhibitor, an immunomodulator, or an immunostimulant, for example, D is

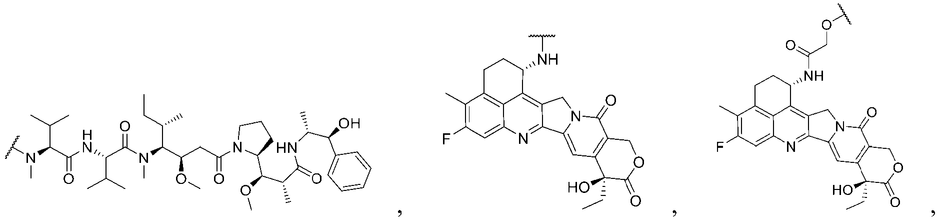

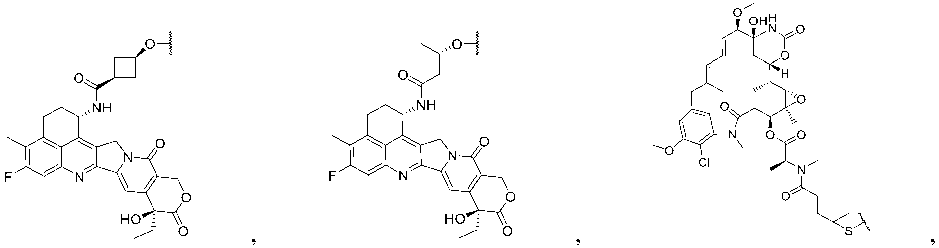

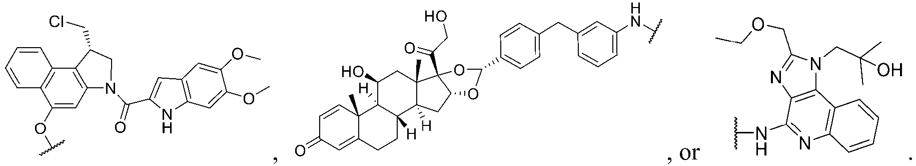

In a preferred embodiment of the present disclosure, in the compound of formula (I-1), T is a target-binding polypeptide, an antibody, or an antigen-binding fragment thereof.

In a preferred embodiment of the present disclosure, in the compound of formula (I-1), T is an antibody or an antigen-binding fragment thereof. In some embodiments, the antibody is a fully human antibody, a humanized antibody, a chimeric antibody, a probody, a bispecific antibody, a multispecific antibody, a monoclonal antibody, or a polyclonal antibody. In some embodiments, the antigen-binding fragment is Fab, Fab', F(ab')₂, Fv, scFv, a diabody, Fd, dAb, VHH, or a complementarity determining region (CDR) fragment.

In a preferred embodiment of the present disclosure, in the compound of formula (I-1), T is a monoclonal antibody.

In a preferred embodiment of the present disclosure, in the compound of formula (I-1), a T-targeted antigen may be: 5T4, AGS-16, ANGPTL4, ApoE, CD19, CTGF, CXCR5, FGF2, MCPT8, MFI2, MS4A7, NCA, Sema5b, SLITRK6, STC2, TGF, 0772P, 5T4, ACTA2, ADGRE1, AG-7, AIF1, AKR1C1, AKR1C2, ASLG659, Axl, B7H3, BAFF-R, BCMA, BMPR1B, BNIP3, C1QA, C1QB, CA6, CADM1, CCD79b, CCL5, CCR5, CCR7, CD1lc, CD123, CD138, CD142, CD147, CD166, CD19, CD19,CD22, CD21, CD20, CD205, CD22, CD223, CD228, CD25, CD30, CD33, CD37, CD38, CD40, CD45, CD45(PTPRC), CD46, CD47, CD49D(ITGA4), CD56, CD66e, CD70, CD71, CD72, CD74, CD79a, CD79b, CD80, CDCP1, CDH11, CDllb, CEA, CEACAM5, c-Met, COL6A3, COL7A1, CRIPTO, CSF1R, CTSD, CTSS, CXCL11, CXCL10, DDIT4, DLL3, DLL4, DR5, E16, EFNA4, EGFR, EGFRvIII, EGLN, EGLN3, EMR2, ENPP3, EpCAM, EphA2, EphB2R, ETBR, FcRH2, FcRH1, FGFR2, FGFR3, FLT3, FOLR-α, GD2, GEDA, GPC-1, GPNMB, GPR20, GZMB, HER2, HER3, HLA-DOB, HMOX1, IFI6, IFNG, IGF-1R, IGFBP3, IL10RA1, IL-13R, IL-2, IL20Ra, IL-3, IL-4, IL-6, IRTA2, KISS1R, KRT33A, LIV-1, LOX, LRP-1, LRRC15, LUM, LY64, LY6E, Ly86, LYPD3, MDP, MMP10, MMP14, MMP16, MPF, MSG783, MSLN, MUC-1, NaPi2b, Napi3b, Nectin-4, Nectin-4, NOG, P2X5, pCAD, P-Cadherin, PDGFRA, PDK1, PD-L1, PFKFB3, PGF, PGK1, PIK3AP1, PIK3CD, PLOD2, PSCA, PSCAhlg, PSMA, PSMA, PTK7, P-cadherin, RNF43, NaPi2b, ROR1, ROR2, SERPINE1, SLC39A6, SLTRK6, STAT1, STEAP1, STEAP2, TCF4, TENB2, TGFB1, TGFB2, TGFBR1, TNFRSF21, TNFSF9, TNF-α, Trop-2, TrpM4, Tyro7, UPK1B, VEGFA, WNT5A, ADAM9, epidermal growth factor, brevican, mesothelin, sodium phosphate cotransporter 2B, Claudin18.2, endothelin receptor, mucin (such as mucin 1 and mucin 16), guanylate cyclase C, integrin a4p7, integrin a5p6, trophoblast glycoprotein, or tissue factor.

In a preferred embodiment of the present disclosure, in the compound of formula (I-1), the T-targeted antigen may be: 5T4, AGS-16, ANGPTL4, ApoE, CD19, CTGF, CXCR5, FGF2, MCPT8, MFI2, MS4A7, NCA, Sema5b, SLITRK6, STC2, TGF, 0772P, 5T4, ACTA2, ADGRE1, AG-7, AIF1, AKR1C1, AKR1C2, ASLG659, Axl, B7H3, BAFF-R, BCMA, BMPR1B, BNIP3, C1QA, C1QB, CA6, CADM1, CCD79b, CCL5, CCR5, CCR7, CD11c, CD123, CD138, CD142, CD147, CD166, CD19, CD19,CD22, CD21, CD20, CD205, CD22, CD223, CD228, CD25, CD30, CD33, CD37, CD38, CD40, CD45, CD45(PTPRC), CD46, CD47, CD49D(ITGA4), CD56, CD66e, CD70, CD71, CD72, CD74, CD79a, CD79b, CD80, CDCP1, CDH11, CDllb, CEA, CEACAM5, c-Met, COL6A3, COL7A1, CRIPTO, CSF1R, CTSD, CTSS, CXCL11, CXCL10, DDIT4, DLL3, DLL4, DR5, E16, EFNA4, EGFR, EGFRvIII, EGLN, EGLN3, EMR2, ENPP3, EpCAM, EphA2, EphB2R, ETBR, FcRH2, FcRH1, FGFR2, FGFR3, FLT3, FOLR-α, GD2, GEDA, GPC-1, GPC-3, ADAM9, GPNMB, GPR20, GZMB, HER2, HER3, HLA-DOB, HMOX1, IFI6, IFNG, IGF-1R, IGFBP3, IL10RA1, IL-13R, IL-2, IL20Ra, IL-3, IL-4, IL-6, IRTA2, KISS1R, KRT33A, LIV-1, LOX, LRP-1, LRRC15, LUM, LY64, LY6E, Ly86, LYPD3, MDP, MMP10, MMP14, MMP16, MPF, MSG783, MSLN, MUC-1, NaPi2b, Napi3b, Nectin-4, Nectin-4, NOG, P2X5, pCAD, P-Cadherin, PDGFRA, PDK1, PD-L1, PFKFB3, PGF, PGK1, PIK3AP1, PIK3CD, PLOD2, PSCA, PSCAhlg, PSMA, PSMA, PTK7, P-cadherin, RNF43, NaPi2b, ROR1, ROR2, SERPINE1, SLC39A6, SLTRK6, STAT1, STEAP1, STEAP2, TCF4, TENB2, TGFB1, TGFB2, TGFBR1, TNFRSF21, TNFSF9, TNF-α, Trop-2, TrpM4, Tyro7, UPK1B, VEGFA, WNT5A, ADAM9, epidermal growth factor, brevican, mesothelin, sodium phosphate cotransporter 2B, Claudin18.2, endothelin receptor, mucin (such as mucin 1 and mucin 16), guanylate cyclase C, integrin a4p7, integrin a5p6, trophoblast glycoprotein, or tissue factor.

In a preferred embodiment of the present disclosure, in the compound of formula (I-1), the T-targeted antigen is: HER2, HER3, B7H3, TNF-α, TROP2, Claudin18.2, CD30, CD33, CD70, ADAM9, or EGFR.

In a preferred embodiment of the present disclosure, in the compound of formula (I-1), the T-targeted antigen is: HER2, HER3, B7H3, TNF-α, TROP2, Claudin18.2, CD30, CD33, CD70, ADAM9, EGFR, GPC-3, or ADAM9.

In a preferred embodiment of the present disclosure, in the compound of formula (I-1), the antibody is an anti-HER2 antibody (e.g., Trastuzumab or Pertuzumab) or a variant thereof, an anti-Trop-2 antibody (Sacituzumab, M1, M2, or M3) or a variant thereof, or an anti-TNF-α antibody (Adalimumab) or a variant thereof.

In a preferred embodiment of the present disclosure, in the compound of formula (I-1), the antibody is Trastuzumab, Sacituzumab, or Adalimumab.

In a preferred embodiment of the present disclosure, in the compound of formula (I-1), the antibody is an anti-HER2 antibody (e.g., Trastuzumab or Pertuzumab) or a variant thereof, an anti-Trop-2 antibody (Sacituzumab, M1, M2, or M3) or a variant thereof, an anti-TNF-α antibody (Adalimumab) or a variant thereof, an anti-GPC-3 antibody (e.g., DB1002 antibody) or a variant thereof, or an anti-ADAM9 antibody (e.g., DB1001 antibody) or a variant thereof.

In a preferred embodiment of the present disclosure, the anti-ADAM9 antibody comprises a heavy chain variable region and a light chain variable region, wherein the heavy chain variable region comprises heavy chain complementarity determining regions HCDR1, HCDR2, and HCDR3, and the light chain variable region comprises light chain complementarity determining regions LCDR1, LCDR2, and LCDR3;
the HCDR1 comprises an amino acid sequence as shown in LYWMX₁, the HCDR2 comprises an amino acid sequence as shown in X₂IIPIFGHTX₃YX₄EKFX₅X₆, the HCDR3 comprises an amino acid sequence as shown in SEQ ID NO: 9, the LCDR1 comprises an amino acid sequence as shown in SEQ ID NO: 10, the LCDR2 comprises an amino acid sequence as shown in SEQ ID NO: 11, and the LCDR3 comprises an amino acid sequence as shown in SEQ ID NO: 12;
wherein X₁ is N, D, H, or E; X₂ is R or D; X₃ is D or K; X₄ is N or E; X₅ is K or R; X₆ is D or N.

In some embodiments of the present disclosure, in the HCDR1, X₁ is N or H, for example, H.

In some embodiments of the present disclosure, in the HCDR2, X₂ is R; X₃ is K; X₄ is N; X₅ is K; X₆ is D or N.

In some specific embodiments of the present disclosure: X₁ is H, X₂ is R; X₃ is K; X₄ is N; X₅ is K; X₆ is N.

In some embodiments of the present disclosure, the HCDR1 comprises an amino acid sequence as shown in SEQ ID NO: 7, the HCDR2 comprises an amino acid sequence as shown in SEQ ID NO: 8, the HCDR3 comprises an amino acid sequence as shown in SEQ ID NO: 9, the LCDR1 comprises an amino acid sequence as shown in SEQ ID NO: 10, the LCDR2 comprises an amino acid sequence as shown in SEQ ID NO: 11, and the LCDR3 comprises an amino acid sequence as shown in SEQ ID NO: 12.

In some specific embodiments of the present disclosure, the amino acid sequence of the HCDR1 is as shown in SEQ ID NO: 7, the amino acid sequence of the HCDR2 is as shown in SEQ ID NO: 8, the amino acid sequence of the HCDR3 is as shown in SEQ ID NO: 9, the amino acid sequence of the LCDR1 is as shown in SEQ ID NO: 10, the amino acid sequence of the LCDR2 is as shown in SEQ ID NO: 11, and the amino acid sequence of the LCDR3 is as shown in SEQ ID NO: 12.

In the present disclosure, the heavy chain variable region and/or the light chain variable region further comprises a framework region, wherein the framework region is a humanized framework region.

In some embodiments of the present disclosure, the heavy chain variable region comprises an amino acid sequence having at least 90%, at least 95%, or at least 99% sequence identity with SEQ ID NO: 13, and/or, the light chain variable region comprises an amino acid sequence having at least 90%, at least 95%, or at least 99% sequence identity with SEQ ID NO: 14.

In the present disclosure, the variable region of the amino acid sequence having at least 90%, at least 95%, or at least 99% sequence identity maintains antigen-binding function at least equivalent to that of an original sequence.

In some specific embodiments of the present disclosure, the amino acid sequence of the heavy chain variable region is as shown in SEQ ID NO: 13; and/or, the amino acid sequence of the light chain variable region is as shown in SEQ ID NO: 14.

In the present disclosure, the ADAM9 is preferably human or monkey ADAM9; in some specific embodiments of the present disclosure, the ADAM9 is human ADAM9.

In some embodiments of the present disclosure, the antibody or the antigen-binding fragment thereof: (1) is a full-length antibody, Fab, Fab', F(ab')₂, Fv, sdAb, or scFv; and/or, (2) is a monoclonal antibody, bispecific antibody, or multispecific antibody.

In the present disclosure, when the antibody or the antigen-binding fragment thereof is a full-length antibody, the full-length antibody comprises a heavy chain constant region of a heavy chain of a human antibody, preferably a heavy chain constant region of a human antibody IgG1; and/or, the full-length antibody comprises a light chain constant region of a light chain of a human antibody, preferably a light chain constant region of a human antibody κ chain.

In some embodiments of the present disclosure, the heavy chain constant region of the human antibody IgG1 has an amino acid sequence as shown in SEQ ID NO: 15 or having at least 90%, at least 95%, or at least 99% sequence identity with SEQ ID NO: 15; and/or, the light chain constant region of the human antibody κ chain has an amino acid sequence as shown in SEQ ID NO: 16 or having at least 90%, at least 95%, or at least 99% sequence identity with SEQ ID NO: 16.

In some embodiments of the present disclosure, the amino acid sequence of the heavy chain constant region of the human antibody IgG1 is as shown in SEQ ID NO: 15, and the amino acid sequence of the light chain constant region of the human antibody κ chain is as shown in SEQ ID NO: 16.

In some embodiments of the present disclosure, the heavy chain of the antibody or the antigen-binding fragment thereof comprises an amino acid sequence having at least 90%, at least 95%, or at least 99% sequence identity with SEQ ID NO: 18, and/or, the light chain of the antibody or the antigen-binding fragment thereof comprises an amino acid sequence having at least 90%, at least 95%, or at least 99% sequence identity with SEQ ID NO: 17.

In the present disclosure, the amino acid sequence having at least 90%, at least 95%, or at least 99% sequence identity maintains antigen-binding function at least equivalent to that of the original sequence.

In some specific embodiments of the present disclosure, the amino acid sequence of the heavy chain of the antibody or the antigen-binding fragment thereof is as shown in SEQ ID NO: 18; and/or, the amino acid sequence of the light chain is as shown in SEQ ID NO: 17.

In a preferred embodiment of the present disclosure, in the compound of formula (I-1), the antibody is Trastuzumab, Sacituzumab, Adalimumab, DB1001 antibody, or DB1002 antibody; preferably, the antibody is Trastuzumab, Sacituzumab, DB1001 antibody, or DB1002 antibody; more preferably, the antibody is DB1001 antibody or DB1002 antibody.

In a preferred embodiment of the present disclosure, in the compound of formula (I-1), k is a natural number or a decimal from 2 to 8, for example, 1.9, 3.6, 3.7, 3.8, 3.9, 4.1, 7.6, 7.7, or 7.9.

In a preferred embodiment of the present disclosure, in the compound of formula (I-1), k is a natural number or a decimal from 2 to 8, for example, 1.9, 3.6, 3.7, 3.8, 3.9, 4.0, 4.1, 4.3, 4.4, 7.4, 7.5, 7.6, 7.7, 7.8, or 7.9.

In a preferred embodiment of the present disclosure, in the compound of formula (I-1), or the mesomer, racemate, enantiomer, diastereomer, or any mixture thereof, or the pharmaceutically acceptable salt thereof, or the solvate thereof, or the pharmaceutically acceptable salt of the solvate thereof, the compound of formula (1-1) is a compound of formula (Ia-1): 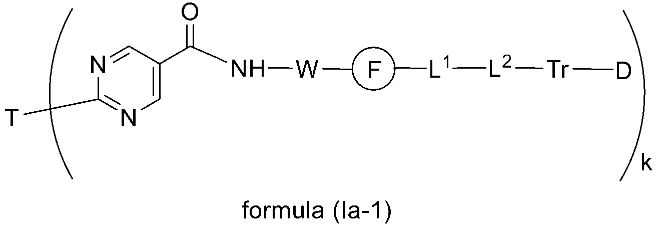 wherein
T and k are as defined in formula (1-1).
W, ring F, L¹, L², Tr, and D are as defined in any one of the above embodiments of formula (I).

In a preferred embodiment of the present disclosure, the compound of formula (I-1) or formula (Ia-1), or the mesomer, racemate, enantiomer, diastereomer, or any mixture thereof, or the pharmaceutically acceptable salt thereof, or the solvate thereof, or the pharmaceutically acceptable salt of the solvate thereof, is selected from:

| | |
|---|---|
| AA1 | 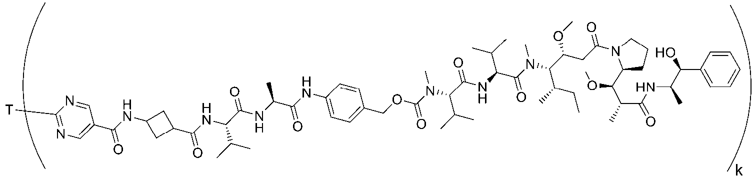 |
| AA2 | 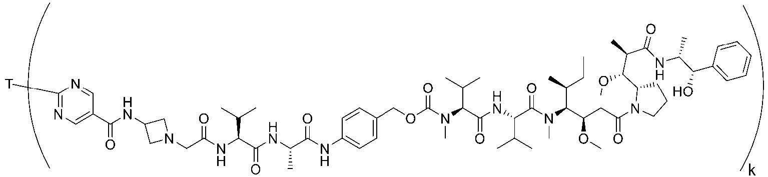 |
| AA3 | 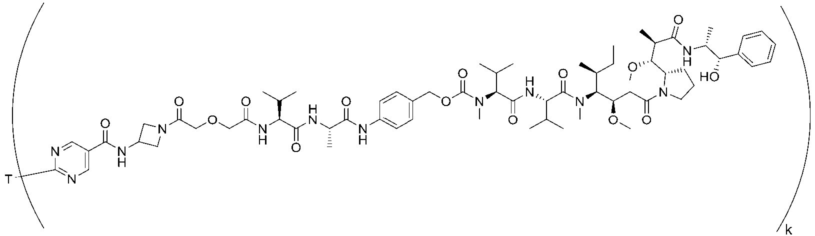 |
| AA4 | 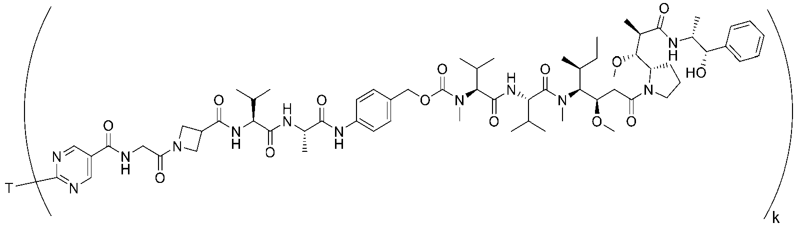 |
| AA5 | 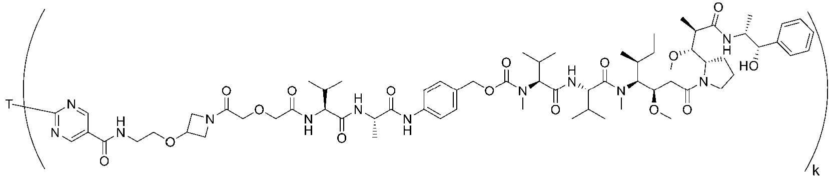 |
| AA6 | 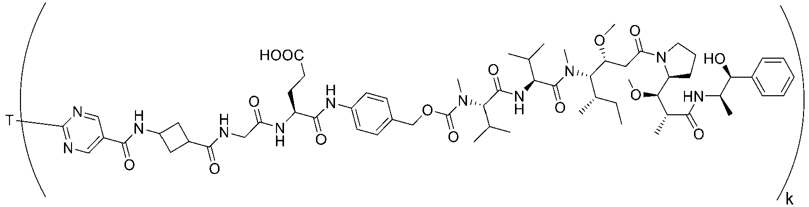 |
| AA7 | 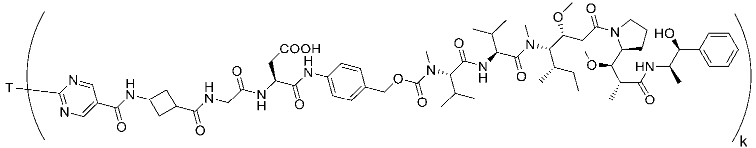 |
| AA8 | 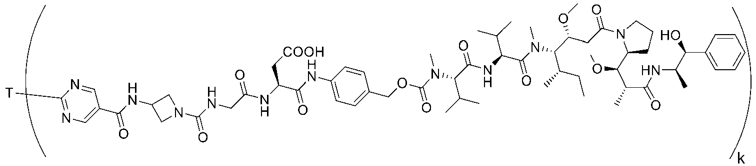 |
| AA9 | 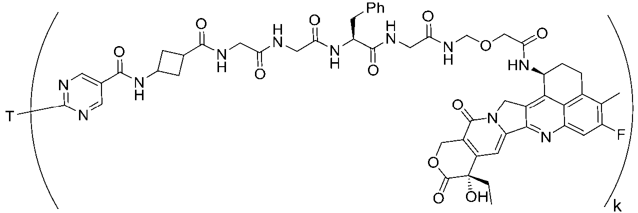 |
| AA10 | 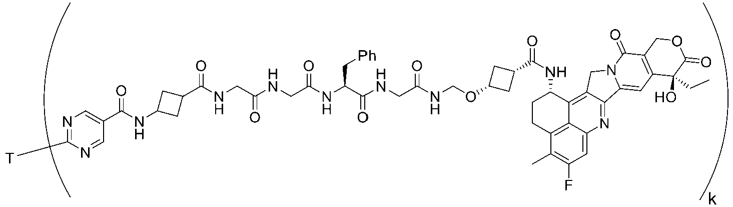 |
| AA11 | 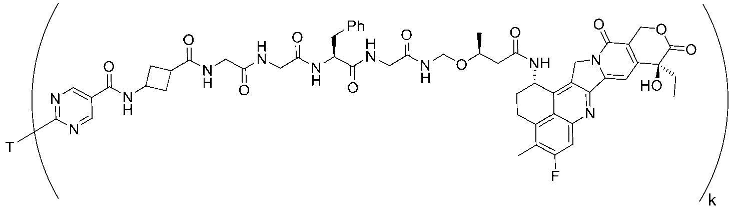 |
| AA12 | 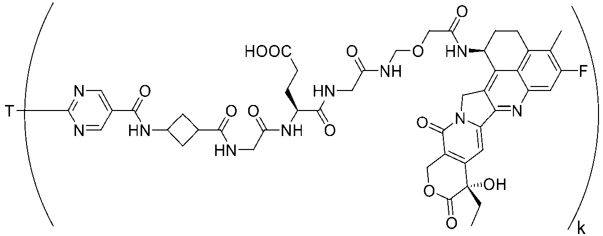 |
| AA13 | 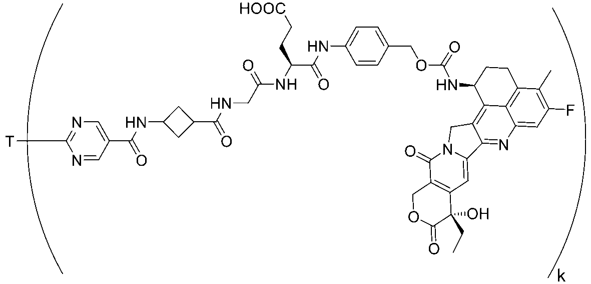 |
| AA14 | 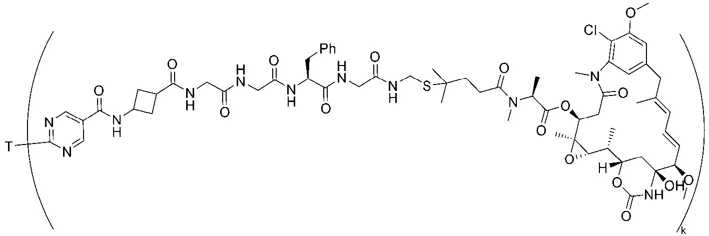 |
| AA15 | 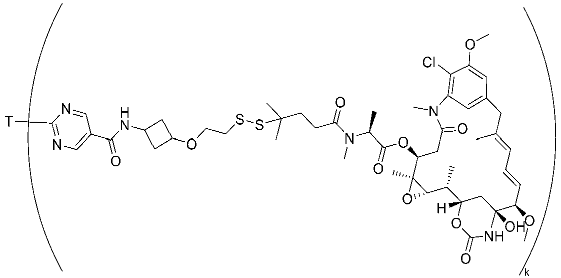 |
| AA16 | 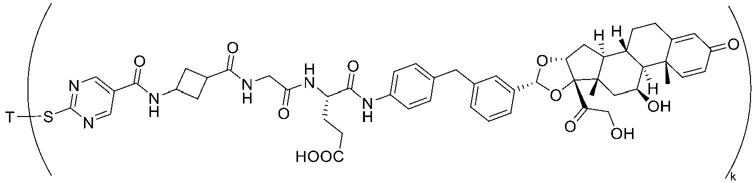 |
| AA17 | 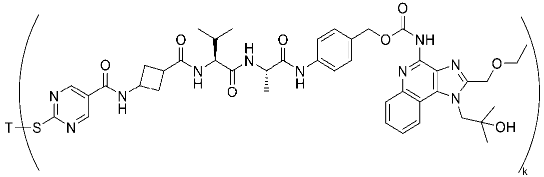 |
| AA18 | 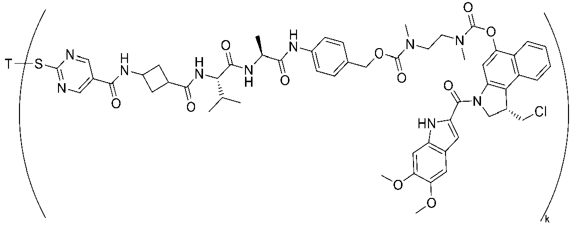 |
| AA19 | 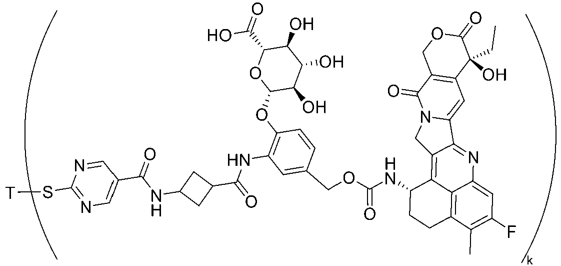 |
| AA20 | 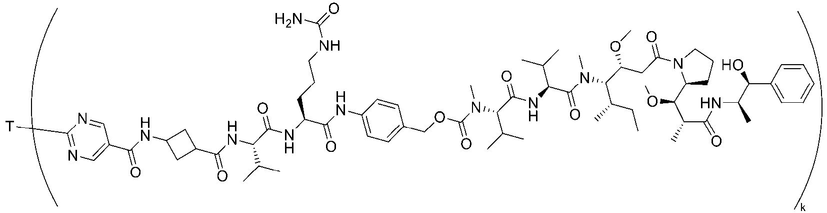 |
| AA21 | 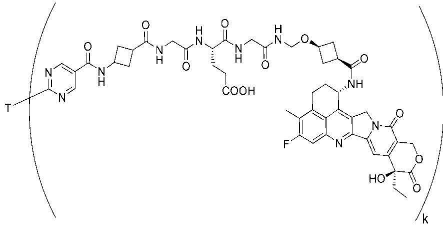 |
| AA22 | 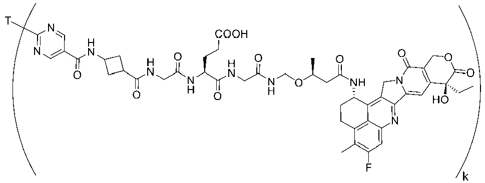 |
| AA23 | 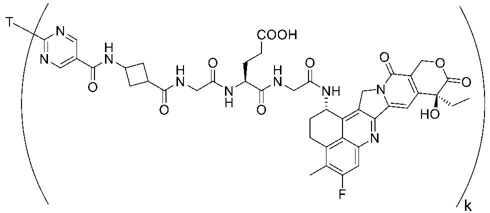 |

wherein T and k are as defined in any one of the embodiments of formula (I-1), respectively.

In some embodiments of the present disclosure, the compound of formula (1-1) or formula (Ia-1), or the mesomer, racemate, enantiomer, diastereomer, or any mixture thereof, or the pharmaceutically acceptable salt thereof, or the solvate thereof, or the pharmaceutically acceptable salt of the solvate thereof, is preferably selected from:

| | |
|---|---|
| ADC-1A | 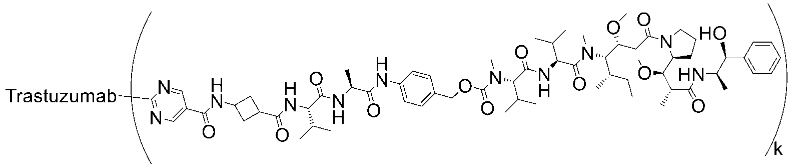 |
| ADC-2A | 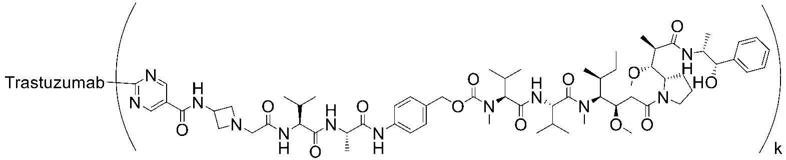 |
| ADC-3A | 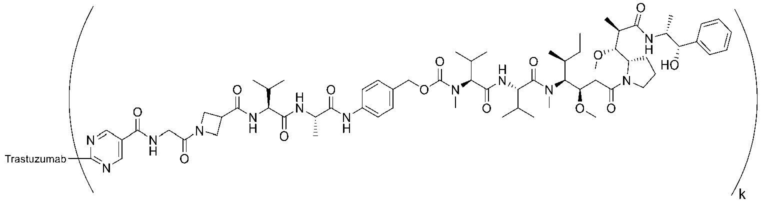 |
| ADC-4A | 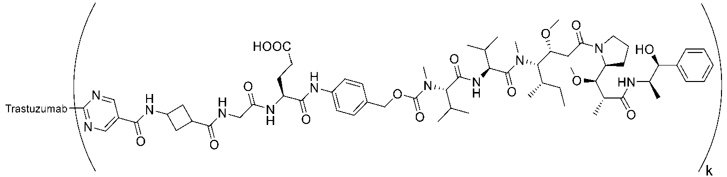 |
| ADC-5A | 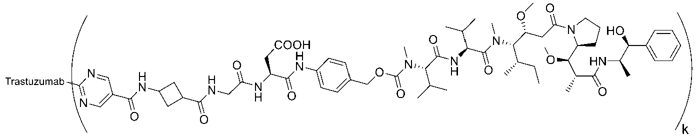 |
| ADC-6A | 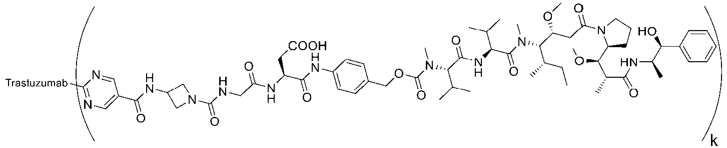 |
| ADC-7A | 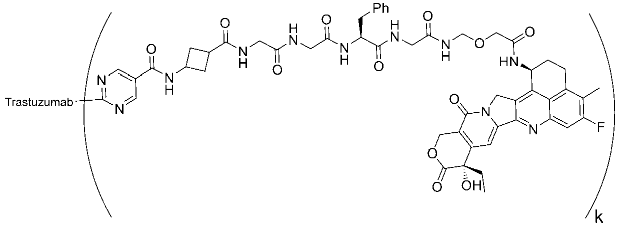 |
| ADC-8A | 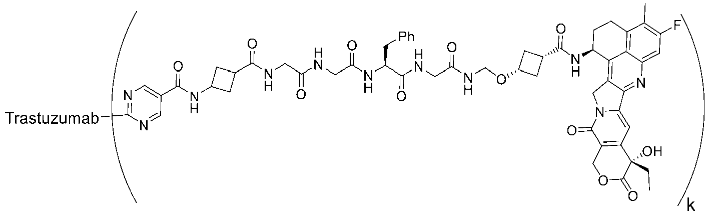 |
| ADC-9A | 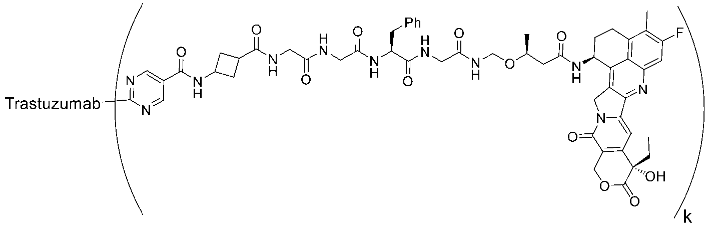 |
| ADC-10A | 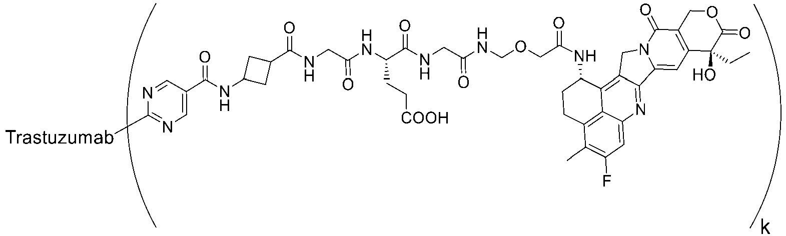 |
| ADC-11A | 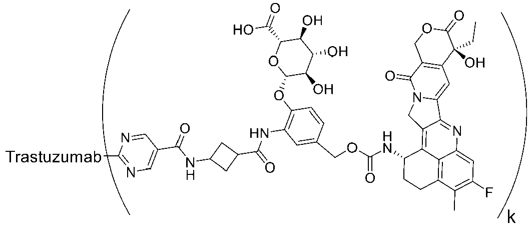 |
| ADC-12A | 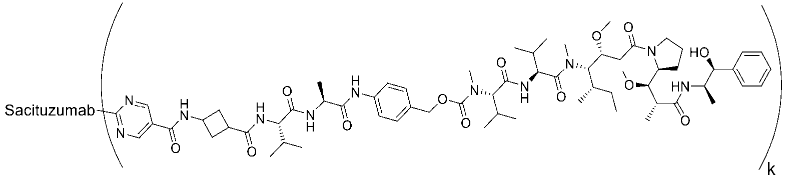 |
| AA-13A | 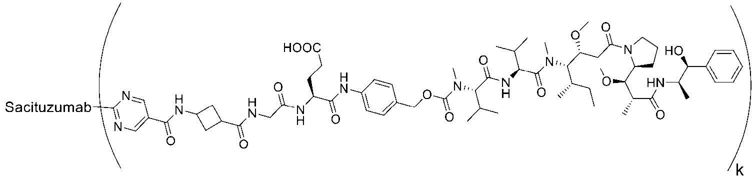 |
| AA-14A | 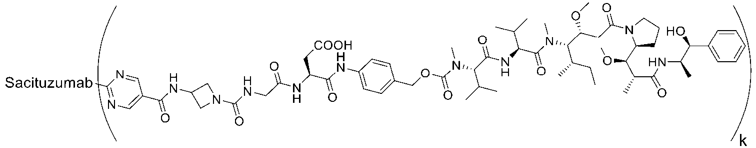 |
| ADC-15A | 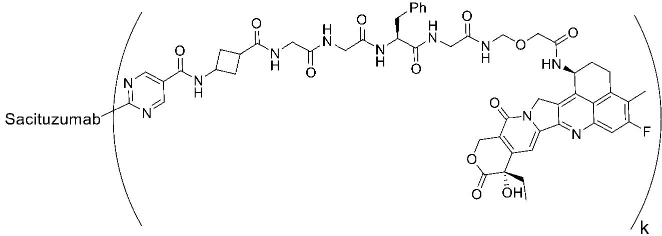 |
| ADC-16A | 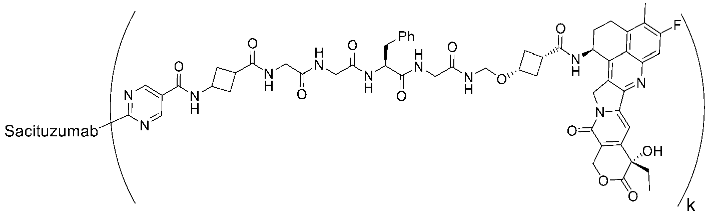 |
| ADC-17A | 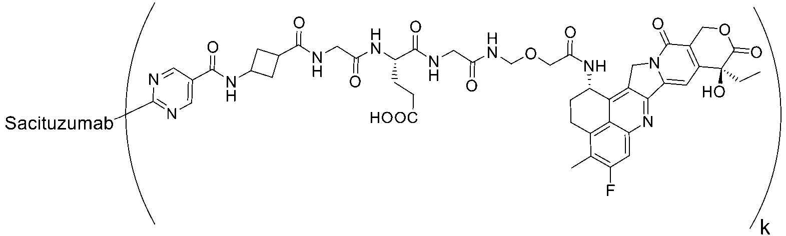 |
| ADC-18A | 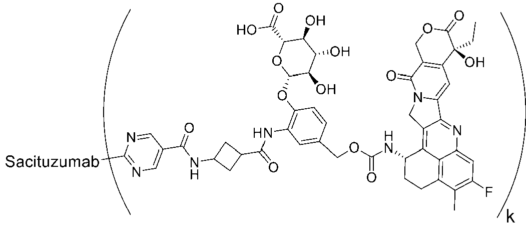 |
| ADC-19A | 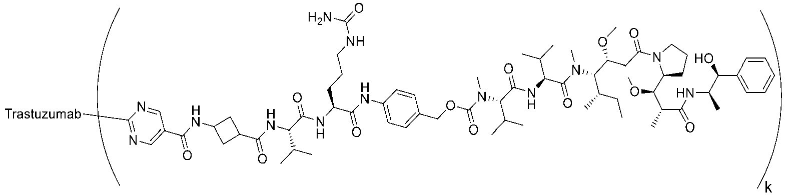 |
| ADC-20A | 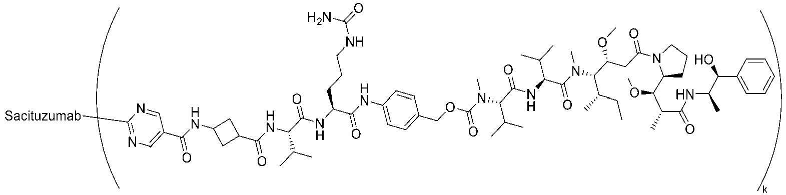 |
| ADC-21A | 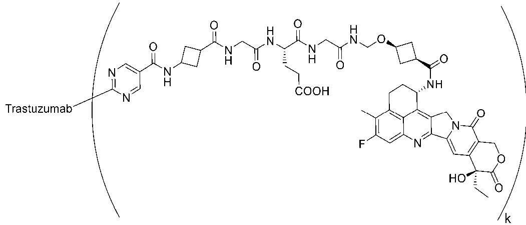 |
| ADC-22A | 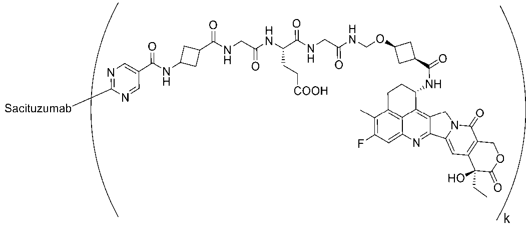 |
| ADC-23A | 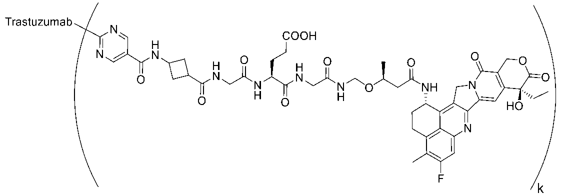 |
| ADC-24A | 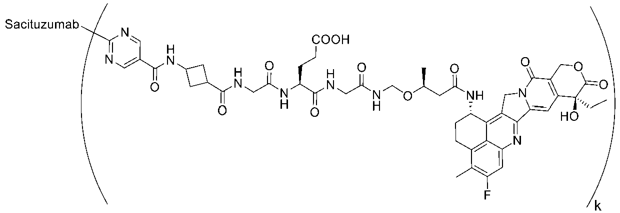 |
| ADC-25A | 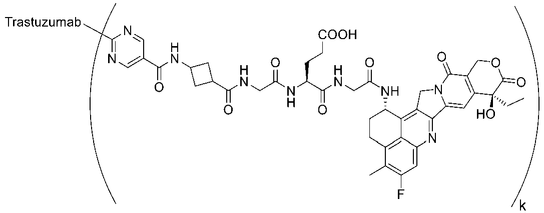 |
| ADC-26A | 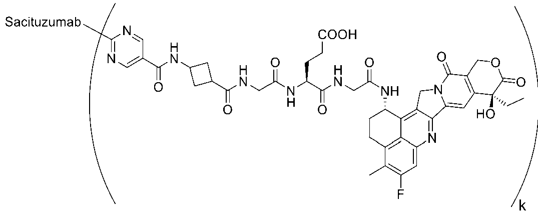 |
| ADC-27A | 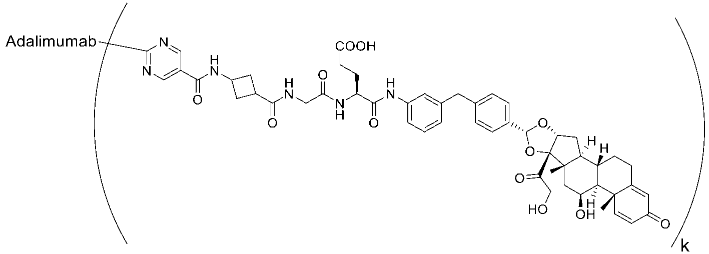 |

wherein k is as defined in any one of the embodiments of formula (1-1).

In another aspect, the present disclosure provides a compound of formula (II-1), or a mesomer, racemate, enantiomer, diastereomer, or any mixture thereof, or a pharmaceutically acceptable salt thereof, or a solvate thereof, or a pharmaceutically acceptable salt of the solvate thereof: 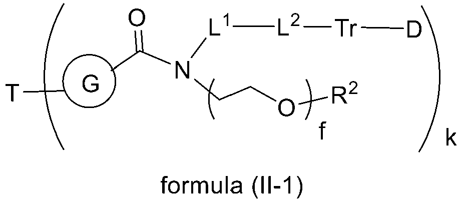 wherein
T and k are as defined in formula (1-1);
ring G, L¹, L², Tr, D, f, and R² are as defined in formula (II), respectively.

In a preferred embodiment of the present disclosure, in the compound of formula (II-1),
R¹ is -C₁₋₆ alkyl;
ring G is 5- to 6-membered heteroaryl, wherein the heteroatom of the 5- to 6-membered heteroaryl may be N and/or O, and the number of heteroatoms is 1, 2, or 3; for example, ring G is 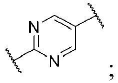
f is a natural number from 1 to 20;
X is -O-;
R² is hydrogen or -C₁₋₆ alkyl;
L¹ is -(CH₂)ₙ-; wherein n is a natural number from 0 to 50; any -CH₂- moiety in L¹ is independently and optionally replaced by the following structural moieties: -NH-, -O-, -C(O)-, or -S-;
L² is a chemical bond, Val-Cit, Val-Ala, Gly-Glu, Gly-Glu-Gly, or Gly-Gly-Phe-Gly;
Tr is a chemical bond, 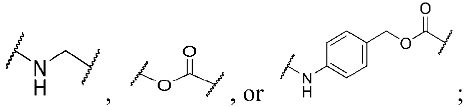
D is a tubulin inhibitor, a topoisomerase inhibitor, a DNA damaging agent, an RNA polymerase inhibitor, an immunomodulator, or an immunostimulant, for example, D is

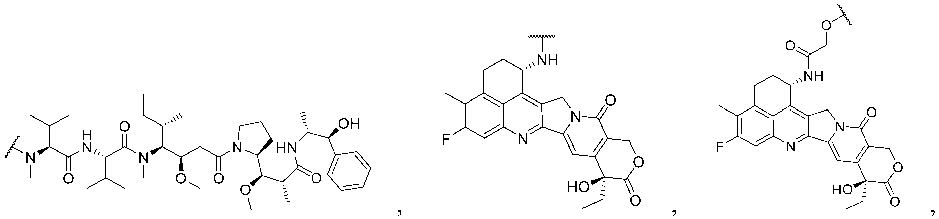

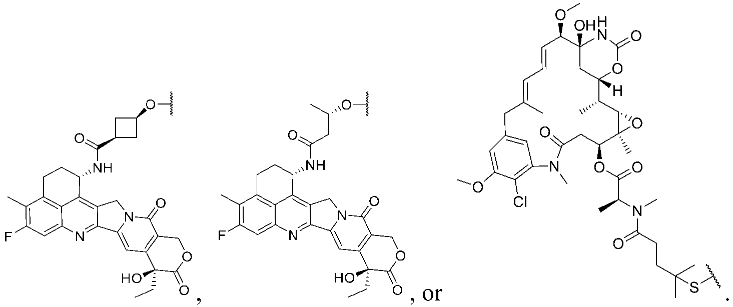

In a preferred embodiment of the present disclosure, in the compound of formula (II-1), or the mesomer, racemate, enantiomer, diastereomer, or any mixture thereof, or the pharmaceutically acceptable salt thereof, or the solvate thereof, or the pharmaceutically acceptable salt of the solvate thereof, the compound of formula (II-1) is a compound of formula (IIa-1): 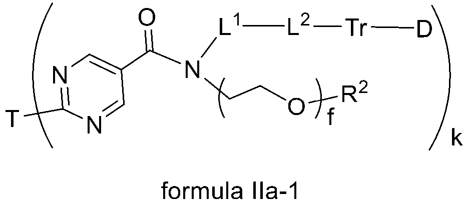 wherein
T and k are as defined in formula (I-1);
L¹, L², Tr, D, f, and R² are as defined in formula (II), respectively.

In a preferred embodiment of the present disclosure, the compound of formula (II-1) or formula (IIa-1), or the mesomer, racemate, enantiomer, diastereomer, or any mixture thereof, or the pharmaceutically acceptable salt thereof, or the solvate thereof, or the pharmaceutically acceptable salt of the solvate thereof, is selected from:

| | |
|---|---|
| BB1 | 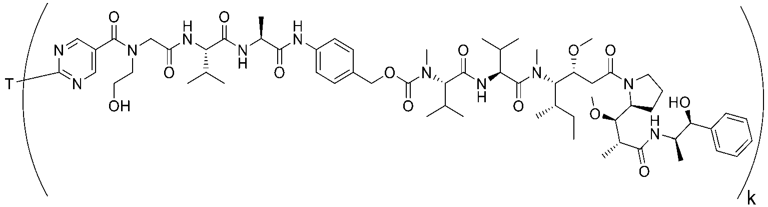 |
| BB2 | 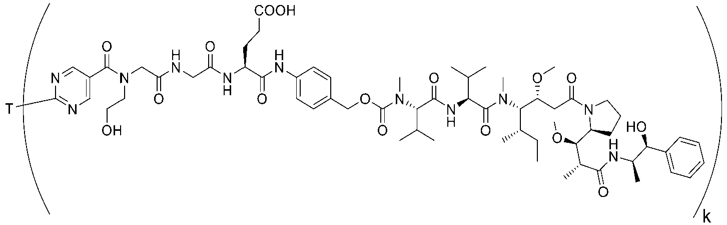 |
| BB3 | 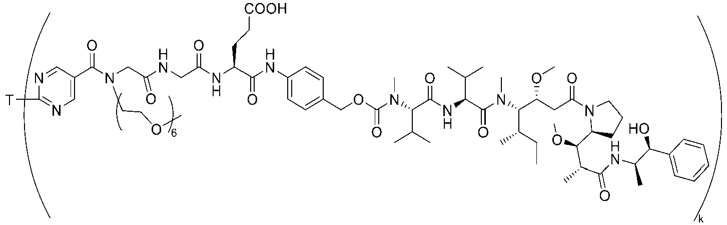 |
| BB4 | 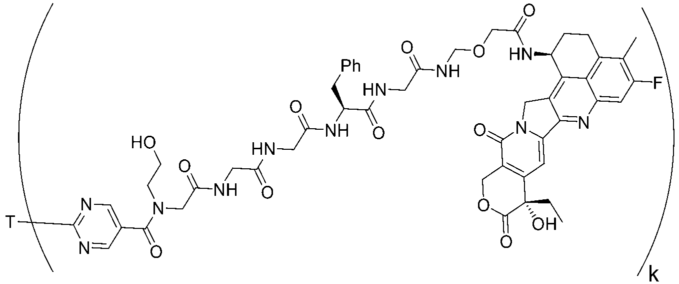 |
| BB5 | 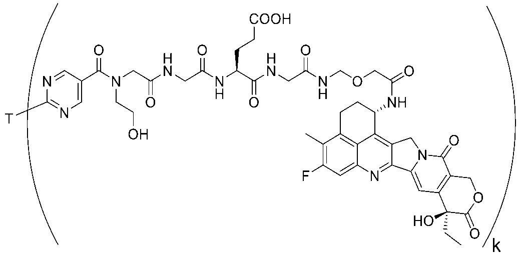 |
| BB6 | 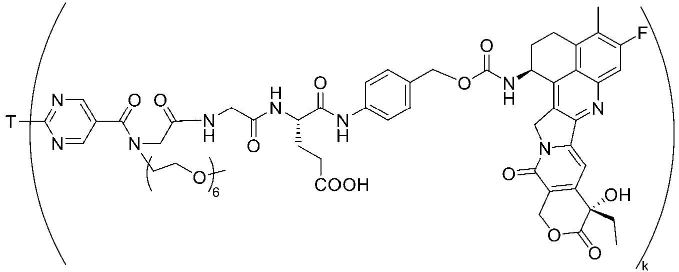 |
| BB7 | 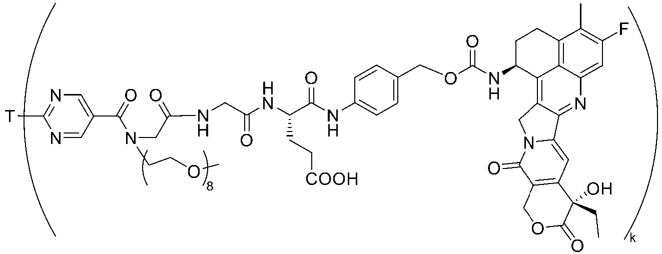 |
| BB8 | 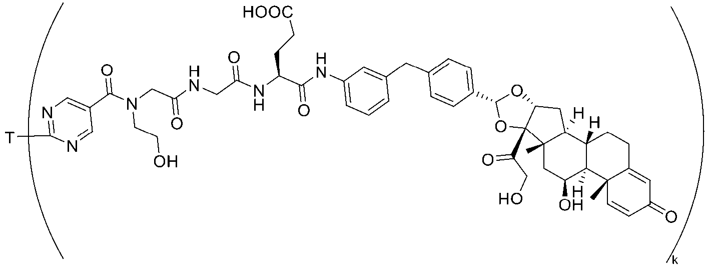 |
| BB9 | 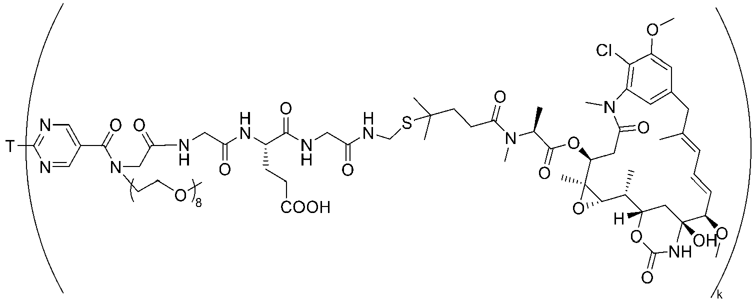 |
| BB10 | 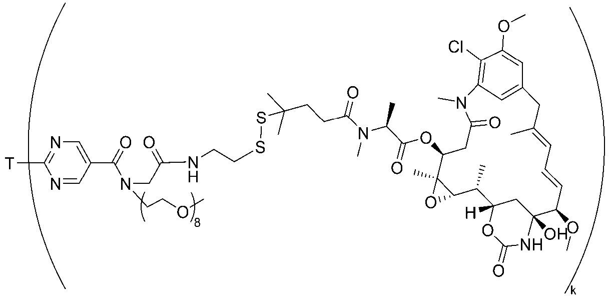 |
| BB11 | 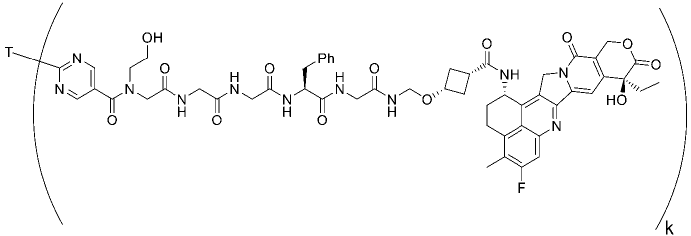 |
| BB12 | 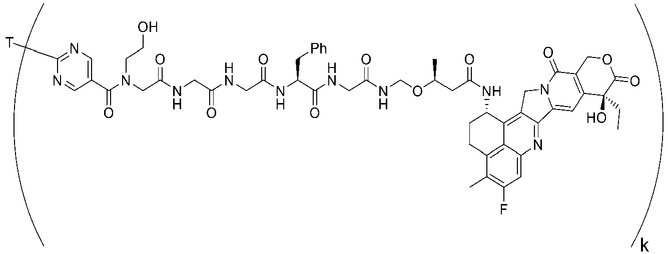 |
| BB13 | 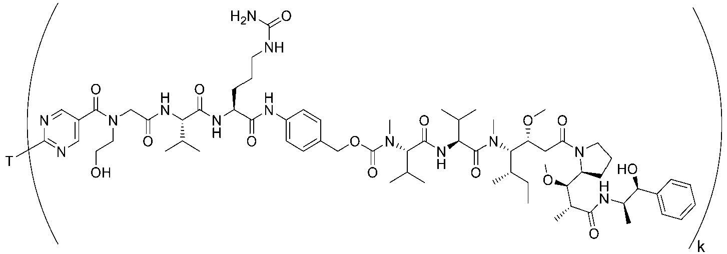 |
| BB14 | 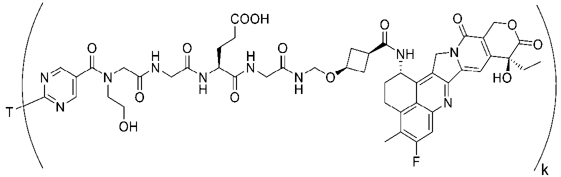 |
| BB15 | 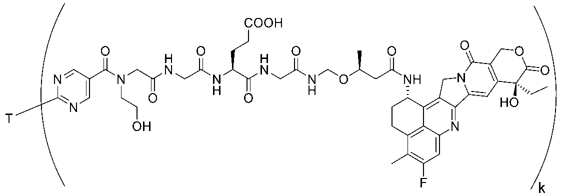 |
| BB16 | 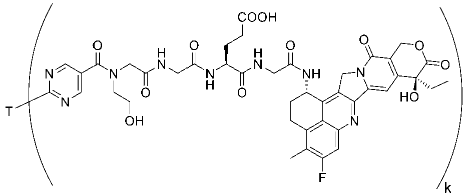 |
| BB17 | 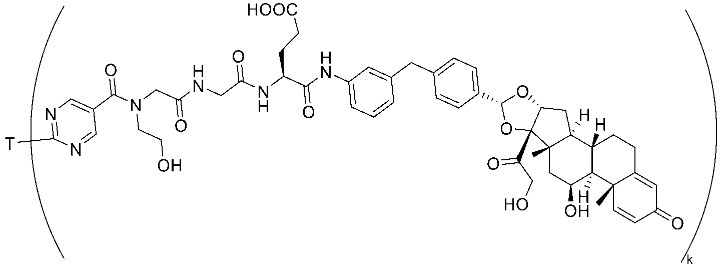 |

wherein T and k are as defined in any one of the embodiments of formula (I-1), respectively.

In some embodiments of the present disclosure, the compound of formula (II-1) or formula (IIa-1), or the mesomer, racemate, enantiomer, diastereomer, or any mixture thereof, or the pharmaceutically acceptable salt thereof, or the solvate thereof, or the pharmaceutically acceptable salt of the solvate thereof, is preferably selected from:

| | |
|---|---|
| ADC-1B | 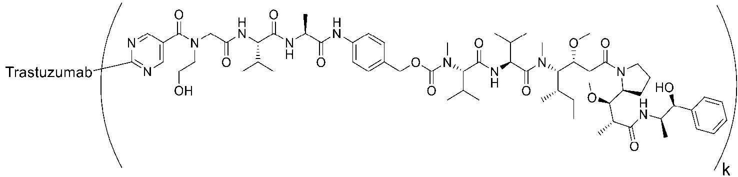 |
| ADC-2B | 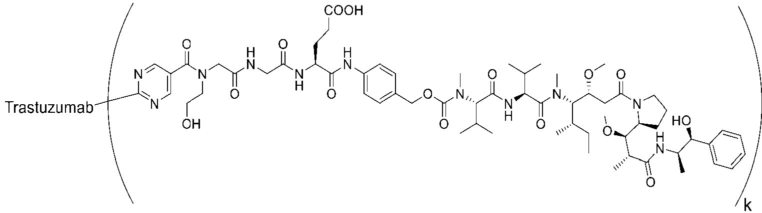 |
| ADC-3B | 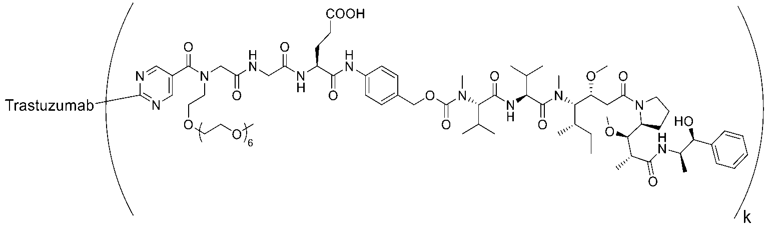 |
| ADC-4B | 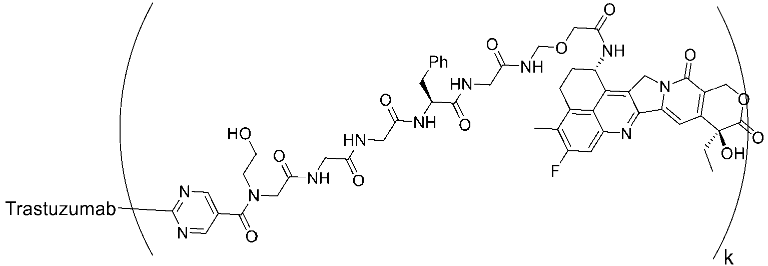 |
| ADC-5B | 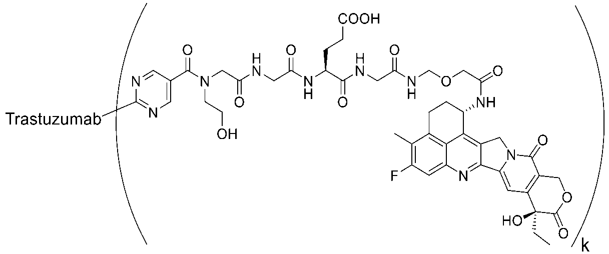 |
| ADC-6B | 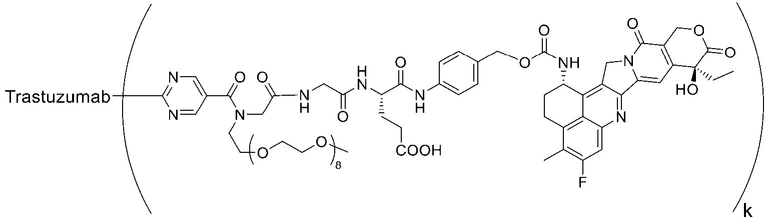 |
| ADC-7B | 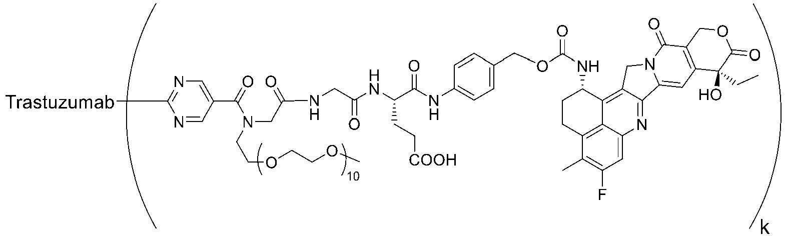 |
| ADC-8B | 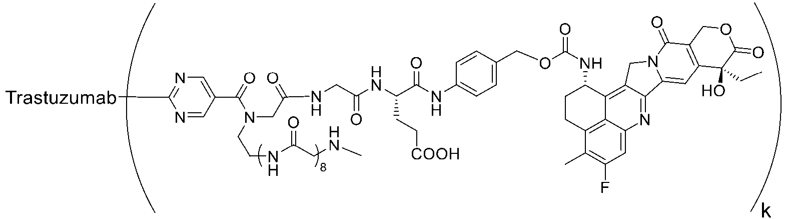 |
| ADC-9B | 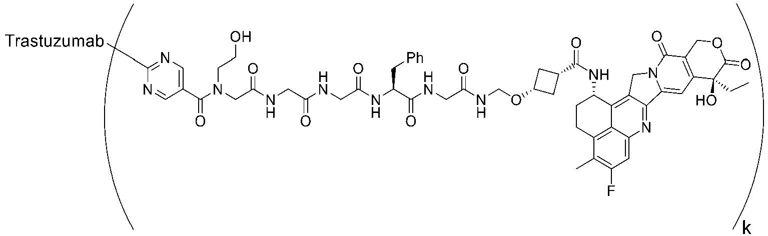 |
| ADC-10B | 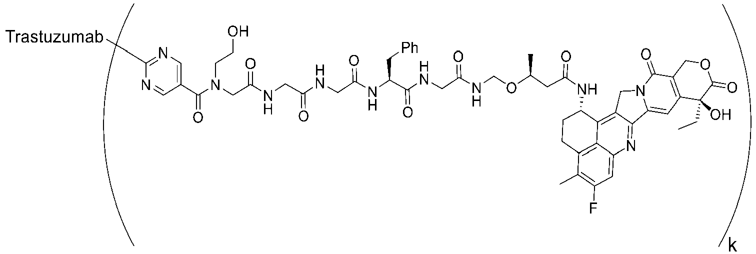 |
| ADC-11B | 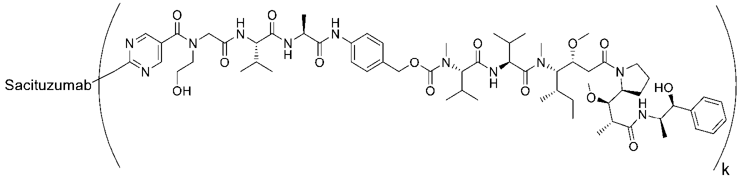 |
| ADC-12B | 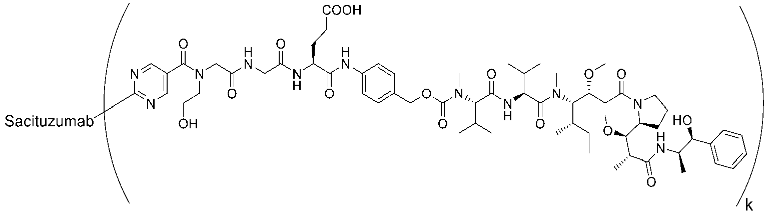 |
| ADC-13B | 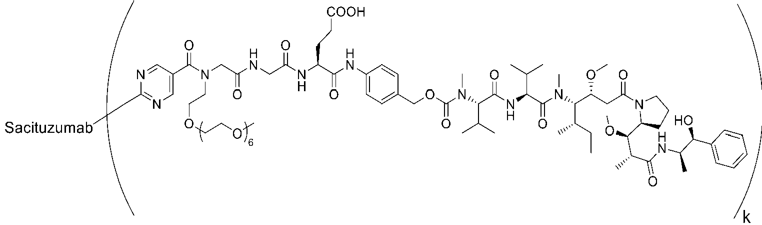 |
| ADC-14B | 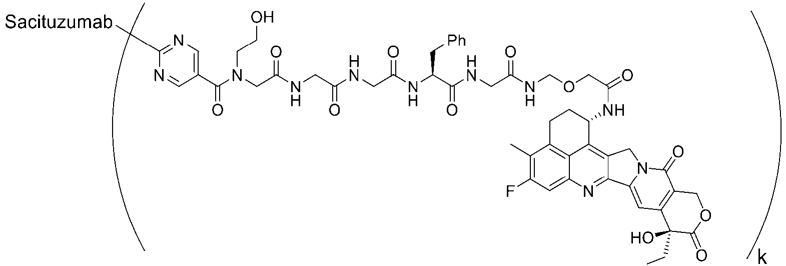 |
| ADC-15B | 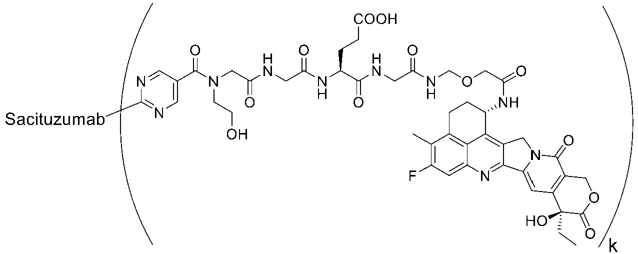 |
| ADC-16B | 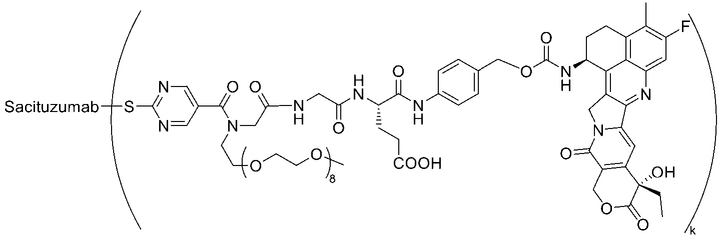 |
| ADC-17B | 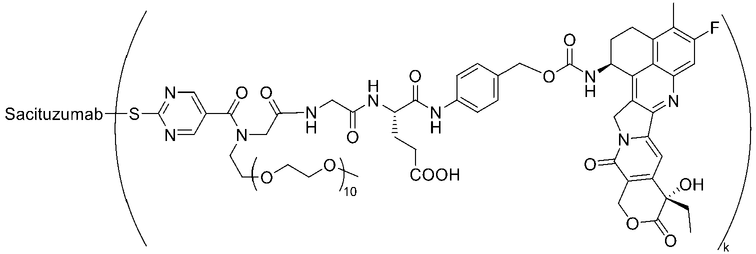 |
| ADC-17B | 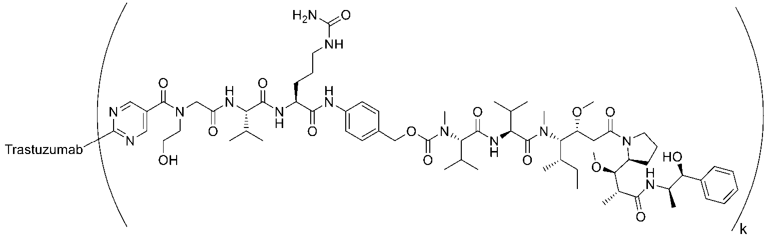 |
| ADC-18B | 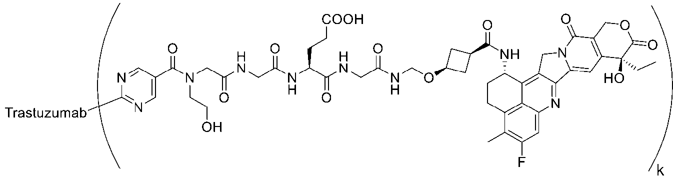 |
| ADC-19B | 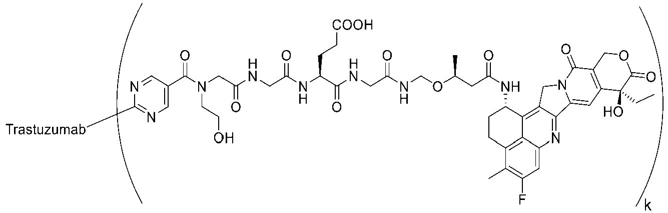 |
| ADC-20B | 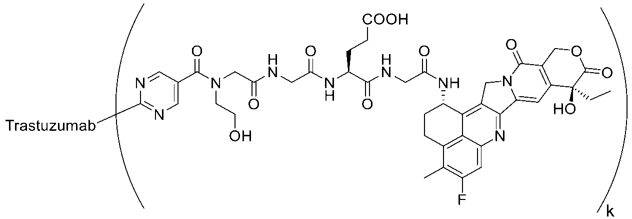 |
| ADC-21B | 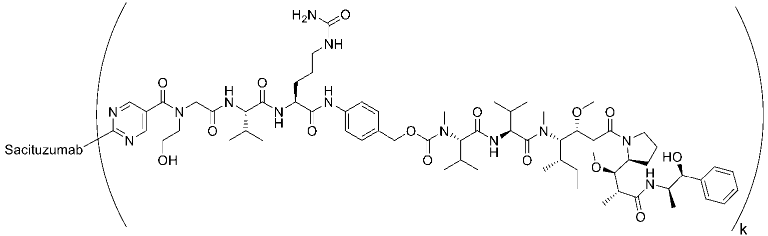 |
| ADC-22B | 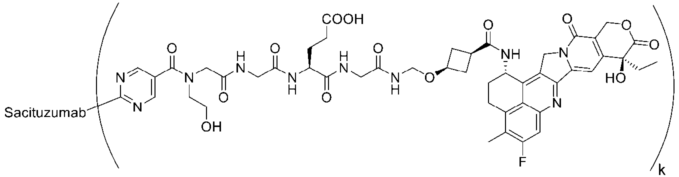 |
| ADC-23B | 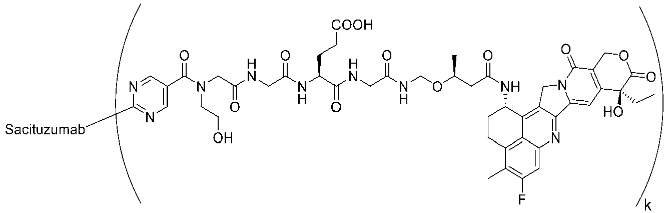 |
| ADC-24B | 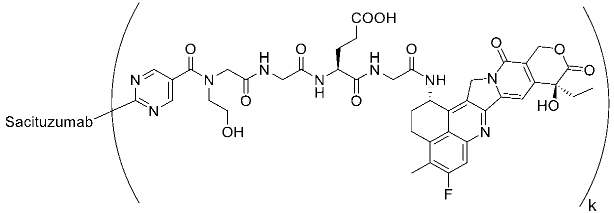 |
| ADC-25B | 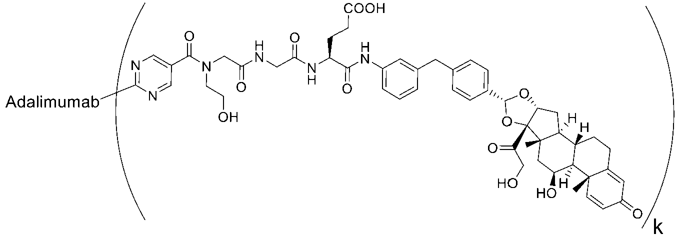 |

wherein k is as defined in any one of the embodiments of formula (I-1).

In another aspect, the present disclosure provides a compound of formula (III-1), or a mesomer, racemate, enantiomer, diastereomer, or any mixture thereof, or a pharmaceutically acceptable salt thereof, or a solvate thereof, or a pharmaceutically acceptable salt of the solvate thereof: 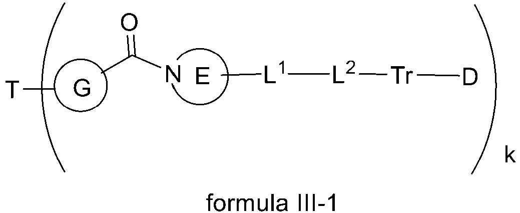 wherein
T and k are as defined in formula (I-1);
ring G, L¹, L², Tr, ring E, and D are as defined in formula (III), respectively.

In a preferred embodiment of the present disclosure, in the compound of formula (III-1),
R¹ is -C₁₋₆ alkyl;
ring G is 5- to 6-membered heteroaryl, wherein the heteroatom of the 5- to 6-membered heteroaryl may be N and/or O, and the number of heteroatoms is 1, 2, or 3; for example, ring G is 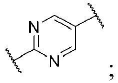
ring E is 4- to 6-membered heterocycloalkylene, wherein the heteroatom of the 4- to 6-membered heterocycloalkylene is N, and the number of heteroatoms is 1 or 2;
the 4- to 6-membered heterocycloalkylene is optionally substituted by one or more - C(O)NH-(CH₂CH₂O)_{y1}CH₃ substituents;
y1 is a natural number from 0 to 30;
L¹ is -(CH₂)ₙ-; wherein n is a natural number from 0 to 50; any -CH₂- moiety in L¹ is independently and optionally replaced by the following structural moieties: -NH-, -C(O)-, -O-, or -S-;
L² is a chemical bond, Val-Ala, Ala-Ala, Gly-Glu, Gly-Asp, Gly-Glu-Gly, or Gly-Gly-Phe-Gly;
Tr is a chemical bond, 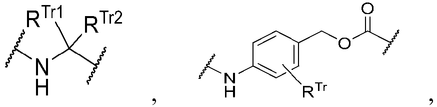 or 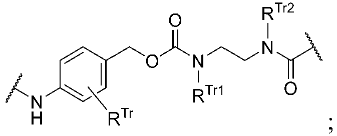 R^{Tr}, R^{Tr1}, and R^{Tr2} are each independently hydrogen or -C₁₋₆ alkyl;
D is a tubulin inhibitor, a topoisomerase inhibitor, a DNA damaging agent, an RNA polymerase inhibitor, an immunomodulator, or an immunostimulant, for example, D is 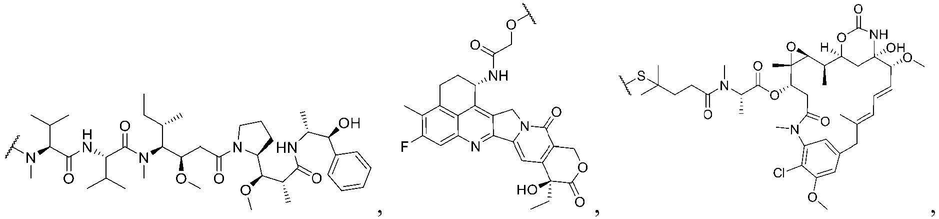 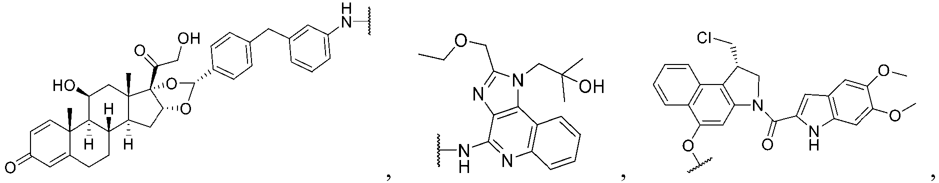 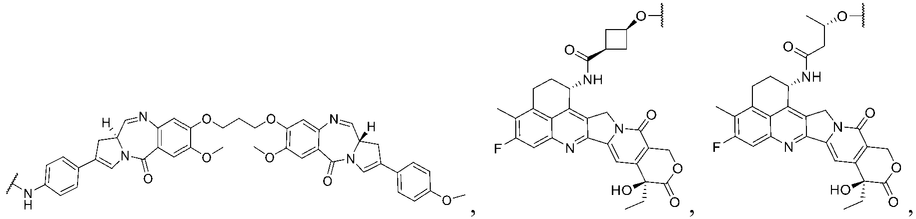 or

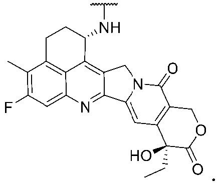

In a preferred embodiment of the present disclosure, in the compound of formula (III-1),
R¹ is -C₁₋₆ alkyl;
ring G is 5- to 6-membered heteroaryl, wherein the heteroatom of the 5- to 6-membered heteroaryl is N, and the number of heteroatoms is 1, 2, or 3;
ring E is 4- to 6-membered heterocycloalkylene, wherein the heteroatom of the 4- to 6-membered heterocycloalkylene is N, and the number of heteroatoms is 1 or 2;
L¹ is 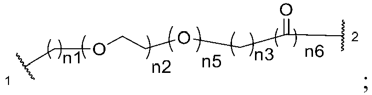 wherein position "1" is connected to ring E, and position "2" is connected to L²;
each of n1 and n2 is 0; n3 is 0, 1, 2, 3, or 4; n5 is 0 or 1; n6 is 1;
L² is Val-Ala, Ala-Ala, Gly-Glu, Gly-Gly-Phe-Gly, or Val-Cit, wherein the N-terminus is connected to ring E, and the C-terminus is connected to L²;
Tr is 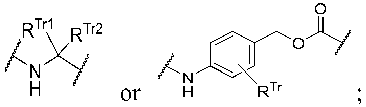 R^{Tr}, R^{Tr1}, and R^{Tr2} are each independently hydrogen or -C₁₋₆ alkyl;
D is 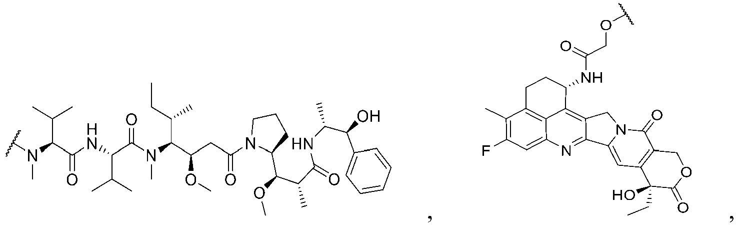 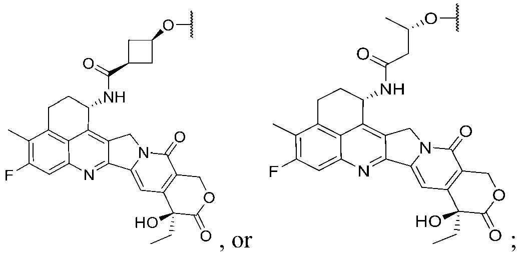
the antibody is Trastuzumab, Sacituzumab, DB1001 antibody, or DB1002 antibody;
k is a natural number or a decimal from 2 to 8.

In a preferred embodiment of the present disclosure, in the compound of formula (III-1),
R¹ is -C₁₋₆ alkyl;
ring G is 5- to 6-membered heteroaryl, wherein the heteroatom of the 5- to 6-membered heteroaryl is N, and the number of heteroatoms is 1, 2, or 3;
ring E is 4- to 6-membered heterocycloalkylene, wherein the heteroatom of the 4- to 6-membered heterocycloalkylene is N, and the number of heteroatoms is 1 or 2;
L¹ is 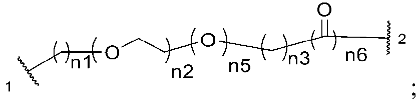 wherein position "1" is connected to ring E, and position "2" is connected to L²;
each of n1 and n2 is 0; n3 is 1, 2, 3, or 4; n5 is 1; n6 is 1;
L² is Gly-Gly-Phe-Gly or Val-Cit; wherein the N-terminus is connected to ring E, and the C-terminus is connected to L²;
Tr is 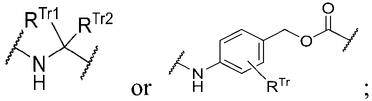 R^{Tr}, R^{Tr1}, and R^{Tr2} are each independently hydrogen or -C₁₋₆ alkyl;
D is 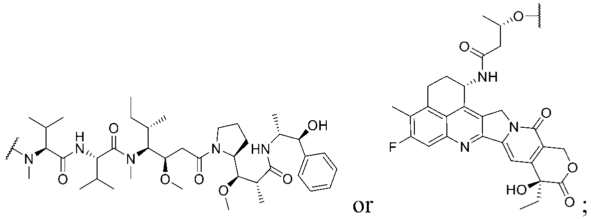
the antibody is DB1001 antibody or DB 1002 antibody;
k is a natural number or a decimal from 2 to 8.

In the compound of formula (III-1), or the mesomer, racemate, enantiomer, diastereomer, or any mixture thereof, or the pharmaceutically acceptable salt thereof, or the solvate thereof, or the pharmaceutically acceptable salt of the solvate thereof, the compound of formula (III-1) is a compound of formula (IIIa-1): 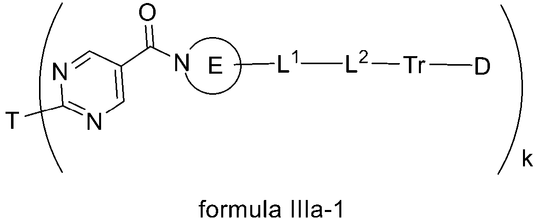 wherein
T and k are as defined in any one of the embodiments of formula (1-1), respectively;
L¹, L², Tr, D, and ring E are as defined in any one of the embodiments of formula (III), respectively.

In a preferred embodiment of the present disclosure, the compound of formula (III-1) is a compound of the following formula: 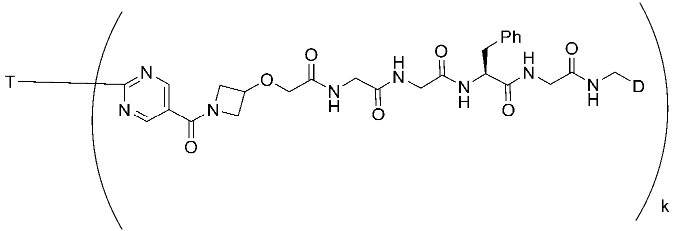 or 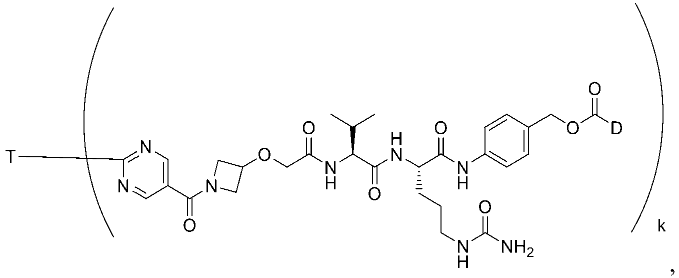 T and k are as defined in any one of the embodiments of formula (1-1), respectively; D is as defined in any one of the embodiments of formula (III).

In a preferred embodiment of the present disclosure, the compound of formula (III-1) or formula (IIIa-1), or the mesomer, racemate, enantiomer, diastereomer, or any mixture thereof, or the pharmaceutically acceptable salt thereof, or the solvate thereof, or the pharmaceutically acceptable salt of the solvate thereof, is selected from:

| | |
|---|---|
| CC1 | 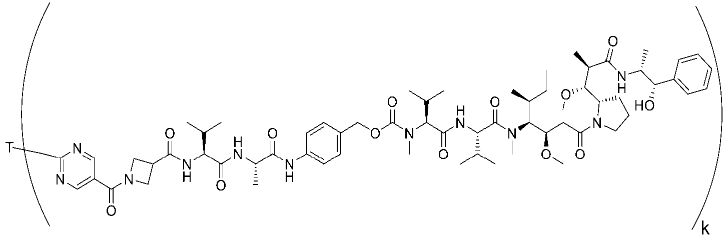 |
| CC2 | 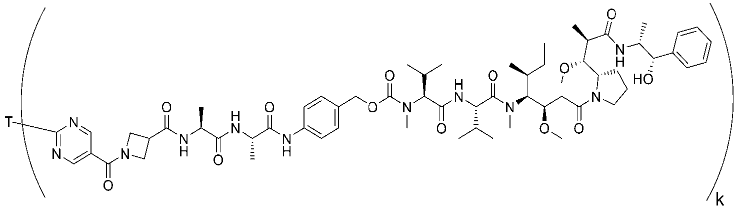 |
| CC3 | 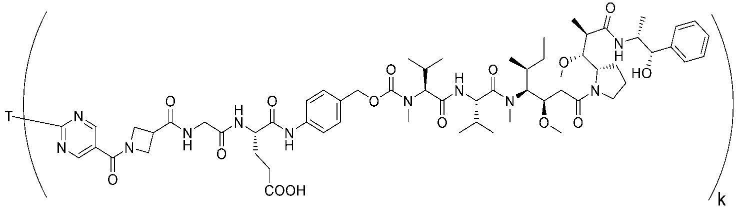 |
| CC4 | 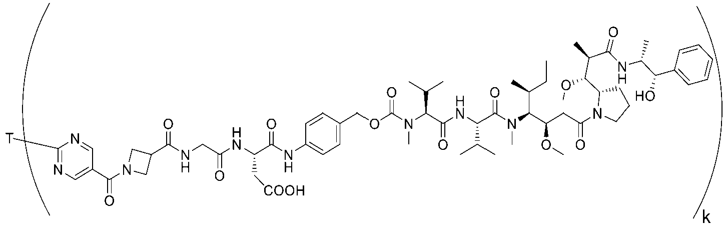 |
| CC5 | 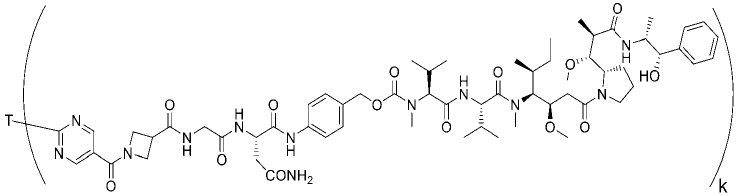 |
| CC6 | 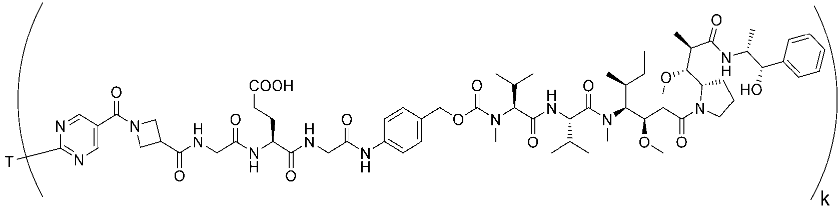 |
| CC7 | 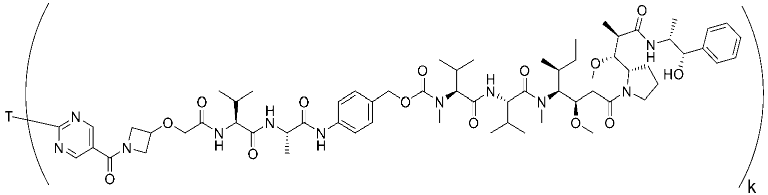 |
| CC8 | 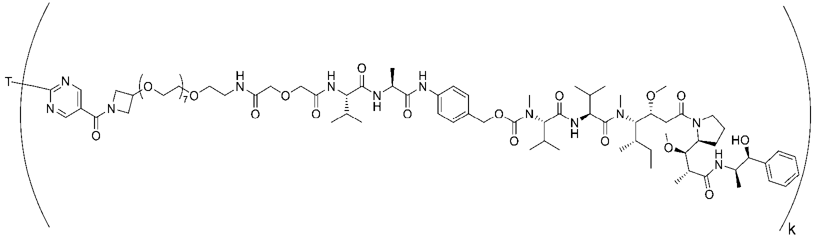 |
| CC9 | 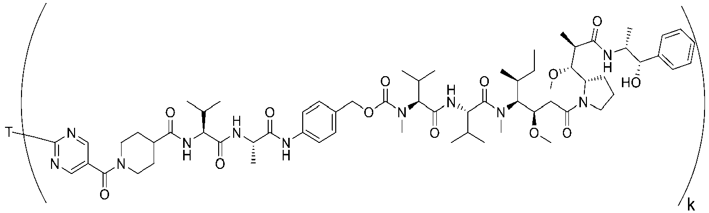 |
| CC10 | 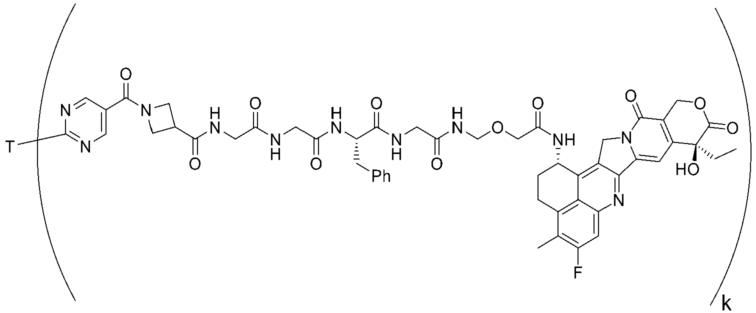 |
| CC11 | 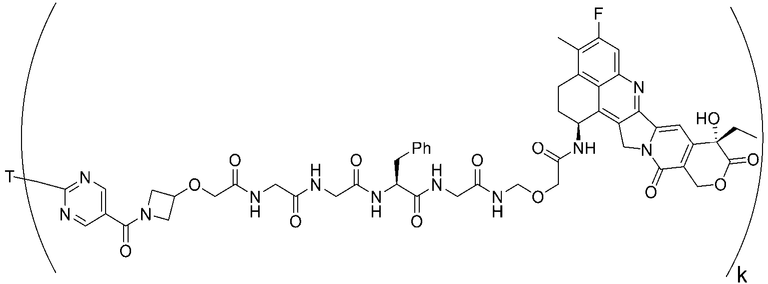 |
| CC12 | 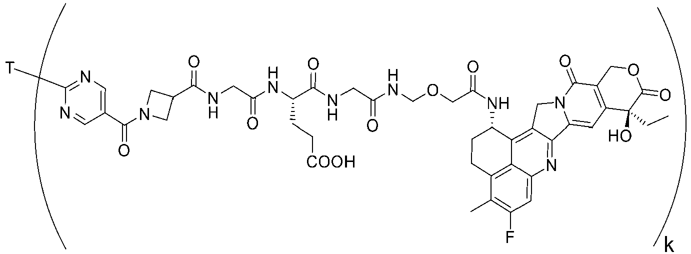 |
| CC13 | 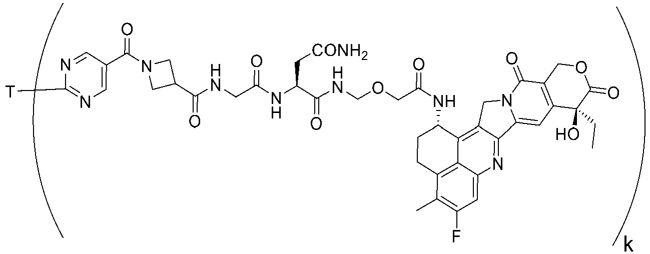 |
| CC14 | 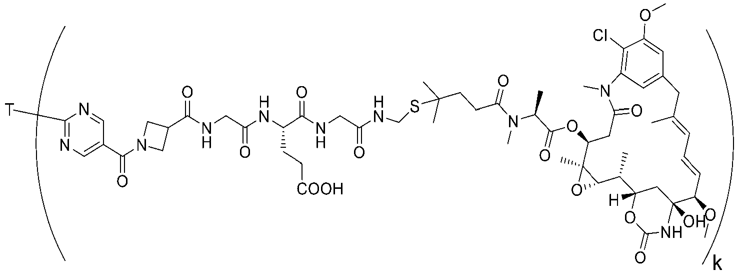 |
| CC15 | 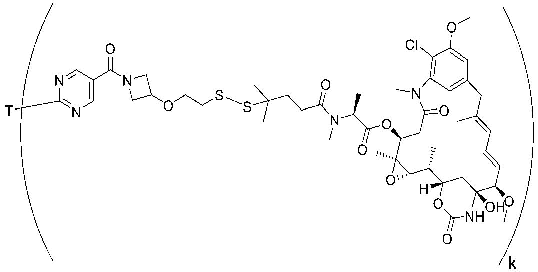 |
| CC16 | 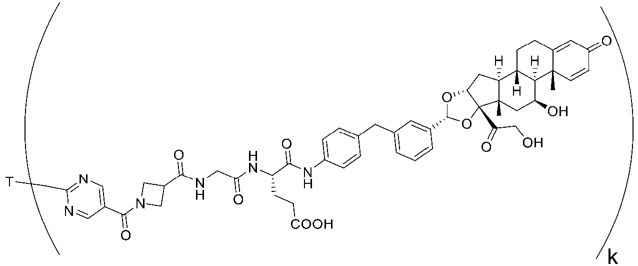 |
| CC17 | 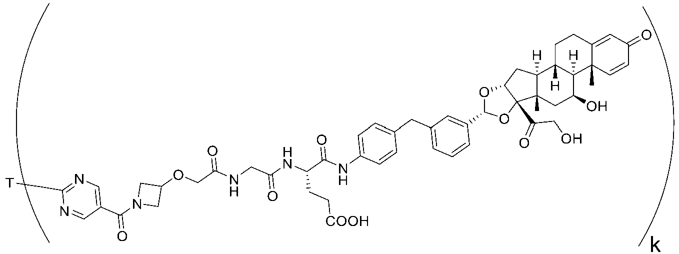 |
| CC19 | 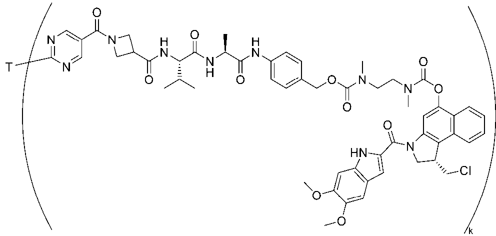 |
| CC20 | 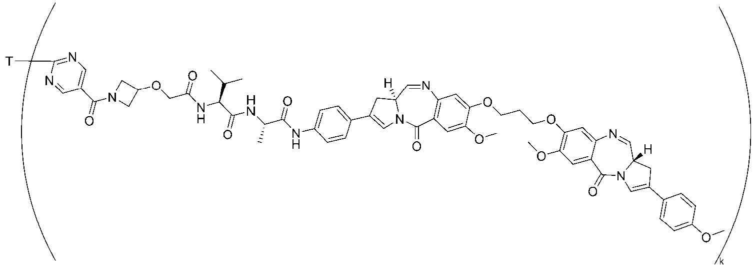 |
| CC22 | 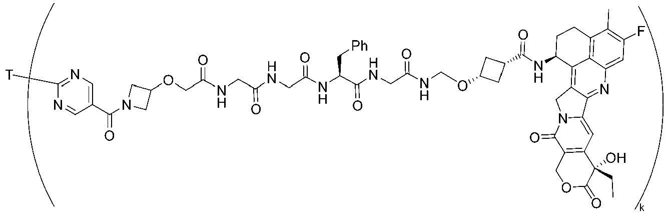 |
| CC23 | 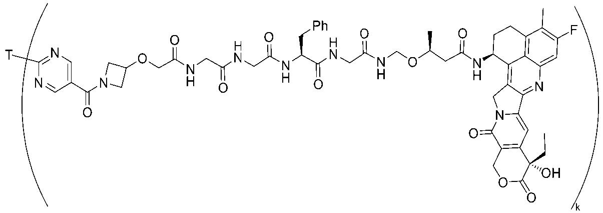 |
| CC24 | 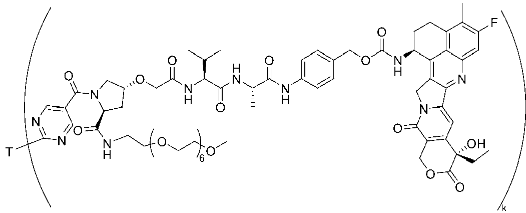 |
| CC25 | 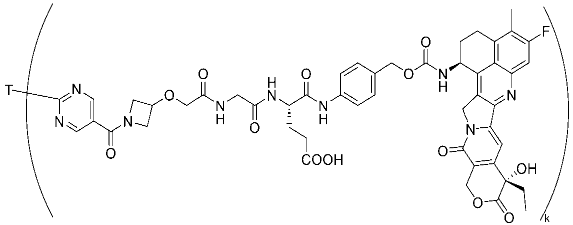 |
| CC26 | 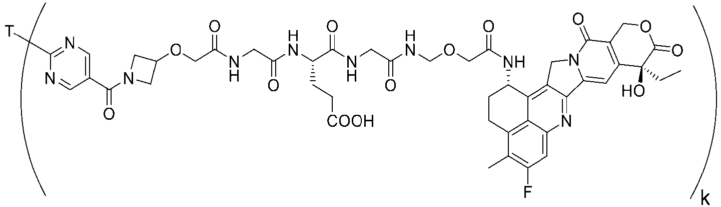 |
| CC27 | 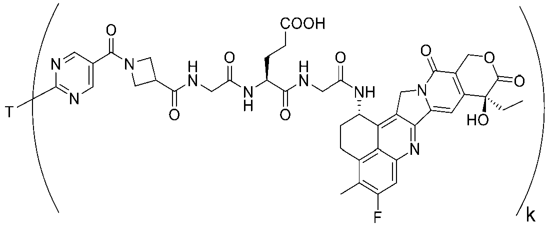 |
| CC28 | 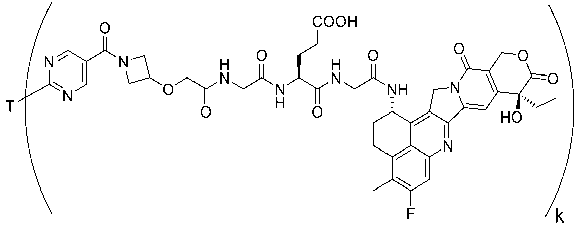 |
| CC29 | 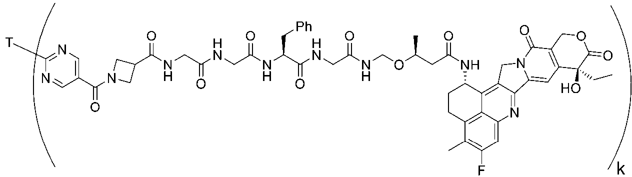 |
| CC30 | 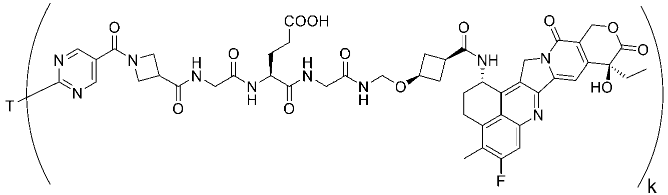 |
| CC31 | 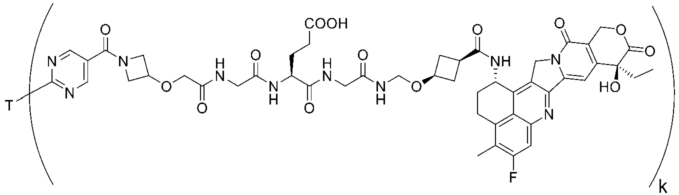 |
| CC32 | 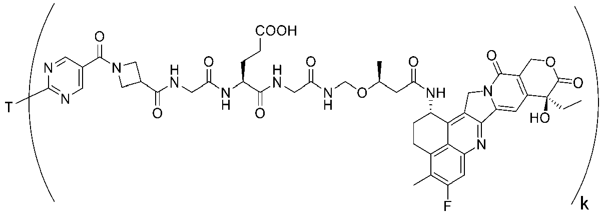 |
| CC33 | 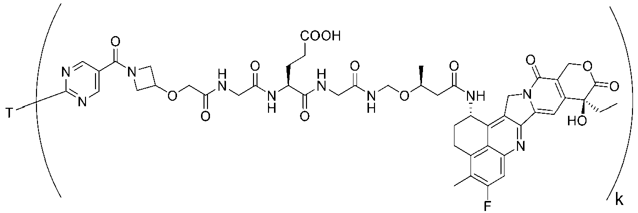 |
| CC34 | 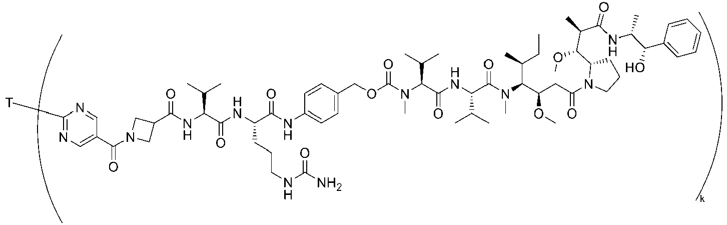 |

wherein T and k are as defined in any one of the embodiments of formula (1-1), respectively.

In some embodiments of the present disclosure, the compound of formula (III-1) or formula (IIIa-1), or the mesomer, racemate, enantiomer, diastereomer, or any mixture thereof, or the pharmaceutically acceptable salt thereof, or the solvate thereof, or the pharmaceutically acceptable salt of the solvate thereof, is preferably selected from:

| | |
|---|---|
| ADC-1C | 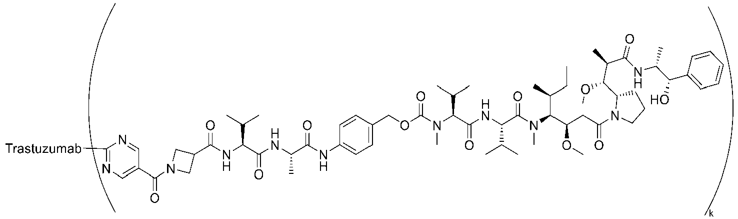 |
| ADC-2C | 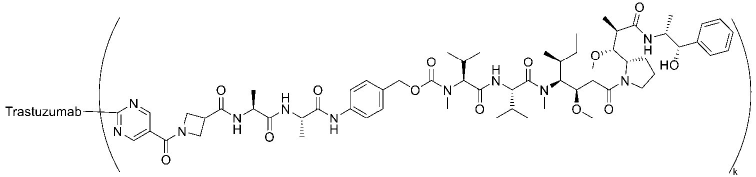 |
| ADC-3C | 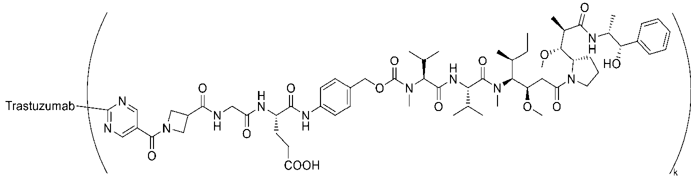 |
| ADC-4C | 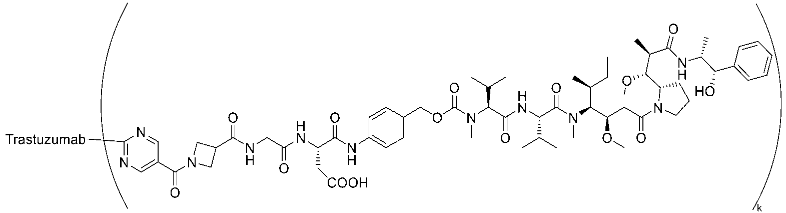 |
| ADC-5C | 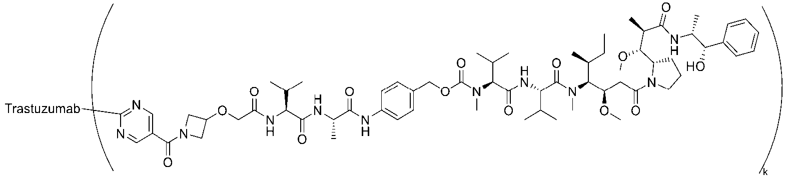 |
| ADC-6C | 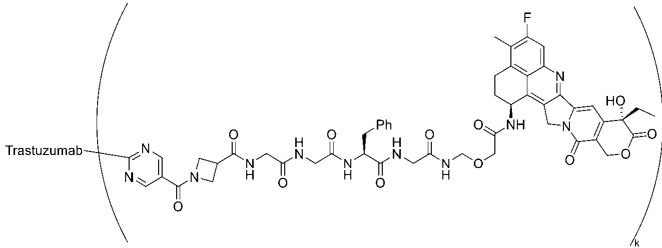 |
| ADC-7C | 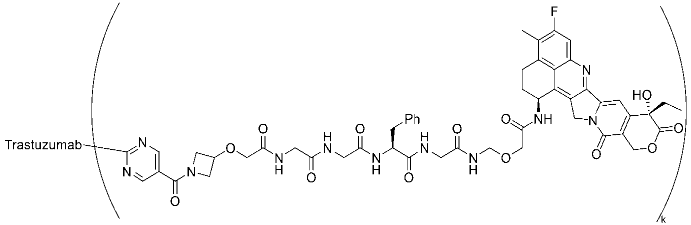 |
| ADC-8C | 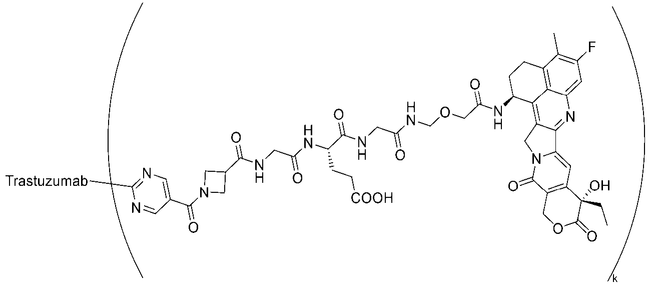 |
| ADC-9C | 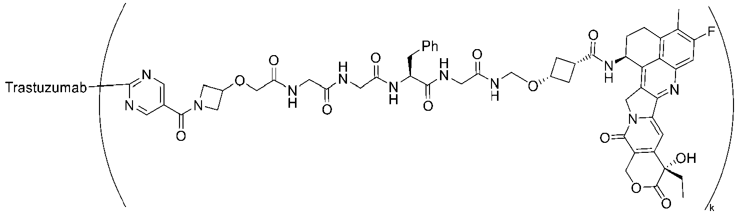 |
| ADC-10C | 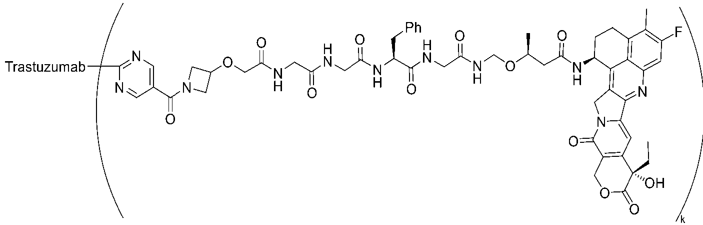 |
| ADC-11C | 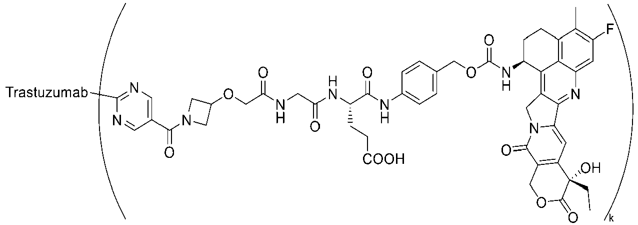 |
| ADC-12C | 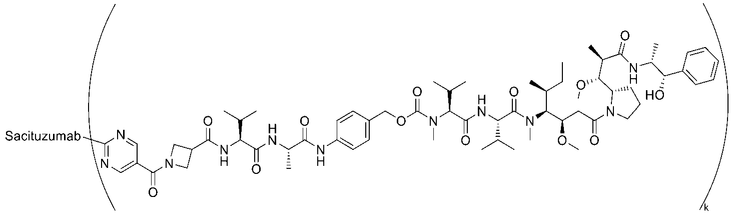 |
| ADC-13C | 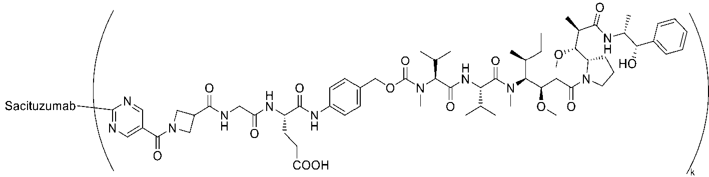 |
| ADC-14C | 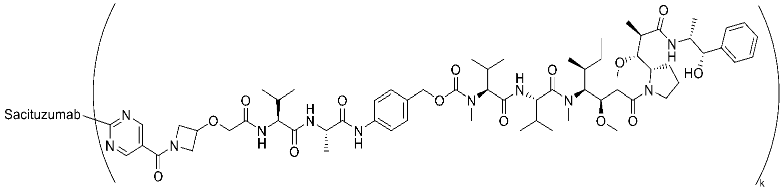 |
| ADC-15C | 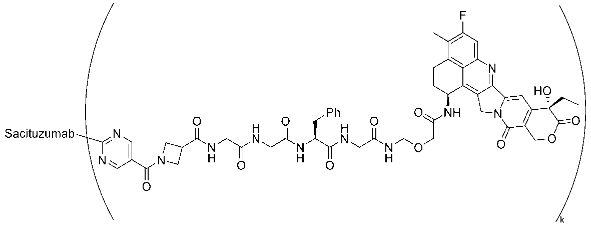 |
| ADC-16C | 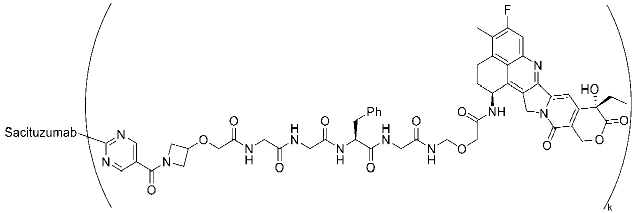 |
| ADC-17C | 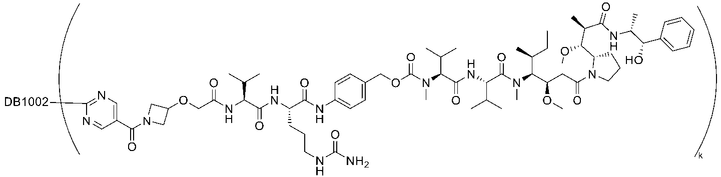 |
| ADC-18C | 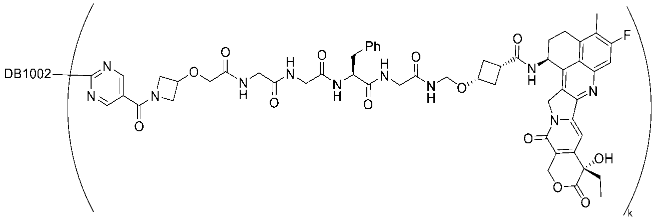 |
| ADC-19C | 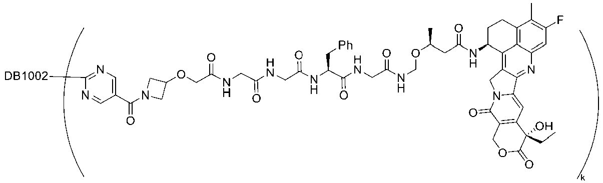 |
| ADC-20C | 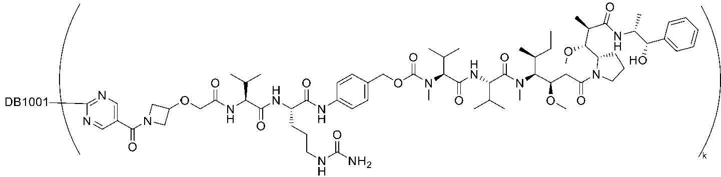 |
| ADC-21C | 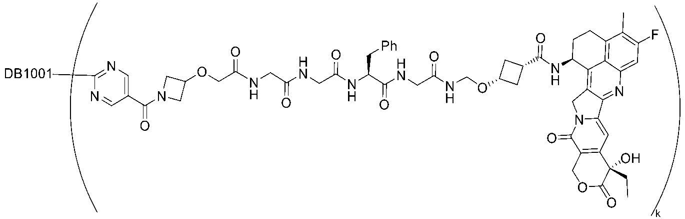 |
| ADC-22C | 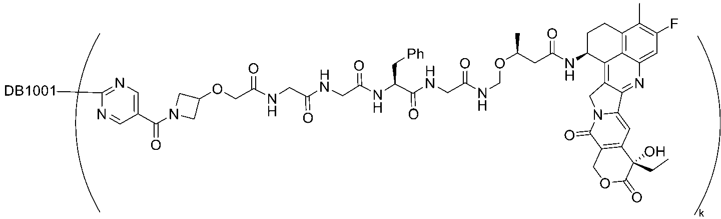 |
| ADC-23C | 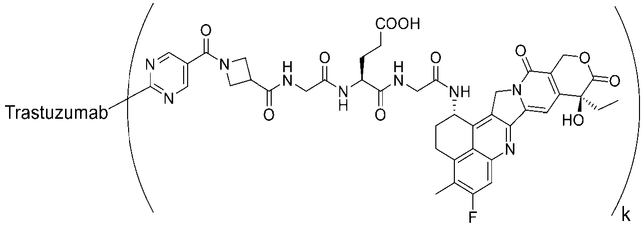 |
| ADC-24C | 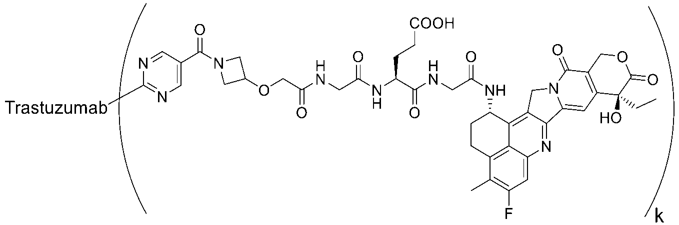 |
| ADC-25C | 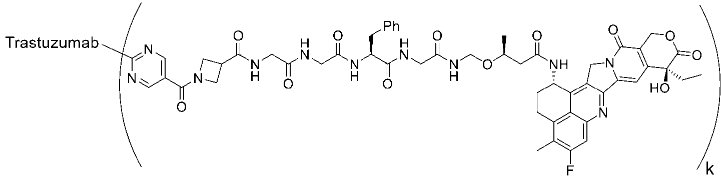 |
| ADC-26C | 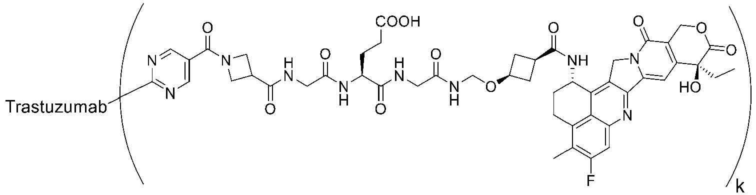 |
| ADC-27C | 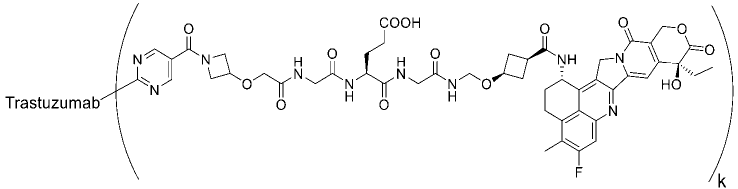 |
| ADC-28C | 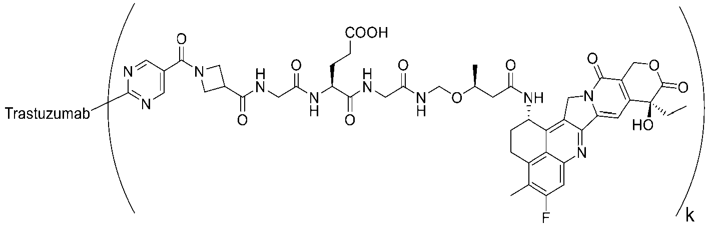 |
| ADC-29C | 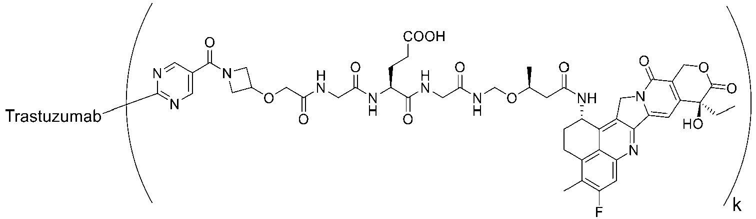 |
| ADC-30C | 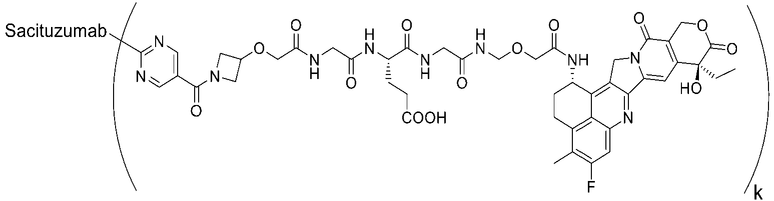 |
| ADC-31C | 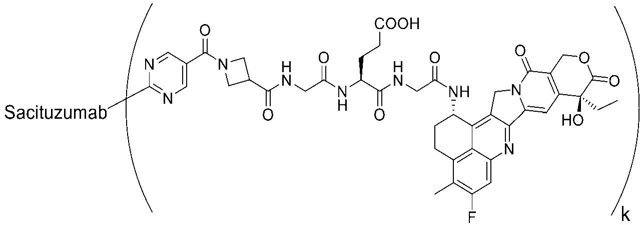 |
| ADC-32C | 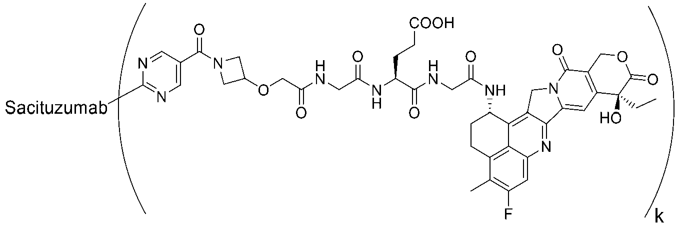 |
| ADC-33C | 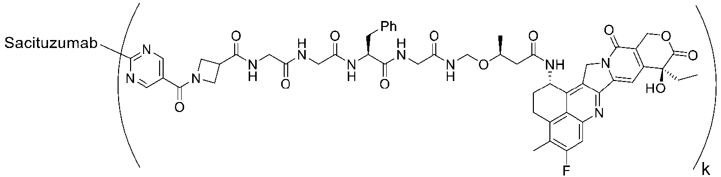 |
| ADC-34C | 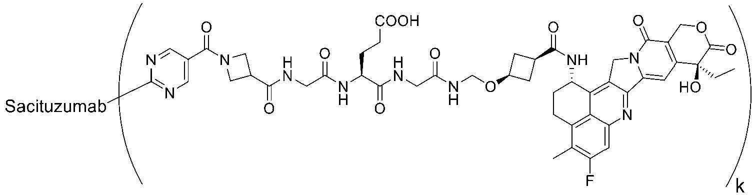 |
| ADC-35C | 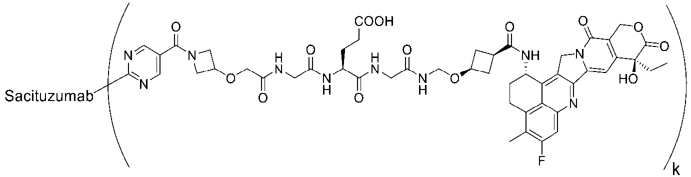 |
| ADC-36C | 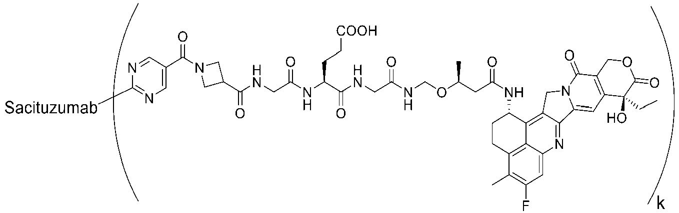 |
| ADC-37C | 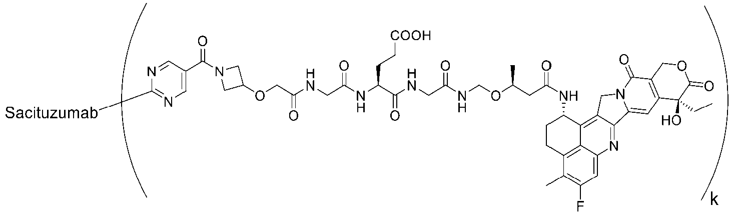 |

wherein k is as defined in any one of the embodiments of formula (I-1).

In a preferred embodiment of the present disclosure, the compound of formula (III-1) or formula (IIIa-1), or the mesomer, racemate, enantiomer, diastereomer, or any mixture thereof, or the pharmaceutically acceptable salt thereof, or the solvate thereof, or the pharmaceutically acceptable salt of the solvate thereof, is selected from:

| No. | Structure |
|---|---|
| ADC-1C-1 | 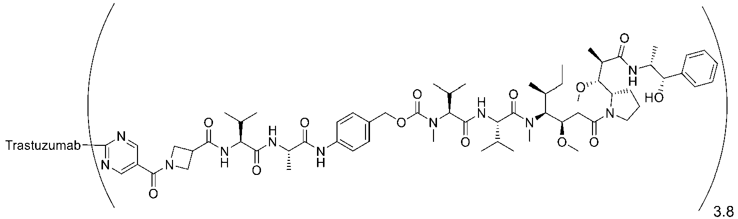 |
| ADC-2C-1 | 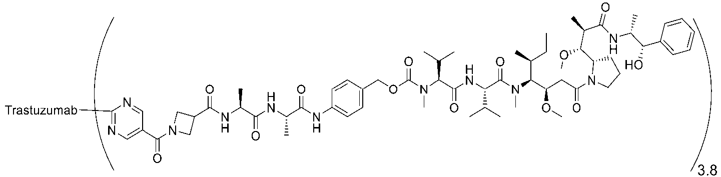 |
| ADC-3C-1 | 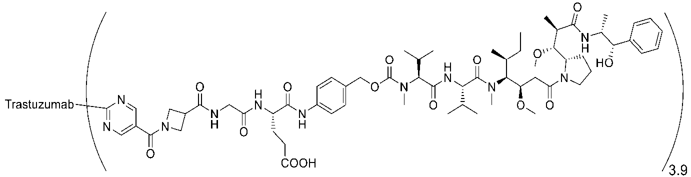 |
| ADC-5C-1 | 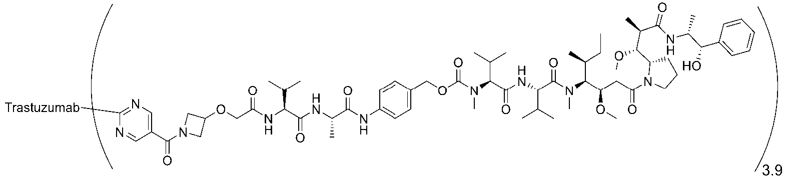 |
| ADC-6C-1 | 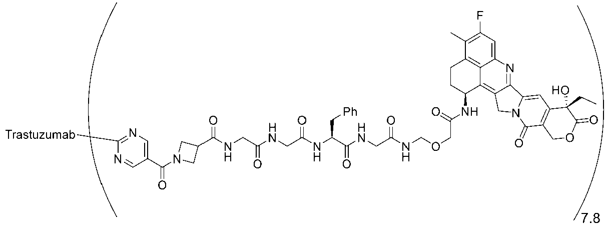 |
| ADC-7C-1 | 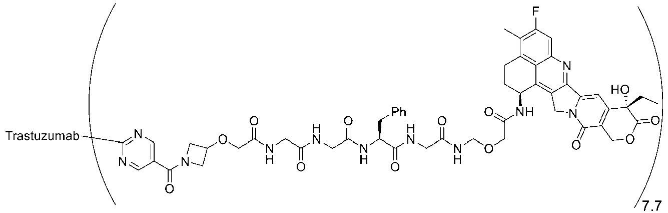 |
| ADC-12C-1 | 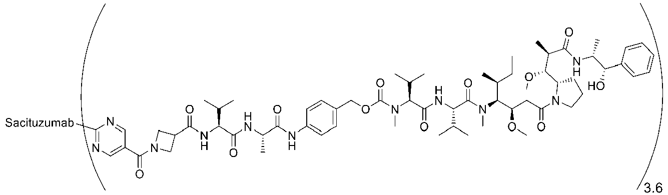 |
| ADC-14C-1 | 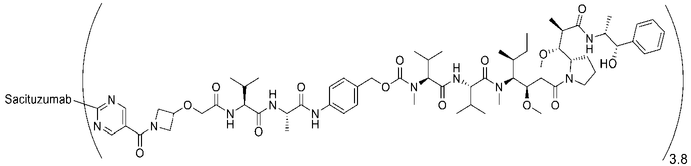 |
| ADC-15C-1 | 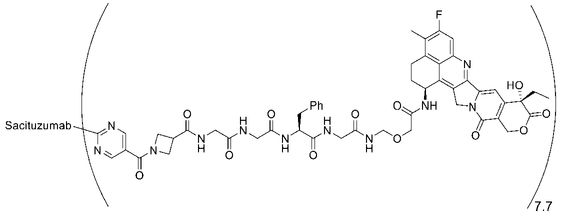 |
| ADC-16C-1 | 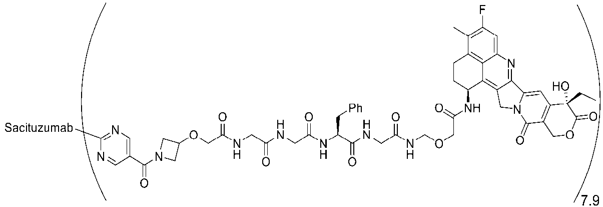 |
| ADC-17C-1 | 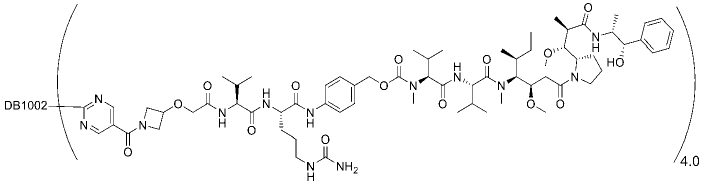 |
| ADC-18C-1 | 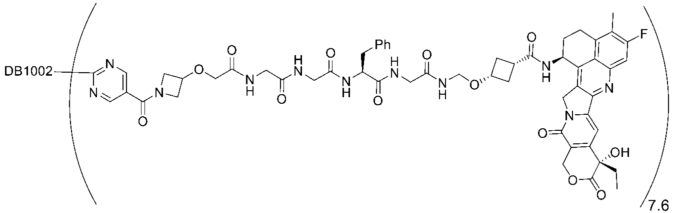 |
| ADC-19C-1 | 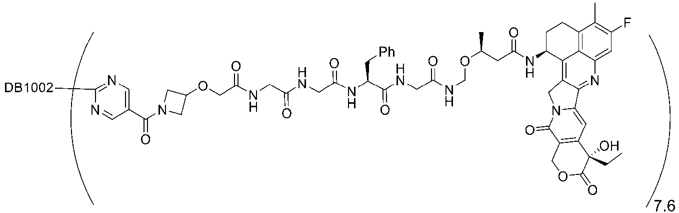 |
| ADC-20C-1 | 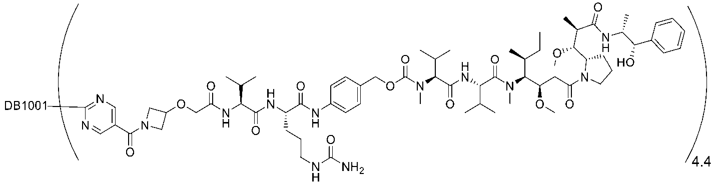 |
| ADC-21C-1 | 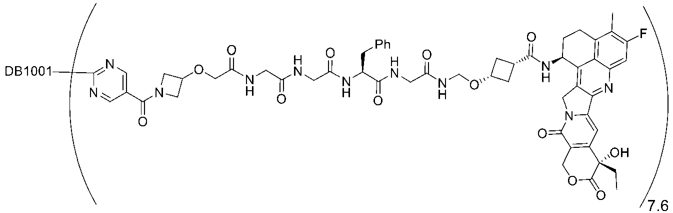 |
| ADC-22C-1 | 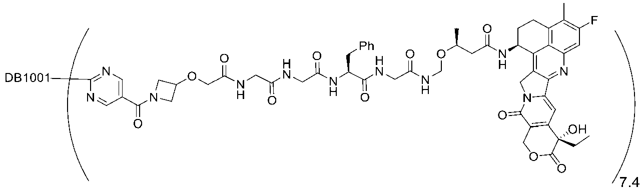 |

In another aspect, the present disclosure provides a compound comprising a structure of formula (I-2), or a mesomer, racemate, enantiomer, diastereomer, or any mixture thereof, or a pharmaceutically acceptable salt thereof, or a solvate thereof, or a pharmaceutically acceptable salt of the solvate thereof: 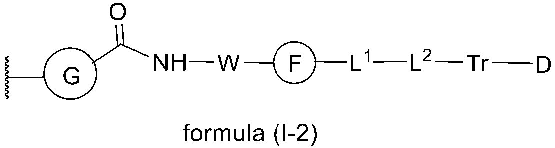 wherein
W, ring G, ring F, L¹, L², Tr, and D are as defined in formula (I), and the ring G is connected to the above ligand.

In a preferred embodiment of the present disclosure, in the compound of formula (I-2),
ring G is 5- to 6-membered heteroaryl, wherein the heteroatom of the 5- to 6-membered heteroaryl may be N and/or O, and the number of heteroatoms is 1, 2, or 3; for example, ring G is 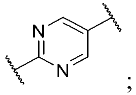
W is -(CH₂)ₜ-, wherein t is a natural number from 0 to 30;
any -CH₂- moiety in W is independently and optionally substituted by -O- or -C(O)-;
ring F is 3- to 6-membered cycloalkylene or 4- to 6-membered heterocycloalkylene; wherein the heteroatom of the 4- to 6-membered heterocycloalkylene is N, and the number of heteroatoms is independently 1 or 2;
L¹ is -(CH₂)ₙ-; wherein n is a natural number from 0 to 50; any -CH₂- moiety in L¹ is independently and optionally replaced by the following structural moieties: -C(O)-, -NH-, -O-, or -S-;
L² is a chemical bond, Val-Cit, Val-Ala, Gly-Glu, Gly-Asp, Gly-Glu-Gly, Gly-Gly-Phe-Gly, or 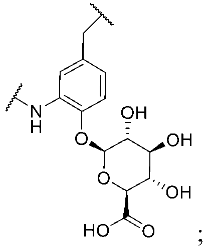
Tr is a chemical bond, 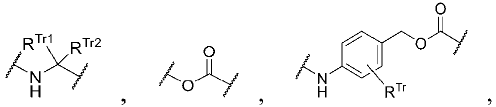 or 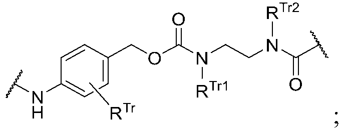 R^{Tr}, R^{Tr1}, and R^{Tr2} are each independently hydrogen or -C₁₋₆ alkyl;
D is a tubulin inhibitor, a topoisomerase inhibitor, a DNA damaging agent, an RNA polymerase inhibitor, an immunomodulator, or an immunostimulant, for example, D is

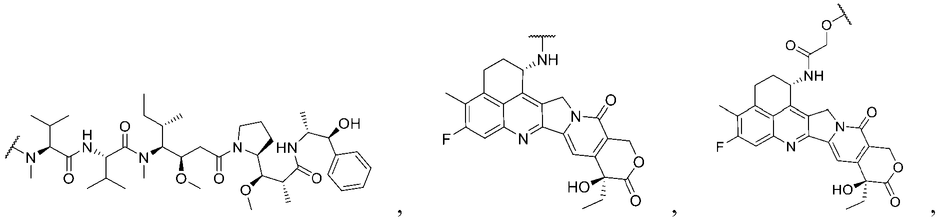

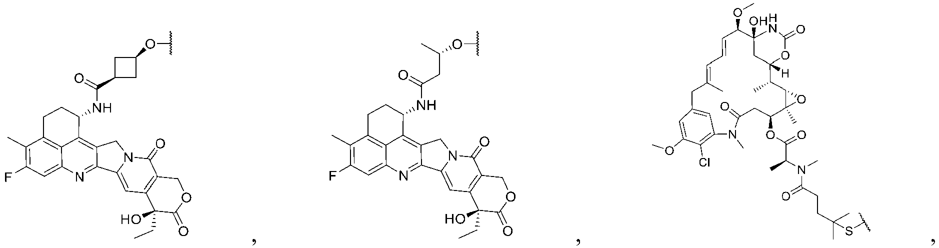

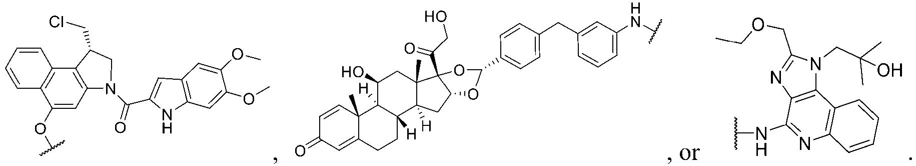

In a preferred embodiment of the present disclosure, in the compound comprising the structure of formula (I-2), or the mesomer, racemate, enantiomer, diastereomer, or any mixture thereof, or the pharmaceutically acceptable salt thereof, or the solvate thereof, or the pharmaceutically acceptable salt of the solvate thereof, the compound comprising the structure of formula (I-2) is a compound comprising a structure of formula (Ia-2): 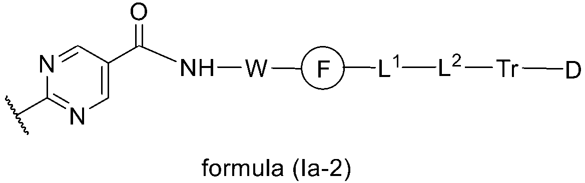 wherein
W, ring F, L¹, L², Tr, and D are as defined in formula (I), and the pyrimidine ring is connected to the above ligand.

In another aspect, the present disclosure provides a compound comprising a structure of formula (II-2), or a mesomer, racemate, enantiomer, diastereomer, or any mixture thereof, or a pharmaceutically acceptable salt thereof, or a solvate thereof, or a pharmaceutically acceptable salt of the solvate thereof: 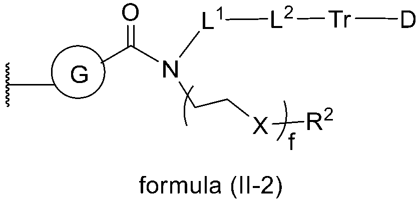 wherein
ring G, L¹, L², Tr, D, X, f, and R² are as defined in formula (II), respectively, and the ring G is connected to the above ligand.

In a preferred embodiment of the present disclosure, in the compound of formula (II-2),
ring G is 5- to 6-membered heteroaryl, wherein the heteroatom of the 5- to 6-membered heteroaryl may be N and/or O, and the number of heteroatoms is 1, 2, or 3; for example, ring G is 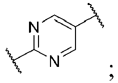
f is a natural number from 1 to 20;
X is -O-;
R² is hydrogen or -C₁₋₆ alkyl;
L¹ is -(CH₂)ₙ-; wherein n is a natural number from 0 to 50; any -CH₂- moiety in L¹ is independently and optionally replaced by the following structural moieties: -NH-, -O-, -C(O)-, or -S-;
L² is a chemical bond, Val-Cit, Val-Ala, Gly-Glu, Gly-Glu-Gly, or Gly-Gly-Phe-Gly;
Tr is a chemical bond, 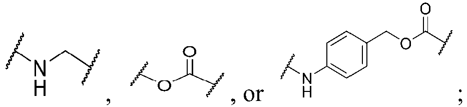
D is a tubulin inhibitor, a topoisomerase inhibitor, a DNA damaging agent, an RNA polymerase inhibitor, an immunomodulator, or an immunostimulant, for example, D is

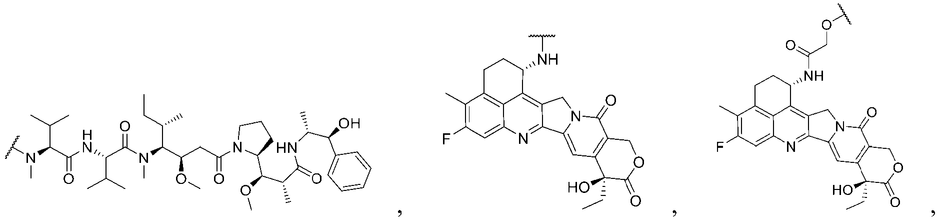

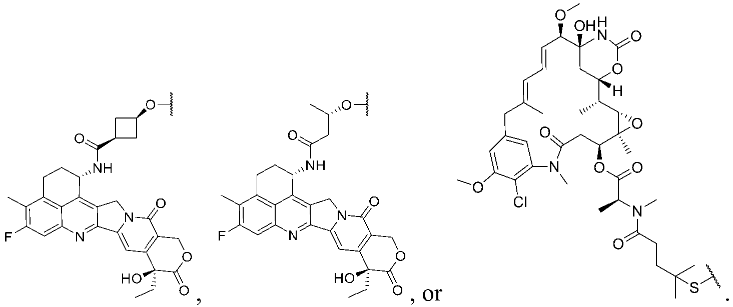

In a preferred embodiment of the present disclosure, in the compound comprising the structure of formula (II-2), or the mesomer, racemate, enantiomer, diastereomer, or any mixture thereof, or the pharmaceutically acceptable salt thereof, or the solvate thereof, or the pharmaceutically acceptable salt of the solvate thereof, the compound comprising the structure of formula (II-2) is a compound comprising a structure of formula (IIa-2): 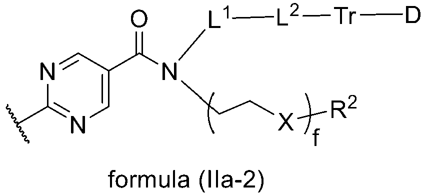 wherein
L¹, L², Tr, D, X, f, and R² are as defined in formula (II), respectively, and the pyrimidine ring is connected to the above ligand.

In another aspect, the present disclosure provides a compound of formula (III-2), or a mesomer, racemate, enantiomer, diastereomer, or any mixture thereof, or a pharmaceutically acceptable salt thereof, or a solvate thereof, or a pharmaceutically acceptable salt of the solvate thereof: 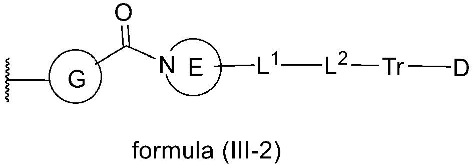 wherein
ring G, L¹, L², Tr, and ring E are as defined in formula (III), respectively, and the ring G is connected to the above ligand.

In a preferred embodiment of the present disclosure, in the compound of formula (III-2),
ring G is 5- to 6-membered heteroaryl, wherein the heteroatom of the 5- to 6-membered heteroaryl may be N and/or O, and the number of heteroatoms is 1, 2, or 3; for example, ring G is 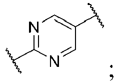
ring E is 4- to 6-membered heterocycloalkylene, wherein the heteroatom of the 4- to 6-membered heterocycloalkylene is N, and the number of heteroatoms is 1 or 2;
the 4- to 6-membered heterocycloalkylene is optionally substituted by one or more - C(O)NH-(CH₂CH₂O)_{y1}CH₃ substituents;
y1 is a natural number from 0 to 30;
L¹ is -(CH₂)ₙ-; wherein n is a natural number from 0 to 50; any -CH₂- moiety in L¹ is independently and optionally replaced by the following structural moieties: -NH-, -C(O)-, -O-, or -S-;
L² is a chemical bond, Val-Ala, Ala-Ala, Gly-Glu, Gly-Asp, Gly-Glu-Gly, or Gly-Gly-Phe-Gly;
Tr is a chemical bond, 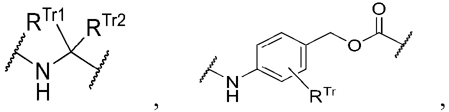 or 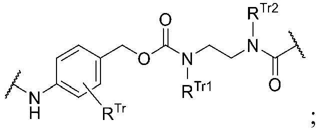 R^{Tr}, R^{Tr1}, and R^{Tr2} are each independently hydrogen or -C₁₋₆ alkyl;
D is a tubulin inhibitor, a topoisomerase inhibitor, a DNA damaging agent, an RNA polymerase inhibitor, an immunomodulator, or an immunostimulant, for example, D is 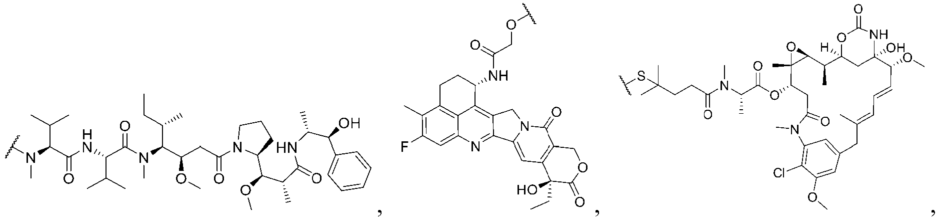 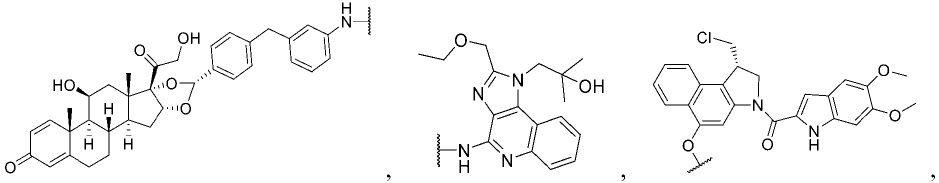 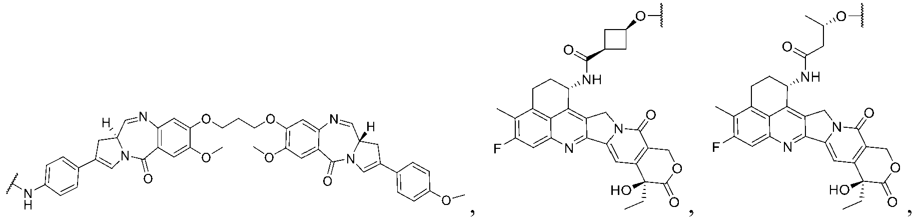 or

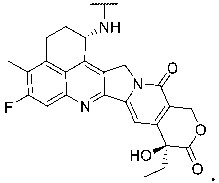

In a preferred embodiment of the present disclosure, in the compound comprising the structure of formula (III-2), or the mesomer, racemate, enantiomer, diastereomer, or any mixture thereof, or the pharmaceutically acceptable salt thereof, or the solvate thereof, or the pharmaceutically acceptable salt of the solvate thereof, the compound comprising the structure of formula (III-2) is a compound comprising a structure of formula (IIIa-2): 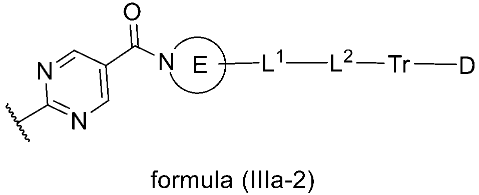 wherein
L¹, L², Tr, and ring E are as defined in formula (III), and the pyrimidine ring is connected to the above ligand.

It should be understood by those skilled in the art that the present disclosure encompasses compounds obtained by any combination of the various embodiments. Embodiments derived from the combination of technical features or preferred technical features in one embodiment with technical features or preferred technical features in another embodiment are also included within the scope of the present disclosure.

In another aspect, the present disclosure provides a preparation method for the compound of formula (1-1), formula (Ia-1), formula (II-1), formula (IIa-1), formula (III-1), or formula (IIIa-1), or the mesomer, racemate, enantiomer, diastereomer, or any mixture thereof, or the pharmaceutically acceptable salt thereof, or the solvate thereof, or the pharmaceutically acceptable salt of the solvate thereof, comprising a step of conjugating the compound of formula (I), formula (Ia), formula (II), formula (IIa), formula (III), or formula (IIIa), or the mesomer, racemate, enantiomer, diastereomer, or any mixture thereof, or the pharmaceutically acceptable salt thereof, or the solvate thereof, or the pharmaceutically acceptable salt of the solvate thereof with an active molecule of T.

In a preferred embodiment of the present disclosure, the method further comprises a step of using a reducing agent (e.g., TCEP) to cleave a disulfide bond of the targeting moiety to obtain a thiol group.

In a preferred embodiment of the present disclosure, the method comprises a step of forming a C-S bond between the compound of formula (I), formula (Ia), formula (II), formula (IIa), formula (III), or formula (IIIa), or the mesomer, racemate, enantiomer, diastereomer, or any mixture thereof, or the pharmaceutically acceptable salt thereof, or the solvate thereof, or the pharmaceutically acceptable salt of the solvate thereof and the thiol group of T.

In a preferred embodiment of the present disclosure, the molar ratio of T to the compound of formula (I), formula (Ia), formula (II), formula (IIa), formula (III), or formula (IIIa), or the mesomer, racemate, enantiomer, diastereomer, or any mixture thereof, or the pharmaceutically acceptable salt thereof, or the solvate thereof, or the pharmaceutically acceptable salt of the solvate thereof is 1:(1-20); preferably, the conjugating is performed in water and/or an organic solvent; preferably, the organic solvent is selected from one or more of N,N-dimethylformamide, dimethyl sulfoxide, N-methylpyrrolidone, nitriles (e.g., acetonitrile), and alcohols (e.g., methanol and ethanol).

In a preferred embodiment of the present disclosure, the method further comprises a step of purifying the conjugation product; preferably, the conjugation product is purified by a chromatography method (e.g., one or more of ion exchange chromatography, hydrophobic chromatography, reverse-phase chromatography, or affinity chromatography).

In another aspect, the present disclosure provides a pharmaceutical composition, which comprises the compound of formula (I), formula (Ia), comprising the structure of formula (I-2), or comprising the structure of formula (Ia-2), or the mesomer, racemate, enantiomer, diastereomer, or any mixture thereof, or the pharmaceutically acceptable salt thereof, or the solvate thereof, or the pharmaceutically acceptable salt of the solvate thereof, or the compound of formula (I-1) or formula (Ia-1), or the mesomer, racemate, enantiomer, diastereomer, or any mixture thereof, or the pharmaceutically acceptable salt thereof, or the solvate thereof, or the pharmaceutically acceptable salt of the solvate thereof, and one or more pharmaceutical excipients.

In another aspect, the present disclosure provides a pharmaceutical composition, which comprises the compound of formula (II), formula (IIa), comprising the structure of formula (II-2), or comprising the structure of formula (IIa-2), or the mesomer, racemate, enantiomer, diastereomer, or any mixture thereof, or the pharmaceutically acceptable salt thereof, or the solvate thereof, or the pharmaceutically acceptable salt of the solvate thereof, or the compound of formula (II-1) or formula (IIa-1), or the mesomer, racemate, enantiomer, diastereomer, or any mixture thereof, or the pharmaceutically acceptable salt thereof, or the solvate thereof, or the pharmaceutically acceptable salt of the solvate thereof, and one or more pharmaceutical excipients.

In another aspect, the present disclosure provides a pharmaceutical composition, which comprises the compound of formula (III), formula (IIIa), comprising the structure of formula (III-2), or comprising the structure of formula (IIIa-2), or the mesomer, racemate, enantiomer, diastereomer, or any mixture thereof, or the pharmaceutically acceptable salt thereof, or the solvate thereof, or the pharmaceutically acceptable salt of the solvate thereof, or the compound of formula (III-1) or formula (IIIa-1), or the mesomer, racemate, enantiomer, diastereomer, or any mixture thereof, or the pharmaceutically acceptable salt thereof, or the solvate thereof, or the pharmaceutically acceptable salt of the solvate thereof, and one or more pharmaceutical excipients.

In another aspect, the present disclosure provides a use of substance P or the above pharmaceutical composition in the manufacture of a medicament for treating a disease associated with abnormal cell activity (e.g., a cancer disease); wherein the substance P is the compound of formula (I), formula (II), or formula (III), or a mesomer, racemate, enantiomer, diastereomer, or any mixture thereof, or a pharmaceutically acceptable salt thereof, or a solvate thereof, or a pharmaceutically acceptable salt of the solvate thereof; or the substance P is the conjugate of formula (1-1), formula (II-1), or formula (III-1), or a mesomer, racemate, enantiomer, diastereomer, or any mixture thereof, or a pharmaceutically acceptable salt thereof, or a solvate thereof, or a pharmaceutically acceptable salt of the solvate thereof; or the substance P is the compound comprising the structure of formula (I-2), formula (II-2), or formula (III-2), or a mesomer, racemate, enantiomer, diastereomer, or any mixture thereof, or a pharmaceutically acceptable salt thereof, or a solvate thereof, or a pharmaceutically acceptable salt of the solvate thereof.

In a preferred embodiment of the present disclosure, the cancer disease is a solid tumor or a non-solid tumor, for example, selected from esophageal cancer (e.g., esophageal adenocarcinoma and esophageal squamous cell carcinoma), brain tumor, lung cancer (e.g., small cell lung cancer and non-small cell lung cancer), squamous cell carcinoma, bladder cancer, gastric cancer, ovarian cancer, peritoneal cancer, pancreatic cancer, breast cancer, head and neck cancer, cervical cancer, endometrial cancer, colorectal cancer, liver cancer, kidney cancer, non-Hodgkin's lymphoma, central nervous system tumor (e.g., neuroglioma, glioblastoma multiforme, glioma, or sarcoma), prostate cancer, and thyroid cancer.

In another aspect, the present disclosure provides a use of substance P or the above pharmaceutical composition in the manufacture of a medicament, wherein the substance P is the compound of formula (I), formula (II), or formula (III), or a mesomer, racemate, enantiomer, diastereomer, or any mixture thereof, or a pharmaceutically acceptable salt thereof, or a solvate thereof, or a pharmaceutically acceptable salt of the solvate thereof; or the substance P is the conjugate of formula (I-1), formula (II-1), or formula (III-1), or a mesomer, racemate, enantiomer, diastereomer, or any mixture thereof, or a pharmaceutically acceptable salt thereof, or a solvate thereof, or a pharmaceutically acceptable salt of the solvate thereof; or the substance P is the compound comprising the structure of formula (I-2), formula (II-2), or formula (III-2), or a mesomer, racemate, enantiomer, diastereomer, or any mixture thereof, or a pharmaceutically acceptable salt thereof, or a solvate thereof, or a pharmaceutically acceptable salt of the solvate thereof;

the medicament may be used for preventing or treating cancer, wherein the cancer disease may be a solid tumor or a non-solid tumor, for example, selected from esophageal cancer (e.g., esophageal adenocarcinoma and esophageal squamous cell carcinoma), brain tumor, lung cancer (e.g., small cell lung cancer and non-small cell lung cancer), squamous cell carcinoma, bladder cancer, gastric cancer, ovarian cancer, peritoneal cancer, pancreatic cancer, breast cancer, head and neck cancer, cervical cancer, endometrial cancer, colorectal cancer, liver cancer, kidney cancer, non-Hodgkin's lymphoma, central nervous system tumor (e.g., neuroglioma, glioblastoma multiforme, glioma, or sarcoma), prostate cancer, and thyroid cancer.

In another aspect, the present disclosure provides a use of substance P or the above pharmaceutical composition in treating a disease associated with abnormal cell activity (e.g., a cancer disease); wherein the substance P is the compound of formula (I), formula (II), or formula (III), or a mesomer, racemate, enantiomer, diastereomer, or any mixture thereof, or a pharmaceutically acceptable salt thereof, or a solvate thereof, or a pharmaceutically acceptable salt of the solvate thereof; or the substance P is the conjugate of formula (I-1), formula (II-1), or formula (III-1), or a mesomer, racemate, enantiomer, diastereomer, or any mixture thereof, or a pharmaceutically acceptable salt thereof, or a solvate thereof, or a pharmaceutically acceptable salt of the solvate thereof; or the substance P is the compound comprising the structure of formula (I-2), formula (II-2), or formula (III-2), or a mesomer, racemate, enantiomer, diastereomer, or any mixture thereof, or a pharmaceutically acceptable salt thereof, or a solvate thereof, or a pharmaceutically acceptable salt of the solvate thereof.

In another aspect, the present disclosure provides a use of substance P or the above pharmaceutical composition in the manufacture of a medicament for treating a disease associated with a target of T, wherein the substance P is the compound of formula (I), formula (II), or formula (III), or a mesomer, racemate, enantiomer, diastereomer, or any mixture thereof, or a pharmaceutically acceptable salt thereof, or a solvate thereof, or a pharmaceutically acceptable salt of the solvate thereof; or the substance P is the conjugate of formula (I-1), formula (II-1), or formula (III-1), or a mesomer, racemate, enantiomer, diastereomer, or any mixture thereof, or a pharmaceutically acceptable salt thereof, or a solvate thereof, or a pharmaceutically acceptable salt of the solvate thereof; or the substance P is the compound comprising the structure of formula (I-2), formula (II-2), or formula (III-2), or a mesomer, racemate, enantiomer, diastereomer, or any mixture thereof, or a pharmaceutically acceptable salt thereof, or a solvate thereof, or a pharmaceutically acceptable salt of the solvate thereof.

In another aspect, the present disclosure provides a method for treating a disease associated with abnormal cell activity (e.g., a cancer disease), comprising administering to an individual in need thereof an effective amount of substance P or the above pharmaceutical composition,

the substance P is the compound of formula (I), formula (II), or formula (III), or a mesomer, racemate, enantiomer, diastereomer, or any mixture thereof, or a pharmaceutically acceptable salt thereof, or a solvate thereof, or a pharmaceutically acceptable salt of the solvate thereof; or the substance P is the conjugate of formula (1-1), formula (II-1), or formula (III-1), or a mesomer, racemate, enantiomer, diastereomer, or any mixture thereof, or a pharmaceutically acceptable salt thereof, or a solvate thereof, or a pharmaceutically acceptable salt of the solvate thereof; or the substance P is the compound comprising the structure of formula (I-2), formula (II-2), or formula (III-2), or a mesomer, racemate, enantiomer, diastereomer, or any mixture thereof, or a pharmaceutically acceptable salt thereof, or a solvate thereof, or a pharmaceutically acceptable salt of the solvate thereof.

In the present application, unless otherwise specified, the scientific and technical terms used herein have meanings commonly understood by those skilled in the art. Moreover, the cell culture, molecular genetics, nucleic acid chemistry, and immunology laboratory procedures used herein are all routine procedures widely used in the corresponding fields. Meanwhile, for a better understanding of the present disclosure, definitions and explanations of relevant terms are provided below.

In the present application, the term "pharmaceutical excipient" refers to the vehicles and additives used in the manufacture of medicaments and the formulation of prescriptions, and refers to the substances contained in a pharmaceutical preparation whose safety has been reasonably evaluated, besides the active ingredients. In addition to shaping, acting as a carrier, and improving stability, the pharmaceutical excipient also has important functions such as solubilization, hydrotropy, sustained and controlled release, and is an important ingredient that may affect the quality, safety, and efficacy of drugs. According to its source, the pharmaceutical excipient can be divided into natural product, semi-synthetic product, and full synthetic product. According to its function and use, the pharmaceutical excipient can be divided into: solvents, propellants, solubilizers, cosolvents, emulsifiers, colorants, binders, disintegrants, fillers, lubricants, wetting agents, osmotic pressure regulators, stabilizers, glidants, flavoring agents, preservatives, suspending agents, coating materials, fragrances, anti-adherents, antioxidants, chelating agents, penetration enhancers, pH adjusters, buffers, plasticizers, surfactants, foaming agents, defoaming agents, thickeners, inclusion agents, humectants, absorbents, diluents, flocculants and deflocculants, filter aids, release blockers, etc. According to its route of administration, the pharmaceutical excipient can be divided into oral administration, injection, mucosal administration, transdermal or topical administration, nasal or oral inhalation administration, ocular administration, etc. The same pharmaceutical excipient can be used in pharmaceutical preparations with different routes of administration, and has different effects and uses.

In the present application, the term "pharmaceutical composition" can be prepared into various suitable dosage forms according to the route of administration. For example, tablets, capsules, granules, oral solutions, oral suspensions, oral emulsions, powders, tinctures, syrups, injections, suppositories, ointments, creams, pastes, ophthalmic preparations, pills, implants, aerosols, powder aerosols, sprays, etc.

The pharmaceutical composition can be administered in the form of injection, including injection solution, sterile powder for injection, and concentrated solution for injection. Herein, useful carriers and solvents include water, Ringer's solution, and isotonic sodium chloride solution. In addition, sterile non-volatile oils can also be used as a solvent or suspending medium, such as monoglycerides or diglycerides.

In the present application, the term "pharmaceutically acceptable salt" or "medicinal salt" typically refers to a salt of the compound or ligand-drug conjugate of the present application, or a salt of the compound described in the present application, which exhibits safety and/or efficacy when administered to mammals and possesses the desired biological activity. The antibody-antibody drug conjugate compounds of the present application may form salts with acids. Non-limiting examples of pharmaceutically acceptable salts include hydrochloride, hydrobromide, hydroiodide, sulfate, bisulfate, citrate, acetate, succinate, ascorbate, oxalate, nitrate, pivalate, hydrogen phosphate, dihydrogen phosphate, salicylate, hydrogen citrate, tartrate, maleate, fumarate, formate, benzoate, methanesulfonate, ethanesulfonate, benzenesulfonate, and*p*-toluenesulfonate.

In the present application, the term "solvate" or "solvent compound" generally refers to a pharmaceutically acceptable solvate formed by the ligand-drug conjugate compound of the present application with one or more solvent molecules. Non-limiting examples of solvent molecules include water, ethanol, acetonitrile, isopropanol, DMSO, and ethyl acetate.

The term "drug loading" generally refers to the average number of cytotoxic drugs loaded onto each ligand, also commonly referred to as the drug-to-antibody ratio, which can also be expressed as the ratio of cytotoxic drug to antibody quantity. The range of cytotoxic drug loading may be a natural number from 0 to 20, such as 1 to 10 cytotoxic drugs. In the embodiments of the present application, the drug-to-antibody ratio is represented by k, and the drug-to-antibody ratio k may be a natural number or a decimal from 1 to 10. k may be a natural number or a decimal from 2 to 8. k may be a natural number or a decimal from 3 to 8. k may be a natural number or a decimal from 1 to 2, 2 to 3, 3 to 4, 4 to 5, 5 to 6, 6 to 7, 7 to 8, 8 to 9, or 9 to 10. k may be 7.8 or 7.9.

In the present application, the term "ligand-drug conjugate" generally refers to a ligand that is connected to a biologically active cytotoxic drug through a stable linker unit. In the present application, a "ligand-drug conjugate" may be an antibody-drug conjugate (ADC), which refers to a monoclonal antibody or an antibody fragment connected to a biologically active cytotoxic drug through a stable linker unit.

In the present application, the term "ligand" generally refers to small molecules, polypeptides, RNA, DNA, carbohydrates, and macromolecular compounds capable of recognizing and binding to antigens or receptors associated with target cells. The function of ligands may be to deliver drugs to target cell populations that bind to the ligand. Such ligands include, but are not limited to, protein hormones, lectins, growth factors, antibodies, or other molecules capable of binding to cells, receptors, and/or antigens. In the present application, the ligand may be represented as T. The ligand forms a linkage bond with the linker unit through a heteroatom (e.g., S or N) on the ligand and may be an antibody or an antigen-binding fragment thereof. The antibody may be selected from a chimeric antibody, a humanized antibody, a fully human antibody, or a murine antibody; the antibody may be a monoclonal antibody. For example, the antibody may be an antibody targeting the following targets: HER2, HER3, B7H3, TROP2, Claudin 18.2, CD30, CD33, CD70, or EGFR. For example, the antibody may be an antibody targeting the following targets: 5T4, AGS-16, ANGPTL4, ApoE, CD19, CTGF, CXCR5, FGF2, MCPT8, MFI2, MS4A7, NCA, Sema5b, SLITRK6, STC2, TGF, 0772P, 5T4, ACTA2, ADGRE1, AG-7, AIF1, AKR1C1, AKR1C2, ASLG659, Axl, B7H3, BAFF-R, BCMA, BMPR1B, BNIP3, C1QA, C1QB, CA6, CADM1, CCD79b, CCL5, CCR5, CCR7, CD1lc, CD123, CD138, CD142, CD147, CD166, CD19, CD19, CD22, CD21, CD20, CD205, CD22, CD223, CD228, CD25, CD30, CD33, CD37, CD38, CD40, CD45, CD45 (PTPRC), CD46, CD47, CD49D (ITGA4), CD56, CD66e, CD70, CD71, CD72, CD74, CD79a, CD79b, CD80, CDCP1, CDH11, CDl1b, CEA, CEACAM5, c-Met, COL6A3, COL7A1, CRIPTO, CSF1R, CTSD, CTSS, CXCL11, CXCL10, DDIT4, DLL3, DLL4, DR5, E16, EFNA4, EGFR, EGFRvIII, EGLN, EGLN3, EMR2, ENPP3, EpCAM, EphA2, EphB2R, ETBR, FcRH2, FcRH1, FGFR2, FGFR3, FLT3, FOLR-α, GD2, GEDA, GPC-1, GPNMB, GPR20, GZMB, HER2, HER3, HLA-DOB, HMOX1, IFI6, IFNG, IGF-1R, IGFBP3, IL10RA1, IL-13R, IL-2, IL20Ra, IL-3, IL-4, IL-6, IRTA2, KISS1R, KRT33A, LIV-1, LOX, LRP-1, LRRC15, LUM, LY64, LY6E, Ly86, LYPD3, MDP, MMP10, MMP14, MMP16, MPF, MSG783, MSLN, MUC-1, NaPi2b, Napi3b, Nectin-4, Nectin-4, NOG, P2X5, pCAD, P-Cadherin, PDGFRA, PDK1, PD-L1, PFKFB3, PGF, PGK1, PIK3AP1, PIK3CD, PLOD2, PSCA, PSCAhlg, PSMA, PSMA, PTK7, P-cadherin, RNF43, NaPi2b, ROR1, ROR2, SERPINE1, SLC39A6, SLTRK6, STAT1, STEAP1, STEAP2, TCF4, TENB2, TGFB1, TGFB2, TGFBR1, TNFRSF21, TNFSF9, TNF-α, Trop-2, TrpM4, Tyro7, UPK1B, VEGFA, WNT5A, ADAM9, epidermal growth factor, brevican, mesothelin, sodium phosphate cotransporter 2B, Claudin18.2, endothelin receptor, mucin (such as mucin 1 and mucin 16), guanylate cyclase C, integrin a4p7, integrin a5p6, trophoblast glycoprotein, or tissue factor.

In the present application, the term "antibody or antigen-binding fragment thereof" generally refers to immunological binding reagents extending to all antibodies from all species, including dimeric, trimeric, and multimeric antibodies; bispecific antibodies; chimeric antibodies; fully human antibodies; humanized antibodies; recombinant and engineered antibodies; and fragments thereof. The term "antibody or fragment thereof" may refer to any antibody-like molecule with an antigen-binding region. This term includes small molecule fragments such as Fab', Fab, F(ab')₂, single-domain antibodies (DABs), Fv, scFv (single-chain Fv), linear antibodies, diabodies, and the like. The term "antigen-binding fragment" may refer to one or more fragments of an antibody that retain the ability to specifically bind to an antigen. For example, fragments of a full-length antibody can be utilized to perform the antigen-binding function of the antibody. Techniques for preparing and using various antibody-based constructs and fragments are well known in the art. The antibody may include one or more of anti-HER2 (ErbB2) antibody, anti-EGFR antibody, anti-B7-H3 antibody, anti-c-Met antibody, anti-HER3 (ErbB3) antibody, anti-HER4 (ErbB4) antibody, anti-CD20 antibody, anti-CD22 antibody, anti-CD30 antibody, anti-CD33 antibody, anti-CD44 antibody, anti-CD56 antibody, anti-CD70 antibody, anti-CD73 antibody, anti-CD105 antibody, anti-CEA antibody, anti-A33 antibody, anti-Cripto antibody, anti-EphA2 antibody, anti-G250 antibody, anti-MUCl antibody, anti-Lewis Y antibody, anti-TNF-α antibody, anti-TROP2 antibody, anti-Claudin 18.2 antibody, anti-VEGFR antibody, anti-GPNMB antibody, anti-Integrin antibody, anti-PSMA antibody, anti-Tenascin-C antibody, anti-SLC44A4 antibody, anti-ADAM9 antibody, or anti-Mesothelin antibody. For example, the antibody may be Trastuzumab, Pertuzumab, Adalimumab.

In the present application, the term "chimeric antibody" generally refers to an antibody formed by fusing the variable region of a murine antibody with the constant region of a human antibody, which can mitigate the immune response induced by murine antibodies. To construct a chimeric antibody, a hybridoma secreting murine specific monoclonal antibodies can be established. The variable region genes can then be cloned from the murine hybridoma cells, and the constant region genes of human antibodies can be cloned as needed. The murine variable region genes and human constant region genes are ligated into a chimeric gene and inserted into an expression vector. The chimeric antibody molecule can be expressed in either a eukaryotic or prokaryotic system.

In the present application, the term "humanized antibody", also known as a "CDR-grafted antibody", generally refers to an antibody produced by transplanting murine CDR sequences into the variable region framework of human antibodies, i.e., the framework sequences of different types of human germline antibodies. It can overcome the heterologous immune responses induced by chimeric antibodies due to their substantial murine protein components. Such framework sequences can be obtained from public DNA databases or published references that include germline antibody gene sequences. For example, germline DNA sequences of human heavy and light chain variable region genes are available in the "VBase" human germline sequence database.

In the present application, the terms "fully human-derived antibody", "fully human antibody", or "completely humanized antibody", also referred to as "fully human monoclonal antibody", indicate that both the variable and constant regions of the antibody are of human origin, eliminating immunogenicity and toxic side effects. The development of monoclonal antibodies has gone four stages: murine monoclonal antibodies, chimeric monoclonal antibodies, humanized monoclonal antibodies, and fully human monoclonal antibodies. The antibody or ligand described in the present application may be a fully human monoclonal antibody. The relevant technologies for preparing fully human antibodies may be: human hybridoma technology, EBV-transformed B lymphocyte technology, phage display technology, transgenic mouse antibody preparation technology, and single B cell antibody preparation technology.

In the present application, the term "CDR" generally refers to one of the six hypervariable regions within the variable domain of an antibody that primarily contributes to antigen binding. One of the most commonly used definitions for the six CDRs is provided by Kabat E.A. et al. (1991) (Sequences of proteins of immunological interest. NIH Publication 91-3242), Chothia et al., "Canonical Structures For the Hypervariable Regions of Immunoglobulins", J. Mol. Biol. 196:901 (1987); and MacCallum et al., "Antibody-Antigen Interactions: Contact Analysis and Binding Site Topography", J. Mol. Biol. 262:732 (1996). As used in the present application, the Kabat definition of CDR may be applied to CDR1, CDR2, and CDR3 of the light chain variable domain (CDRL1, CDRL2, CDRL3, or L1, L2, L3), as well as CDR1, CDR2, and CDR3 of the heavy chain variable domain (CDRH1, CDRH2, CDRH3, or H1, H2, H3).

The term "one or more" or similar expressions such as "at least one" may denote, for example, 1, 2, 3, 4, 5, 6, 7, 8, 9, 10, or more;

When the lower and upper limits of a numerical range are disclosed, any numerical value falling within that range and any included range are specifically disclosed. In particular, each range of values disclosed herein (in the form of "about a to b", or equivalently, "approximately a to b", or equivalently, "about a-b") shall be understood to encompass each numerical value and range within the broader range;

For example, the expression "C₁₋₆" shall be understood to encompass any sub-range and each point value therein, such as C₂₋₅, C₃₋₄, C₁₋₂, C₁₋₃, C₁₋₄, C₁₋₅, as well as C₁, C₂, C₃, C₄, C₅, or C₆. For example, the expression "C₃₋₁₀" shall also be understood in a similar manner, for example, it may encompass any sub-range and point value included therein, such as C₃₋₉, C₆₋₉, C₆₋₈, C₆₋₇, C₇₋₁₀, C₇₋₉, C₇₋₈, C₈₋₉, as well as C₃, C₄, C₅, C₆, C₇, C₈, C₉, or C₁₀. As another example, the expression "3- to 10-membered" shall be understood to encompass any sub-range and each point value therein, such as 3- to 4-membered, 3- to 5-membered, 3- to 6-membered, 3- to 7-membered, 3- to 8-membered, 3- to 9-membered, 4- to 5-membered, 4- to 6-membered, 4- to 7-membered, 4- to 8-membered, 5- to 7-membered, 5- to 8-membered, 6- to 7-membered, as well as 3-, 4-, 5-, 6-, 7-, 8-, 9-, or 10-membered. As yet another example, the expression "5- to 10-membered" shall also be understood in a similar manner, for example, it may encompass any sub-ranges and point values included therein, such as 5- to 6-membered, 5- to 7-membered, 5- to 8-membered, 5- to 9-membered, 5- to 10-membered, 6- to 7-membered, 6-to 8-membered, 6- to 9-membered, 6- to 10-membered, 7- to 8-membered, as well as 5-, 6-, 7-, 8-, 9-, or 10-membered.

In the present application, the term "alkyl" refers to a saturated straight or branched hydrocarbon group. As used herein, the term "C₁₋₆ alkyl" refers to a saturated straight or branched hydrocarbon group having 1-6 carbon atoms (e.g., 1, 2, 3, 4, 5, or 6 carbon atoms). The "C₁₋₆ alkyl" is, for example, methyl, ethyl, *n*-propyl, isopropyl, *n*-butyl, isobutyl, *sec*-butyl, *tert*-butyl, *n*-pentyl, isopentyl, neopentyl, or *n*-hexyl. The alkyl group in the present disclosure is optionally substituted by one or more substituents of the alkyl group described in the present disclosure.

In the present application, the term "alkylene" refers to a saturated straight or branched divalent hydrocarbon group. As used herein, the term "C₁₋₆ alkylene" refers to a saturated straight or branched divalent hydrocarbon group having 1-6 carbon atoms. The "C₁₋₆ alkylene" includes, for example, but is not limited to, methylene, ethylene, propylene, or butylene, and the like. The alkylene in the present disclosure is optionally substituted by one or more substituents described in the present disclosure.

In the present application, the term "alkenyl" refers to a straight or branched aliphatic hydrocarbon group having one or more carbon-carbon double bonds. For example, the term "C₂₋₆ alkenyl" as used herein refers to an alkenyl group having 2-6 carbon atoms and one, two, or three (preferably one) carbon-carbon double bonds (e.g., vinyl, 1-propenyl, 2-propenyl, 2-butenyl, 3-butenyl, 2-pentenyl, 3-pentenyl, 4-pentenyl, 2-hexenyl, 3-hexenyl, 4-hexenyl, 5-hexenyl, 2-methyl-2-propenyl, or 4-methyl-3-pentenyl), which is optionally substituted by one or more (e.g., 1-3) substituents described herein.

In the present application, the term "alkenylene" refers to a straight or branched divalent aliphatic hydrocarbon group having one or more carbon-carbon double bonds, wherein the two groups (or fragments) attached thereto may be connected to the same carbon atom or different carbon atoms. For example, the term "C₂₋₆ alkenylene" as used herein refers to an alkenylene group having 2-6 carbon atoms (e.g., 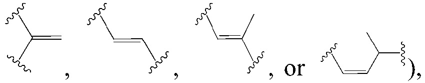 which is optionally substituted by one or more (e.g., 1-3) substituents described herein.

In the present application, the term "alkynyl" refers to a straight or branched aliphatic hydrocarbon group having one or more carbon-carbon triple bonds. For example, the term "C₂₋₆ alkynyl" as used herein refers to an alkynyl group having 2-6 carbon atoms and one, two, or three (preferably one) carbon-carbon triple bonds (e.g., ethynyl, 1-propynyl, 2-propynyl, 2-butynyl, 3-butynyl, 2-pentynyl, 3-pentynyl, 4-pentynyl, 2-hexynyl, 3-hexynyl, 4-hexynyl, or 5-hexynyl), which is optionally substituted by one or more (e.g., 1-3) substituents described herein.

**In** the present application, the term "alkynylene" refers to a straight or branched divalent aliphatic hydrocarbon group having one or more carbon-carbon triple bonds, wherein the two groups (or fragments) attached thereto are respectively connected to different carbon atoms. For example, the term "C₂₋₆ alkynylene" as used herein refers to an alkynylene group having 2-6 carbon atoms (e.g., 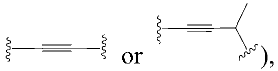 which is optionally substituted by one or more (e.g., 1-3) substituents described herein.

In the present application, the term "aryl" refers to a monocyclic or fused-ring aromatic hydrocarbon group having a conjugated π-electron system. For example, the term "C₆₋₁₂ aryl" as used herein refers to an aryl group having 6-12 carbon atoms (e.g., phenyl or naphthyl), which is optionally substituted by one or more substituents described herein (e.g., substituted by halogen).

In the present application, the term "arylene" refers to a monocyclic or fused-ring divalent aromatic hydrocarbon group having a conjugated π-electron system. For example, the term "C₆₋₁₀ arylene" as used herein refers to an arylene group having 6-10 carbon atoms, which is optionally substituted by one or more substituents described herein (e.g., substituted by halogen).

In the present application, the term "heteroaryl" or "heteroaromatic ring" refers to a monocyclic or fused heterocyclic system having one or more conjugated π-electron systems, wherein one or more (e.g., 1, 2, or 3) ring atoms are heteroatoms selected from N, O, P, and S, and the remaining ring atoms are C. The heteroaryl or heteroaromatic ring may be characterized by the number of ring atoms. For example, 5- to 12-membered heteroaryl may contain 5 to 12 (e.g., 5, 6, 7, 8, 9, 10, 11, or 12) ring atoms, particularly 5, 6, 9, or 10 ring atoms. Examples of heteroaryl include thienyl, furyl, pyrrolyl, oxazolyl, thiazolyl, imidazolyl, pyrazolyl, pyridyl, pyrazinyl, isoxazolyl, isothiazolyl, oxadiazolyl, triazolyl, thiadiazolyl, indolyl, etc., which are optionally substituted by one or more substituents described herein.

In the present application, the term "heteroarylene" refers to a monocyclic or fused-ring divalent aromatic group having a conjugated π-electron system, wherein the ring has one or more carbon atoms (e.g., 1, 2, 3, 4, 5, 6, 9, or 10 carbon atoms) and one or more (e.g., 1, 2, 3, or 4) heteroatoms each independently selected from N, O, P, and S, for example, having a total of 5 to 12 (preferably 5 to 10, more preferably 5, 6, 9, or 10) ring atoms. For example, the term "5- to 12-membered heteroarylene" as used herein refers to a heteroarylene having 5 to 12 ring atoms, which is optionally substituted by one or more substituents described herein (e.g., 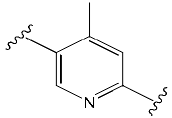 substituted by C₁₋₆ alkyl or 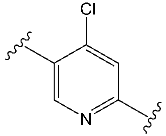 substituted by halogen).

In the present application, the term "cycloalkyl" refers to a saturated or partially saturated, monocyclic or polycyclic (e.g., bicyclic) non-aromatic hydrocarbon group. For example, "C₃₋₁₂ cycloalkyl" or "3- to 12-membered cycloalkyl" refers to a cycloalkyl group having 3-12 ring carbon atoms (e.g., 3, 4, 5, 6, 7, 8, 9, 10, 11, or 12). Common cycloalkyl groups include (but are not limited to) monocyclic cycloalkyl groups such as cyclopropyl, cyclobutyl, cyclopentyl, cyclohexyl, cycloheptyl, cyclobutene, cyclopentene, or cyclohexene; or bicyclic cycloalkyl groups, including fused, bridged, or spiro rings, such as bicyclo[1.1.1]pentyl, bicyclo[2.2.1]heptyl, bicyclo[3.2.1]octyl, bicyclo[5.2.0]nonyl, or decalinyl, which are optionally substituted by one or more substituents described herein.

In the present application, the term "cycloalkylene" refers to a saturated or partially saturated, monocyclic or polycyclic (e.g., bicyclic) non-aromatic divalent cyclic group. For example, "C₃₋₁₂ cycloalkylene" or "3- to 12-membered cycloalkylene" refers to a cycloalkylene group having 3-12 ring carbon atoms (e.g., 3, 4, 5, 6, 7, 8, 9, 10, 11, or 12). Common cycloalkylene groups include (but are not limited to) monocyclic cycloalkyl groups, such as cyclopropylene, cyclobutylene, cyclopentylene, cyclohexylene, cycloheptylene, cyclobutenylene, cyclopentenylene, or cyclohexenylene; or bicyclic cycloalkylene groups, including fused, bridged, or spiro rings, such as bicyclo[1.1.1]pentylene, bicyclo[2.2.1]heptylene, bicyclo[3.2.1]octylene, bicyclo[5.2.0]nonylene, or decalinylene, which are optionally substituted by one or more substituents described herein.

The term "heterocycloalkyl" refers to a saturated or partially saturated non-aromatic cyclic group containing at least one ring member selected from N, O, P, and S as a heteroatom. Preferably, the number of heteroatoms is 1, 2, 3, or 4. For example, 3- to 8-membered or 3-to 6-membered heterocycloalkyl. Specific examples include, but are not limited to, oxiranyl, oxocyclobutyl, pyrrolidinyl, tetrahydrofuranyl, piperidinyl, piperazinyl, tetrahydropyranyl, homopiperazinyl, etc., which are optionally substituted by one or more oxo groups or substituents described herein.

The term "heterocycloalkylene" refers to a saturated or partially saturated non-aromatic divalent cyclic group containing at least one ring member selected from N, O, P, and S as a heteroatom. Preferably, the number of heteroatoms is 1, 2, 3, or 4. For example, 3-to 8-membered or 3- to 6-membered heterocycloalkylene. Specific examples include, but are not limited to, oxiranylene, oxocyclobutylene, pyrrolidinylene, tetrahydrofuranylene, piperidinylene, piperazinylene, tetrahydropyranylene, homopiperazinylene, etc., which are optionally substituted by one or more oxo groups or substituents described herein.

The term "fused ring (fused ring system)" refers to a polycyclic structure formed by two or more (e.g., 3, 4, or 5) carbocyclic rings or heterocyclic rings sharing common ring edges, wherein the carbocyclic rings include cycloalkyl and aryl, and the heterocyclic rings include heteroaryl and heterocycloalkyl. The fused ring system includes, but is not limited to: a fused ring system formed by cycloalkyl and cycloalkyl, a fused ring system formed by cycloalkyl and heterocycloalkyl, a fused ring system formed by cycloalkyl and an aromatic ring, a fused ring system formed by cycloalkyl and a heteroaromatic ring, a fused ring system formed by heterocycloalkyl and a heteroaromatic ring, a fused ring system formed by heterocycloalkyl and an aromatic ring, a fused ring system formed by a heteroaromatic ring and a heteroaromatic ring, a fused ring system formed by a heteroaromatic ring and an aromatic ring, etc.

In the present application, the term "halogen" generally refers to fluorine, chlorine, bromine, or iodine, for example, it may be fluorine or chlorine.

In the present application, the term "each independently" means that at least two groups (or fragments) with identical or similar value ranges in a structure may have the same or different meanings in specific circumstances. For example, if substituent X and substituent Y are each independently hydrogen, halogen, hydroxyl, cyano, alkyl, or aryl, then when substituent X is hydrogen, substituent Y may be hydrogen or alternatively halogen, hydroxyl, cyano, alkyl, or aryl; similarly, when substituent Y is hydrogen, substituent X may be hydrogen or alternatively halogen, hydroxyl, cyano, alkyl, or aryl.

In the present application, the term "optional" or "optionally" generally means that the described event or condition may but does not necessarily occur, and that the description includes both cases where the event or condition does or does not occur. For example, "heterocyclyl optionally substituted by an alkyl group" means that the alkyl group may but not necessarily be present, and the description includes both cases where the heterocyclyl is substituted by an alkyl group and where it is not.

In the present application, the term "substituted" and its other variants herein mean that one or more (e.g., 1, 2, 3, or 4) atoms or atomic groups (e.g., hydrogen atoms) on a specified atom are replaced by other equivalents, provided that the normal valency of the specified atom or atomic group under the current circumstances is not exceeded and a stable compound can be formed. If an atom or atomic group is described as "optionally substituted by...," it may be either substituted or unsubstituted. Unless otherwise specified, the connecting site of a substituent herein may be at any suitable position of the substituent. When the connecting bond in a substituent is shown as passing through a chemical bond between two interconnected atoms in a ring system, it indicates that the substituent may be attached to any ring-forming atom in the ring system.

In the present application, the term 0 or more (e.g., 0 or 1 or more, 0 or 1, 0) methylene units are "replaced" generally means that when the structure contains 1 or more methylene units, the one or more methylene units may not be replaced or replaced by one or more groups described herein (e.g., -NHC(O)-, -C(O)NH-, -C(O)-, -OC(O)-, -C(O)O-, -NH-, -O-, -S-, -SO-, -SO₂-, -PH-, -P(=O)H-, -NHSO₂-, -SO₂NH-, -C(=S)-, -C(=NH)-, -N=N-, -C=N-, -N=C-, or - C(=N₂)-).

A bond in a structural formula represented herein by a wavy line " 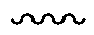 " is intended to indicate that the structure represents a *cis* or *trans* isomer, or a mixture of *cis* and *trans* isomers in any ratio.

When used alone or in combination with other groups herein, the term "oxo" refers to =O.

In the present application, one or more hydrogen atoms (for example, up to 5, for another example, 1 to 3 hydrogen atoms) are independently substituted in a group by a corresponding number of substituents. Substituents are only located at their possible chemical positions, and a person skilled in the art is able to determine (by experiment or theory) possible or impossible substitutions without undue effort. For example, an amino or hydroxyl group with a free hydrogen may be unstable when bonded to a carbon atom with unsaturated (e.g., olefinic) bonds.

In the present application, the term "amino acid" refers to both natural and unnatural amino acids, and the writing of conventional amino acids follows conventional usage. See, for example, Immunology-A Synthesis (2nd Edition, E.S. Golub and D.R. Gren, Eds., Sinauer Associates, Sunderland, Mass. (1991)), which is incorporated herein by reference. As used herein, the terms "polypeptide" and "protein" have the same meaning and are used interchangeably. Furthermore, in the present application, amino acids are generally represented by the single-letter and three-letter abbreviations commonly known in the art. For example, alanine can be represented by A or Ala; arginine can be represented by R or Arg; glycine can be represented by G or Gly; and glutamine can be represented by Q or Gln;

In the present application, the term "unnatural amino acid" has the following structure: 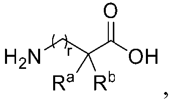 wherein r is selected from 0, 1, 2, 3, 4, and 5; wherein R^{a} and R^{b} are each independently selected from -C₁₋₆ alkyl-NH₂, -C₁₋₆ alkyl-NH-C₁₋₆ alkyl, -C₁₋₆ alkyl-N(C₁₋₆ alkyl)₂, -C₁₋₆ alkyl-NH-C₃₋₁₀ cycloalkyl, -C₁₋₆ alkyl-N(3- to 10-membered cycloalkyl)(C₁₋₆ alkyl), -C₁₋₆ alkyl-C₃₋₁₀ cycloalkyl, -C₁₋₆ alkyl-(3- to 10-membered heterocycloalkyl), -C₁₋₆ alkyl-NHCOC₁₋₆ alkyl, -C₁₋₆ alkyl-NHCOOC₁₋₆ alkyl, -C₁₋₆ alkyl-NHS(O)₂C₁₋₆ alkyl, -C₁₋₆ alkyl-S(O)₂-C₁₋₆ alkyl, -C₁₋₆ alkyl-S(O)₂-C₃₋₁₀ cycloalkyl, -C₁₋₆ alkyl-S(O)₂-NH₂, -C₁₋₆ alkyl-COOH, -C₁₋₆ alkyl-CONH₂, -C₁₋₆ alkyl-CONHC₁₋₆ alkyl, -C₁₋₆ alkyl-CO(3- to 10-membered heterocycloalkyl), 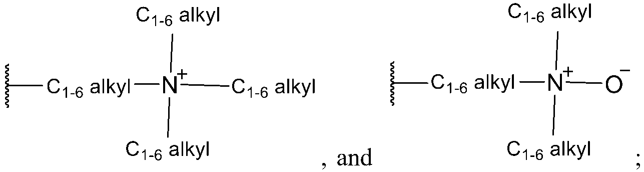 the alkyl, cycloalkyl, and heterocycloalkyl are each optionally substituted by one or more substituents selected from H, halogen, -OH, -NH₂, -SH, -NO₂, CN, -COOH, and oxo; or any R^{a} and R^{b}, together with the atom to which they are attached, form a 3- to 10-membered heterocycloalkyl group or a 3- to 10-membered cycloalkyl group; the cycloalkyl and heterocycloalkyl are each optionally substituted by one or more substituents selected from H, halogen, -OH, -NH₂, -SH, -NO₂, CN, -COOH, and oxo;

In the present application, the term "compound" generally refers to a substance having two or more different elements. For example, the compounds of the present application may be organic compounds. For example, the compound of the present application may be a compound with a molecular weight below 500, a compound with a molecular weight below 1000, a compound with a molecular weight above 1000, a compound with a molecular weight above 10000, or a compound with a molecular weight above 100000. In the present application, the compound may also refer to a compound connected by chemical bonds, for example, a compound in which one or more molecules with a molecular weight below 1000 are connected to a biomacromolecule by chemical bonds, and the biomacromolecule may be a polysaccharide, protein, nucleic acid, polypeptide, etc. For example, the compounds of the present application may include a compound in which a protein is connected to one or more molecules with a molecular weight below 1000, a compound in which a protein is connected to one or more molecules with a molecular weight below 10000, and a compound in which a protein is connected to one or more molecules with a molecular weight below 100000.

In the present application, the term "stereoisomer" refers to an isomer formed by at least one asymmetric center. For compounds having one or more (e.g., one, two, three, or four) asymmetric centers, they may produce racemic mixtures, single enantiomers, diastereomeric mixtures, and individual diastereomers. Specific individual molecules may also exist as geometric isomers (*cis*/*trans*)*.*

The compounds of the present disclosure may exist as mixtures of two or more structurally distinct forms in rapid equilibrium (commonly referred to as tautomers). Representative examples of tautomers include keto-enol tautomers, phenol-keto tautomers, nitroso-oxime tautomers, imine-enamine tautomers, etc. It is to be understood that the scope of the present application encompasses all such isomers or mixtures thereof in any proportion (e.g., 60%, 65%, 70%, 75%, 80%, 85%, 90%, 95%, 96%, 97%, 98%, or 99%).

Herein, the carbon-carbon bonds of the compounds of the present disclosure may be depicted using solid lines ( 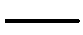 ), solid wedges ( 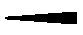 ), or dashed wedges ( 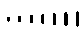 ). The use of solid lines to depict bonds to an asymmetric carbon atom is intended to indicate that all possible stereoisomers at that carbon atom (e.g., specific enantiomers or racemic mixtures) are included. The use of solid or dashed wedges to depict bonds to an asymmetric carbon atom is intended to indicate the presence of the depicted stereoisomer. When present in a racemic mixture, the use of solid and dashed wedges defines relative stereochemistry rather than absolute stereochemistry. Unless otherwise specified, the compounds of the present disclosure are intended to exist in the form of stereoisomers (including *cis* and *trans* isomers, optical isomers (e.g., R and *S* enantiomers), diastereomers, geometric isomers, rotational isomers, conformational isomers, atropisomers, and mixtures thereof). The compounds of the present disclosure may exhibit more than one type of isomerism and consist of mixtures thereof (e.g., racemic mixtures and diastereomeric pairs).

In the present application, the term "comprise", "contain", or "include" generally means including the explicitly stated features but does not exclude the presence of other elements. The terms "above" and "below" usually refer to the case where the number itself is included.

In the present application, the term "about" generally refers to a variation within the range of 0.5% to 10% above or below a specified value, such as a variation within the range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

Unless otherwise indicated, the structures described herein may also include compounds that differ only in the presence or absence of one or more isotopically enriched atoms. For example, compounds in which hydrogen atoms are substituted by deuterium or tritium, or carbon atoms are substituted by carbon-13 or carbon-14, but are otherwise identical to the structures in the present application, are within the scope of the present application.

The terms "active ingredient", "therapeutic agent", "active substance", or "active agent" refer to a chemical entity that can effectively treat one or more symptoms of a target disorder or condition.

The term "effective amount" (e.g., "therapeutically effective amount" or "prophylactically effective amount") as used herein refers to the amount of an active ingredient that, upon administration, achieves the intended effect to some extent, such as alleviating one or more symptoms of the treated disorder or preventing the occurrence of the disorder or its symptoms.

Unless otherwise specified, as used herein, the term "treating" means reversing, alleviating, inhibiting the progression of the disorder or condition to which such term applies or one or more symptoms of such disorder or condition, or preventing such disorder or condition or one or more symptoms of such disorder or condition.

As used herein, "individual" includes human or non-human animals. Exemplary human individuals include human individuals (referred to as patients) with a disease *(e.g.,* a disease described herein) or normal individuals. In the present disclosure, "non-human animal" includes all vertebrates, such as non-mammals (*e.g*., birds, amphibians, reptiles) and mammals, such as non-human primates, livestocks, and/or domesticated animals (*e.g*., sheep, dogs, cats, cows, pigs, *etc.*)*.*

Those skilled in the art will appreciate that since nitrogen requires available lone pair electrons to oxidize into oxides, not all nitrogen-containing heterocycles are capable of forming nitrogen oxides. Those skilled in the art will recognize nitrogen-containing heterocycles that are capable of forming nitrogen oxides. Those skilled in the art will also recognize that tertiary amines are capable of forming nitrogen oxides. Synthetic methods for preparing nitrogen oxides of heterocycles and tertiary amines are well known to those skilled in the art, including the oxidation of heterocycles and tertiary amines with peroxyacids such as peracetic acid and meta-chloroperbenzoic acid (mCPBA), hydrogen peroxide, alkyl hydroperoxides such as *tert*-butyl hydroperoxide, sodium perborate, and dioxiranes such as dimethyldioxirane. These methods for preparing nitrogen oxides have been extensively described and reviewed in the literature; see, for example: T. L. Gilchrist, Comprehensive Organic Synthesis, vol. 7, pp 748-750 (A. R. Katritzky and A. J. Boulton, Eds., Academic Press); and G. W. H. Cheeseman and E. S. G. Werstiuk, Advances in Heterocyclic Chemistry, vol. 22, pp 390-392 (A. R. Katritzky and A. J. Boulton, Eds., Academic Press).

In any process for preparing the compounds of the present disclosure, it may be necessary and/or desirable to protect sensitive or reactive groups on any relevant molecule, thereby forming a chemically protected form of the compounds of the present disclosure. This can be achieved by conventional protecting groups, such as those described in T.W. Greene & P.G.M. Wuts, Protective Groups in Organic Synthesis, John Wiley & Sons, 2006, the contents of which are incorporated herein by reference. The protecting groups can be removed at an appropriate subsequent stage using methods known in the art.

The present disclosure also encompasses the preparation methods for the compounds described herein. It should be understood that the compounds of the present disclosure can be synthesized using the methods described below, as well as synthetic methods known in the field of synthetic organic chemistry or variations thereof as appreciated by those skilled in the art. Preferred methods include, but are not limited to, those described below. The reactions may be carried out in a solvent or solvent mixture suitable for the reagents and materials employed and suitable for the transformation.

When the enumerative linking group does not indicate the direction for linking, the direction for linking follows the same order as the reading sequence from left to right. For example, for the group 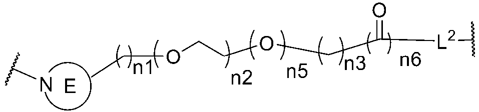 within L¹, the reading sequence of the group is from left to right, with the left side of the group being ring E and the right side of the group being L². The group links ring E and L² in the same direction as the left-to-right reading sequence, thereby forming

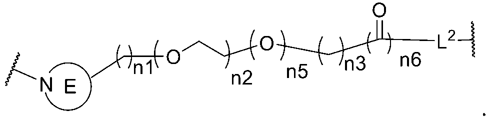

On the basis of not violating common knowledge in the art, the above preferred conditions can be arbitrarily combined to obtain preferred examples of the present disclosure.

The reagents and raw materials used in the present disclosure are all commercially available.

Beneficial effects of the present disclosure:
The heteroaromatic ring linker, linker components, and ligand-drug conjugates used in the present disclosure have one or more of the following advantages:
   (1) exhibiting extremely high stability;
   (2) exhibiting extremely high conjugation efficiency, wherein in some embodiments, the conjugation efficiency can reach 98%;
   (3) the conjugation reaction is insensitive to pH, maintaining high conjugation efficiency across a pH range of 4.0 to 9.0;
The antibody-drug conjugate of the present disclosure has one or more of the following advantages:
   (1) the conjugate obtained by the conjugation method of the present disclosure can effectively enhance the stability of drug molecules in circulation, reduce the shedding of drugs in non-target cells, and reduce off-target toxicity;
   (2) The conjugate exhibits favorable tumor tissue targeting properties;
   (3) The conjugate demonstrates favorable therapeutic efficacy in tumor animal models.

In addition, the conjugation method of the present disclosure has a wide range of applications and can be widely used for conjugating bioactive molecules with antibodies or targeted small molecule ligands.

In summary, the linker and ligand-drug conjugate of the present disclosure have significant clinical value.

### DETAILED DESCRIPTION OF THE PREFERRED EMBODIMENT

The present disclosure is further illustrated below by means of examples, but the present disclosure is not limited to the scope of the examples. The experimental methods for which specific conditions are not indicated in the following examples are selected according to conventional methods and conditions, or according to the product instructions.

In the conventional synthesis methods, preparation examples, as well as examples and intermediate synthesis examples, the meaning of each abbreviation is shown in the table below.

| **Abbr.** | **Meaning** | **Abbr.** | **Meaning** |
|---|---|---|---|
| DMF | *N*,*N*-Dimethylformamide | LiOH | Lithium hydroxide |
| DIEA | Diisopropylethylamine | LC-MS | Liquid chromatography-mass spectrometry |
| HPLC | High-performance liquid chromatography | TLC | Thin-layer chromatography |
| HATU | *O*-(7-Azabenzotriazol-1-yl)-*N*,*N*,*N'*,*N'*-tetramethyluronium hexafluorophosphate | THF | Tetrahydrofuran |
| TFA | Trifluoroacetic acid | EEDQ | 2-Ethoxy-1-ethoxycarbonyl-1,2-dihydroquinoline |
| DCM | Dichloromethane | HOBT | 1-Hydroxybenzotriazole |
| TCEP | Tris(2-carboxyethyl)phosphine hydrochloride | EDCI | 1-Ethyl-(3-dimethylaminopropyl)carbodiimide hydrochloride |
| DTPA | Diethylenetriaminepentaacetic acid | NaCl | Sodium chloride |
| DMSO | Dimethyl sulfoxide | DTT | Dithiothreitol |
| RP-HPLC | Reversed-phase high-performance liquid chromatography | NH₄HC O₃ | Ammonium bicarbonate |
| SEC | Size exclusion chromatography | GSH | Reduced glutathione |
| Nanodrop | Spectrophotometer | | |

The structures of the compounds described in the following examples were determined by nuclear magnetic resonance (¹H-NMR) and/or mass spectrometry (MS).

The instrument for measuring nuclear magnetic resonance (¹H-NMR) is a Bruker 400 MHz NMR instrument; the measurement solvent is deuterated methanol (CD₃OD), deuterated chloroform (CDCl₃), hexadeuterated dimethyl sulfoxide(DMSO-*d*₆), or deuterated water (D₂O); the internal standard substance is tetramethylsilane (TMS).

The abbreviations in the nuclear magnetic resonance (NMR) data in the following examples have the following meanings:
s: singlet, d: doublet, t: triplet, q: quartet, dd: double doublet, qd: quartet doublet, ddd: double double doublet, ddt: double double triplet, dddd: double double double doublet, m: multiplet, br: broad, *J*: coupling constant, Hz: Hertz, *δ*: chemical shift.

All chemical shift (*δ*) values are given in parts per million (ppm).

Mass spectrometry (MS) is measured using an Agilent (ESI) mass spectrometer, manufacturer: Agilent, model: Agilent 6120B.

Preparative high-performance liquid chromatography (HPLC) is carried out using a Shimadzu LC-8A preparative liquid chromatograph (YMC, ODS, 250 × 20 mm chromatographic column).

Thin-layer chromatography purification is performed using GF 254 (0.4-0.5 nm) silica gel plates produced in Yantai.

The reaction is monitored by thin-layer chromatography (TLC) or liquid chromatography-mass spectrometry (LC-MS). The developing solvent systems used include, but are not limited to: dichloromethane-methanol systems, n-hexane-ethyl acetate systems, and petroleum ether-ethyl acetate systems. The volume ratios of the solvents are adjusted according to the polarity of the compounds, or adjusted by adding triethylamine or the like.

Column chromatography generally uses 200-300 mesh silica gel (Qingdao Haiyang) as the stationary phase. The eluent systems include, but are not limited to, dichloromethane-methanol system and n-hexane-ethyl acetate system. The volume ratios of the solvents are adjusted according to the polarity of the compounds, or adjusted by adding small amounts of triethylamine or the like.

Unless otherwise specified in the examples, the reaction temperature is room temperature (20°C-30°C).

Unless otherwise indicated, the reagents used in the examples are purchased from companies such as Acros Organics, Aldrich Chemical Company, Nanjing Pharmaron Scientific, or Shanghai Shuya Medical Technology.

The above examples do not limit the scope of the present application in any way. In addition to those described herein, various modifications of the present disclosure will be apparent to those skilled in the art based on the foregoing description. Such modifications are also intended to fall within the scope of the appended claims. All references cited in the present application (including all patents, patent applications, journal articles, books, and any other publications) are incorporated herein by reference in their entirety.

### Example 1: Preparation of linker-tryptamine of ligand-drug conjugate for evaluating conjugation efficiency

### Example 1.1: Preparation of compound LP1

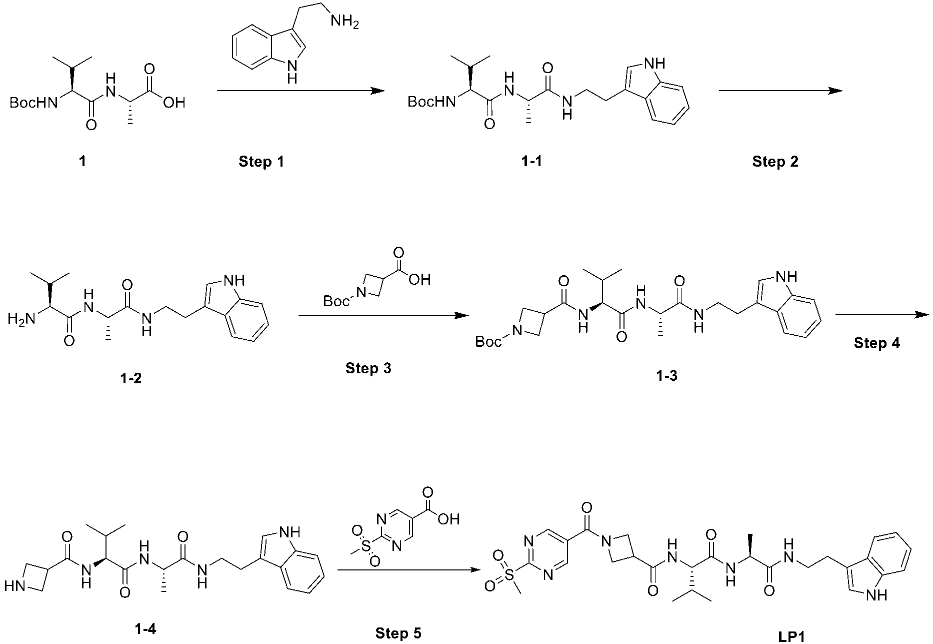

### Step 1: Preparation of compound 1-1

Compound 1 (200 mg, 0.694 mmol) was dissolved in DMF (1.5 mL), followed by the addition of HATU (96 mg, 1.04 mmol) and tryptamine (122 mg, 0.76 mmol). DIEA (358 mg, 2.78 mmol) was then added. The mixture was stirred at room temperature for 25 minutes. After completion of the reaction, water was added, followed by extraction with ethyl acetate. The organic phase was washed twice with saturated brine, dried, and concentrated. The mixture was concentrated and then purified by a silica gel column to obtain compound **1-1** (270 mg, yield: 90%).

### Step 2: Preparation of compound 1-2

Compound **1-1** (100 mg, 0.23 mmol) was dissolved in a dioxane hydrochloride solution (3 mL, 4 M) and stirred at 25°C for 30 minutes. After completion of the reaction, the pH of the reaction mixture was adjusted to 8 with a saturated aqueous sodium bicarbonate solution. The aqueous phase was extracted with ethyl acetate. The organic phase was washed once with saturated brine, dried, concentrated, and then purified by column chromatography to obtain compound **1-2** (71 mg, yield: 93%).

### Step 3: Preparation of compound 1-3

1-Boc-azetidine-3-carboxylic acid (200 mg, 1 mmol) was dissolved in DMF (5 mL), followed by the addition of HATU (567 mg, 1.49 mmol), DIEA (513 mg, 3.98 mmol), and compound **1-2** (361 mg, 1.09 mmol). The mixture was then stirred at room temperature for 30 minutes. After completion of the reaction, water was added, and the mixture was extracted with ethyl acetate. The organic phase was washed twice with saturated brine, dried, concentrated, and then purified by column chromatography to obtain compound **1-3** (450 mg, yield: 87%).

### Step 4: Preparation of compound 1-4

Compound **1-3** (1.75 g, mmol) was dissolved in TFA/DCM (40 mL, 1/3) and stirred at room temperature for 30 minutes. The pH of the solution was adjusted to 8 with an aqueous sodium bicarbonate solution. The mixture was extracted with ethyl acetate, and the organic phase was dried and concentrated. After concentration, the residue was purified by column chromatography to obtain compound **1-4** (0.9 g, yield: 63%).

### Step 5: Preparation of compound LP1

2-Methylsulfonyl-5-pyrimidinecarboxylic acid (20 mg, 0.08 mmol) was dissolved in DMF (2 mL), followed by the addition of EDCI (56 mg, 0.3 mmol) and HOBT (26 mg, 0.2 mmol). The mixture was stirred at 25°C for 30 minutes. Then, compound **1-4** (40 mg, 0.1 mmol) was added, and the mixture was stirred for 1 h. After completion of the reaction, water was added to quench the reaction, and the mixture was extracted with ethyl acetate. The organic phase was dried, concentrated, and then purified by reverse-phase HPLC to obtain compound **LP1** (3 mg, yield: 6%).

MS (ESI) m/z: 598.3 [M+H]⁺_{∘}

¹H NMR (400 MHz, DMSO-*d*₆) δ 10.79 (s, 1H), 9.26 (d, *J* = 2.6 Hz, 2H), 8.44 (s, 1H), 8.15 (d, *J* = 7.8 Hz, 1H), 8.04 (s, 1H), 7.90 (s, 1H), 7.51 (t, *J* = 6.9 Hz, 1H), 7.32 (d, *J* = 8.0 Hz, 1H), 7.13 (d, *J=* 4.2 Hz, 1H), 7.05 (t, *J =* 7.5 Hz, 1H), 6.99 - 6.92 (m, 1H), 4.55 (t, *J =* 8.8 Hz, 1H), 4.45 - 4.36 (m, 1H), 4.23 (dd, *J =* 15.0, 7.6 Hz, 3H), 4.17 - 4.06 (m, 1H), 3.62 (t, *J* = 7.2 Hz, 1H), 3.43 (d, *J =* 7.2 Hz, 3H), 2.79 (q, *J =* 7.4 Hz, 2H), 2.69 - 2.65 (m, 1H), 2.35 - 2.29 (m, 1H), 1.98 (s, 1H), 1.18 (dd, *J* = 9.9, 7.1 Hz, 3H), 0.84 (ddd, *J =* 14.8, 6.4, 4.8 Hz, 6H).

### The following compounds were synthesized with reference to a method similar to that of Example 1.1.

| No. | Structure | MS and ¹HNMR |
|---|---|---|
| LP2 | 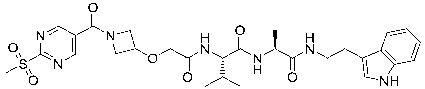 | MS (ESI) m/z: 628.3[M+H]⁺ |
| | | ¹H NMR (400 MHz, DMSO-*d*₆) δ 10.80 (s, 1H), 9.41 (d, *J =* 6.8 Hz, 1H), 9.37 (s, 2H), 8.17 (d, *J =* 5.6 Hz, 1H), 7.94 (t, *J =* 5.6 Hz, 1H), 7.56 (dd, *J =* 28.3, 8.6 Hz, 2H), 7.32 (d, *J =* 8.0 Hz, 1H), 7.14 (s, 1H), 7.01 (dt, *J =* 34.9, 7.3 Hz, 2H), 4.57 (dd, *J =* 13.6, 6.8 Hz, 1H), 4.28 - 4.19 (m, 2H), 3.73 (s, 2H), 3.45 (s, 3H), 3.19 (dd, *J* = 13.6, 6.4 Hz, 4H), 2.80 (t, *J =* 7.3 Hz, 2H), 1.99 (dd, *J =* 13.5, 6.7 Hz, 1H), 1.18 (d, *J =* 7.0 Hz, 3H), 0.83 (dd, *J =* 19.2, 6.7 Hz, 6H). |
| LP3 | 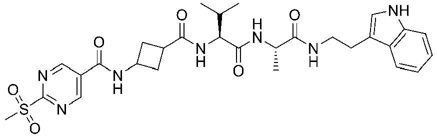 | MS (ESI) m/z: 612.2[M+H]⁺ |
| | | ¹H NMR (400 MHz, DMSO- *d*₆) δ10.80 (s, 1H), 9.48 - 9.30 (m, 2H), 9.23 (dd, *J =* 20.5, 7.4 Hz, 1H), 8.32 (s, 1H), 7.93 (ddd, *J =* 26.9, 17.5, 7.9 Hz, 3H), 7.52 (d, *J =* 7.9 Hz, 1H), 7.32 (d, *J* = 8.0 Hz, 1H), 7.14 (s, 1H), 7.05 (t, *J* = 7.2 Hz, 1H), 6.96 (t, *J* = 7.4 Hz, 1H), 4.50 (ddd, *J* = 25.0, 16.1, 8.2 Hz, 1H), 4.28 - 4.13 (m, 2H), 3.45 (d, *J =* 2.7 Hz, 3H), 3.33 (dd, *J* = 13.1, 7.4 Hz, 3H), 3.13 (s, 1H), 2.81 (d, *J* = 7.4 Hz, 2H), 2.42 - 2.23 (m, 3H), 1.99 (dt, *J =* 16.9, 6.8 Hz, 1H), 1.19 (t, *J =* 6.4 Hz, 3H), 0.83 (dd, *J =* 19.2, 6.7 Hz, 6H) |
| LP4 | 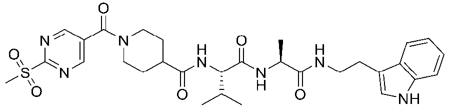 | MS (ESI) m/z: 626.3[M+H]⁺ |
| | | ¹H NMR (400 MHz, DMSO-*d*₆) δ 10.80 (s, 1H), 9.15 (s, 2H), 7.94 - 7.85 (m, 3H), 7.52 (t, *J =* 5.6 Hz, 1H), 7.32 (d, *J =* 8.0 Hz, 1H), 7.13 (s, 1H), 7.06 (t, *J =* 6.4 Hz, 1H), 6.99 - 6.94 (m, 1H), 4.47 (d, *J =* 11.4 Hz, 1H), 4.25 - 4.15 (m, 2H), 3.61 - 3.58 (m, 1H), 3.43 (s, 3H), 3.28 - 3.26 (m, 2H), 3.15 (t, *J* = 7.2 Hz, 2H), 2.90 - 2.77 (m, 3H), 2.67 - 2.58 (m, 1H), 2.03 - 1.51 (m, 5H), 1.26 - 1.17 (m, 3H), 0.83 (dd, *J =* 19.2, 6.4 Hz, 6H). |
| LP5 | 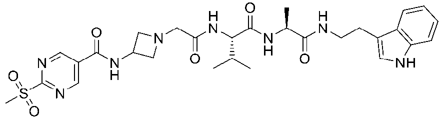 | MS (ESI) m/z:627.3[M+H]⁺ |
| | | ¹H NMR (400 MHz, DMSO- *d*₆) δ 10.80 (s, 1H), 9.41 (d, *J =* 6.8 Hz, 1H), 9.37 (s, 2H), 8.17 (d, *J =* 5.6 Hz, 1H), 7.94 (t, *J =* 5.6 Hz, 1H), 7.56 (dd, *J =* 28.3, 8.6 Hz, 2H), 7.32 (d, *J =* 8.0 Hz, 1H), 7.14 (s, 1H), 7.01 (dt, *J =* 34.9, 7.3 Hz, 2H), 4.57 (dd, *J =* 13.6, 6.8 Hz, 1H), 4.28 - 4.19 (m, 2H), 3.73 (s, 2H), 3.45 (s, 3H), 3.19 (dd, *J* = 13.6, 6.4 Hz, 4H), 2.80 (t, *J =* 7.3 Hz, 2H), 1.99 (dd, *J =* 13.6, 6.8 Hz, 1H), 1.18 (d, *J =* 7.0 Hz, 3H), 0.83 (dd, *J =* 19.2, 6.8 Hz, 6H). |
| LP6 | 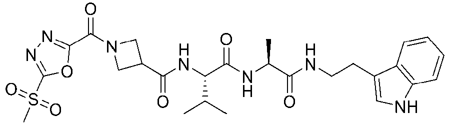 | MS (ESI) m/z: 588.2 [M+H]⁺ |
| | | ¹H NMR (400 MHz, DMSO-*d*₆) δ 9.02 (s, 1H), 7.59 (dd, *J =* 7.6, 4.4 Hz, 1H), 7.16 - 7.04 (m, 4H), 6.91 - 6.84 (m, 3H), 4.82 - 4.66 (m, 2H), 4.36 - 4.16 (m, 4H), 3.67 - 3.62 (m, 1H), 3.49 - 3.44 (m, 4H), 2.92 (q, *J =* 6.8 Hz, 2H), 1.28 (dd, *J =* 9.8, 7.2 Hz, 3H), 0.84 (ddd, *J =* 14.8, 6.4, 4.8 Hz, 6H). |
| LP7 | 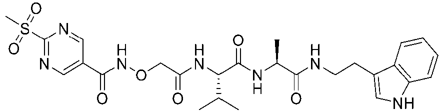 | MS (ESI) m/z: 588.2[M+H]⁺ |
| | | ¹H NMR (400 MHz, DMSO- d*₆*)δ 12.63 (s, 1H), 10.79 (s, 1H), 9.31 (s, 2H), 8.14 (dd, *J =* 26.7, 8.1 Hz, 2H), 7.91 (t, *J* = 5.5 Hz, 1H), 7.52 (d, *J =* 7.8 Hz, 1H), 7.33 (d, *J* = 8.1 Hz, 1H), 7.13 (d, *J* = 2.1 Hz, 1H), 7.06 (t, *J =* 7.0 Hz, 1H), 6.97 (t, *J* = 7.0 Hz, 1H), 4.53 (s, 2H), 4.38 - 4.16 (m, 2H), 3.44 (s, 3H), 2.79 (t, *J =* 7.3 Hz, 2H), 2.09 - 1.81 (m, 1H), 1.17 (d, *J =* 7.0 Hz, 3H), 0.85 (dd, *J =* 15.3, 6.7 Hz, 6H). |
| LP9 | 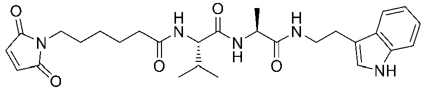 | MS (ESI) m/z: 524.3[M+H]⁺ |
| | | ¹H NMR (400 MHz, DMSO- *d*₆) δ 10.80 (s, 1H), 7.96 - 7.88 (m, 2H), 7.80 (d, *J =* 8.4 Hz, 1H), 7.52 (d, *J =* 8.0 Hz, 1H), 7.32 (d, *J =* 8.0 Hz, 1H), 7.14 (d, *J =* 2.1 Hz, 1H), 7.08 - 7.04 (m, 1H), 7.01 - 6.94 (m, 3H), 4.26 - 4.21 (m, 1H), 4.15 - 4.12 (m, 1H), 3.36 - 3.29 (m, 4H), 2.81 (dd, *J =* 14.2, 6.8 Hz, 4H), 2.22 - 2.08 (m, 2H), 2.08 - 1.86 (m, 1H), 1.56 - 1.42 (m, 4H), 1.22 - 1.14 (m, 5H), 0.83 (dd, *J =* 19.2, 6.8 Hz, 6H). |
| LP10 | 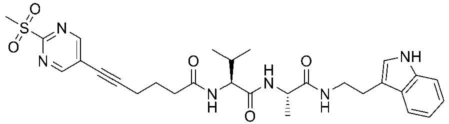 | MS (ESI) m/z: 581.2 [M+H]⁺ |
| | | ¹H NMR (400 MHz, DMSO-*d*₆) δ 10.71 (s, 1H), 9.10 (s, 1H), 9.03 (s, 1H), 8.31 - 8.29 (m, 0.5H), 8.09 - 8.08 (m, 0.5H), 7.99 - 7.90 (m, 2H), 7.52 (d, *J=* 8.0 Hz, 1H), 7.32 (t, *J =* 7.8 Hz, 1H), 7.14 (s, 1H), 7.07 - 6.90 (m, 2H), 4.28 - 4.03 (m, 2H), 3.41 (s, 1H), 2.81 - 2.77 (m, 2H), 2.57 - 2.53 (m, 4H), 2.44 - 2.33 (m, 2H), 2.01 - 1.78 (m, 3H), 1.18 (dd, *J =* 9.9, 7.1 Hz, 3H), 0.84 (ddd, *J =* 14.8, 6.4, 4.8 Hz, 6H). |

### Example 1.2: Preparation of compound LP8

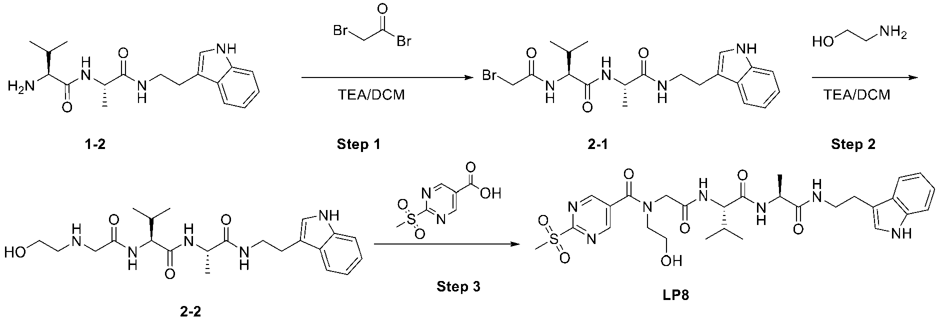

### Step 1: Preparation of compound 2-1

Under a nitrogen atmosphere, DIEA (46 mg, 0.45 mmol) was added to a solution of compound **1-2** (100 mg, 0.3 mol) and bromoacetyl bromide (61 mg, 0.3 mmol) in DMF (5 mL). The mixture was stirred at room temperature for 1 h. The mixture was quenched by adding water and extracted with ethyl acetate, and the combined organic phases were dried over sodium sulfate and concentrated under reduced pressure. The residue was purified by column chromatography to obtain compound **2-1** (35 mg, yield: 25.7%).

### Step 2: Preparation of compound 2-2

Under a nitrogen atmosphere, DIEA (12 mg, 0.12 mmol) was added to a solution of compound **2-1** (35 mg, 0.08 mol) and ethanolamine (5 mg, 0.08 mmol) in DMF (2 mL). The mixture was stirred at room temperature for 1 h. The mixture was concentrated under reduced pressure. The residue was purified by column chromatography to obtain compound **2-2** (13 mg, yield: 39.4%).

### Step 3: Preparation of compound LP8

Compound **LP8** was synthesized with reference to a method similar to that described in Step 5 of Example 1.1.

MS (ESI) m/z: 616.3 [M+H]⁺_{∘}

¹H NMR (400 MHz, DMSO-*d*₆) δ 10.79 (s, 1H), 9.12 (d, *J* = 36.6 Hz, 2H), 8.29 - 7.97 (m, 2H), 7.88 (d, *J =* 6.9 Hz, 1H), 7.52 (d, *J* = 7.8 Hz, 1H), 7.33 (d, *J* = 8.1 Hz, 1H), 7.14 (s, 1H), 7.06 (t, *J* = 7.5 Hz, 1H), 6.97 (t, *J* = 7.4 Hz, 1H), 5.22 - 4.55 (m, 2H), 4.39 - 3.93 (m, 4H), 3.68 - 3.46 (m, 4H), 3.42 (d, *J* = 13.0 Hz, 3H), 2.81 (d, *J* = 8.2 Hz, 2H), 1.97 (dq, *J* = 47.5, 6.8 Hz, 2H), 1.27 - 1.10 (m, 3H), 0.96 - 0.57 (m, 6H)_{∘}

### Example 2: Preparation of ligand-linker-tryptamine conjugate

The antibody was prepared according to conventional methods. For example, after vector construction, eukaryotic cells such as HEK293 cells and CHO cells could be transfected for purification and expression. Trastuzumab and Sacituzumab were used as examples to prepare the ligand-drug conjugates.

### Amino acid sequence of Trastuzumab:

**Light chain (SEQ ID NO: 1)** 
DIQMTQSPSSLSASVGDRVTITCRASQDVNTAVAWYQQKPGKAPKLLIYSASF
LYSGVPSRFSGSRSGTDFTLTISSLQPEDFATYYCQQHYTTPPTFGQGTKVEIKRTVAAP
SVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC
**Heavy chain (SEQ ID NO: 2)**

EVQLVESGGGLVQPGGSLRLSCAASGFNIKDTYIHWVRQAPGKGLEWVARI
YPTNGYTRYADSVKGRFTISADTSKNTAYLQMNSLRAEDTAVYYCSRWGGDGFYAM
DYWGQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKS
CDKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIE
KTISKAKGQPREPQVYTLPPSREEMTKN

### Example 2.1: Preparation of compounds TLP1-TLP8

### 1. Experimental procedure for reduction reaction

Using a reaction system with 1 mg of Trastuzumab antibody, a reaction concentration of 5 mg/mL, and a total volume of 200 µL as an example: 25.9 µL of antibody solution (38.60 mg/mL) was added to a 1.5 mL EP tube, followed by the addition of 106.9 µL of a 20 mM PB solution and 20.0 µL of a 10 mM DTPA trisodium solution (final DTPA concentration: 1 mM). Finally, 47.2 µL of a 1 mM TCEP solution (TCEP:Ab = 7:1) was slowly added. After completion of the addition, the mixture was thoroughly mixed by pipetting and placed in a constant-temperature shaking mixer at 25°C with an oscillation speed of 400 rpm. The timing was initiated after the completion of TCEP addition, and the reaction was carried out for 2 h.

### 2. Experimental procedure for pH adjustment

After the completion of the reduction reaction, the pH of the system was adjusted using 0.5 M acetic acid solution. Approximately 10-20 µL of 0.5 M acetic acid solution was intermittently added using a pipette, and the mixture was shaken and mixed thoroughly after each addition. The pH of the system was monitored with a pH meter (direct measurement) until the pH was reduced to 7.4 ± 0.1, 5.8 ± 0.1, or 6.5 ± 0.1.

### 3. Experimental procedure for conjugation reaction

Prior to the conjugation reaction, the temperature of the reaction system was appropriately reduced using an ice-water bath. To the pH-adjusted system was slowly added 18.9 µL of DMSO, and the mixture was thoroughly mixed by pipetting. While stirring, 12.1 µL of an LP solution in DMSO (5 mM, LP:Ab = 9:1, final DMSO concentration in the system: 15%) was slowly added.. After the completion of the addition, the mixture was thoroughly mixed by pipetting, and the clarity of the system was observed. The mixture was placed in a 22°C constant-temperature mixer with an oscillation speed of 400 rpm. The timing was initiated after the completion of LP addition, and the reaction was carried out for 1 h.

### 4. Experimental procedure for buffer exchange by ultrafiltration

After the completion of the conjugation reaction, the sample was loaded into an ultrafiltration tube (Amicon Ultra-0.5 mL, 30 kDa) and subjected to four times of buffer exchange with 20 mM PB by centrifugation (13000 rpm, 2 minutes). Each centrifugation was performed until approximately 150 µL of the sample remained, followed by replenishment with PB to 500 µL. The exchanged samples TLP1-TLP8 were collected, and the volume of the recovered samples was recorded. The protein concentration of the samples was measured by Nanodrop using 20 mM PB as a blank. A portion of the samples was taken for HPLC detection, and the remaining samples were stored at -80 ± 10°C.

The DAR (k) was detected and calculated by RP-HPLC analysis, and the aggregation level was detected by SEC. The results are shown in Table 1.

**Table 1: ADC structure, conjugation efficiency, and aggregation level**

| No. | Structure | RP DAR (k) | Monomer/% |
|---|---|---|---|
| TLP9 (Control 1) | 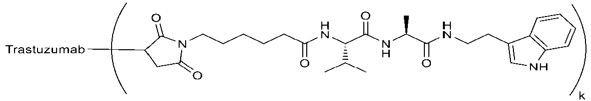 | 5.69 | 95.9 |
| TLP10 (Control 2) | 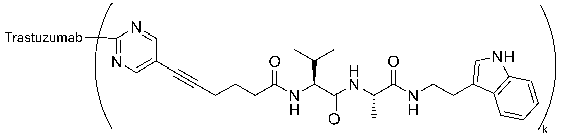 | 6.25 | 98.7 |
| TLP1 | 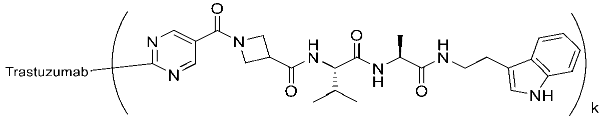 | 6.64 | 99.0 |
| TLP2 | 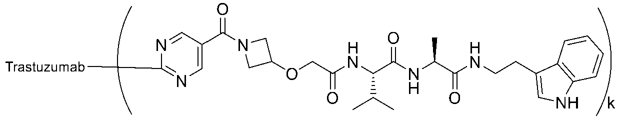 | 7.75 | 98.9 |
| TLP3 | 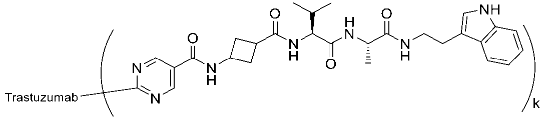 | 7.77 | 99.0 |
| TLP4 | 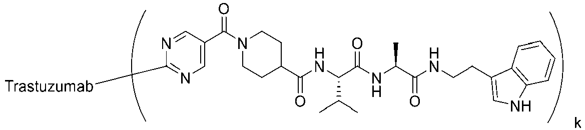 | 7.39 | 99 |
| TLP5 | 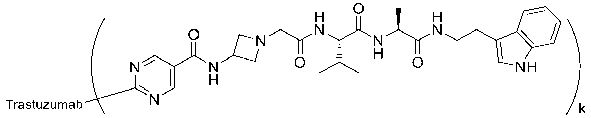 | NA | 99.1 |
| TLP6 | 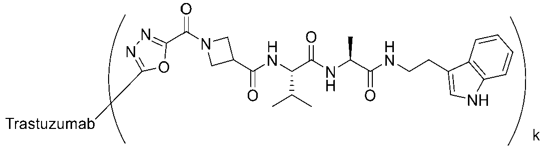 | 4.59 | 98.7 |
| TLP7 | 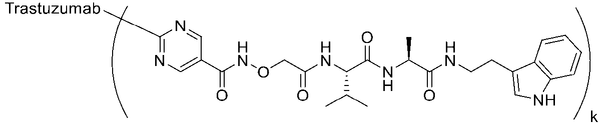 | 5.07 | 99.0 |
| TLP8 | 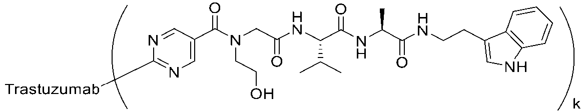 | 7.84 | 99.0 |

According to the above method, TLP1, TLP2, TLP8, and TLP10 (Control 2) were prepared in buffers with pH 5.8, 6.5, and 7.4, respectively. The DAR was detected and calculated by RP-HPLC analysis, and the aggregation level was detected by SEC. The results are shown in Table 2.

**Table 2: Conjugation efficiency and aggregation level under different pH conditions**

| Compound | pH | RP DAR | Monomer/% |
|---|---|---|---|
| TLP1 | 5.8 | 6.45 | 99.1 |
| | 6.5 | 6.52 | 99.1 |
| | 7.4 | 6.64 | 99.0 |
| TLP2 | 5.8 | 7.17 | 99.0 |
| | 6.5 | 7.17 | 98.9 |
| | 7.4 | 7.75 | 98.9 |
| TLP8 | 5.8 | 7.45 | 98.9 |
| | 6.5 | 7.80 | 98.7 |
| | 7.4 | 7.84 | 99.0 |
| TLP10 (Control 2) | 5.8 | 4.74 | 99.2 |
| | 6.5 | 4.55 | 97.6 |
| | 7.4 | 6.25 | 98.7 |

The results indicate that most of the novel linkers of the present disclosure have extremely high conjugation efficiency and the resulting ADCs have low aggregation levels. Furthermore, it is surprisingly found that the novel linkers of the present disclosure have high conjugation efficiency under high, medium, and low pH conditions, particularly showing high conjugation efficiency at low pH, which is significantly superior to the control TLP10.

### Example 3: GSH exchange stability test

In the present disclosure, the ADC compound was placed in PBS buffer (pH 7.4), and GSH was used to replace HSA in plasma. The incubation was performed under a nitrogen atmosphere at 40°C. The DAR values of the ADC compound were measured at 0 h, 1 d, and 4 d, respectively, to simulate and evaluate the stability of the linker of the ADC compound in plasma.

The ADC concentration was 0.001 µmol/mL, and the GSH concentration was 0.954 µmol/mL (equivalent to 1.5 times the HSA concentration in human plasma (0.636 µmol/mL)).

### Example 3.1: Exchange stability test of TLP1 and control example TLP9.

49.2 µL (0.45 mg, 9.15 mg/mL) of each ADC solution of TLP1 and TLP9 was placed in a 4 mL EP tube, followed by the addition of 2860.8 µL of 20 mM PBS solution and 90 µL of 10 mg/mL GSH solution, resulting in a final ADC concentration of 0.15 mg/mL and a final GSH concentration of 0.3 mg/mL in the system. After the completion of the addition, the mixture was thoroughly mixed by shaking and aliquoted into 2 mL cryotubes, with 500 µL per tube (6 tubes in total). One tube of T0 sample was stored in a -80°C freezer, while the other 5 tubes of samples were light-shielded with aluminum foil and incubated in a 40°C incubator. One tube was removed for testing at each time point (1 d and 4 d). The exchange test results are shown in Table 3.

**Table 3: DAR values incubated in GSH at pH 7.4**

| Compound | Time | RP DAR |
|---|---|---|
| TLP9 (Control 1) | 0 h | 5.73 |
| | Day 1 | 5.44 |
| | Day 4 | 5.16 |
| TLP1 | 0 h | 6.70 |
| | Day 1 | 6.77 |
| | Day 4 | 6.74 |

TLP1 exhibited good long-term stability in GSH and was superior to the control example TLP9. It can be concluded that the linker of the present disclosure has favorable stability in plasma.

### Example 3.2: Exchange stability tests of TLP2, TLP3, TLP4, and TLP8.

The method of Example 3.1 was referenced to evaluate the DAR value changes of TLP2, TLP3, TLP4, and TLP8 at different time points during incubation in GSH. The results showed that all four ADC compounds exhibited good stability, with their DAR values remaining unchanged.

### Example 4: Preparation of linker-payload of antibody-drug conjugate

### Intermediate Preparation

### Intermediate Preparation Example 1:

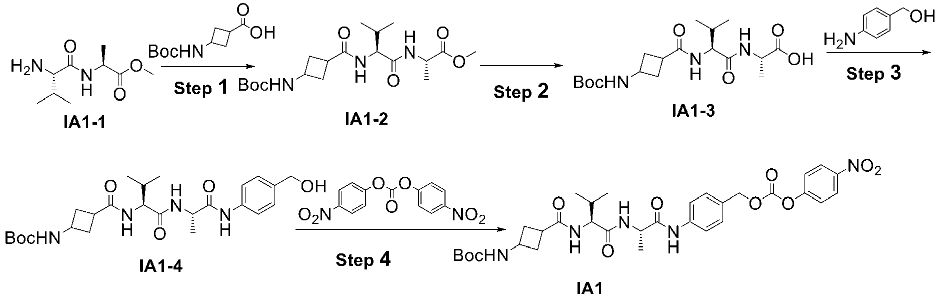

### Step 1: Preparation of compound IA1-2

1-Boc-azetidine-3-carboxylic acid (200 mg, 1 mmol) was dissolved in DMF (5 mL), followed by the addition of HATU (567 mg, 1.49 mmol), DIEA (513 mg, 3.98 mmol), and compound **IA1-1** (222 mg, 1.09 mmol). The mixture was then stirred at room temperature for 30 minutes. After completion of the reaction, water was added, and the mixture was extracted with ethyl acetate. The organic phase was washed twice with saturated brine, dried, concentrated, and then purified by column chromatography to obtain compound **IA1-2** (339 mg, yield: 85%).

### Step 2: Preparation of compound IA1-3

Compound **IA1-2** (339 mg, 0.85 mmol) was dissolved in methanol (3 mL), followed by the addition of an aqueous LiOH solution (10 mL, 2 N). The mixture was stirred at room temperature for 1 h. After completion of the reaction, the pH of the solution was adjusted to 8 with an aqueous citric acid solution (2 N). The mixture was extracted with ethyl acetate, and the organic phase was dried and concentrated. After concentration, the crude product of compound **IA1-3** (327 mg) was obtained.

### Step 3: Preparation of compound IA1-4

Compound **IA1-3** (327 mg, 0.85 mmol) was dissolved in DMF (10 mL), followed by the addition of EEDQ (420 mg, 1.70 mmol) and p-aminobenzyl alcohol (209 mg, 1.7 mmol). The mixture was then stirred at room temperature for 24 h. After completion of the reaction, water was added, and the mixture was extracted with ethyl acetate. The organic phase was washed twice with saturated brine, dried, concentrated, and then purified by column chromatography to obtain compound **IA1-4** (334 mg, yield: 80%).

### Step 4: Preparation of compound IA1

Compound **IA1-4** (334 mg, 0.68 mmol) was dissolved in DMF (10 mL), followed by the addition of bis(p-nitrophenyl) carbonate (249 mg, 0.82 mmol) and DIEA (335 mg, 2.72 mmol). The mixture was then stirred at room temperature for 24 h. After completion of the reaction, water was added, and the mixture was extracted with ethyl acetate. The organic phase was washed twice with saturated brine, dried, concentrated, and then purified by column chromatography to obtain compound **IA1** (401 mg, yield: 90%).

### Referring to the method described in Intermediate Preparation Example 1, the following intermediates were synthesized using different protected or unprotected peptide chains:

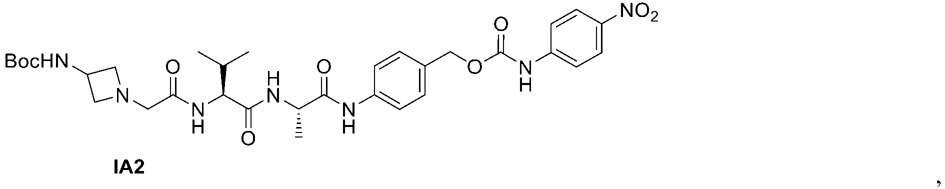

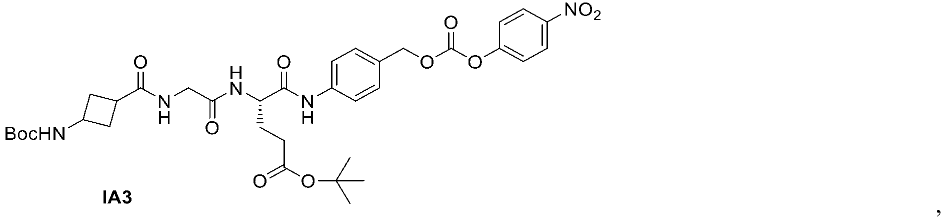

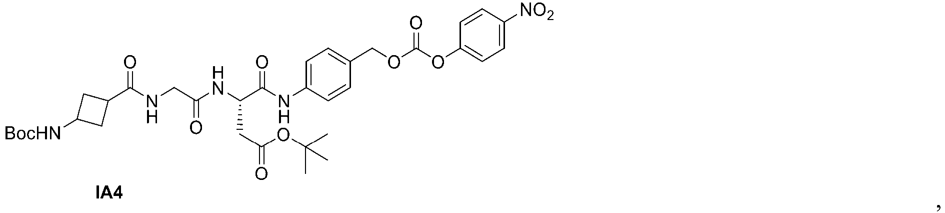

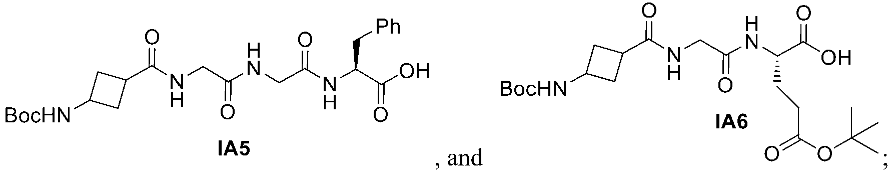

### Intermediate Preparation Example 2: Synthesis of compound IB1

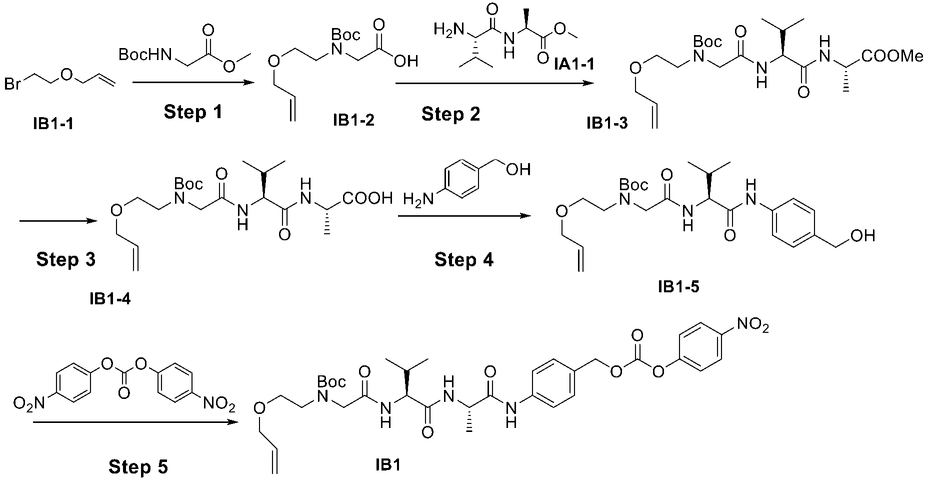

### Step 1: Preparation of compound IB1-2

Under a nitrogen atmosphere, methyl Boc-glycinate (2 g, 10.5 mmol) was dissolved in anhydrous tetrahydrofuran (30 mL), and potassium carbonate (2.9 g, 21.1 mmol) was added. The mixture was heated to 40°C and stirred for 30 minutes, followed by the addition of compound IB1-1 (1.9 g, 11.6 mmol), and the reaction was continued for 2 h. The mixture was then cooled to room temperature, and a 2 N aqueous LiOH solution (30 mL) was added, followed by stirring for another 2 h. After completion of the reaction, methyl tert-butyl ether was added. The mixture was stirred and the phases were separated. The organic phase was discarded. The pH of the aqueous phase was adjusted to 5 with a 2 N aqueous hydrochloric acid solution. The organic phase was separated, dried over sodium sulfate, and concentrated under reduced pressure to obtain compound **IB1-2** (2.3 g, yield: 85%), which was directly used in the next step.

### Steps 2 to 5: Preparation of compound IB1

Compound **IB1** was synthesized with reference to the method described in Intermediate Preparation Example 1.

Referring to **the method described in Intermediate Preparation Example 2, the following intermediates were synthesized using different protected or unprotected** peptide chains: 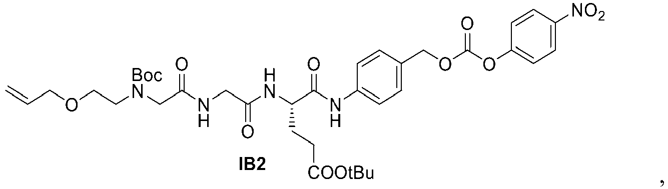 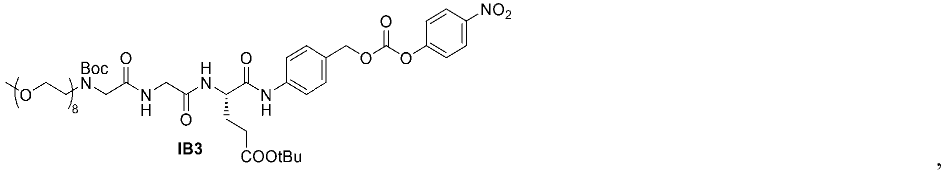 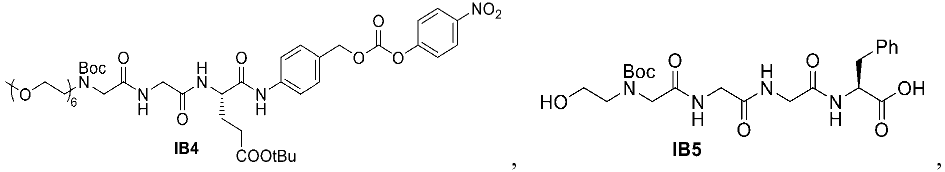 and

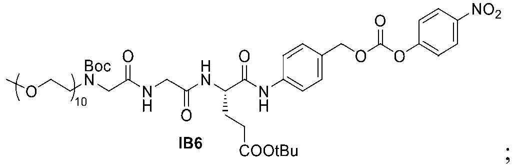

### Intermediate Preparation Example 3: Synthesis of compound IC5

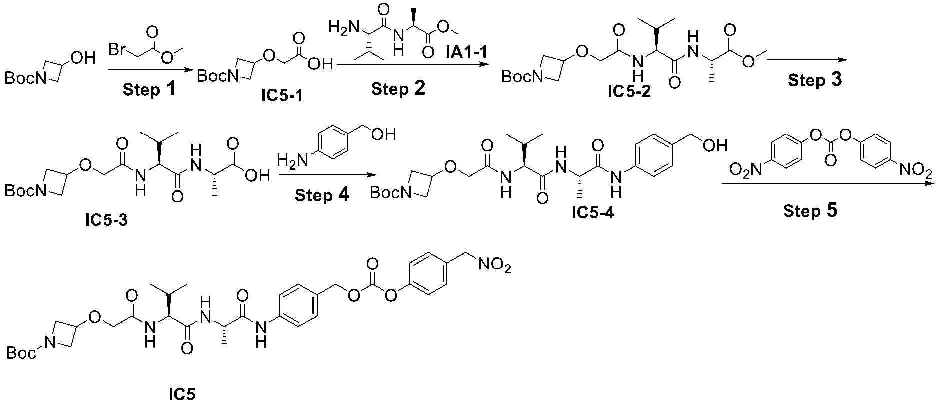

### Step 1: Preparation of compound IC5-1

Under a nitrogen atmosphere, *N*-Boc-3-hydroxyazetidine (2 g, 11.5 mmol) was dissolved in anhydrous tetrahydrofuran (30 mL), and potassium carbonate (3.2 g, 23.1 mmol) was added. The mixture was heated to 40°C and stirred for 30 minutes, followed by the addition of methyl bromoacetate (1.8 g, 11.6 mmol), and the reaction was continued for 2 h. The mixture was then cooled to room temperature, and a 2 N aqueous LiOH solution (30 mL) was added, followed by stirring for another 2 h. After completion of the reaction, methyl *tert-*butyl ether was added. The mixture was stirred and the phases were separated. The organic phase was discarded. The pH of the aqueous phase was adjusted to 5 with a 2 N aqueous hydrochloric acid solution. The organic phase was separated, dried over sodium sulfate, and concentrated under reduced pressure to obtain compound **IC5-1** (2.4 g, yield: 90%), which was directly used in the next step.

### Steps 2 to 5: Preparation of compound IC5

Compound **IC5** was synthesized with reference to the method described in Intermediate Preparation Example 1.

### Intermediate Preparation Example 4: Synthesis of compound IC9

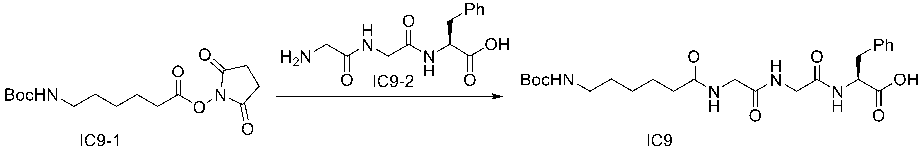

Under a nitrogen atmosphere, IC9-1 (2.0 g, 6.09 mmol) and IC9-2 (1.7 g, 6.09 mmol) were dissolved in anhydrous DMSO (30 mL), and the mixture was stirred at room temperature for 16 h. After completion of the reaction, water was added, and the mixture was extracted with ethyl acetate. The organic phase was washed twice with saturated brine, dried, concentrated, and then purified by column chromatography to obtain compound **IC9** (2 g, yield: 66%).

Referring to the method described in Intermediate Preparation Example 1, Preparation Example 3 or 4, the following intermediates were synthesized using different protected or unprotected peptide chains:

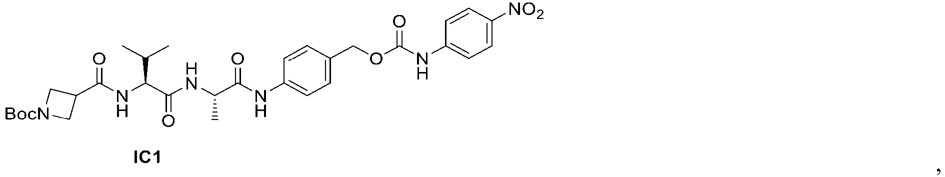

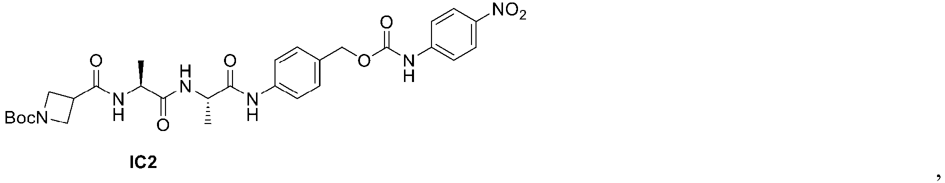

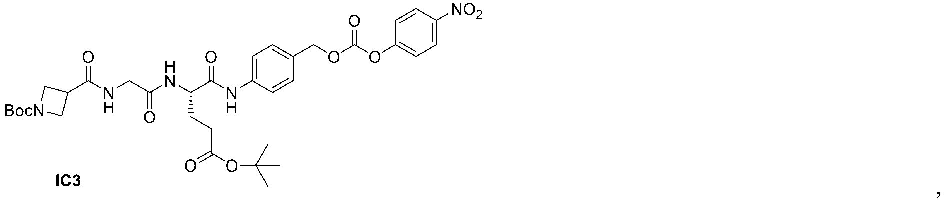

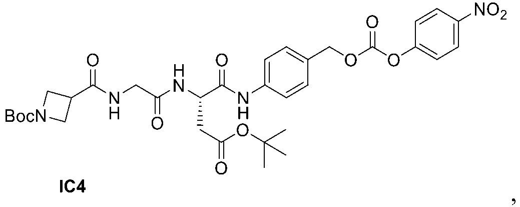

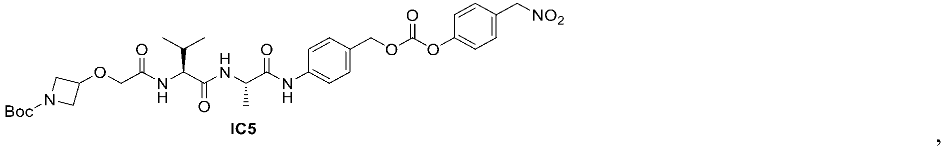

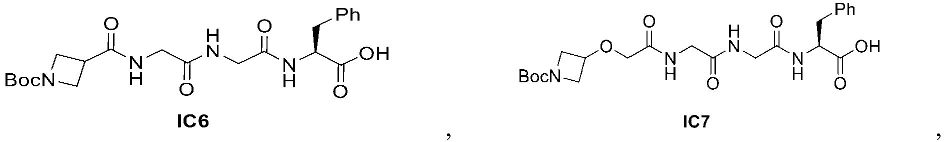

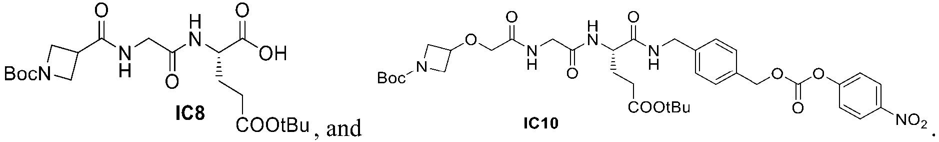

### Intermediate Preparation Example 5:

Intermediates CP1, CP2, and CP3 were synthesized with reference to the method described in WO2022068878A1;

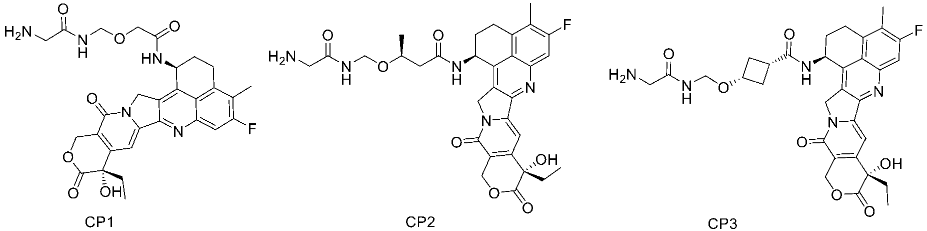

VA-PAB-MMAE was synthesized with reference to the method reported in patent WO2018031690;

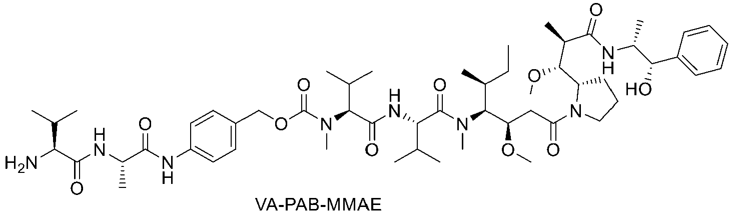

GGFG-AM-DXD was synthesized with reference to the method reported in patent WO2015155998;

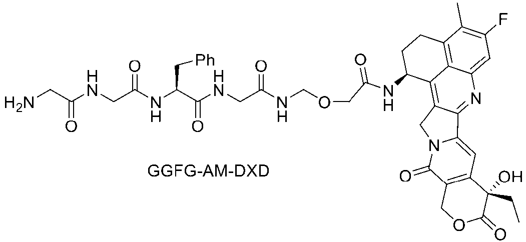

### Example 4.1.1 Preparation of compound A1

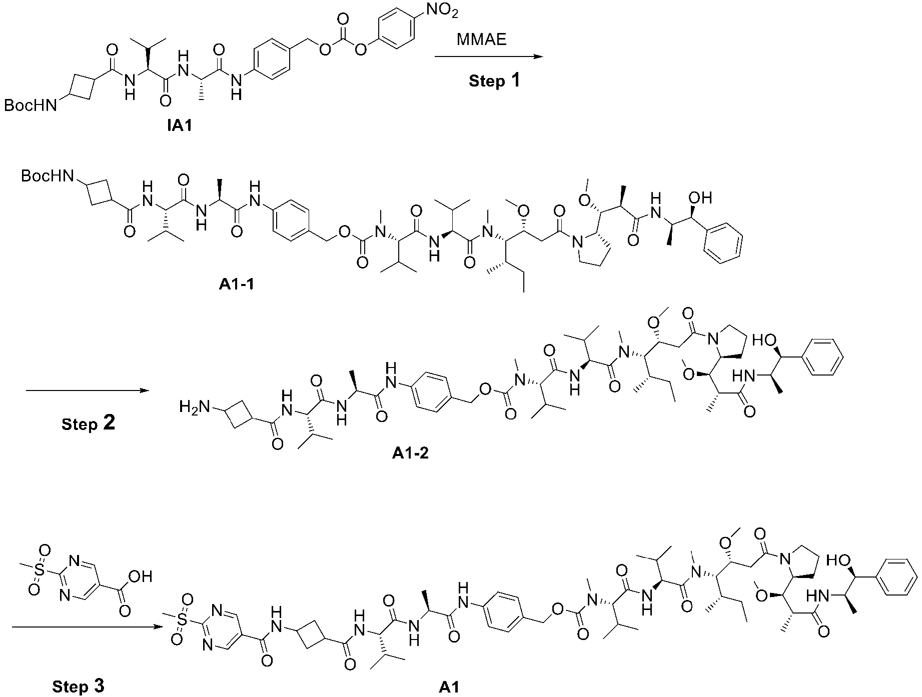

### Step 1: Preparation of compound A1-1

Compound **IA1** (200 mg, 0.31 mmol) was dissolved in DMF (3 mL), followed by the addition of MMAE (201 mg, 0.36 mmol) and HOBT (24 mg, 0.17 mmol). The mixture was stirred at 25°C for 10 minutes, and then DIEA (0.12 mL, 0.69 mmol) was added dropwise. The reaction was continued for 24 h after completion of the addition. After the completion of the reaction was detected by LCMS, water was added, and the mixture was extracted with ethyl acetate. The organic phase was washed with saturated brine, then dried over anhydrous sodium sulfate, and concentrated. The residue was purified by column chromatography to obtain compound **A1-1** (344 mg, yield: 90%).

### Step 2: Preparation of compound A1-2

Compound **A1-1** (344 mg, 0.28 mmol) was dissolved in a dioxane hydrochloride solution (3 mL, 4 M) and stirred at 25°C for 30 minutes. After completion of the reaction, the pH of the reaction mixture was adjusted to 8 with a saturated aqueous sodium bicarbonate solution. The aqueous phase was extracted with ethyl acetate. The organic phase was washed once with saturated brine, dried, concentrated, and then purified by column chromatography to obtain compound **A1-2** (269 mg, yield: 85%).

### Step 3: Preparation of compound A1

2-Methylsulfonyl-5-pyrimidinecarboxylic acid (20 mg, 0.08 mmol) was dissolved in DMF (2 mL), followed by the addition of EDCI (56 mg, 0.3 mmol) and HOBT (26 mg, 0.2 mmol). The mixture was stirred at 25°C for 30 minutes. Then compound **A1-2** (91 mg, 0.08 mmol) was added and stirred for 1 h. After completion of the reaction, water was added to quench the reaction, and the mixture was extracted with ethyl acetate. The organic phase was dried, concentrated, and then purified by reverse-phase HPLC to obtain compound **A1** (20 mg, yield: 22%).

MS (ESI) m/z: 1318.7 [M+H]⁺_{∘}

### Example 4.1.2 Preparation of compound A9

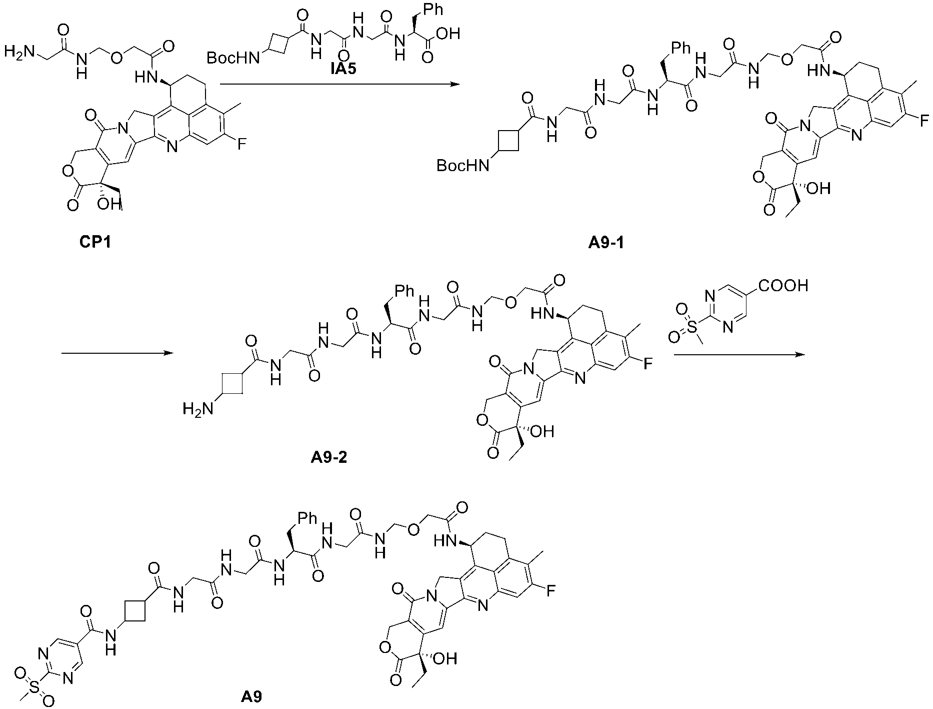

### Step 1: Preparation of compound A9-1

At 25°C, compound IA5 (50.0 mg, 0.105 mmol) was dissolved in DMF (5 mL), followed by the addition of HATU (48.5 mg, 0.126 mmol) and DIEA (39.8 mg, 0.315 mmol). After stirring for 30 minutes, CP1 (60.6 mg, 0.105 mmol) was added, and the reaction was continued for 2 h. After the completion of the reaction was detected by TLC, the mixture was purified by reverse-phase preparative HPLC to obtain compound **A9-1** (71 mg, yield: 65%).

### Step 2: Preparation of compound A9-2

Compound **A9-1** (71 mg, 0.07 mmol) and anhydrous zinc bromide (315 mg, 1.4 mmol) were dissolved in nitromethane (5 mL), and the mixture was stirred at 40°C for 1 h. After completion of the reaction, the mixture was concentrated to dryness under reduced pressure and purified by reverse-phase HPLC to obtain compound **A9-2** (37 mg, yield: 55%).

### Step 3: Preparation of compound A9

2-Methylsulfonyl-5-pyrimidinecarboxylic acid (10 mg, 0.04 mmol) was dissolved in DMF (2 mL), followed by the addition of EDCI (28 mg, 0.15 mmol) and HOBT (13 mg, 0.10 mmol). The mixture was stirred at 25°C for 30 minutes. Then compound **A9-2** (37 mg, 0.08 mmol) was added, and the mixture was stirred for 1 h. After completion of the reaction, water was added to quench the reaction, and the mixture was extracted with ethyl acetate. The organic phase was dried, concentrated, and then purified by reverse-phase HPLC to obtain compound **A9** (5 mg, yield: 11%).

MS (ESI) m/z: 1122.4 [M+H]⁺_{∘}

**The following compounds were synthesized by using different intermediates with reference to methods similar to those described in Examples 4.1.1 and 4.1.2.**

| No. | Structure | MS, [M+H]⁺ |
|---|---|---|
| A2 | 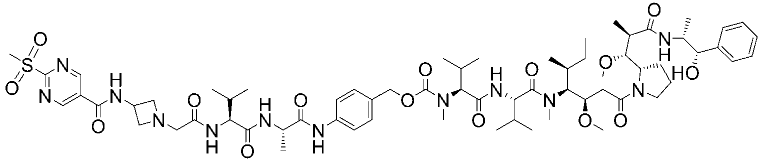 | 1333.7 |
| A3 | 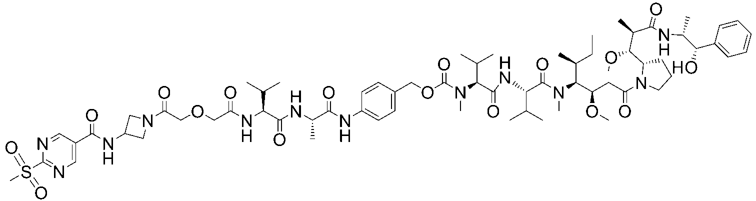 | 1391.7 |
| A6 | 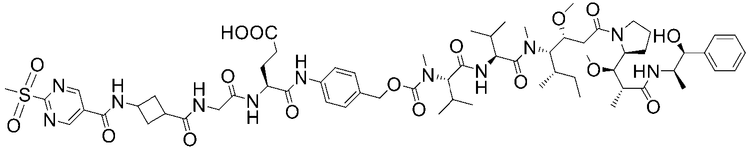 | 1334.7 |
| A7 | 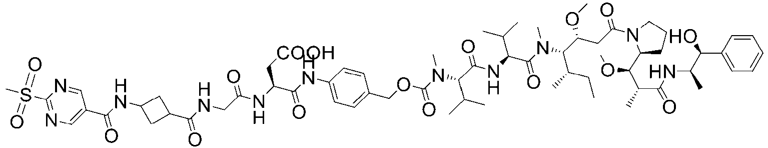 | 1320.6 |
| A10 | 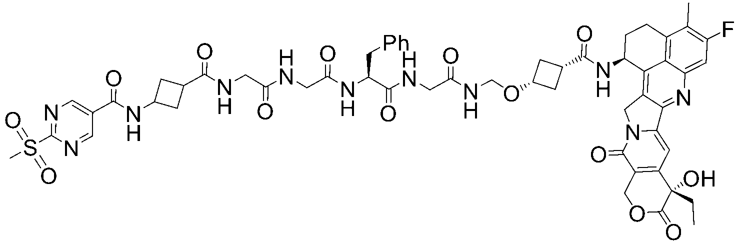 | 1162.4 |
| A11 | 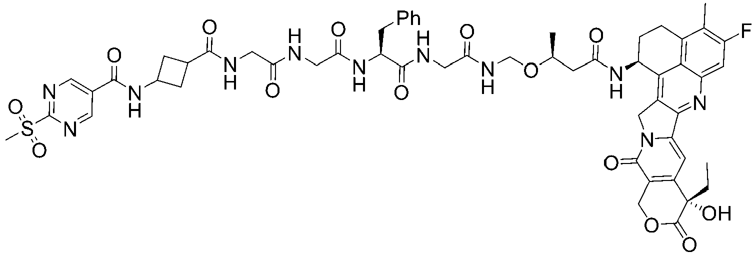 | 1150.4 |
| A12 | 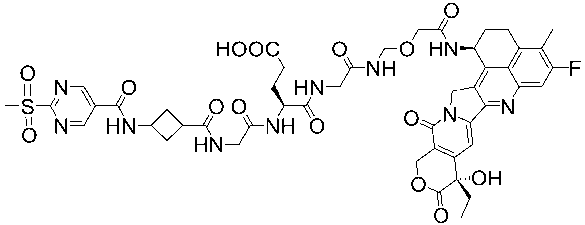 | 1047.3 |
| A13 | 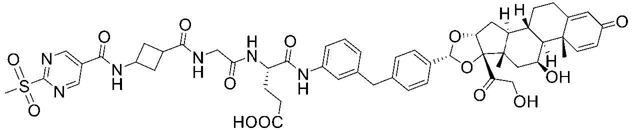 | 1037.4 |

### Example 4.2.1 Preparation of compound B1

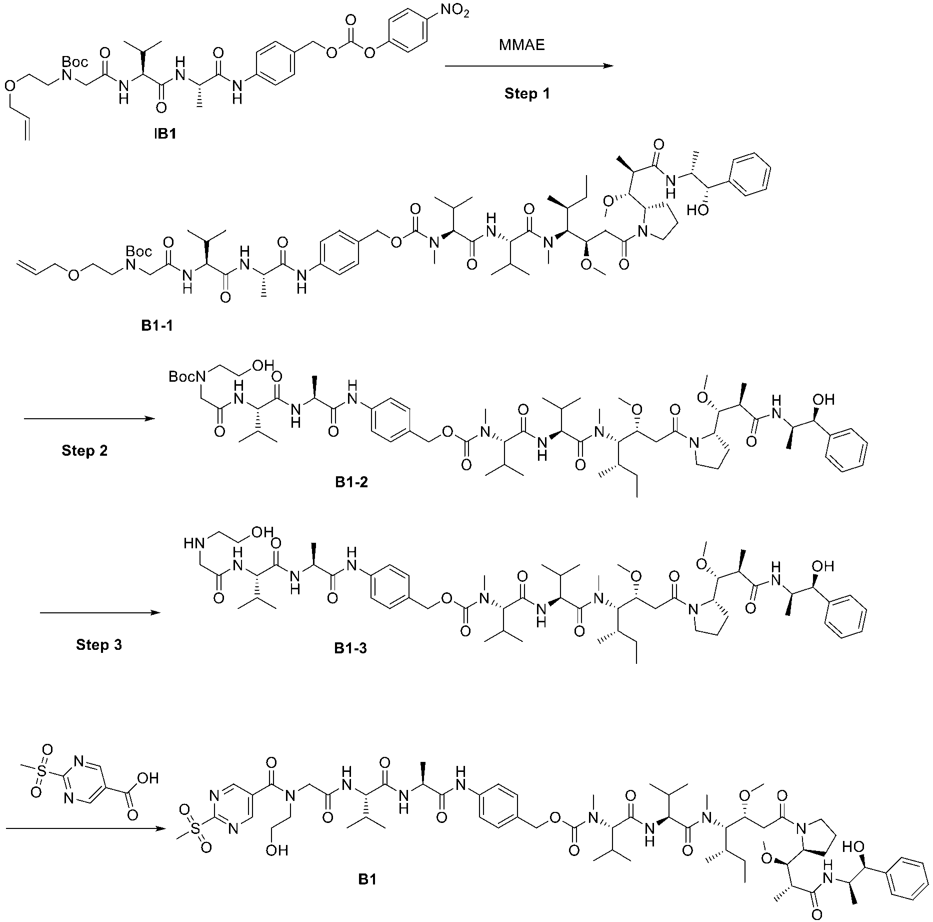

### Step 1: Preparation of compound B1-1

Compound **IB1** (210 mg, 0.30 mmol) was dissolved in DMF (3 mL), followed by the addition of MMAE (201 mg, 0.36 mmol) and HOBT (24 mg, 0.17 mmol). The mixture was stirred at 25°C for 10 minutes, and then DIEA (0.12 mL, 0.69 mmol) was added dropwise. The reaction was continued for 24 h after completion of the addition. After the completion of the reaction was detected by LCMS, water was added, and the mixture was extracted with ethyl acetate. The organic phase was washed with saturated brine, then dried over anhydrous sodium sulfate, and concentrated. The residue was purified by column chromatography to obtain compound **B1-1** (326 mg, yield: 84%).

### Step 2: Preparation of compound B1-2

At 25°C, compound **B1-1** (326.0 mg, 0.25 mmol) was dissolved in anhydrous tetrahydrofuran (5 mL), followed by the addition of *N*-methylmorpholine (52.5 mg, 0.50 mmol) and tetrakis(triphenylphosphine)palladium (28.8 mg, 0.025 mmol). After nitrogen purging, the reaction was continued at room temperature for 2 h. After LCMS showed the completion of the reaction, the reaction mixture was concentrated to dryness. The residue was purified by preparative HPLC to obtain compound **B1-2** (200 mg, yield: 65%).

### Step 3: Preparation of compound B1-3

Compound **B1-2** (200 mg, 0.16 mmmol) was dissolved in a dioxane hydrochloride solution (5 mL, 4 M) and stirred at 25°C for 30 minutes. After completion of the reaction, the pH of the reaction mixture was adjusted to 8 with a saturated aqueous sodium bicarbonate solution. The aqueous phase was extracted with ethyl acetate. The organic phase was washed once with saturated brine, dried, concentrated, and then purified by column chromatography to obtain compound **B1-3** (91 mg, yield: 50%).

### Step 3: Preparation of compound B1

2-Methylsulfonyl-5-pyrimidinecarboxylic acid (20 mg, 0.08 mmmol) was dissolved in DMF (2 mL), followed by the addition of EDCI (56 mg, 0.3 mmol) and HOBT (26 mg, 0.2 mmol). The mixture was stirred at 25°C for 30 minutes. Then compound **B1-3** (91 mg, 0.08 mmol) was added, and the mixture was stirred for 1 h. After completion of the reaction, water was added to quench the reaction, and the mixture was extracted with ethyl acetate. The organic phase was dried, concentrated, and then purified by reverse-phase HPLC to obtain compound **B1** (10 mg, yield: 10%).

MS (ESI) m/z: 1322.7 [M+H]⁺_{∘}

### Example 4.2.2 Preparation of compound B4

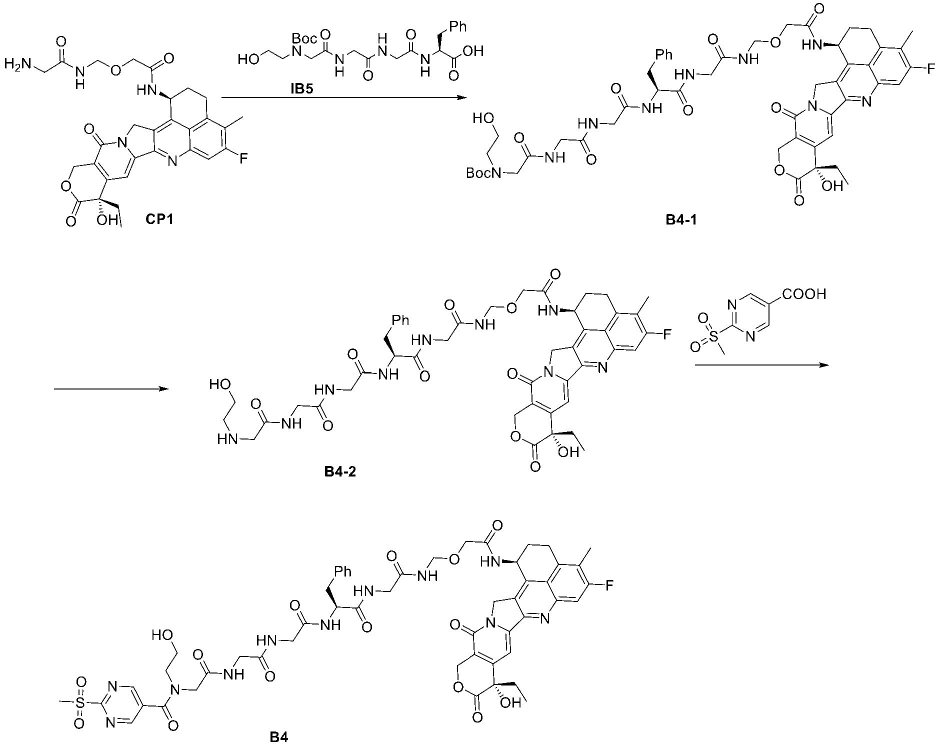

### Step 1: Preparation of compound B4-1

At 25°C, compound **IB5** (100.0 mg, 0.210 mmol) was dissolved in DMF (8 mL), followed by the addition of HATU (97.1 mg, 0.252 mmol) and DIEA (79.6 mg, 0.63 mmol). After stirring for 30 minutes, CP1 (121.2 mg, 0.21 mmol) was added, and the reaction was continued for 2 h. After the completion of the reaction was detected by TLC, the mixture was purified by reverse-phase preparative HPLC to obtain compound **B4-1** (131 mg, yield: 60%).

### Step 2: Preparation of compound B4-2

Compound **B4-1** (131 mg, 0.13 mmol) and anhydrous zinc bromide (584 mg, 2.6 mmol) were dissolved in nitromethane (5 mL), and the mixture was stirred at 40°C for 1 h. After completion of the reaction, the mixture was concentrated to dryness under reduced pressure and purified by reverse-phase HPLC to obtain compound **B4-2** (60 mg, yield: 49%).

### Step 3: Preparation of compound B4

2-Methylsulfonyl-5-pyrimidinecarboxylic acid (13 mg, 0.063 mmmol) was dissolved in DMF (2 mL), followed by the addition of EDCI (36 mg, 0.190 mmol) and HOBT (17 mg, 0.126 mmol). The mixture was stirred at 25°C for 30 minutes. Then compound **B4-2** (60 mg, 0.063 mmol) was added, and the mixture was stirred for 1 h. After completion of the reaction, water was added to quench the reaction, and the mixture was extracted with ethyl acetate. The organic phase was dried, concentrated, and then purified by reverse-phase HPLC to obtain compound **B4** (5 mg, yield: 7%).

MS (ESI) m/z: 1126.4 [M+H]⁺_{∘}

**The following compounds were synthesized by using different intermediates with reference to methods similar to those described in Examples 4.2.1 and 4.2.2.**

| No. | Structure | MS, [M+H]⁺ |
|---|---|---|
| B2 | 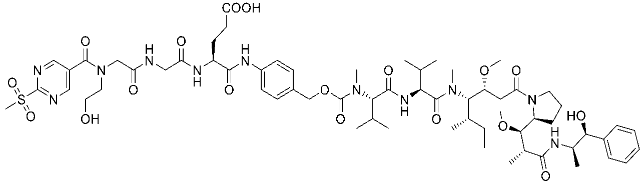 | 1338.7 |
| B3 | 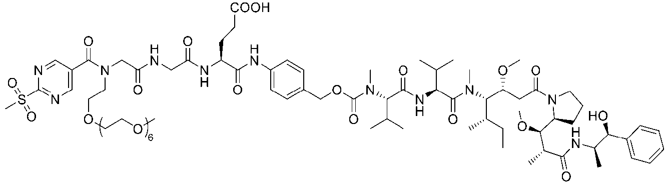 | 1616.8 |
| B5 | 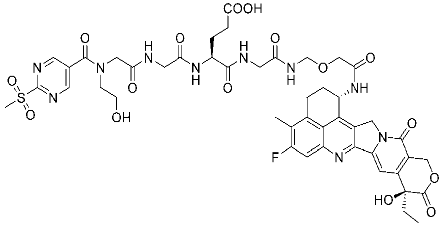 | 1051.3 |
| B6 | 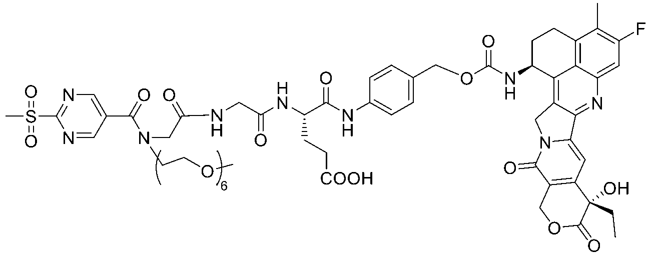 | 1290.5 |
| B7 | 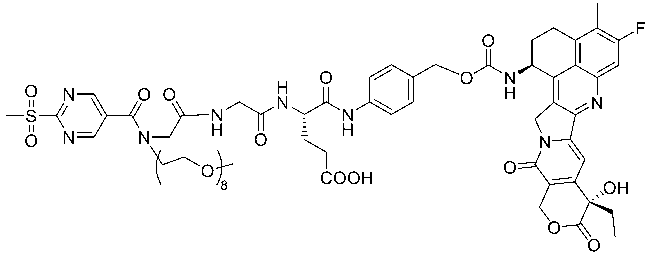 | 1378.5 |
| B8 | 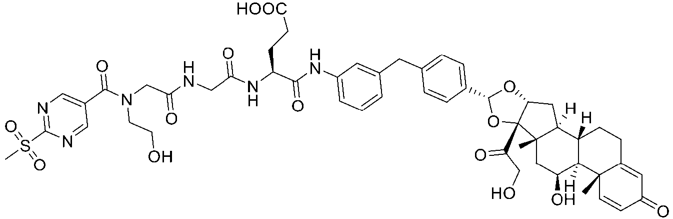 | 1041.4 |
| B11 | 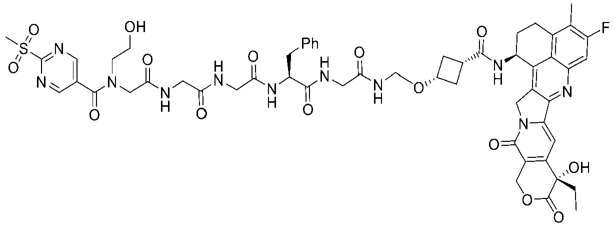 | 1166.4 |
| B12 | 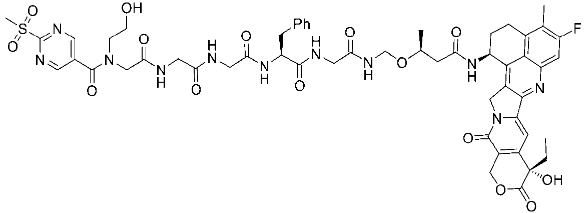 | 1154.4 |

### Example 4.3.1 Preparation of compound C1

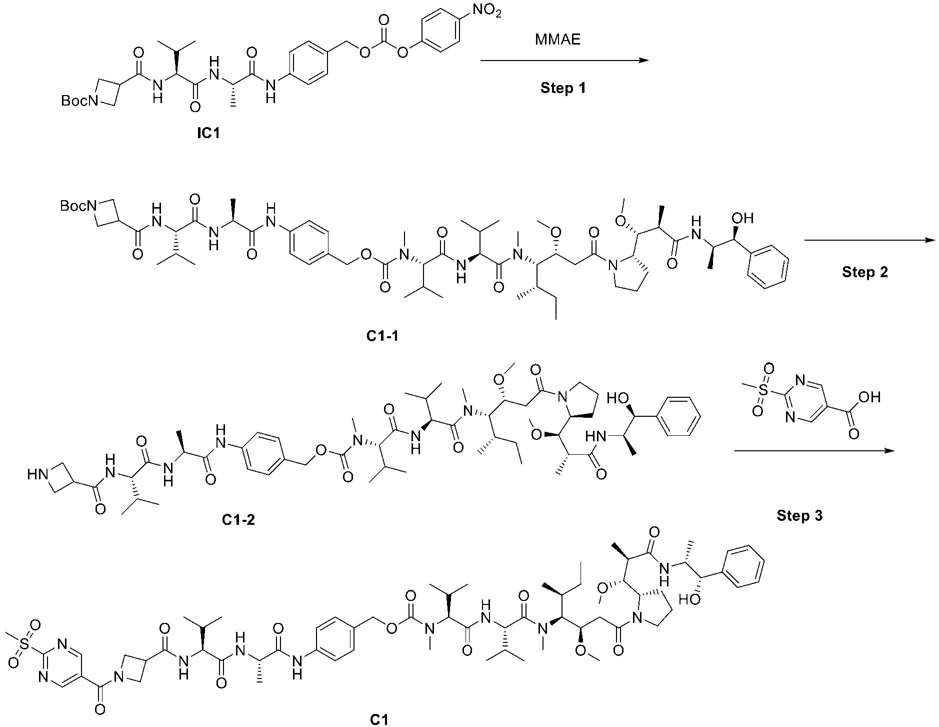

### Step 1: Preparation of compound C1-1

Compound **IC1** (100 mg, 0.17 mmol) was dissolved in DMF (3 mL), followed by the addition of MMAE (110 mg, 0.20 mmol) and HOBT (13 mg, 0.10 mmol). The mixture was stirred at 25°C for 10 minutes, and then DIEA (0.12 mL, 0.69 mmol) was added dropwise. The reaction was continued for 24 h after completion of the addition. After the completion of the reaction was detected by LCMS, water was added, and the mixture was extracted with ethyl acetate. The organic phase was washed with saturated brine, then dried over anhydrous sodium sulfate, and concentrated. The residue was purified by column chromatography to obtain compound **C1-1** (183 mg, yield: 88%).

### Step 2: Preparation of compound C1-2

Compound **C1-1** (183 mg, 0.15 mmmol) was dissolved in a dioxane hydrochloride solution (3 mL, 4 M) and stirred at 25°C for 30 minutes. After completion of the reaction, the pH of the reaction mixture was adjusted to 8 with a saturated aqueous sodium bicarbonate solution. The aqueous phase was extracted with ethyl acetate. The organic phase was washed once with saturated brine, dried, concentrated, and then purified by column chromatography to obtain compound **C1-2** (143 mg, yield: 85%).

### Step 3: Preparation of compound C1

2-Methylsulfonyl-5-pyrimidinecarboxylic acid (20 mg, 0.08 mmmol) was dissolved in DMF (2 mL), followed by the addition of EDCI (56 mg, 0.3 mmol) and HOBT (26 mg, 0.2 mmol). The mixture was stirred at 25°C for 30 minutes. Then compound **C1-2** (90 mg, 0.08 mmol) was added, and the mixture was stirred for 1 h. After completion of the reaction, water was added to quench the reaction, and the mixture was extracted with ethyl acetate. The organic phase was dried, concentrated, and then purified by reverse-phase HPLC to obtain compound **C1** (11 mg, yield: 10%).

MS (ESI) m/z: 1304.7 [M+H]⁺_{∘}

### Example 4.3.2 Preparation of compound C11

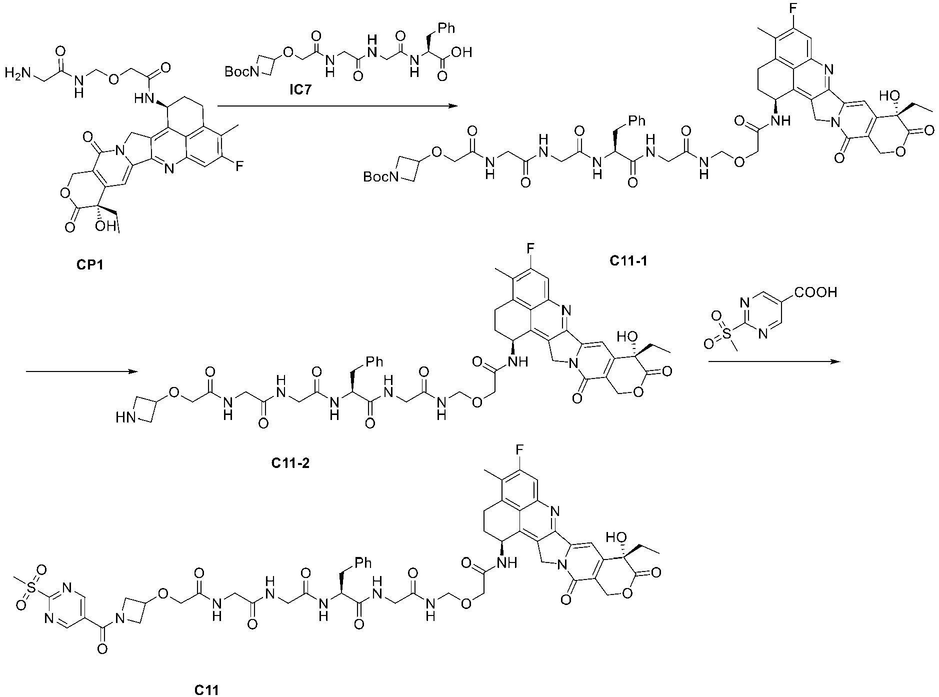

### Step 1: Preparation of compound C11-1

At 25°C, compound IC7 (100.0 mg, 0.203 mmol) was dissolved in DMF (8 mL), followed by the addition of HATU (93.8 mg, 0.244 mmol) and DIEA (77.0 mg, 0.609 mmol). After stirring for 30 minutes, CP1 (117.2 mg, 0.203 mmol) was added, and the reaction was continued for 2 h. After the completion of the reaction was detected by TLC, the mixture was purified by reverse-phase preparative HPLC to obtain compound **C11-1** (154 mg, yield: 72%).

### Step 2: Preparation of compound C11-2

Compound **C11-1** (154 mg, 0.15 mmol) and anhydrous zinc bromide (584 mg, 2.6 mmol) were dissolved in nitromethane (5 mL). After completion of the reaction, the mixture was concentrated to dryness under reduced pressure and purified by reverse-phase HPLC to obtain compound **C11-2** (64 mg, yield: 45%).

### Step 3: Preparation of compound C11

2-Methylsulfonyl-5-pyrimidinecarboxylic acid (13 mg, 0.063 mmol) was dissolved in DMF (2 mL), followed by the addition of EDCI (36 mg, 0.190 mmol) and HOBT (17 mg, 0.126 mmol). The mixture was stirred at 25°C for 30 minutes. Then compound **C11-2** (60 mg, 0.063 mmol) was added, and the mixture was stirred for 1 h. After completion of the reaction, water was added to quench the reaction, and the mixture was extracted with ethyl acetate. The organic phase was dried, concentrated, and then purified by reverse-phase HPLC to obtain compound **C11** (4 mg, yield: 5.6%).

MS (ESI) m/z: 1138.4 [M+H]⁺_{∘}

### Example 4.3.3 Preparation of compound C34

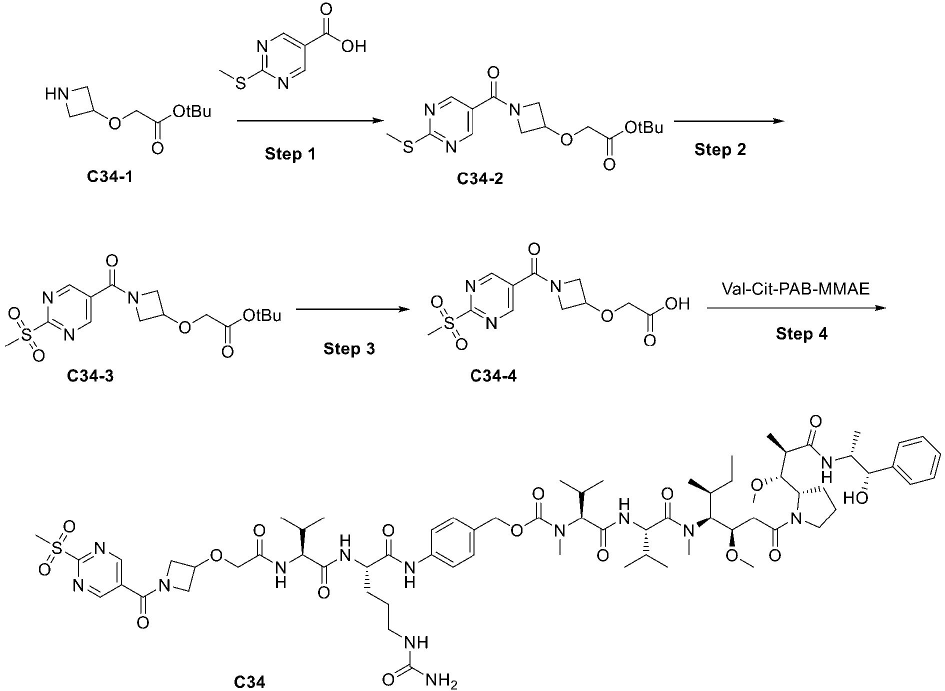

### Step 1: Preparation of compound C34-2

Under a nitrogen atmosphere, DIEA (510.5 mg, 3.96 mmol) was added to a solution of **C34-1** (370 mg, 1.98 mol), 2-methylthiopyrimidine-5-carboxylic acid (336.4 mg, 1.98 mmol), and HATU (1.13 g, 2.97 mmol) in DMF (5 mL). The reaction mixture was stirred at room temperature for 1 h. Saturated brine was added to the reaction mixture, and the mixture was extracted with ethyl acetate. The organic phase was dried over anhydrous sodium sulfate, filtered, and subjected to rotary evaporation until dryness to obtain a mixture. The mixture was purified by column chromatography to obtain compound **C34-2** (400 mg, yield: 74%).

### Step 2: Preparation of compound C34-3

To a solution of compound **C34-2** (360 mg, 1.06 mmol) in tetrahydrofuran (20 mL) was added an aqueous solution (20 mL) of Oxone (1.96 g, 3.08 mmol). The reaction mixture was stirred at room temperature for 1 h under a nitrogen atmosphere. After the completion of the reaction was detected by LCMS, water and ethyl acetate were added to the reaction mixture, followed by stirring and phase separation. The organic phase was separated, washed with saturated brine, dried over anhydrous sodium sulfate, and concentrated under reduced pressure to obtain compound **C34-3** (270 mg, yield: 68%).

### Step 3: Preparation of compound C34-4

Compound **C34-3** (100 mg, 0.26 mmol) was dissolved in 4 mL of anhydrous dichloromethane at room temperature under a nitrogen atmosphere, followed by the addition of 1.5 mL of trifluoroacetic acid with stirring. The mixture was stirred for another 1 h. The reaction mixture was then subjected to rotary evaporation until dryness. The residue was purified by reversed-phase HPLC to obtain compound **C34-3** (30 mg, yield: 35%).

MS (ESI) m/z: 316.1 [M+H]⁺_{∘}

¹H NMR (400 MHz, CDCl₃) δ 9.24 (s, 2H), 4.67 - 4.51 (m, 4H), 4.36 - 4.23 (m, 3H), 3.48 (s, 3H).

### Step 4: Preparation of compound C34

A solution of compound **C34-4** (130 mg, 0.4127 mmol), HOBt (55.7 mg, 0.4127 mmol), EDCI (79.11 mg, 0.4127 mmol), and **Val-Cit-PAB-MMAE** (100 mg, 0.1 mmol) in DMF (1 mL) was stirred at room temperature for 1 h. The reaction mixture was purified by reverse-phase HPLC to obtain compound **C34** (16.9 mg, yield: 15%).

MS (ESI) m/z: 1420.5 [M+H]⁺_{∘}

¹H NMR (400 MHz, DMSO-*d*₆) δ 10.04 (s, 1H), 9.27 (s, 2H), 8.33 (d, *J =* 7.4 Hz, 1H), 8.13 - 7.84 (m, 1H), 7.74 - 7.47 (m, 4H), 7.40 - 7.15 (m, 7H), 5.99 (s, 1H), 5.43 (s, 3H), 5.14 - 4.93 (m, 2H), 4.80 - 4.21 (m, 8H), 4.02 - 3.91 (m, 4H), 3.45 (s, 6H), 3.28 - 3.10 (m, 10H), 3.09 - 2.81 (m, 7H), 2.41 (d, *J =* 16.1 Hz, 1H), 2.32 - 2.21 (m, 1H), 2.15 - 1.91 (m,4H), 1.84 - 1.23 (m, 9H), 1.05 - 0.96 (m, 6H), 0.95 - 0.69 (m, 25H).

### Example 4.3.4 Preparation of compound C23

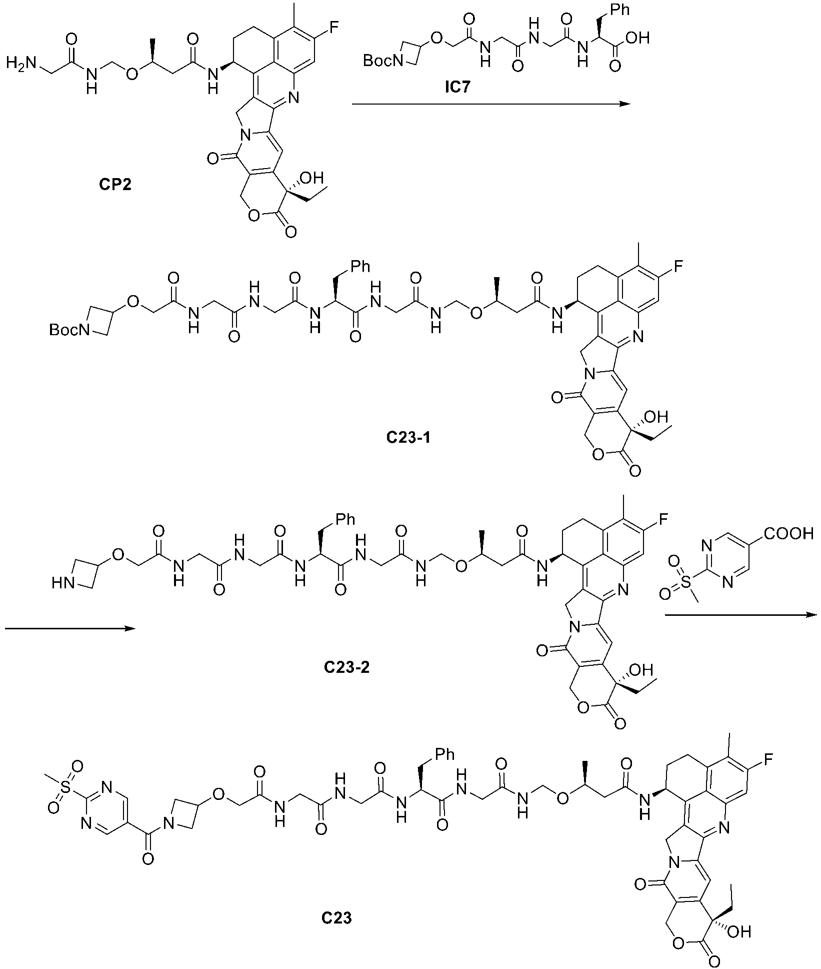

**Compound C23 was synthesized with reference to a method similar to that described in Example 4.3.2**

MS (ESI) m/z: 1166.3 [M+H]⁺_{∘}

¹H NMR (400 MHz, DMSO-*d*₆) δ 9.26 (s, 2H), 8.53 - 8.44 (m, 2H), 8.29 (t,*J* = 5.5 Hz, 1H), 8.14 - 8.05 (m, 3H), 7.80 (d, *J=* 11.0 Hz, 1H), 7.33 (s, 1H), 7.24 - 7.12 (m, 5H), 6.53 (s, 1H), 5.59 - 5.54 (m, 1H), 5.43 (d, *J* = 3.8 Hz, 2H), 5.31- 5.14 (m, 2H), 4.66 - 4.46 (m, 5H), 4.39 - 4.29 (m, 2H), 4.05 - 3.98 (m, 2H), 3.94 (s, 2H), 3.77 - 3.69 (m, 5H), 3.60 (dd, *J =* 16.7, 5.5 Hz, 1H), 3.45 (s, 3H), 3.17 - 3.14 (m, 2H), 3.02 (dd, *J =* 13.7, 4.4 Hz, 1H), 2.78 - 2.72 (m, 1H), 2.41 (s, 3H), 2.37 - 2.32 (m, 1H), 2.23 (dd, *J =* 14.0, 6.0 Hz, 1H), 2.15 - 2.07 (m, 2H), 1.91 - 1.79 (m, 2H), 1.12 (d, *J=* 6.1 Hz, 3H), 0.87 (t, *J=* 7.3 Hz, 3H).

### Example 4.3.5 Preparation of compound C22

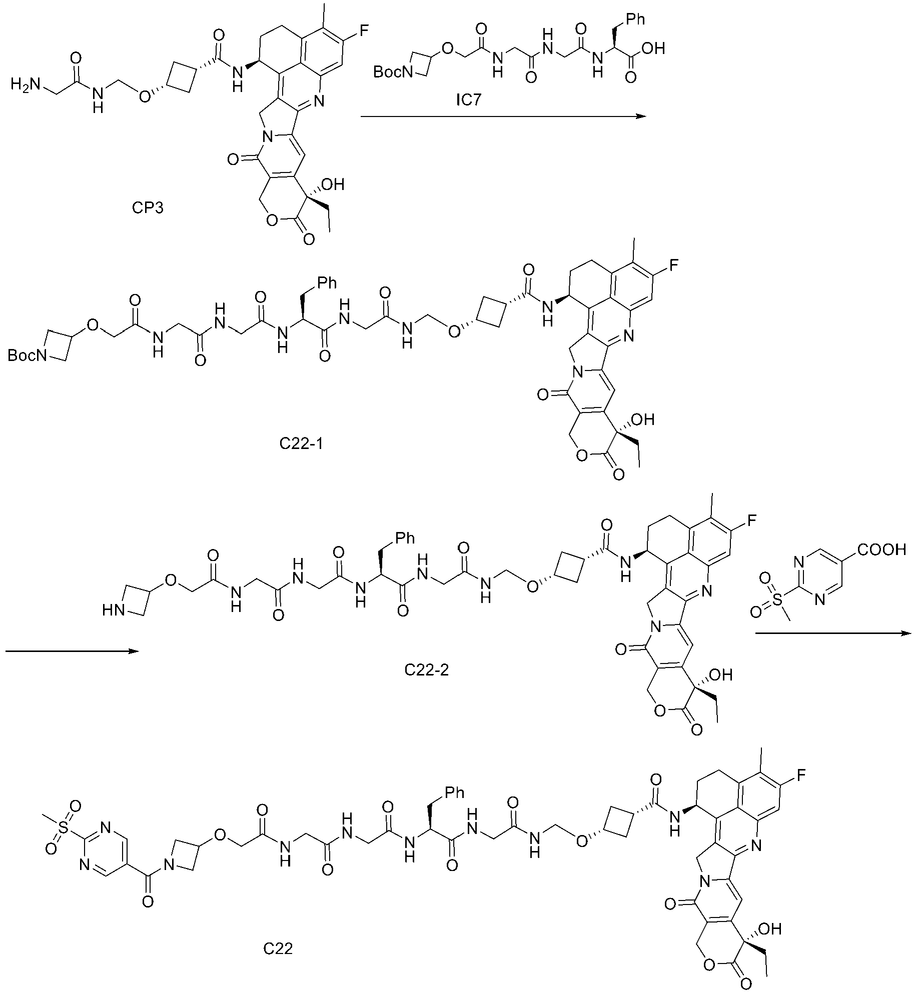

**Compound C22** was synthesized with reference to a method similar to that described in **Example 4.3.2**

MS (ESI) m/z: 1179.3 [M+H]⁺ₒ

¹H NMR (400 MHz, DMSO-*d*₆) δ 9.28 (s, 2H), 8.47 (d, *J* = 7.1 Hz, 2H),8.32 (t,*J* = 5.5 Hz, 1H), 8.19 - 8.07 (m, 3H), 7.81 (d, *J* = 11.0 Hz, 1H), 7.32 (s, 1H), 7.25 - 7.16 (m, 5H), 6.54 (s, 1H), 5.60 - 5.54 (m, 1H), 5.43 (d, *J =* 3.8 Hz, 2H), 5.15 (d, *J =* 5.4 Hz, 2H), 4.61 - 4.47 (m, 5H), 4.43 - 4.31 (m, 2H), 4.05 - 4.01 (m, 2H), 3.96 (s, 2H), 3.94 - 3.92 (m, 1H), 3.80 - 3.59 (m, 6H), 3.47 (s, 3H), 3.22 - 3.14 (m, 2H), 3.09 - 3.04 (m, 1H), 2.84 - 2.77 (m, 1H), 2.62 - 2.57 (m, 1H), 2.41 (s, 3H), 2.38 - 2.33 (m, 1H), 2.20 - 2.07 (m, 4H), 1.91 - 1.81 (m, 2H), 0.89 (t, *J =* 7.3 Hz, 3H).

**The following compounds were synthesized by using different intermediates with reference to methods similar to those described in Examples 4.3.1 and 4.3.2.**

| No. | Structure | MS, [M+H]⁺ |
|---|---|---|
| C2 | 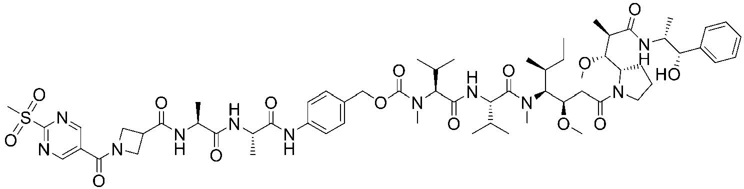 | 1276.7 |
| C3 | 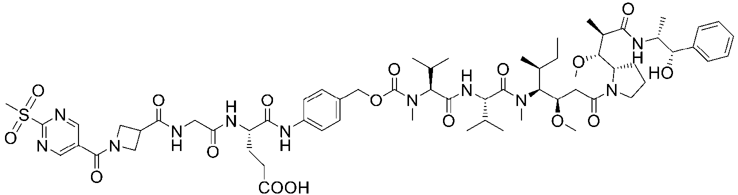 | 1320.6 |
| C4 | 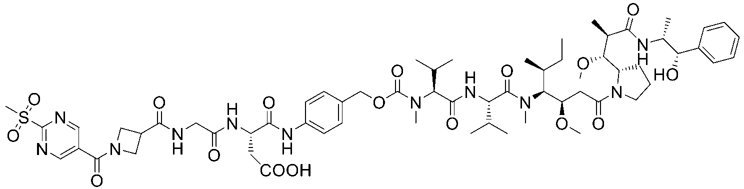 | 1306.6 |
| C7 | 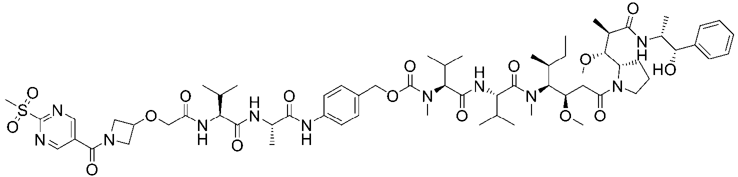 | 1334.7 |
| C10 | 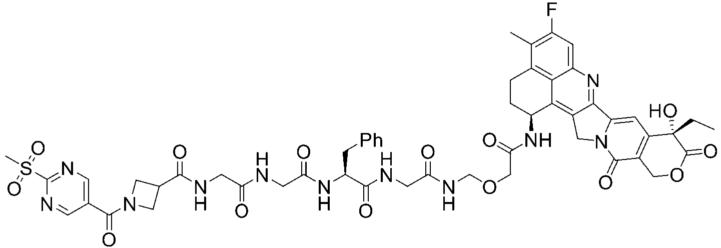 | 1108.4 |
| C12 | 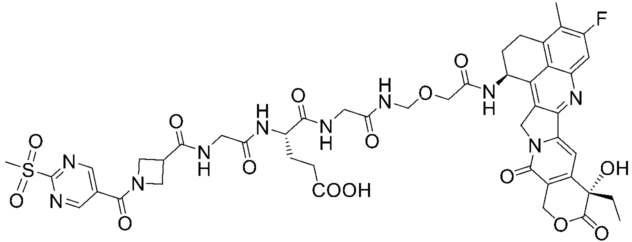 | 1033.3 |
| C25 | 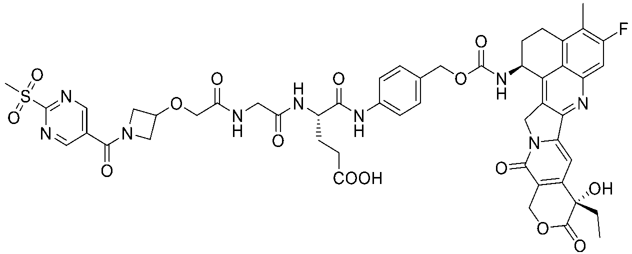 | 1068.3 |

### Example 4.3.6 Preparation of control compound D1

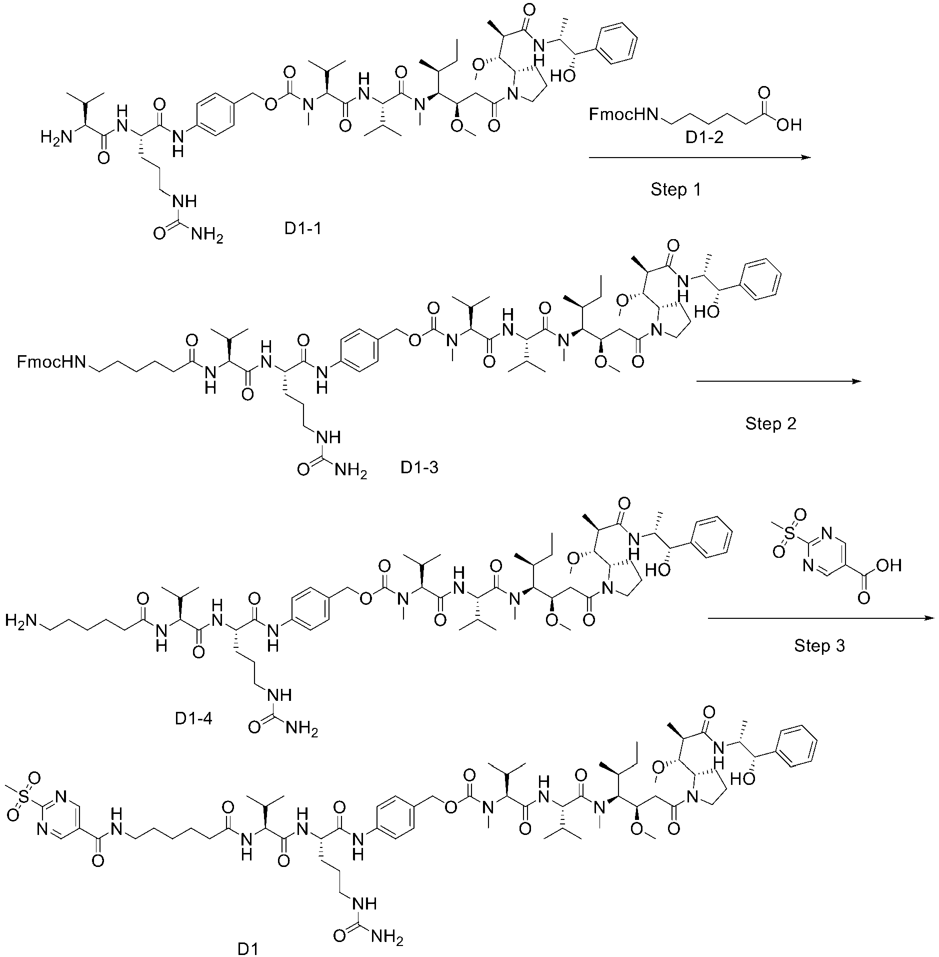

### Step 1: Preparation of compound D1-3

Compound **D1-1** (100 mg, 0.04 mmol), HATU (22.80 mg, 0.06 mmol), and DIEA (34.83 mg, 0.27 mmol) were dissolved in 3 mL of DMF at room temperature. After stirring for 5 minutes, compound **D1-2** (50 mg, 0.04 mmol) was added. After completion of the addition, stirring was continued for 2 h. After completion of the reaction, the mixture was concentrated under reduced pressure. The residue was purified by reverse-phase column chromatography to obtain compound **D1-3** (50.2 mg, yield: 77%).

### Step 2: Preparation of compound D1-4

Compound **D1-3** (50 mg, 0.03 mmol) was dissolved in anhydrous dichloromethane (2 mL), cooled to 0°C, and then piperidine (0.5 mL) was added. The reaction was continued at this temperature for 1 h. After completion of the reaction, the mixture was concentrated under reduced pressure. The residue was purified by reverse-phase column chromatography to obtain compound **D1-4** (30 mg, yield: 71%).

### Step 3: Preparation of control compound D1

Compound 2-methylthiopyrimidine-5-carboxylic acid (4 mg, 0.02 mmol), HATU (11.40 mg, 0.03 mmol) and DIEA (8.0 mg, 0.06 mmol) were dissolved in 2 mL of anhydrous DMF at room temperature. After stirring for 15 minutes, compound **D1-4** (96.83 mg, 0.06 mmol) was added. After completion of the addition, stirring was continued for 2 h. After completion of the reaction, the mixture was concentrated under reduced pressure. The residue was purified by reverse-phase HPLC to obtain compound **D1** (13.9 mg, yield: 40%).

MS (ESI) m/z: 1421.6[M+H]⁺_{∘}

¹H NMR (400 MHz, DMSO-*d*₆) δ 9.99 (s, 1H), 9.36 (s, 2H), 8.99 (t, *J =* 5.5 Hz, 1H), 8.12 - 8.01 (m, 2H), 7.90 - 7.81 (m, 2H), 7.61 - 7.57 (m, 2H), 7.34 - 7.22 (m, 6H), 7.22 - 7.13 (m, 1H), 5.98 (t, *J =* 5.8 Hz, 1H), 5.42 - 5.34 (m, 3H), 5.10 - 4.92 (m, 2H), 4.78 - 4.59 (m, 1H), 4.52 - 4.33 (m, 3H), 4.31 - 4.16 (m, 2H), 4.04 - 3.91 (m, 2H), 3.62 - 3.51 (m, 1H), 3.45 (s, 3H), 3.31 (d, *J* = 6.3 Hz, 2H), 3.28 - 3.11 (m, 7H), 3.08 - 2.79 (m, 7H), 2.43 - 2.41 (m, 1H), 2.28 - 1.90 (m, 7H), 1.81 - 1.64 (m, 4H), 1.58 - 1.47 (m, 6H), 1.35 - 1.27 (m, 4H), 1.07 - 0.95 (m, 6H), 0.91 - 0.71 (m, 25H).

### Example 4.3.7 Preparation of control compound D2

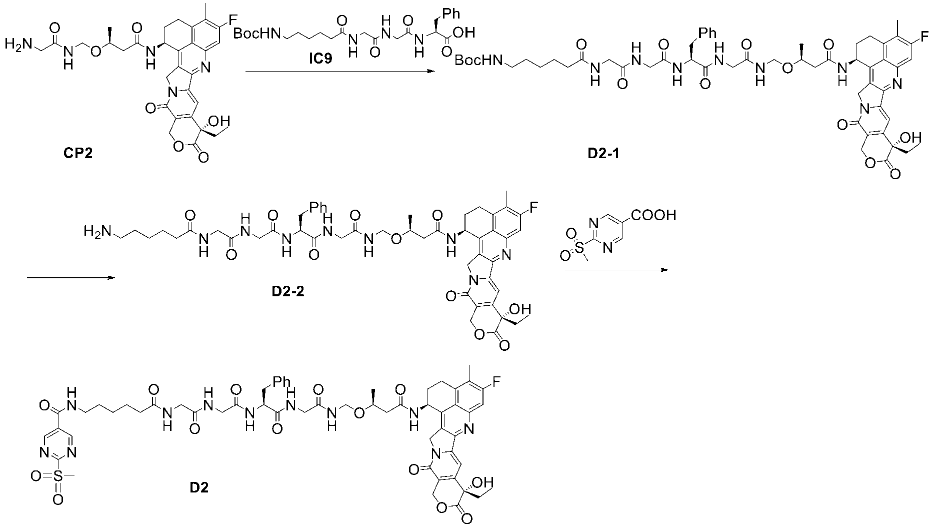

**Control compound 2 was synthesized with reference to a method similar to that described in Example 4.3.2**

MS (ESI) m/z: 1166.3 [M+H]⁺_{∘}

¹H NMR (400 MHz, DMSO-*d*₆) δ 9.35 (s, 2H), 8.98 (t, *J =* 5.6 Hz, 1H), 8.49 (t,*J* = 9.1 Hz, 2H), 8.30 (t, *J* = 5.5 Hz, 1H), 8.18 - 7.92 (m, 3H), 7.81 (d, *J* = 11.0 Hz, 1H), 7.33 (s, 1H), 7.25 - 7.05 (m, 5H), 6.55 (s, 1H), 5.64 - 5.52 (m, 1H), 5.52 - 5.35 (m, 2H), 5.23 (dd, *J =* 42.2, 19.0 Hz, 2H), 4.71 - 4.41 (m, 3H), 4.03 (dd, *J =* 12.7, 6.2 Hz, 1H), 3.80 - 3.51 (m, 6H), 3.45 (s, 3H), 3.31 - 3.24 (m, 2H), 3.16 (d, *J =* 5.5 Hz, 2H), 3.02 (dd, *J =* 13.7, 4.4 Hz, 1H), 2.84 - 2.66 (m, 1H), 2.43 - 2.31 (m, 4H), 2.23 (dd, *J =* 14.0, 6.0 Hz, 1H), 2.14 (t, *J =* 7.4 Hz, 4H), 1.85 (td, *J* = 14.3, 7.0 Hz, 2H), 1.53 (dd, *J* = 13.7, 7.1 Hz, 4H), 1.41 - 1.24 (m, 2H), 1.12 (d, *J=* 6.1 Hz, 3H), 0.87 (t, *J=* 7.3 Hz, 3H).

### Example 5: Preparation of ligand-drug conjugate

The antibody was prepared according to conventional methods. For example, after vector construction, eukaryotic cells such as HEK293 cells and CHO cells could be transfected for purification and expression. Trastuzumab, Sacituzumab, and anti-GPC-3 antibody DB1002 (prepared with reference to WO2006006693) were used as examples to prepare the ligand-drug conjugate.

### Amino acid sequence of Trastuzumab:

Light chain (SEQ ID NO: 1) 
DIQMTQSPSSLSASVGDRVTITCRASQDVNTAVAWYQQKPGKAPKLLIYSASF
LYSGVPSRFSGSRSGTDFTLTISSLQPEDFATYYCQQHYTTPPTFGQGTKVEIKRTVAAP
SVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC
Heavy chain (SEQ ID NO: 2)

EVQLVESGGGLVQPGGSLRLSCAASGFNIKDTYIHWVRQAPGKGLEWVARI
YPTNGYTRYADSVKGRFTISADTSKNTAYLQMNSLRAEDTAVYYCSRWGGDGFYAM
DYWGQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKS
CDKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIE
KTISKAKGQPREPQVYTLPPSREEMTKN

### Amino acid sequence of Sacituzumab:

Light chain (SEQ ID NO: 3) 
DIQLTQSPSSLSASVGDRVSITCKASQDVSIAVAWYQQKPGKAPKLLIYSASY
RYTGVPDRFSGSGSGTDFTLTISSLQPEDFAVYYCQQHYITPLTFGAGTKVEIKRTVAA
PSVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC
Heavy chain (SEQ ID NO: 4)

QVQLQQSGSELKKPGASVKVSCKASGYTFTNYGMNWVKQAPGQGLKWM
GWINTYTGEPTYTDDFKGRFAFSLDTSVSTAYLQISSLKADDTAVYFCARGGFGSSYW
YFDVWGQGSLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKRVEP
KSCDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQP
ENNYKTTPPVLDSDGSFFLYSKLTVDKSRWQQGNVFSCSVMHEALHNHYTQKSLSLS
PGK

### Amino acid sequence of anti-GPC-3 antibody DB1002

Light chain (SEQ ID NO: 5) 
DVVMTQSPLSLPVTPGEPASISCRSSQSLVHSNRNTYLHWYLQKPGQSPQLLI
YKVSNRFSGVPDRFSGSGSGTDFTLKISRVEAEDVGVYYCSQNTHVPPTFGQGTKLEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
Heavy chain (SEQ ID NO: 6)

QVQLVQSGAEVKKPGASVKVSCKASGYTFTDYEMHWVRQAPGQGLEWMG
ALDPKTGDTAYSQKFKGRVTLTADKSTSTAYMELSSLTSEDTAVYYCTRFYSYTYWG
QGTLVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTH
TCPPCPAPELLGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTISKA
KGQPREPQVYTLPPSRDELTKNQVSLTCLVKGFYPSDIAVEWESNGQPENNYKTTPPV
LDSDGSFFLYSKLTVDKSRWQQGNVFSCSVMHEALHNHYTQKSLSLSPGK

### Example 5.1: Preparation method of ADAM9 antibody DB1001

The murine anti-ADAM9 monoclonal antibody MAB-A antibody (heavy chain variable region SEQ ID NO: 7 and light chain variable region SEQ ID NO: 11 sequences disclosed in patent WO2018119196A1) was rehumanized to improve the solubility of the humanized version of the antibody, reduce the hydrophobicity of the antibody, thereby improving its conjugation yield and druggability as an ADC drug. The humanized version antibody hMAB-A (2I.2) of MAB-A (sequence information of heavy chain SEQ ID NO: 52 and light chain SEQ ID NO: 68 disclosed in Patent WO2018119196A1), which entered the clinical research phase, exhibited issues with strong hydrophobicity.

The rehumanization of the murine antibody was performed according to methods disclosed in the literature of this field, that is, the murine antibody constant domains were replaced by human constant domains, and the CDR regions were modified for the purpose of optimizing affinity and druggability. The MAB-A antibody was rehumanized by selecting human germline antibody sequences based on the homology between the parental murine MAB-A antibody of hMAB-A (2I.2) and human antibodies. The specific method was: comparing the variable region sequences of the heavy and light chains with a human antibody germline database based on the typical structure of the VH/VL CDRs of the murine antibody MAB-A to obtain highly homologous human germline templates; grafting the CDR regions of the murine antibody onto the selected corresponding humanized templates; subsequently, based on the three-dimensional structure of the murine antibody, performing back mutations on embedded residues, residues directly interacting with the CDR regions, and residues significantly influencing the conformation of VL and VH, as well as optimizing chemically unstable amino acid residues in the CDR regions; and through expression testing and comparison of the number of back mutations, selecting the designed antibody composed of the humanized heavy chain variable region HCVR and light chain variable region LCVR sequences. The amino acid sequences of the CDRs of the humanized antibody and the variable region sequences of the heavy and light chains of the humanized antibody were as follows:
VH CDR1 SEQ ID NO: 7
LYWMH
VH CDR2 SEQ ID NO: 8
RIIPIFGHTKYNEKFKN
VH CDR3 SEQ ID NO: 9
GGYYYYPREGFLDY
VL CDR1 SEQ ID NO: 10
RSSQSVDYSGDSYMN
VL CDR2 SEQ ID NO: 11
AASDRES
VL CDR3 SEQ ID NO: 12
QQSREDPFT
Heavy chain variable region: SEQ ID NO: 13 
QVQLQQPGTEVVKPGASVKLSCKASGFTFPLYWMHWVRQRPGRGLEWIGR
IIPIFGHTKYNEKFKNKATLTADESSSTAYMELRSLTSDDSAVYYCARGGYYYYPREGF
LDYWGQGTSVTVSS
Light chain variable region: SEQ ID NO: 14

DIVMTQSPASLSVSPGERASISCRSSQSVDYSGDSYMNWYQQKPGQSPRLLI

YAASDRESGVPDRFSGSGSGTDFTLKISRVEEEDAGTYYCQQSREDPFTFGQGTKVDI

K

The designed heavy chain and light chain variable region sequences were respectively connected to the heavy chain constant region and light chain constant region sequences of a human antibody, finally resulting in the anti-ADAM9 antibody DB1001.

Exemplarily, the antibody heavy chain constant region was selected from the human IgG1 heavy chain constant region with the sequence as shown in SEQ ID NO: 15; the antibody light chain constant region was selected from the constant region of the human κ chain with the sequence as shown in SEQ ID NO: 16. The antibody constant region sequences are shown below.
Human IgG1 heavy chain constant region SEQ ID NO: 15
Human light chain κ chain constant region SEQ ID NO: 16

### Amino acid sequence of anti-ADAM9 antibody DB1001

**Light chain (SEQ ID NO: 17)** 
DIVMTQSPASLSVSPGERASISCRSSQSVDYSGDSYMNWYQQKPGQSPRLLI
YAASDRESGVPDRFSGSGSGTDFTLKISRVEEEDAGTYYCQQSREDPFTFGQGTKVDI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
**Heavy chain (SEQ ID NO: 18)**

QVQLQQPGTEVVKPGASVKLSCKASGFTFPLYWMHWVRQRPGRGLEWIGR
IIPIFGHTKYNEKFKNKATLTADESSSTAYMELRSLTSDDSAVYYCARGGYYYYPREGF
LDYWGQGTSVTVSSASTKGPSVFPLAPSSKSTSGGTAALGCLVKDYFPEPVTVSWNS

GALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAP
IEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK

The amino acid sequence of anti-ADAM9 antibody DB1001 was codon-optimized to synthesize cDNA and ligated into the PTT5 plasmid (commissioned to Nanjing GenScript Biotech Co., Ltd.). The PTT5 plasmids corresponding to the heavy and light chains of the antibody were co-transfected into CHO cells using PEImax 40000. After 5 days of culture, the cell culture supernatant was collected, and the antibody component was purified by protein A (MabSelectTM PrismA), and the antibody was quantified by OD280nm (commissioned to Nanjing GenScript Biotech Co., Ltd.). The antibody yield expressed in the 4 mL system was 0.936 mg. The antibody was further expressed in a 500 mL system, and the antibody expression levels are shown in the table below.

| Antibodies | Expression level in 4 mL system (mg) | SEC purity | Proportion of high molecular weight polymer |
|---|---|---|---|
| DB1001 | 112 mg | 98% | 1% |

### Example 5.2: Preparation of compound ADC-1A-1

To a buffer of antibody Trastuzumab (14.0 mM succinic acid-sodium hydroxide + 108 mM NaCl, pH 7.4; 20 mg, 10.0 mg/mL, 0.135 µmol) was added the prepared tris(2-carboxyethyl)phosphine hydrochloride (10 mM, 0.135 mL, 1.35 µmol) at 37°C. The mixture was placed in a water bath shaker and shaken at 37°C for 3 h. After the reaction was stopped, excess TCEP was removed by ultrafiltration exchange with a buffer of 14.0 mM succinic acid-sodium hydroxide + 108 mM NaCl, pH 6.0.

Compound **AA1** (1.215 µmol, 5 eq) was dissolved in 0.243 mL of DMSO and added to the above solution. The mixture was placed in a water bath shaker, shaken at 22°C for 2 h, and then the reaction was stopped. The reaction mixture was desalted and purified using a Sephadex G25 gel column (elution phase: 20 mM histidine-hydrochloric acid, pH 5.5) to obtain the product **ADC-1A-1,** which was stored at 4°C.

LCMS analysis detected that DAR(k) = 3.9.

Referring to conditions similar to those of ADC-1A-1, different equivalents of TCEP and linker-payload were used to conjugate with different antibodies to obtain the following compounds. The results are shown in Table 4A.

**Table 4A: ADC structures and DAR (k) values**

| No. | Structure | DAR (k) |
|---|---|---|
| ADC-1A-**1** | 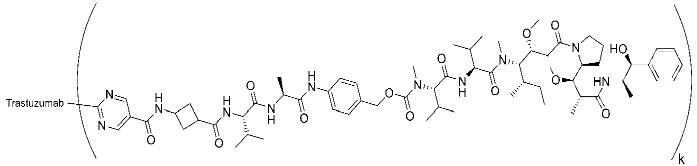 | 3.9 |
| ADC-2A-**1** | 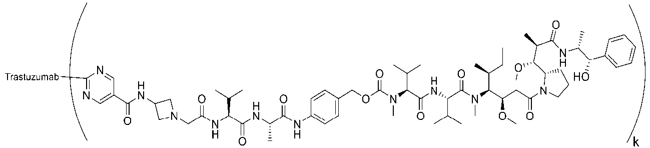 | 3.8 |
| ADC-4A-**1** | 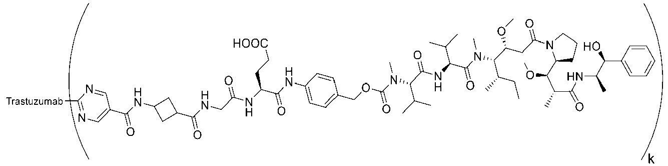 | 3.7 |
| ADC-7A-**1** | 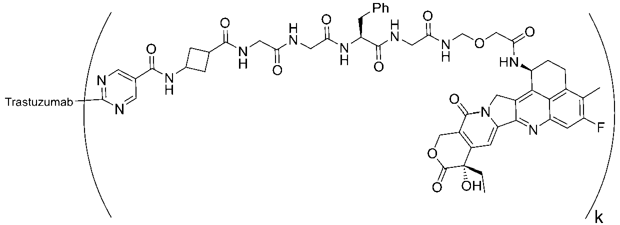 | 7.9 |
| ADC-12A-**1** | 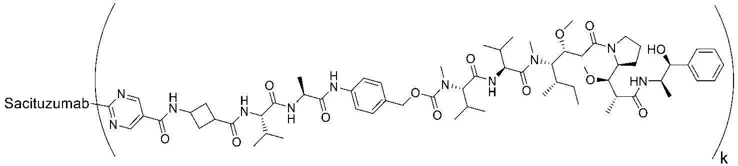 | 1.9 |
| ADC-13A-**1** | 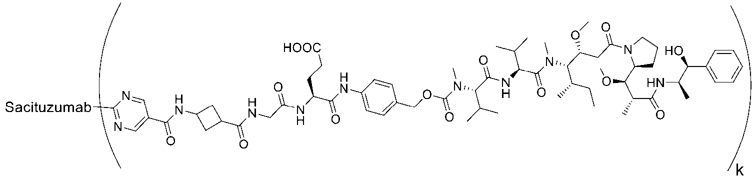 | 3.8 |
| ADC-15A-**1** | 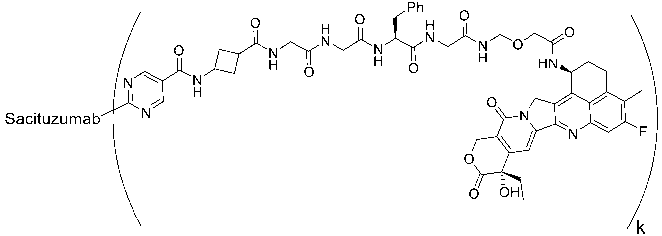 | 7.6 |
| ADC-1B-**1** | 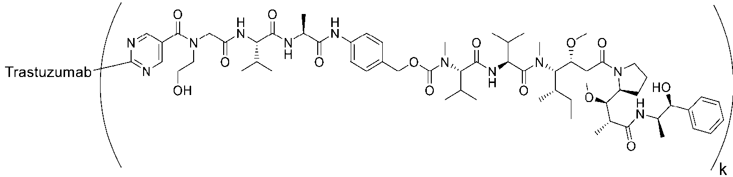 | 3.9 |
| ADC-2B-**1** | 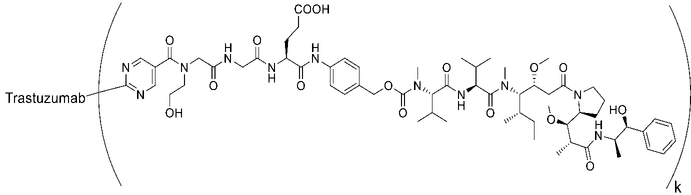 | 4.1 |
| ADC-4B-**1** | 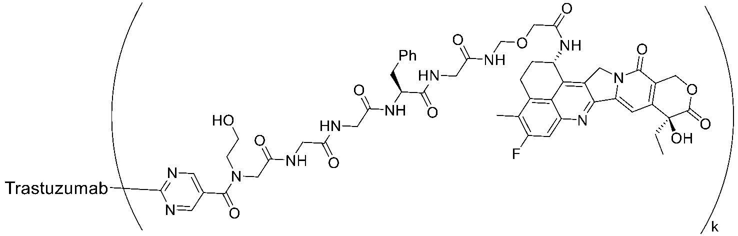 | 7.9 |
| ADC-11B-**1** | 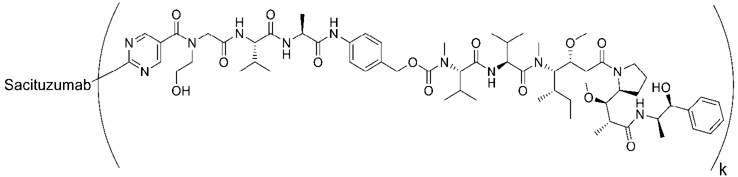 | 3.7 |
| ADC-14B-**1** | 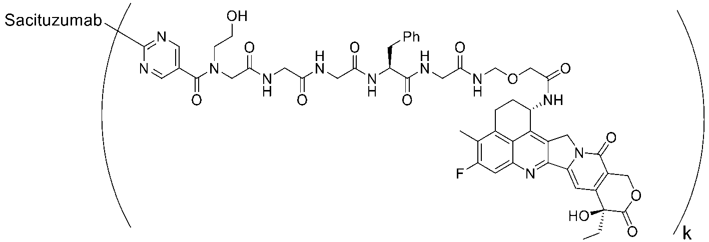 | 7.9 |
| ADC-1C-**1** | 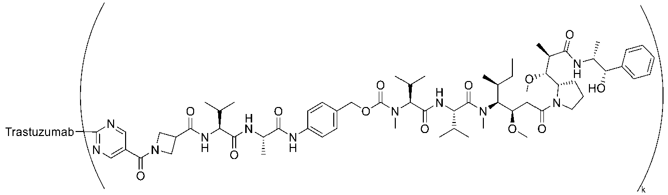 | 3.8 |
| ADC-2C-**1** | 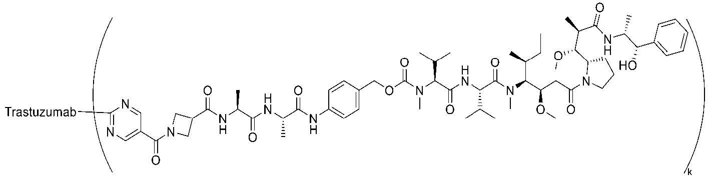 | 3.8 |
| ADC-3C-**1** | 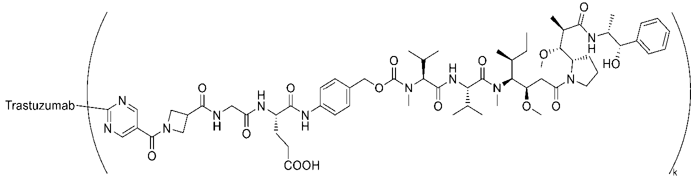 | 3.9 |
| ADC-5C-**1** | 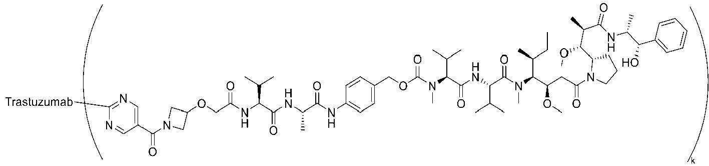 | 3.9 |
| ADC-6C-**1** | 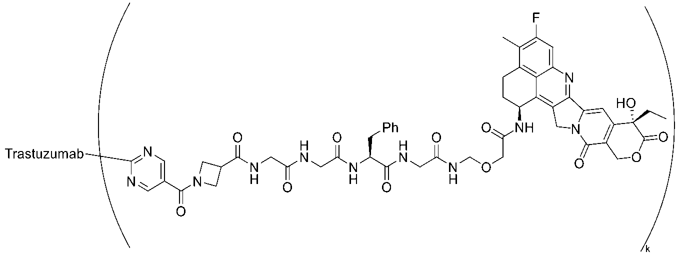 | 7.8 |
| ADC-7C-**1** | 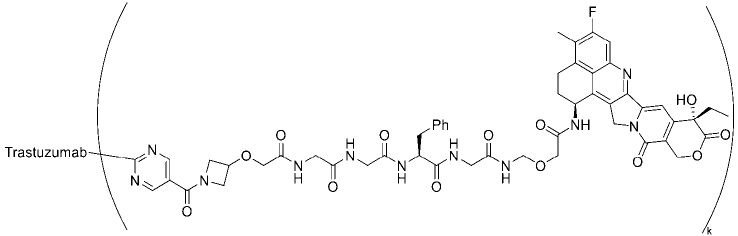 | 7.7 |
| ADC-12C-**1** | 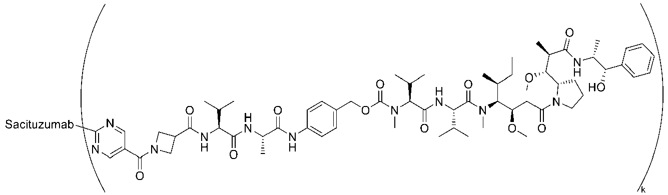 | 3.6 |
| ADC-14C-**1** | 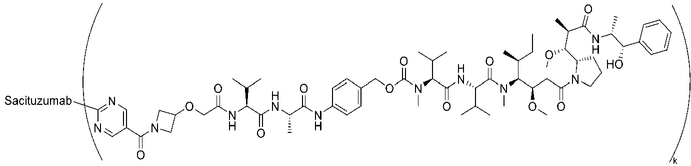 | 3.8 |
| ADC-15C-**1** | 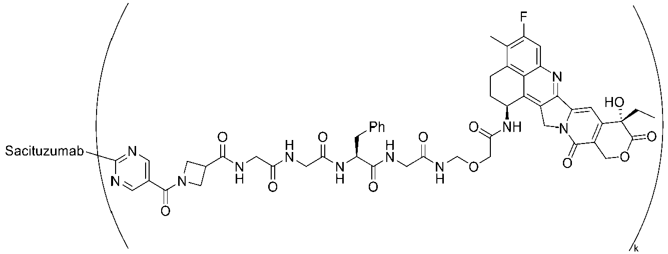 | 7.7 |
| ADC-16C-**1** | 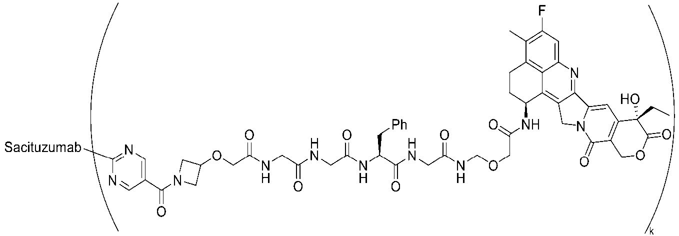 | 7.9 |
| ADC-17C-**1** | 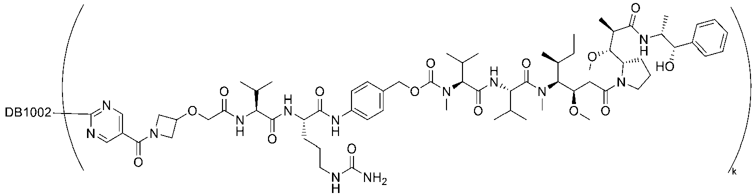 | 4.0 |
| ADC-18C-**1** | 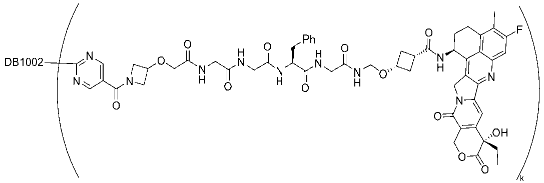 | 7.6 |
| ADC-19C-**1** | 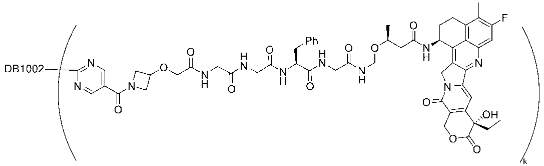 | 7.6 |
| ADC-20C-**1** | 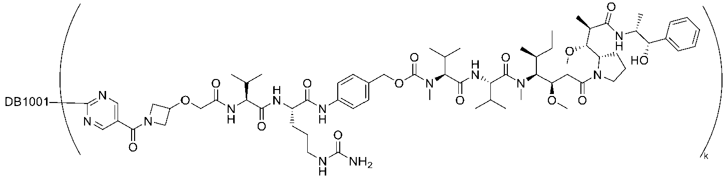 | 4.4 |
| ADC-21C-**1** | 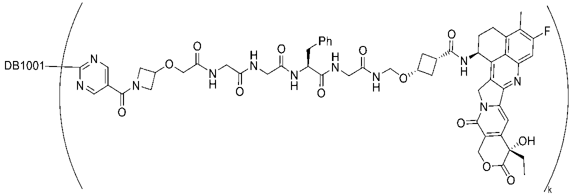 | 7.6 |
| ADC-22C-**1** | 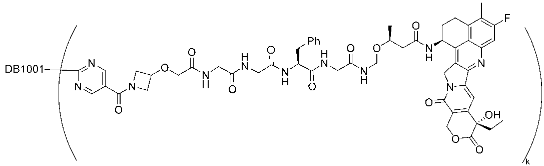 | 7.4 |

Referring to conditions similar to those of ADC-1A-**1**, different equivalents of TCEP and control linker-payload were conjugated with DB 1001 to obtain the following control ADCs. The results are shown in Table 4B.

**Table 4B: Structures and DAR (k) values of control ADCs**

| | | |
|---|---|---|
| ADC-1D-**1** (Reference ADC-20C) | 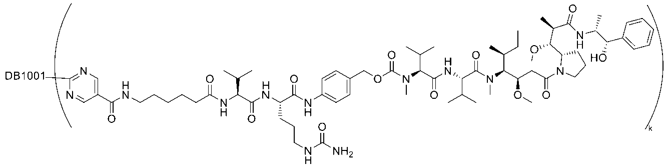 | 4.4 |
| ADC-2D-**1** (Reference ADC-20C) | 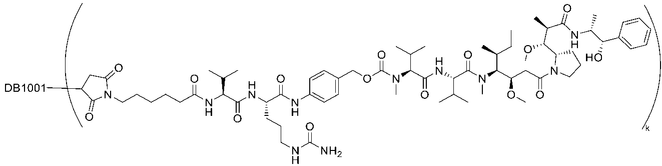 | 4.3 |
| ADC-3D-**1** (Reference ADC-22C) | 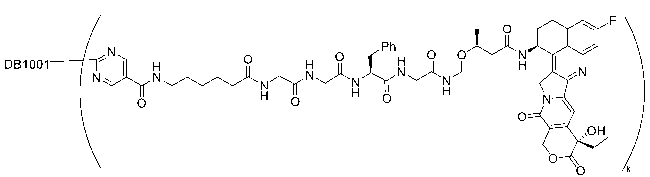 | 7.5 |
| ADC-4D-**1** (Reference ADC-22C) | 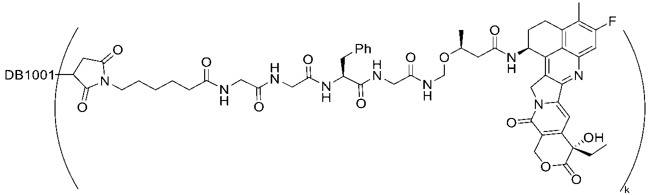 | 7.6 |

### Example 6: Plasma stability test of antibody-drug conjugate

To evaluate the plasma stability of the antibody-drug conjugate of the present disclosure, the antibody-drug conjugate of the present disclosure was incubated in human plasma for 7 days. Samples were taken at 0 h, 8 h, 1 day, 4 days, and 7 days, respectively, to detect the drug-to-antibody ratio (DAR value) and free drug.

| Reagent | Supplier |
|---|---|
| Human plasma | Shanghai Pufen Biotechnology Co., Ltd. |
| Water | Millipore Milli-Q |
| PBS tablets | Sigma-Aldrich |
| Acetonitrile | Merck |
| Biotinylated Human Adam9 Protein, His Tag | Acro Biosystems |
| Formic acid | TCI |
| Streptavidin magnetic beads | ThermoFisher |
| 2.0 mL Protein low-binding tube | Eppendorf |
| BioResolve RP mAb column | Waters |
| Acquity UPLC BEH C18 Column | Waters |

### Incubation of antibody-drug conjugate in plasma

The antibody-drug conjugate ADC-20C of the present disclosure, control ADC-1D, and ADC-3D were each diluted with human plasma to a final concentration of 100 µg/mL and incubated in the dark at 37°C for 7 days. Samples were collected at T0 (the sample was immediately frozen at -70°C within 30 minutes after dilution), 2 h, 8 h, day 1 (24 h), day 4, and day 7. All samples were stored in a -70°C freezer prior to analysis.

Free drugs were analyzed by LC-MS/MS.

### Standard curve and QC sample preparation

The free drug standard was prepared as a 1 mg/mL stock solution in DMSO. The free drug stock solution was diluted in blank plasma matrix by gradient dilution to prepare a standard curve with concentrations ranging from 1.0 to 100 ng/mL, as well as QC samples at three concentration levels, namely 3.0 ng/mL (LQC), 40.0 ng/mL (MQC), and 75.0 ng/mL (HQC).

### Sample pretreatment

100 µL of each of the study sample, standard curve sample, and QC sample were mixed with acetonitrile. The samples were thoroughly mixed by vortex for 1 minute and centrifuged in a centrifuge at 12000 rpm for 5 minutes to precipitate proteins. The supernatant was transferred to a 96-well plate for LC-MS/MS analysis.

### LC-MS instrument detection method

LC-MS instrument detection method for free drugs

| HPLC parameters | | |
|---|---|---|
| Instrument | Vanquish UHPLC system | |
| Chromatographic column | ACQUITY UPLC BEH C18 column, 130Å, 1.7 µm, 2.1 mm × 50 mm | |
| Column temperature | 45°C | |
| Autosampler Temperature | 8°C | |
| Mobile phase | A: 0.1% formic acid/water | |
| | B: 0.1% formic acid/acetonitrile | |
| Flow rate | 0.3 mL/min | |
| | Time | Mobile phase B% |
| | 0.00 | 20 |
| | 0.5 | 20 |
| LC gradient | 06 | 35 |
| | 3.5 | 40 |
| | 4.0 | 100 |
| | 5.5 | 100 |
| | 5.6 | 20 |
| | 7.0 | 20 |
| Mass spectrometry parameters | | |
| Mass spectrometry | TSQTM Quantis | |
| Scan mode | Positive | |
| Source parameters | Capillary voltage: 3 kV; cone gas flow: 50 L/h; source temperature: 120°C; Desolvation temperature: 500°C; High mass: 4000 m/z; Low mass: 600 m/z; scan time: 1 s | |
| Analyte | MMAE | |

### Data processing

Data were acquired and analyzed using ChromeleonTM software. The results are shown in Table 5.

**Table 5**

| Time | MMAE release ratio (%) | | |
|---|---|---|---|
| | ADC-20C | ADC-1D | ADC-2D |
| 0 | 0 | 0 | 0 |
| 2h | A | A | A |
| 8h | A | A | A |
| 1d | A | A | A |
| 4d | A | A | A |
| 7d | A | A | A |

| | | | |
|---|---|---|---|
| Note: A < 1 | | | |

The results show that the antibody-drug conjugate of the present disclosure exhibit a slow release rate of free drug after plasma incubation, with a free drug release rate in plasma below 1% on day 7.

The drug-to-antibody ratio was determined by LC-MS

Antibody-drug conjugates were captured by immobilized magnetic beads

25 µL of streptavidin magnetic beads was added to each well of a 96-well plate, and the storage buffer was discarded. The beads were eluted with 200 µL of PBS buffer, then treated with 80 µL of biotin-labeled antigen protein and another 100 µL of PBS buffer at room temperature for 120 minutes. After complete immobilization of the antigen protein, the beads were eluted twice with 200 µL of PBS buffer. 20 µL of antibody-drug conjugate plasma sample and 180 µL of PBS buffer were added to each well, followed by shaking at room temperature for 120 minutes to ensure complete capture of the antibody-drug conjugates in the plasma. The supernatant was then removed. The beads were washed twice with PBS buffer, and 1% formic acid/30% acetonitrile aqueous elution buffer was added, followed by treatment at room temperature for 20 minutes. Subsequently, 5 µL of neutralization buffer (1M NH₄HCO₃, pH 8.5) and 5 µL of DTT (1M) were added to reduce the captured antibody-drug conjugates at room temperature for 60 minutes, followed by analysis via LC-MS.

The LC-MS parameters are as follows:

| | | |
|---|---|---|
| Instrument | ACQUITY UPLC I-Class | |
| Mobile phase | A: 0.1% formic acid/water | |
| | B: 0.1% formic acid/acetonitrile | |
| Chromatographic column | BioResolve RP mAb Polyphenyl, 450Å, 2.7 µm 2.1 × 50 mm | |
| Flow rate | 0.3 mL/min | |
| Gradient | Time | Mobile phase B% |
| | 0.00 | 25 |
| | 1.00 | 25 |
| | 15.00 | 40 |
| | 15.50 | 90 |
| | 15.60 | 25 |
| | 16.00 | 90 |
| | 16.10 | 25 |
| | 18.00 | 25 |
| Mass spectrometry parameters | | |
| Mass spectrometry | Xevo G2-XS Qtof | |
| Scan mode | Positive | |
| Source parameters | Capillary voltage: 3 kV; cone gas flow: 50 L/h; source temperature: 120°C; Desolvation temperature: 500°C; High mass: 4000 m/z; Low mass: 600 m/z; scan time: 1 s | |

### Data processing

Data were acquired and analyzed using UNIFI software (V1.9.4, Waters). The results are shown in Table 6.

**Table 6**

| Time | DAR value | | |
|---|---|---|---|
| | ADC-20C-1 | ADC-1D-1 | ADC-2D-1 |
| 0 | 4.4 | 4.4 | 4.3 |
| 2h | A | A | A |
| 8h | A | A | B |
| 1d | A | A | B |
| 4d | A | A | C |
| 7d | A | A | C |

| | | | |
|---|---|---|---|
| Note: A: 3.5-4.5; B: 2.5-3.5; C: < 2.5 | | | |

The results show that after incubation in plasma for 7 days, the drug-to-antibody ratio of the antibody-drug conjugate of the present disclosure exhibit no significant change. The antibody-drug conjugate of the present disclosure have extremely high plasma stability, indicating excellent safety of the antibody-drug conjugate of the present disclosure.

### Example 7: In vitro cell proliferation inhibitory activity test of antibody-drug conjugate

The CellTiter-Glo^{®} chemiluminescent cell viability assay (i.e., CTG method) was used to evaluate the inhibitory effect of the ADC drugs disclosed herein on cell proliferation after incubation for 6 days in ADAM9-positive cells (Calu-3, Ls174T) or GPC3-positive cells (Huh7, HepG2).

Cells in the logarithmic growth phase were collected and plated at a density of 6000 cells/well. The cell plate was placed in a 37°C, 5% CO₂ incubator and cultured overnight. On the second day of the experiment, the corresponding ADC drugs were diluted with complete medium in a 3-fold serial dilution to obtain 9 concentration gradients, starting with a maximum concentration of 300 nM. The obtained solutions were added to the cell culture plate at 100 µL/well with complete medium used as a blank control, and 3 replicate wells were set up. The plate was incubated for another 6 days in a 37°C, 5% CO₂ incubator. At the end of the incubation, the cell culture plate was removed and equilibrated to room temperature. 50 µL of CTG detection reagent (Promega, Cat#: G7573) was added to each well, and the mixture was shaken and mixed well. After the mixture was left in the dark for 10 minutes, the signal values were detected and read using a microplate reader. GraphPad Prism software was used to plot the S-shaped dose-response curve using a nonlinear regression model, and the IC₅₀ values were calculated. The cell viability was calculated using the formula: cell viability = (Lum**_{test compound}** - Lum**_{blank control}**)/(Lum_{vehicle control} - Lum_{blank control}) × 100% The results are shown in Tables 7A and 7B.

**Table 7A: Inhibitory activity of antibody-drug conjugates on the proliferation of GPC3-positive cells**

| ADC | IC₅₀ (nM) | |
|---|---|---|
| | Huh7 | HepG2 |
| ADC-17C**-1** | 0.089 | 0.171 |
| ADC-19C**-1** | 0.026 | 0.224 |

**Table 7B: Inhibitory activity of antibody-drug conjugates on the proliferation of ADAM9-positive cells (Calu-3, Ls174T)**

| ADC | IC₅₀ (nM) | |
|---|---|---|
| | Calu-3 | Ls174T |
| ADC-20C**-1** | 0.090 | 5.385 |
| ADC-1D**-1** | / | 15.30 |
| ADC-2D-**1** | / | 27.12 |
| ADC-22C-**1** | 0.26 | 5.571 |
| ADC-3D-**1** | 0.34 | / |
| ADC-4D-**1** | 0.78 | / |

**Experimental conclusion:** The antibody-drug conjugates of the present application have significant proliferation inhibitory activity on tumor cells compared to control molecules; ADC-20C**-1** and ADC-22C**-1** have significant inhibitory advantages in Ls174T and Calu-3 cells.

## Claims

1. A compound of formula (III), or a mesomer, racemate, enantiomer, diastereomer, or any mixture thereof, or a pharmaceutically acceptable salt thereof, or a solvate thereof, or a pharmaceutically acceptable salt of the solvate thereof: 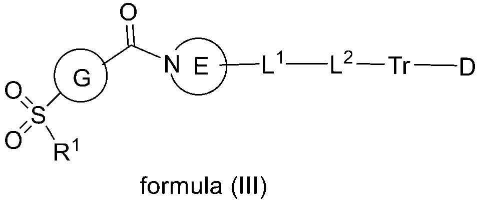 wherein
R¹ is -C₁₋₆ alkyl, -C₃₋₈ cycloalkyl, -C₆₋₁₀ aryl, or 5- to 10-membered heteroaryl, wherein the alkyl, cycloalkyl, aryl, and heteroaryl are each optionally substituted by one or more substituents selected from hydrogen, deuterium, halogen, hydroxyl, cyano, nitro, -OC₁₋₆ alkyl, -C₂₋₆ alkenyl, and -C₂₋₆ alkynyl;
ring G is 5- to 10-membered heteroaryl, wherein the heteroaryl is optionally substituted by one or more substituents selected from hydrogen, deuterium, halogen, hydroxyl, amino, cyano, nitro, -C₁₋₆ alkyl, -OC₁₋₆ alkyl, -NHC₁₋₆ alkyl, and -N(C₁₋₆ alkyl)₂;
ring E is 4- to 12-membered heterocycloalkylene; wherein the 4- to 12-membered heterocycloalkylene is optionally substituted by one or more substituents selected from hydrogen, deuterium, halogen, hydroxyl, amino, cyano, nitro, -NHC₁₋₆ alkyl, -N(C₁₋₆ alkyl)₂, and -C(O)NH-(CH₂CH₂O)_{y1}CH₃;
y1 is a natural number;
the heteroatom of the 4- to 12-membered heterocycloalkylene is selected from one or more types of N, O, and S, and the number of heteroatoms is 1, 2, 3, or 4;
L¹ is -(C(R^{L11})₂)ₙ-; n is a natural number;
any -_{C(}R^{L11})₂- moiety in L¹ is independently and optionally replaced by the following structural moieties: -Cy-, -NR^{L12}-, -C(O)-, -O-, -S-, -SO-, -SO₂-, -P(R^{L12})-, -P(=O)(R^{L12})-, -
C(=S)-, -C(=NR^{L12})-, -N=N-, -C=N-, -N=C-, 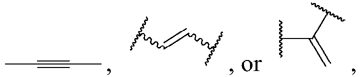 -Cy- is phenylene, 5- to 8-membered heteroarylene, 3- to 10-membered heterocycloalkylene, or 3- to 10-membered cycloalkylene, wherein each -Cy- is independently unsubstituted or substituted by one or more R^{cx},
R^{L11}, R^{L12}, and R^{cx} are each independently hydrogen, deuterium, halogen, -NO₂, -CN, - OR^{L1a}, -SR^{L1a}, -N(R^{L1a})₂, -N⁺(R^{L1a})₃, -C(O)R^{L1a}, -CO₂R^{L1a}, -C(O)C(O)R^{L1a}, - C(O)CH₂C(O)R^{L1a}, -S(O)R^{L1a}, -S(O)₂R^{L1a}, -C(O)N(R^{L1a})₂, -SO₂N(R^{L1a})2, -OC(O)R^{L1a}, - N(R^{L1a})SO₂R^{L1b}, -N(R^{L1a})COR^{L1b}, -(CH₂)_{y}-CO-(N(Me)CH₂C(O))ₘ-OR^{L1a}, -(CH₂)_{y}-CO-(N(Me)CH₂C(O))ₘ-NHR^{L1a}, -(CH₂)_{y}-CO-(N(Me)CH₂C(O))ₘ-N⁺(R^{L1a})₃, -(CH₂)_{y}-NHCOCH₂(OCH₂CH₂)OR^{L1a}, -(CH₂)_{y}-NH(COCH₂(N(Me))ₘ-R^{L1a}, -(CH₂)_{y}-CONH-(CH₂CH₂O)ₘ-R^{L1a}, -(CH₂)_{y}-NHCO-(CH₂CH₂O)ₘ-R^{L1a}, -(CH₂CH₂O)ₘ-R^{L1a}, - (COCH₂N(Me))ₘ-R^{L1a}, -COCH₂(OCH₂CH₂)ₘ-OR^{L1a}, -CO-(CH₂CH₂O)ₘ-R^{L1a}, -CO-(CH₂)_{y}-CONH-(CH₂CH₂O)ₘ-R^{L1a}, -CO-(CH2)y-NHCO-(CH₂CH₂O)ₘ₋R^{L1a}, -C₁₋₆ alkyl, -C₂₋₆ alkenyl, - C₂₋₆ alkynyl, 3- to 8-membered cycloalkyl, 4- to 10-membered heterocycloalkyl, 6- to 10-membered aryl, or 5- to 10-membered heteroaryl, wherein the -C₁₋₆ alkyl, -C₂₋₆ alkenyl, -C₂₋₆ alkynyl, 3- to 8-membered cycloalkyl, 4- to 10-membered heterocycloalkyl, 6- to 10-membered aryl, and 5- to 10-membered heteroaryl are each independently and optionally substituted by one or more R^{L1a};
m and y are each independently a natural number,
R^{L1a} and R^{L1b} are each independently hydrogen, deuterium, halogen, -NO₂, -CN, -OH, - SH, -NH₂, -N(Me)₂, -CO₂H, -S(O)₂Me, -S(O)₂OH, -C(O)NH₂, -SO₂NH₂, -C₁₋₆ alkyl, -C₂₋₆ alkenyl, -C₂₋₆ alkynyl, 3- to 8-membered cycloalkyl, 4- to 10-membered heterocycloalkyl, 6-to 10-membered aryl, or 5- to 10-membered heteroaryl;
L² is a chemical bond or selected from one or more of an amino acid residue, a short peptide composed of 2 to 10 amino acid residues, 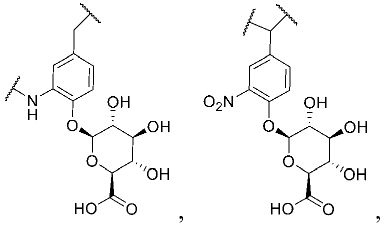 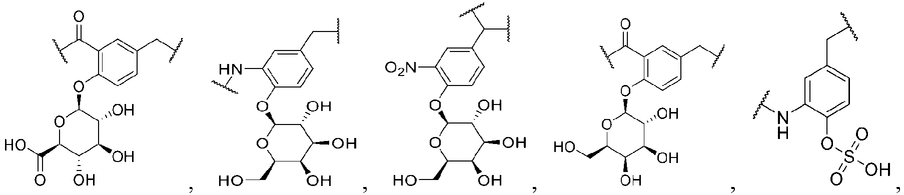 and 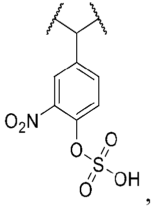 wherein the amino acid residue is a natural amino acid residue or an unnatural amino acid residue;
Tr is a chemical bond or selected from one or more of 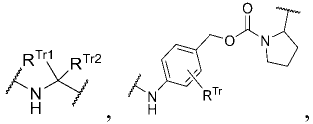 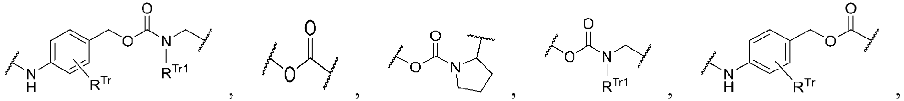 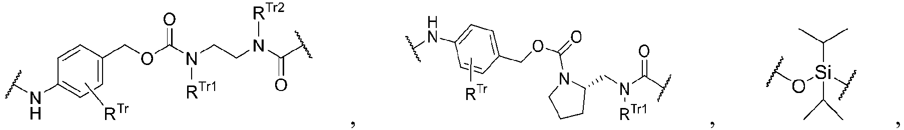 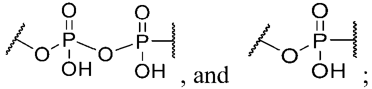
R^{Tr}, R^{Tr1}, and R^{Tr2} are each independently hydrogen, deuterium, halogen, -NO₂, -CN, -OH, -SH, -NH₂, -CO₂H, -S(O)₂OH, -C(O)NH₂, -SO₂NH₂, -OC(O)NH₂, -CH₂CO-(N(Me)CH₂C(O))_{z}-OR^{Tra}, -CH₂CO-(N(Me)CH₂C(O))_{z}-NHR^{Tra}, -(CH₂CH₂O)_{z}-R^{Tra}, -CONH-(CH₂CH₂O)_{z}-R^{Tra}, -C₁₋₆ alkyl, -C₂₋₆ alkenyl, -C₂₋₆ alkynyl, 3- to 8-membered cycloalkyl, 4- to 10-membered heterocycloalkyl, 6- to 10-membered aryl, or 3- to 10-membered heteroaryl, wherein the -C₁₋₆ alkyl, -C₂₋₆ alkenyl, -C₂₋₆ alkynyl, 3- to 8-membered cycloalkyl, 4- to 10-membered heterocycloalkyl, 6- to 10-membered aryl, and 3- to 10-membered heteroaryl are each independently and optionally substituted by one or more R^{Tra};
each R^{Tra} is independently hydrogen, deuterium, halogen, -NO₂, -CN, -OH, -SH, -NH₂, - N(Me)₂, -S(O)₂Me, -CO₂H, -S(O)₂OH, -C(O)NH₂, -SO₂NH₂, -C₁₋₆ alkyl, -C₂₋₆ alkenyl, -C₂₋₆ alkynyl, 3- to 8-membered cycloalkyl, 4- to 10-membered heterocycloalkyl, 6- to 10-membered aryl, or 5- to 10-membered heteroaryl;
z is a natural number;
D is a drug active molecule;
heteroatoms in the 5- to 10-membered heteroaryl, 5- to 8-membered heteroarylene, 3- to 10-membered heterocycloalkylene, and 4- to 10-membered heterocycloalkyl are each independently selected from one or more types of N, O, and S, and the number of heteroatoms is independently 1, 2, 3, or 4.

2. The compound of formula (III), or the mesomer, racemate, enantiomer, diastereomer, or any mixture thereof, or the pharmaceutically acceptable salt thereof, or the solvate thereof, or the pharmaceutically acceptable salt of the solvate thereof according to claim 1, wherein the compound of formula (III), or the mesomer, racemate, enantiomer, diastereomer, or any mixture thereof, or the pharmaceutically acceptable salt thereof, or the solvate thereof, or the pharmaceutically acceptable salt of the solvate thereof satisfies one or more of the following conditions,
(1) R¹ is -C₁₋₆ alkyl or -C₃₋₈ cycloalkyl, wherein the alkyl and cycloalkyl are each independently and optionally substituted by one or more substituents selected from hydrogen, deuterium, and halogen; preferably, R¹ is -C₁₋₆ alkyl; further preferably, R¹ is -Me, -Et, - isopropyl, or -cyclopropyl; more preferably, R¹ is -Me;
(2) ring G is pyridine, pyrimidine, pyridazine, pyrazine, oxazole, isoxazole, thiazole, isoxazole, oxadiazole, thiadiazole, benzothiazole, or benzoxazole; preferably, ring G is 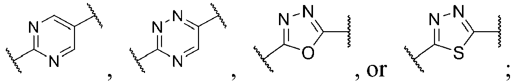 further preferably, ring G is 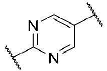 or 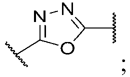 more preferably, ring G is 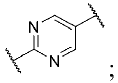
(3) L¹ is -(CHR^{L11})ₙ-; n is a natural number from 0 to 50;
any -CHR^{L11}- moiety in L¹ is independently and optionally replaced by the following structural moieties: -Cy-, -NR^{L12}-, -C(O)-, -S-, -O-, 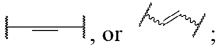
-Cy- is phenylene, 5- to 6-membered heteroarylene, 4- to 10-membered heterocyclylene, or 3- to 6-membered cycloalkylene, wherein each -Cy- is independently unsubstituted or substituted by 1 to 3 R^{cx},
R^{L11}, R^{L12}, and R^{cx} are each independently hydrogen, halogen, -OR^{L1a}, -N(R^{L1a})₂, - C(O)R^{L1a}, -S(O)₂R^{L1a}, -C(O)N(R^{L1a})₂, -SO₂N(R^{L1a})₂, -N(R^{L1a})SO₂R^{L1b}, -N(R^{L1a})COR^{L1b}, - (CH₂)_{y}-CO-(N(Me)CH₂C(O))ₘ-OR^{L1a}, -(CH₂)_{y}-CO-(N(Me)CH₂C(O))ₘ-NHR^{L1a}, -(CH₂)_{y}-CONH-(CH₂CH₂O)ₘ-R^{L1a}, -(CH₂)y-NHCO-(CH₂CH₂O)ₘ₋R^{L1a}, -(CH₂)_{y}-NHCOCH₂(OCH₂CH₂)OR^{L1a}, -(CH₂)_{y}-NH(COCH₂(N(Me))ₘ-R^{L1a}, -(CH₂)_{y}-NHCO-(CH₂CH₂O)ₘ-R^{L1a}, -(CH₂CH₂O)ₘ₋R^{L1a}, -(COCH₂N(Me))ₘ-R^{L1a}, -COCH₂(OCH₂CH₂)m-OR^{L1a}, -CO-(CH₂CH₂O)ₘ-R^{L1a}, -C₁₋₆ alkyl, -C₂₋₆ alkenyl, -C₂₋₆ alkynyl, 3- to 8-membered cycloalkyl, 4- to 10-membered heterocycloalkyl, 6- to 10-membered aryl, or 5- to 10-membered heteroaryl; wherein the -C₁₋₆ alkyl, -C₂₋₆ alkenyl, -C₂₋₆ alkynyl, 3- to 8-membered cycloalkyl, 4- to 10-membered heterocycloalkyl, 6- to 10-membered aryl, and 5- to 10-membered heteroaryl are each independently and optionally substituted by one or more R^{L1a};
m is a natural number from 0 to 8;
y is 0, 1, 2, 3, or 4;
R^{L1a} and R^{L1b} are each independently hydrogen, halogen, -CN, -OH, -NH₂, -N(Me)₂, - CO₂H, -C(O)NH₂, or -C₁₋₆ alkyl;
preferably, L¹ is -(CH₂)ₙ-; n is a natural number from 0 to 50;
any -CH₂- moiety in L¹ is independently and optionally replaced by the following structural moieties: phenylene, 5- to 6-membered heteroarylene, 4- to 6-membered heterocyclylene, 3- to 6-membered cycloalkylene, -NR^{L12}-, -C(O)-, -S-, or -O-;
each R^{L12} is independently hydrogen, -OR^{L1a}, -C(O)R^{L1a}, -S(O)₂R^{L1a}, -C(O)N(R^{L1a})₂, - SO₂N(R^{L1a})₂, -(CH₂)_{y}-CO-(N(Me)CH₂C(O))ₘ-OR^{L1a}, -(CH₂)_{y}-CO-(N(Me)CH₂C(O))ₘ-NHR^{L1a}, -(CH₂)_{y}-CONH-(CH₂CH₂O)ₘ-R^{L1a}, -(CH₂)_{y}-NHCOCH₂(OCH₂CH₂)OR^{L1a}, - (CH₂CH₂O)ₘ-R^{L1a}, -(COCH₂N(Me))ₘ-R^{L1a}, -COCH₂(OCH₂CH₂)ₘ-OR^{L1a}, -CO-(CH₂CH₂O)ₘ-R^{L1a}, or -C₁₋₆ alkyl optionally substituted by R^{L1a};
m is a natural number from 0 to 8;
y is 0, 1, 2, 3, or 4;
each R^{L1a} is independently hydrogen, halogen, -CN, -OH, -NH₂, -N(Me)₂, -CO₂H, - C(O)NH₂, or -C₁₋₆ alkyl;
preferably, L¹ is -(CH₂)ₙ-; n is a natural number from 0 to 50;
any -CH₂- moiety in L¹ is independently and optionally replaced by the following structural moieties: -NH-, -NMe-, -C(O)-, -S-, or -O-; for example, L¹ is: 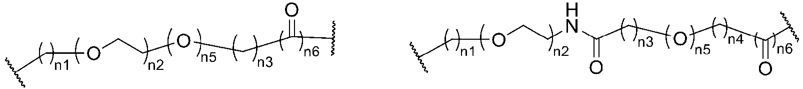 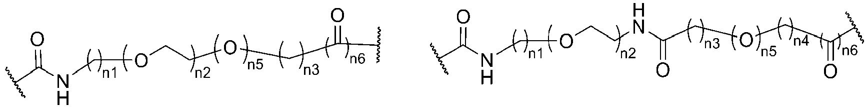 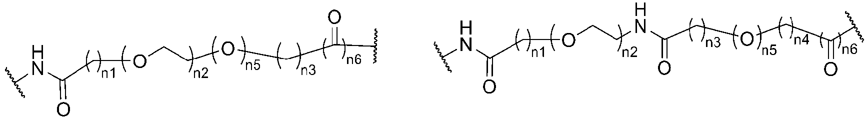 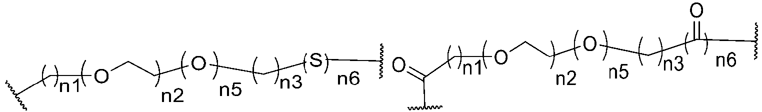 wherein
n1, n2, n3, and n4 are each independently a natural number from 0 to 8;
n5 and n6 are each independently 0 or 1;
(4) L² is a chemical bond or an amino acid residue, a short peptide composed of 2 to 7 amino acid residues, 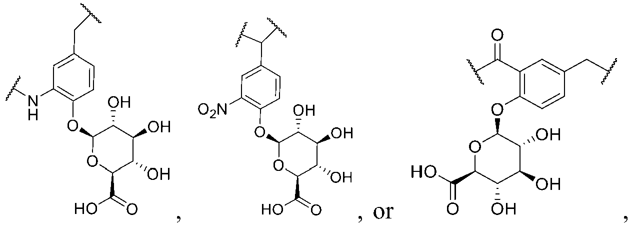 wherein the amino acid residue is Val, D-Val, Nva, Phe, Lys, Leu, Ile, Gly, Ala, D-Ala, Cit, Asp, Asn, Glu, Gln, Pro, Arg, Lys(Ac), Lys(Me), Lys(Et), Lys(nPr), Nva, or AA;
AA is 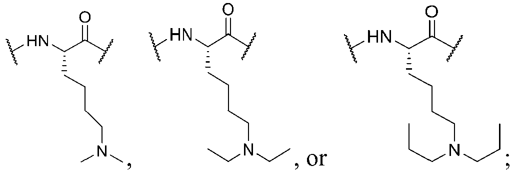
preferably, L² is a chemical bond, Val, D-Val, Phe, Lys, Leu, Ile, Gly, Ala, D-Ala, Cit, Asp, Asn, Glu, Gln, Val-Cit, Val-Ala, Val-Lys, Val-Lys(Ac), Phe-Lys, Phe-Lys(Ac), Leu-Lys, Leu-Lys(Ac), Ala-Ala, Ala-Lys, D-Ala-Ala, Gly-Glu, Gly-Asp, Gly-Asn, Val-Glu, Val-Asp, Asp-Asp, Asn-Asn, Glu-Val-Cit, Ala-Ala-Ala, Ala-(D-Ala)-Ala, Ala-Ala-Asn, Ala-(D-Ala)-Asn, Ala-Ala-Asp, Val-Lys-Gly, D-Val-Leu-Lys, Gly-Gly-Arg, Gly-Gly-Gly, Lys-Ala-Asn, Gly-Phe-Gly, Gly-Gly-Phe, Asn-Pro-Val, Ala-Lys-Gly, Gly-Lys-Gly, Gly-Glu-Gly, Gly-Gly-Gly-Gly, Gly-Gly-Phe-Gly, Gly-Gly-Glu-Gly, Lys-Ala-Ala-Asn, Lys-Ala-Ala-Asp, Ala-Ala-Pro-Val, Ala-Ala-Pro-Nva, 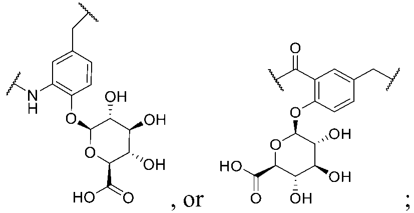
preferably, L² is a chemical bond, Lys, Cit, Asp, Asn, Glu, Gln, Val-Cit, Val-Ala, Asp-Asp, Ala-Ala, Gly-Glu, Gly-Asp, Gly-Asn, Asn-Asn, Gly-Glu-Gly, Gly-Gly-Glu-Gly, Gly-Gly-Phe-Gly, or Gly-Gly-Gly-Gly;
(5) Tr is a chemical bond or selected from one or more of 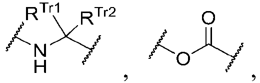 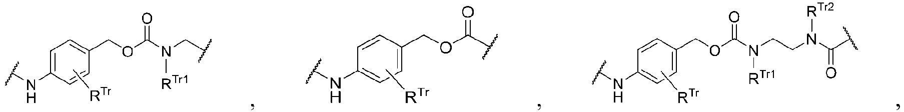 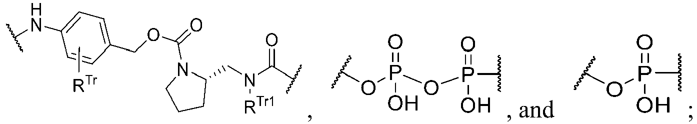
R^{Tr}, R^{Tr1}, and R^{Tr2} are each independently hydrogen, halogen, -NO₂, -CN, -OH, -NH₂, - CO₂H, -S(O)₂H, -C(O)NH₂, -SO₂NH₂, -OC(O)NH₂, -CH₂CO-(N(Me)CH₂C(O))_{z}-OR^{Tra}-, - CH₂CO-(N(Me)CH₂C(O))_{z}-NHR^{Tra}, -(CH₂CH₂O)_{z}-R^{Tra}, -CONH-(CH_{z}CH₂O)_{z}-R^{Tra}, or -C₁₋₆ alkyl optionally substituted by R^{Tra};
R^{Tra} is hydrogen, deuterium, halogen, -NO₂, -CN, -OH, -NH₂, -N(Me)₂, -S(O)₂Me, -CO₂H, -S(O)₂OH, -C(O)NH₂, -SO₂NH₂, or -C₁₋₆ alkyl;
z is a natural number;
preferably, Tr is a chemical bond or selected from one or more of 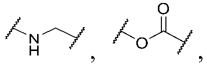 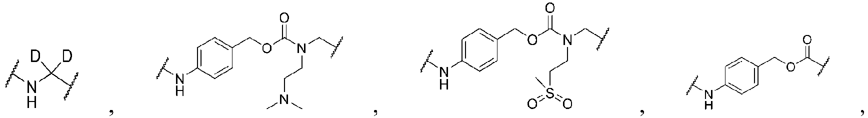 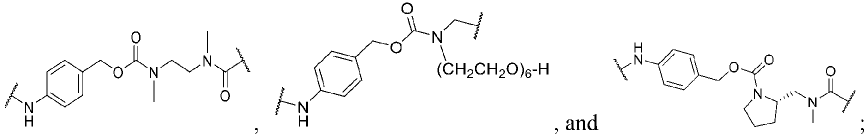
preferably, Tr is a chemical bond, 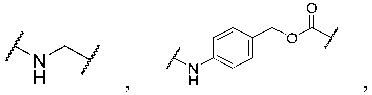 or 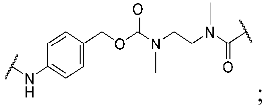 and
(6) D is a tubulin inhibitor, a topoisomerase inhibitor, a DNA damaging agent, an RNA polymerase inhibitor, an immunomodulator, or an immunostimulant;
the tubulin inhibitor may be Auristatins (e.g., MMAE, MMAF, or MMAD), Maytansinoids (e.g., DM1 or DM4), Vinblastines, Taxols, Eribulins, Tubulysins, Colchicines, or Hemiasterlin; or a stereoisomer, isostere, analog, or derivative thereof; for example, 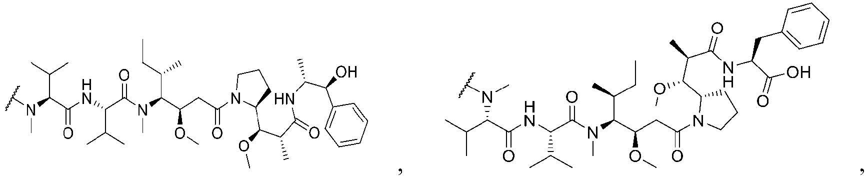 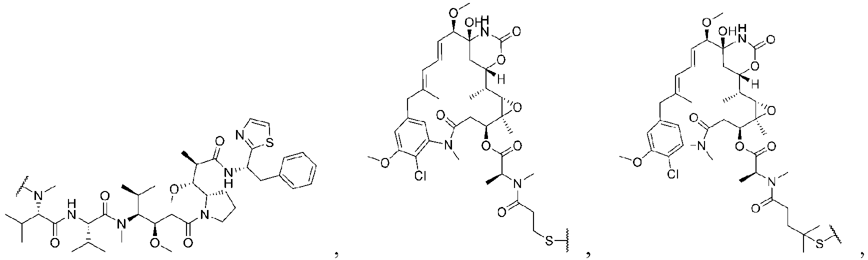 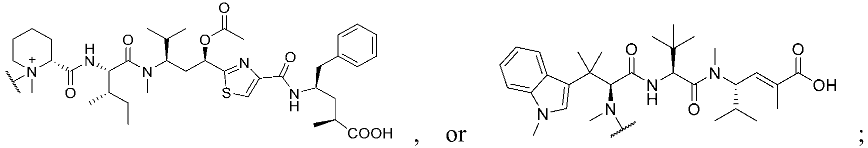 more preferably 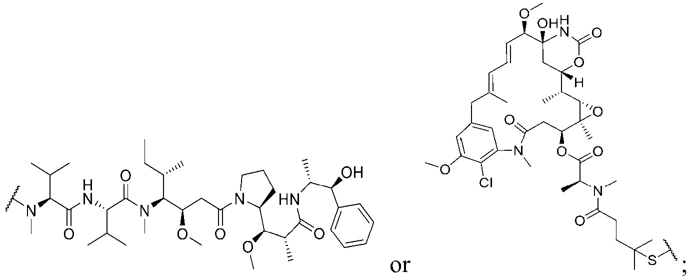
the topoisomerase inhibitor may be camptothecin (e.g., SN38 or DX8951F); for example, 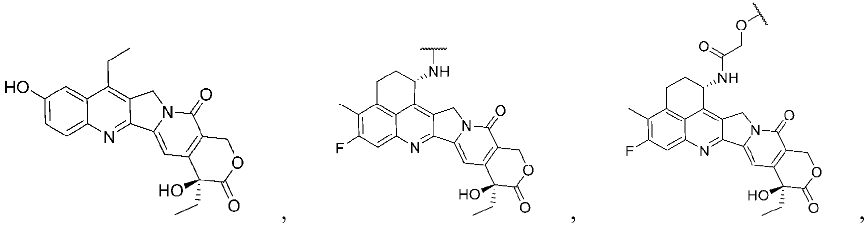 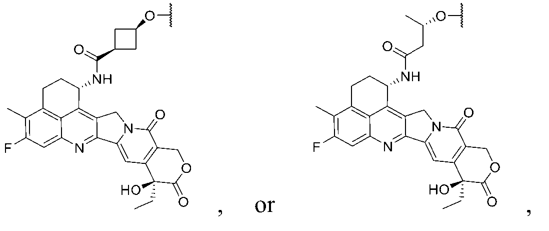 preferably 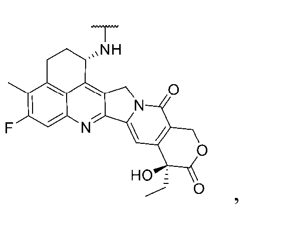 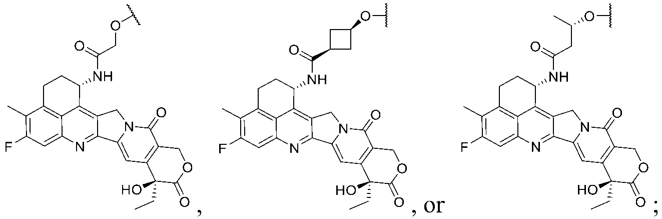
the DNA damaging agent may be Calicheamicins, Mitomycins, anthracyclines (e.g., doxorubicin), methotrexates, Duocarmycins, a CBI dimer, a CPI dimer, a CTI dimer, a PBD dimer, an IGN dimer, a PDD dimer, or a Duocarmycin-PBD dimer; for example, 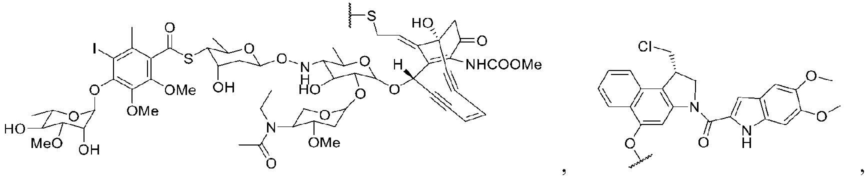 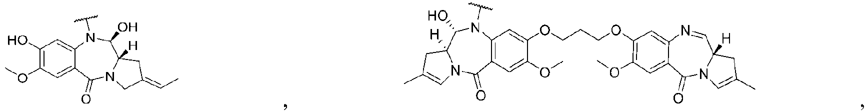 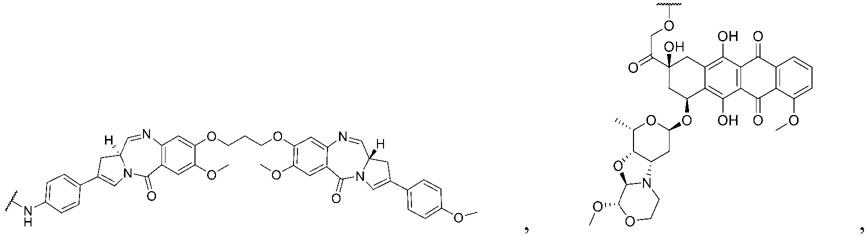
or 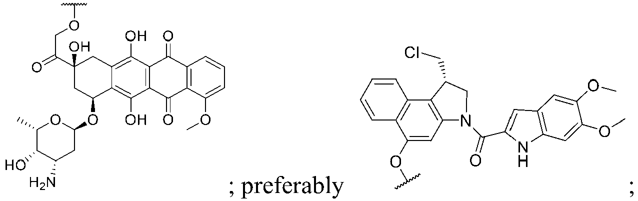
the RNA polymerase inhibitor may be Amatoxins or a spliceosome inhibitor (e.g., Thailanstatin A); for example, 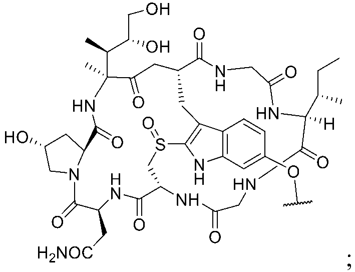
the immunomodulator may be a glucocorticoid; for example, 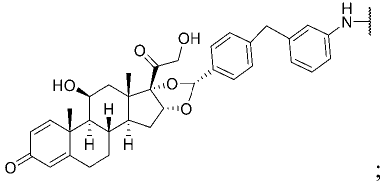
the immunostimulant may be (e.g., Sting or TLR7/8 agonist), a protein toxin (e.g., interleukin, interferon, or tumor necrosis factor TNF-α), a selective or non-selective kinase
inhibitor, a Bcl-x1 inhibitor, and a Bcl-2/Bcl-X1 inhibitor; for example,

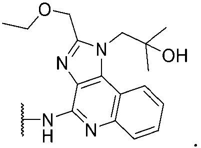

3. The compound of formula (III), or the mesomer, racemate, enantiomer, diastereomer, or any mixture thereof, or the pharmaceutically acceptable salt thereof, or the solvate thereof, or the pharmaceutically acceptable salt of the solvate thereof according to claim 2, wherein 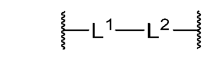 is: 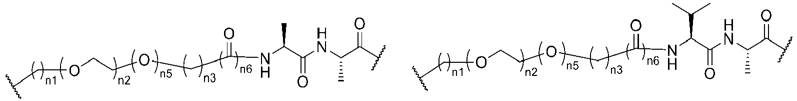 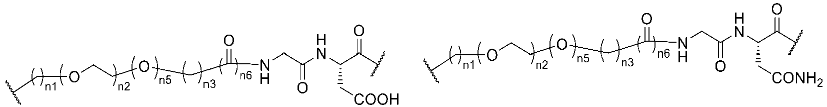 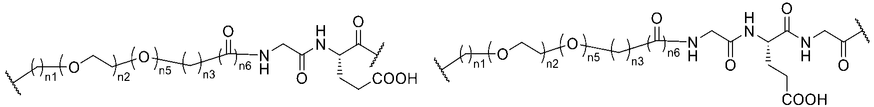 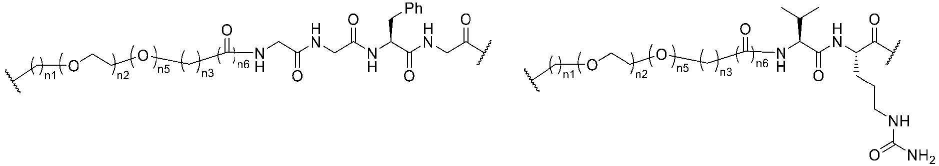 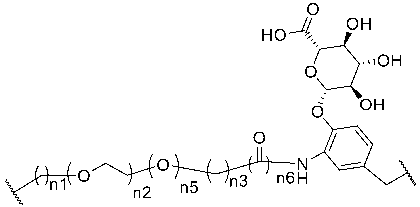 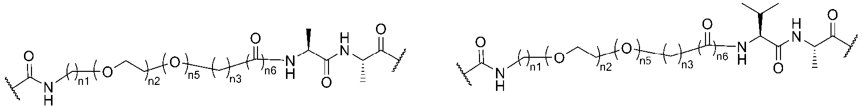 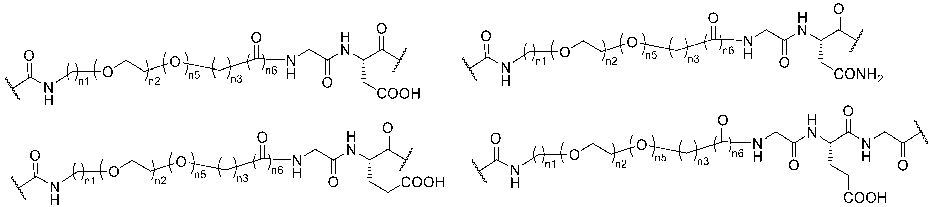 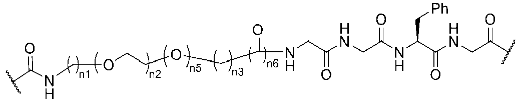 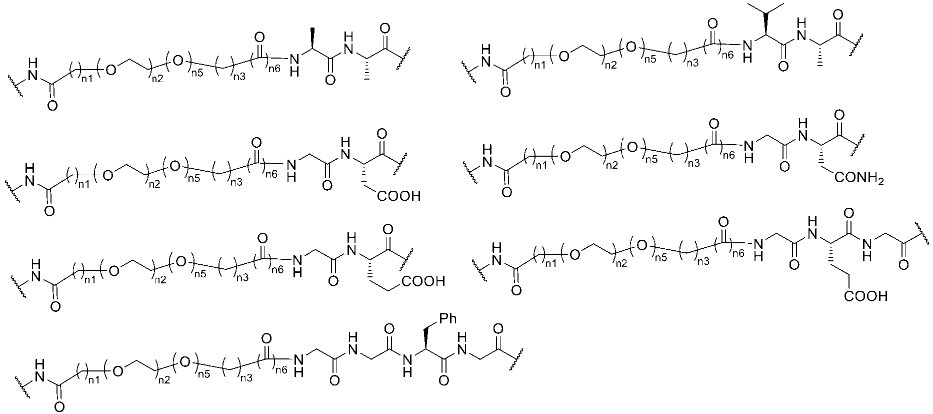 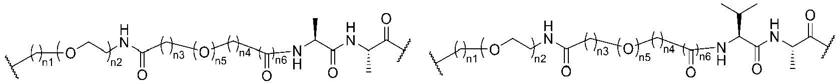 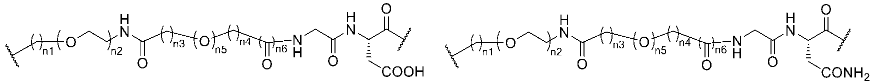 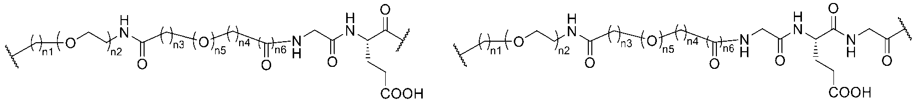 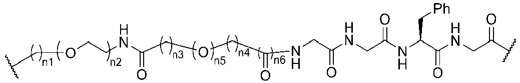 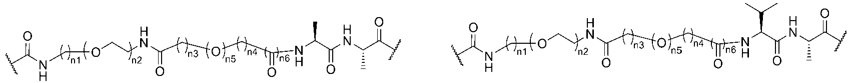 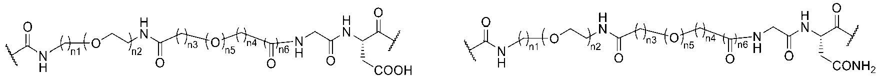 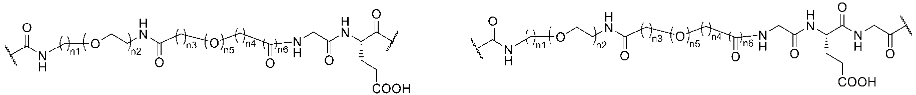 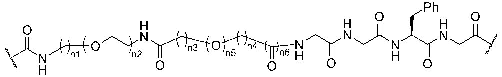 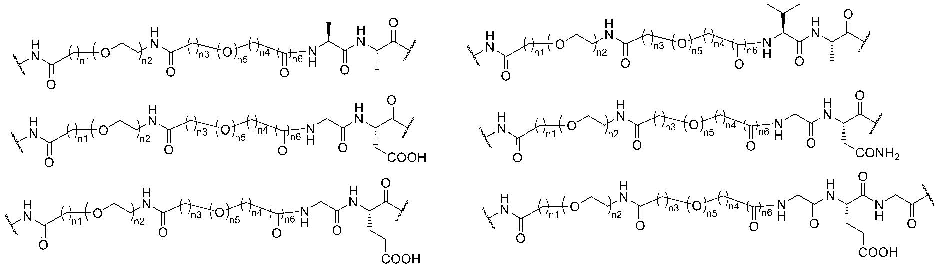 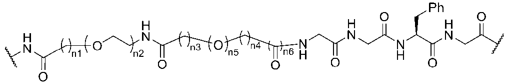 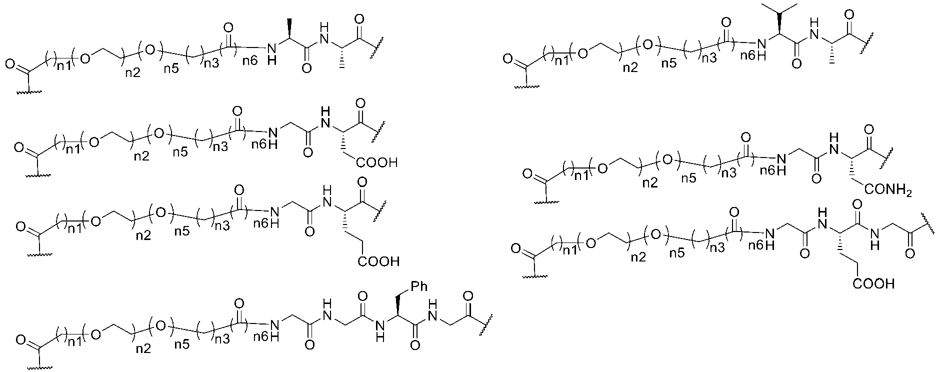 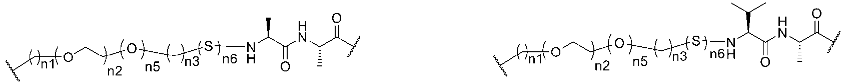 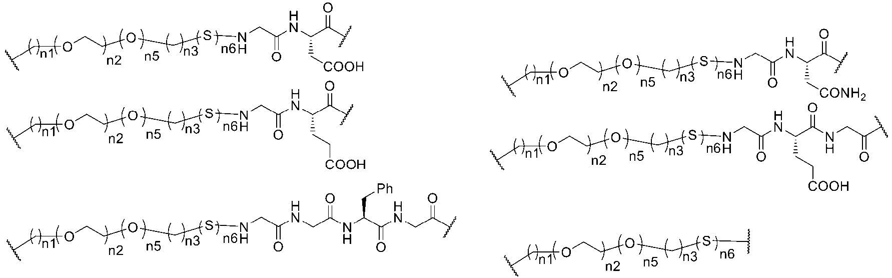
wherein n1, n2, n3, and n4 are each independently a natural number from 0 to 8;
n5 and n6 are each independently 0 or 1;
preferably, 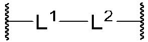 is Scheme 1 or Scheme 2: 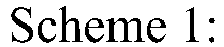 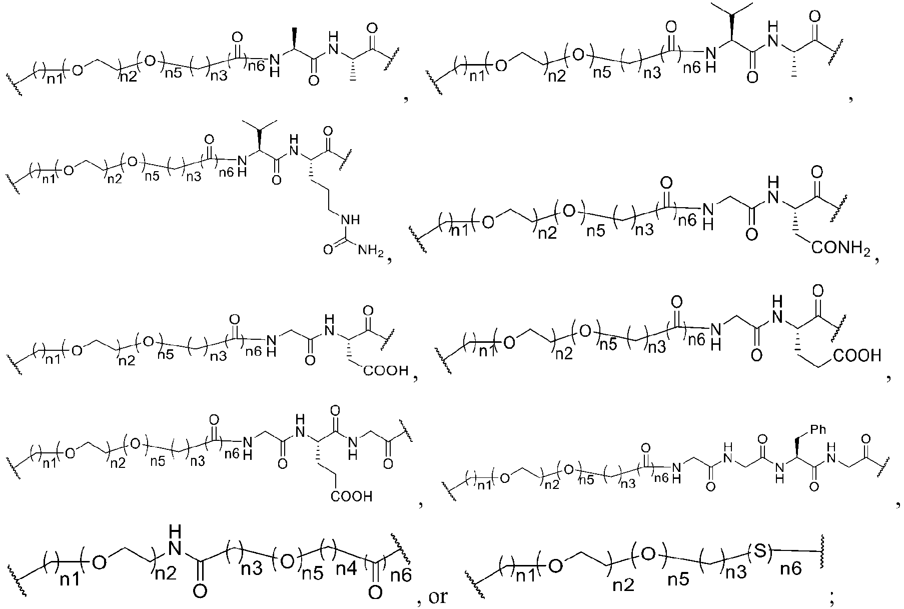
n1, n2, n3, and n4 are each independently 0, 1, 2, 3, 4, 5, 6, 7, or 8;
n5 and n6 are each independently 0 or 1;
for example, 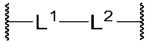 is: 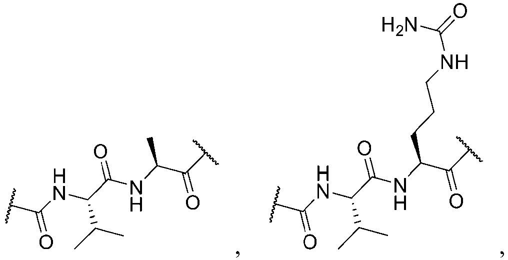 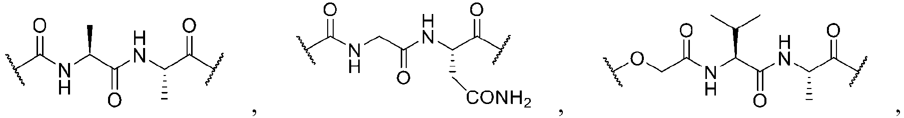 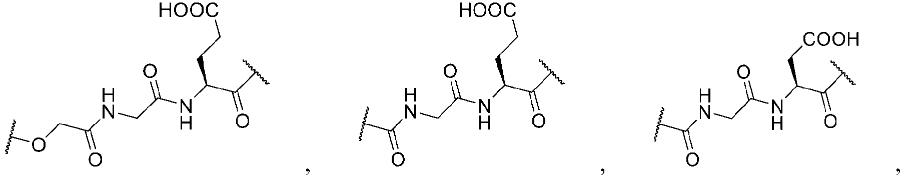 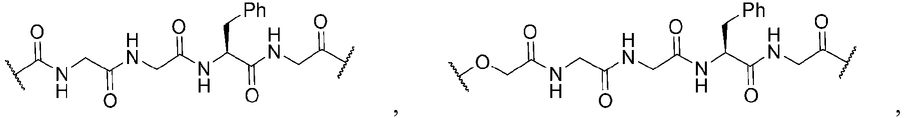 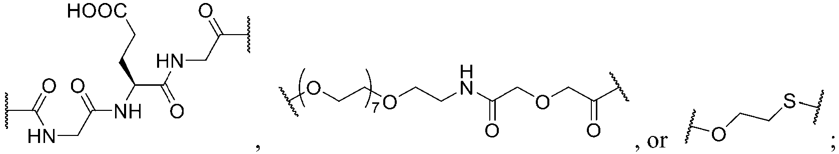 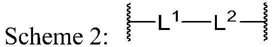 is: 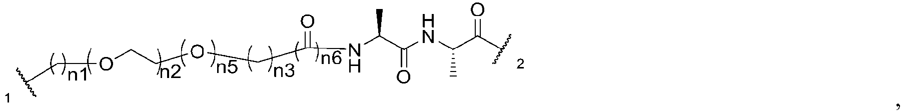 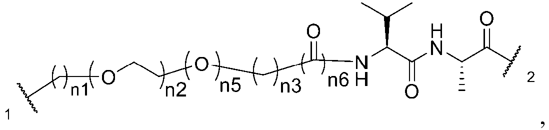 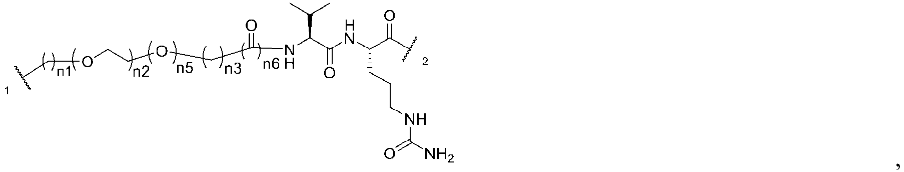 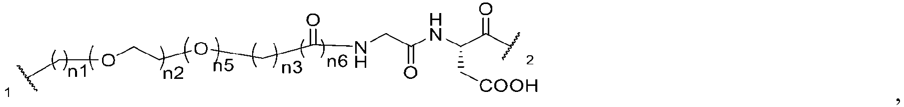 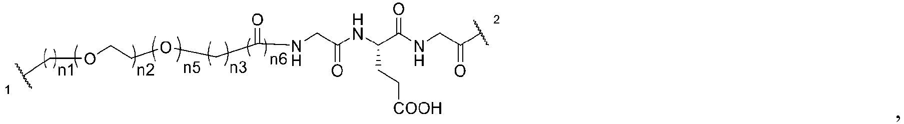 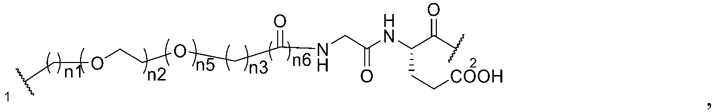 or 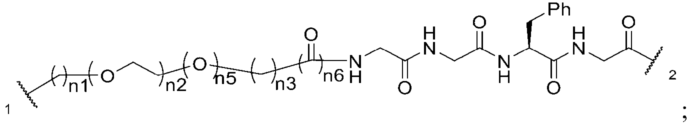
wherein position "1" is connected to ring E, and position "2" is connected to Tr; each of n1 and n2 is 0; n3 is 0, 1, 2, 3, or 4; n5 is 0 or 1; n6 is 1;
preferably, 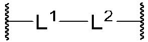 is: 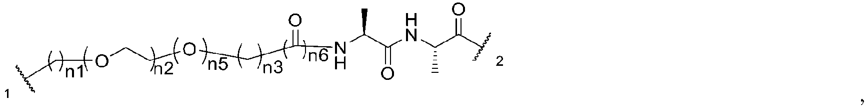 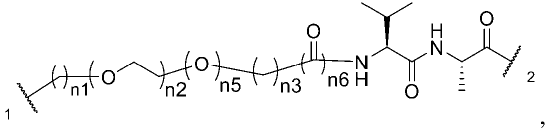 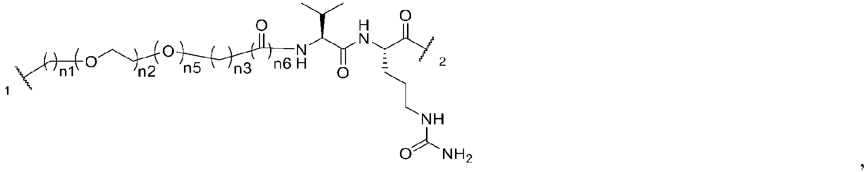 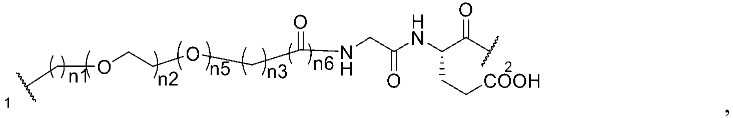 or 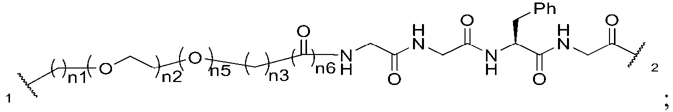
wherein position "1" is connected to ring E, and position "2" is connected to Tr;
each of n1 and n2 is 0; n3 is 0, 1, 2, 3, or 4; n5 is 0 or 1; n6 is 1;
more preferably, 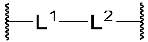 is: 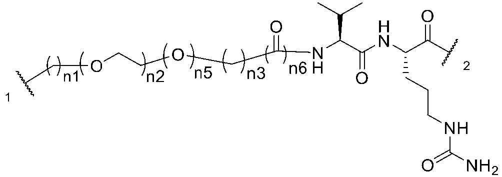 or 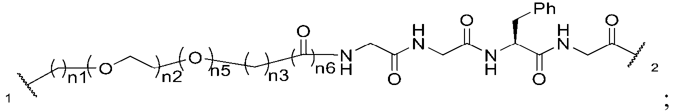
wherein position "1" is connected to ring E, and position "2" is connected to Tr; each of n1 and n2 is 0; n3 is 0, 1, 2, 3, or 4; n5 is 0 or 1; n6 is 1;
for example, 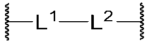 is: 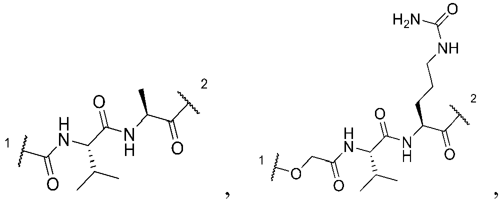 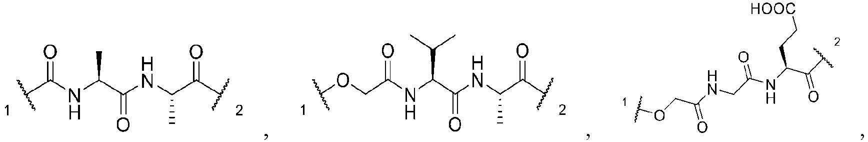 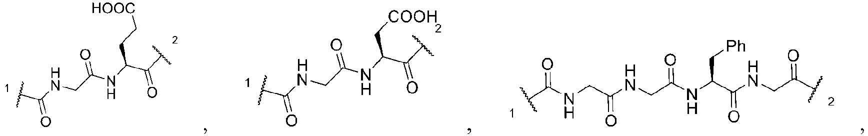 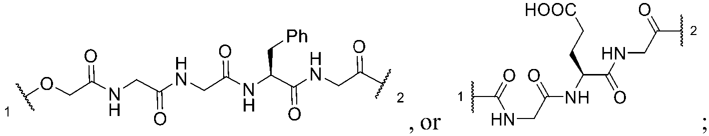 wherein position "1" is connected to ring E, and position "2" is connected to Tr;
preferably, 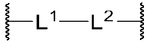 is: 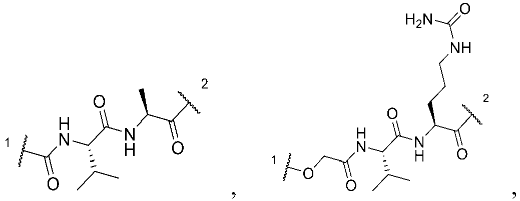 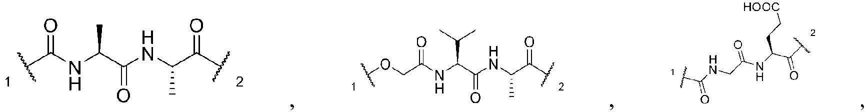 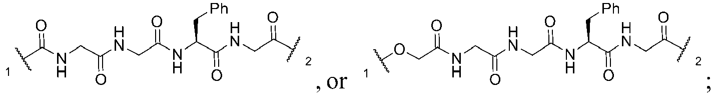 wherein position "1" is connected to ring E, and position "2" is connected to Tr;
more preferably, 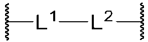 is 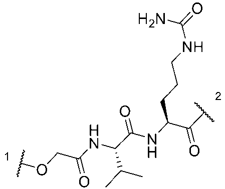 or 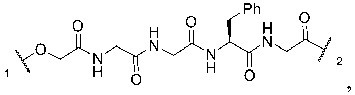 wherein position "1" is connected to ring E, and position "2" is connected to Tr.

4. The compound of formula (III), or the mesomer, racemate, enantiomer, diastereomer, or any mixture thereof, or the pharmaceutically acceptable salt thereof, or the solvate thereof, or the pharmaceutically acceptable salt of the solvate thereof according to claim 1, wherein the compound of formula (III), or the mesomer, racemate, enantiomer, diastereomer, or any mixture thereof, or the pharmaceutically acceptable salt thereof, or the solvate thereof, or the pharmaceutically acceptable salt of the solvate thereof satisfies one or more of the following conditions,
(1) in ring G, the 5- to 10-membered heteroaryl is 5- to 6-membered heteroaryl;
(2) in ring G, the heteroatom of the 5- to 10-membered heteroaryl is N and/or O, and the number of heteroatoms is 1, 2, or 3; preferably, the heteroatom of the 5- to 10-membered heteroaryl is N, and the number of heteroatoms is 1, 2, or 3, for example, 2;
(3) in L¹, n is a natural number from 0 to 50; preferably, n is a natural number from 1 to 10, for example, 2 or 6;
(4) in L¹, each of R^{L11} and R^{L12} is hydrogen;
(5) in L¹, any -C(R^{L11})₂- moiety in L¹ is independently and optionally replaced by the following structural moieties: -NH-, -C(O)-, -O-, or -S-; preferably, any -C(R^{L11})₂- in L¹ may be each independently replaced by the following structural moieties: -C(O)-, -O-, or -S-;
(6) R^{Tr}, R^{Tr1}, and R^{Tr2} in Tr are each independently hydrogen or methyl; preferably hydrogen;
(7) Tr is a chemical bond or selected from one or more of 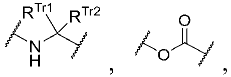 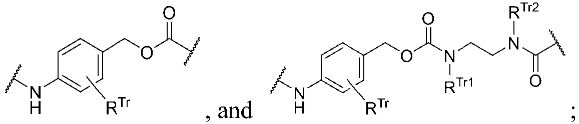
(8) the 4- to 12-membered heterocycloalkylene in ring E is 4- to 6-membered heterocycloalkylene;
(9) the heteroatom of the 4- to 12-membered heterocycloalkylene in ring E is N, and the number of heteroatoms is 1 or 2;
(10) the 4- to 12-membered heterocycloalkylene in ring E is optionally substituted by one or more -C(O)NH-(CH₂CH₂O)_{y1}CH₃ substituents;
(11) y1 in ring E is a natural number from 0 to 30, preferably a natural number from 0 to 10; for example, 6 or 7; for another example, -C(O)NH-(CH₂CH₂O)_{y1}CH₃ is

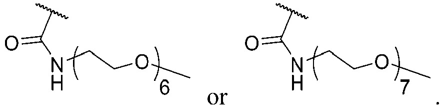

5. The compound of formula (III), or the mesomer, racemate, enantiomer, diastereomer, or any mixture thereof, or the pharmaceutically acceptable salt thereof, or the solvate thereof, or the pharmaceutically acceptable salt of the solvate thereof according to claim 4, wherein the compound of formula (III), or the mesomer, racemate, enantiomer, diastereomer, or any mixture thereof, or the pharmaceutically acceptable salt thereof, or the solvate thereof, or the pharmaceutically acceptable salt of the solvate thereof satisfies one or more of the following conditions,
(1) ring E is 4- to 6-membered heterocycloalkylene, wherein the heterocycloalkylene is optionally substituted by one or more substituents selected from hydrogen, deuterium, halogen, hydroxyl, amino, cyano, nitro, and -C(O)NH-(CH₂CH₂O)_{y1}CH₃; preferably, the heteroatom of the 4- to 6-membered heterocycloalkylene is N, and the number of heteroatoms is 1 or 2; preferably, the 4- to 6-membered heterocycloalkylene is optionally substituted by one or more -C(O)NH-(CH₂CH₂O)_{y1}CH₃ substituents, wherein y1 is a natural number from 0 to 30; more preferably, ring E is 4- to 6-membered heterocycloalkylene, wherein the heteroatom of the 4-to 6-membered heterocycloalkylene is N, and the number of heteroatoms is 1;
preferably, ring E is 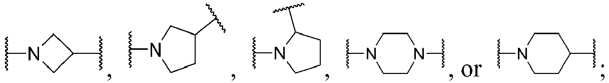 further preferably, ring E is 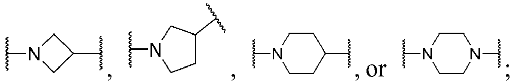 still further preferably, ring E is 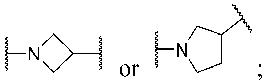
(2) L¹ is -(CH₂)ₙ-; n is a natural number from 0 to 50; any -CH₂- moiety in L¹ is independently and optionally replaced by the following structural moieties: -NH-, -C(O)-, -O-, or -S-;
preferably, L¹is Scheme 1 or Scheme 2:
Scheme 1: L¹ is 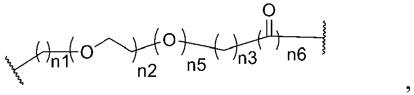 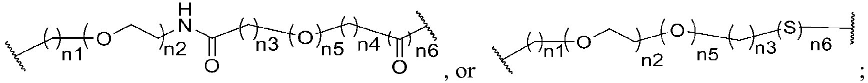
wherein
n1, n2, n3, and n4 are each independently 0, 1, 2, 3, 4, 5, 6, 7, or 8;
n5 and n6 are each independently 0 or 1;
for example, L¹ is 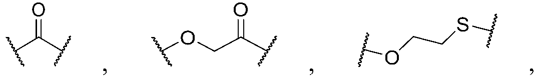
or 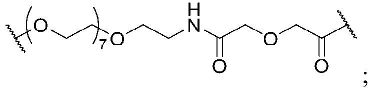
Scheme 2: L¹ is 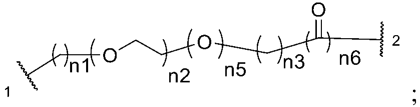 wherein position "1" is connected to ring E, and position "2" is connected to L²;
n1 and n2 are 0; n5 is 0 or 1; n3 is 0, 1, 2, 3, or 4; n6 is 1; preferably, n5 is 1;
for example, L¹ is 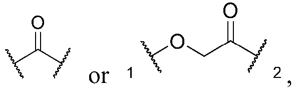 wherein position "1" is connected to ring E, and position "2" is connected to L²; preferably 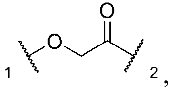 wherein position "1" is connected to ring E, and position "2" is connected to L²;
(3) L² is Scheme 1 or Scheme 2; Scheme 1: L² is a chemical bond, Val-Ala, Val-Cit, Ala-Ala, Gly-Glu, Gly-Asp, Gly-Glu-Gly, or Gly-Gly-Phe-Gly; Scheme 2: L² is Val, Ala, Gly, Glu, Cit, Asp, Phe, Val-Ala, Ala-Ala, Gly-Glu, Gly-Asp, Gly-Glu-Gly, Gly-Gly-Phe-Gly, or Val-Cit; wherein the N-terminus is connected to ring E, and the C-terminus is connected to L²; preferably, L² is Val-Ala, Ala-Ala, Gly-Glu, Gly-Asp, Gly-Glu-Gly, Gly-Gly-Phe-Gly, or Val-Cit, wherein the N-terminus is connected to ring E, and the C-terminus is connected to L²; more preferably, L² is Val-Ala, Ala-Ala, Gly-Glu, Gly-Gly-Phe-Gly, or Val-Cit, wherein the N-terminus is connected to ring E, and the C-terminus is connected to L²; most preferably, L² is Gly-Gly-Phe-Gly or Val-Cit, wherein the N-terminus is connected to ring E, and the C-terminus is connected to L²;
(4) Tr is Scheme 1 or Scheme 2: Scheme 1: Tr is a chemical bond, 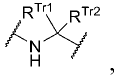 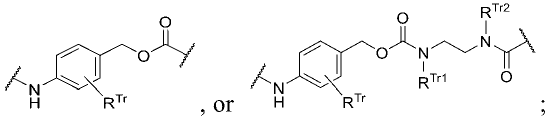 preferably, R^{Tr}, R^{Tr1}, and R^{Tr2} are each independently hydrogen or -C₁₋₆ alkyl; for example, Tr is a chemical bond, 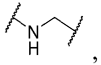 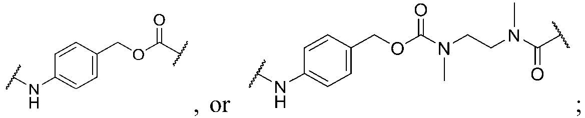 Scheme 2: Tr is 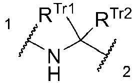 or 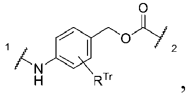 wherein position "1" is connected to L², and position "2" is connected to D; R^{Tr}, R^{Tr1}, and R^{Tr2} are each independently hydrogen or -C₁₋₆ alkyl, for example, Tr is 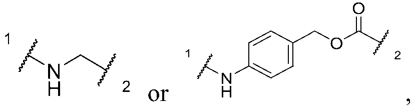 wherein position "1" is connected to L², and position "2" is connected to D;
(5) D is a tubulin inhibitor, a topoisomerase inhibitor, a DNA damaging agent, an RNA polymerase inhibitor, an immunomodulator, or an immunostimulant, for example, D is 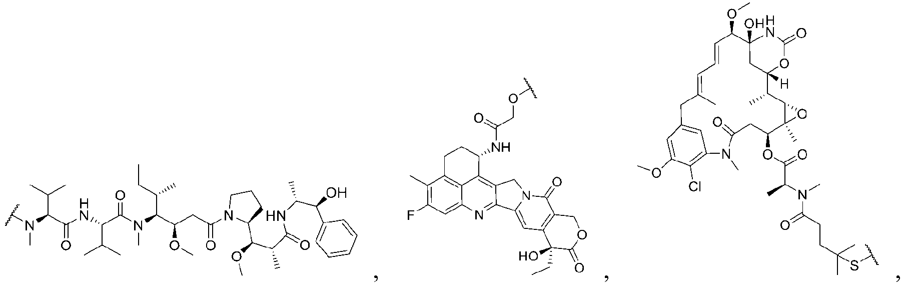 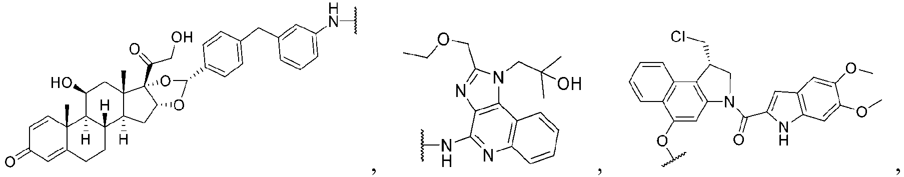 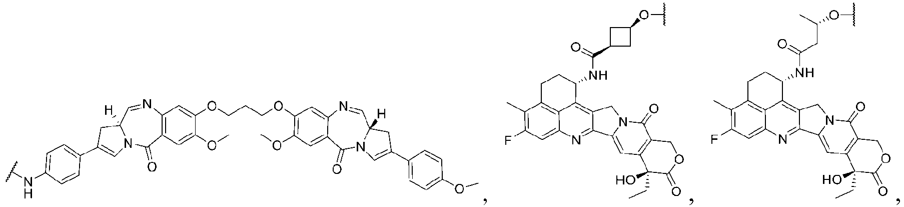 or 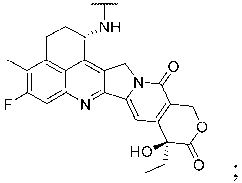 preferably, D is 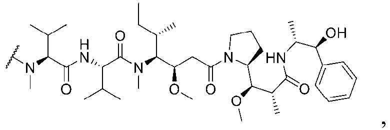 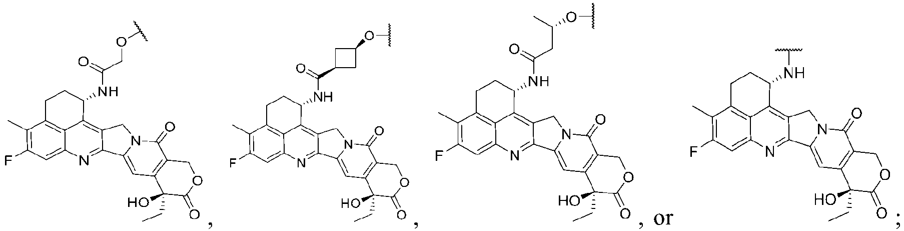 more preferably, D is 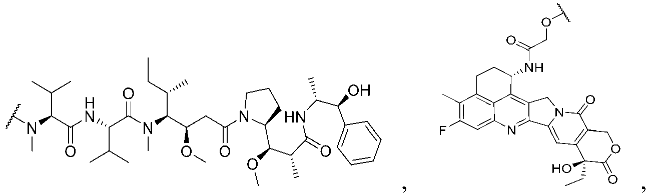 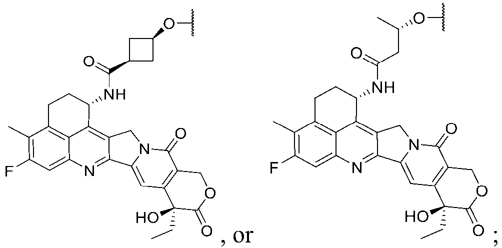 most preferably, D is 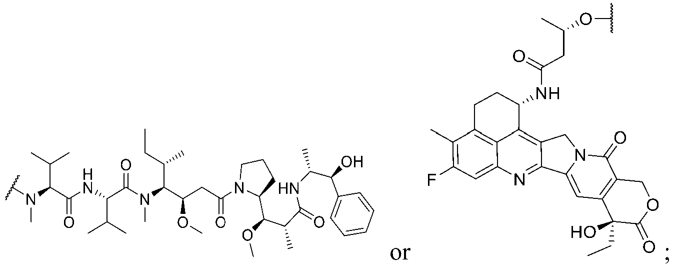
(6) ring G is 5- to 6-membered heteroaryl, wherein the heteroatom of the 5- to 6-membered heteroaryl is N and/or O, and the number of heteroatoms is 1, 2, or 3; preferably, ring G is 5-to 6-membered heteroaryl, wherein the heteroatom of the 5- to 6-membered heteroaryl is N, and the number of heteroatoms is 1, 2, or 3, for example, 2.

6. The compound of formula (III), or the mesomer, racemate, enantiomer, diastereomer, or any mixture thereof, or the pharmaceutically acceptable salt thereof, or the solvate thereof, or the pharmaceutically acceptable salt of the solvate thereof according to claim 1, wherein the compound of formula (III) is selected from the following Scheme 1, Scheme 2, Scheme 3, Scheme 4, or Scheme 5:
Scheme 1: R¹ is -C₁₋₆ alkyl;
ring G is 5- to 6-membered heteroaryl, wherein the heteroatom of the 5- to 6-membered heteroaryl may be N and/or O, and the number of heteroatoms is 1, 2, or 3; for example, ring G is 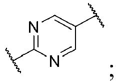
ring E is 4- to 6-membered heterocycloalkylene, wherein the heteroatom of the 4- to 6-membered heterocycloalkylene is N, and the number of heteroatoms is 1 or 2;
the 4- to 6-membered heterocycloalkylene is optionally substituted by one or more - C(O)NH-(CH₂CH₂O)_{y1}CH₃ substituents;
y1 is a natural number from 0 to 30;
L¹ is -(CH₂)ₙ-; wherein n is a natural number from 0 to 50; any -CH₂- moiety in L¹ is independently and optionally replaced by the following structural moieties: -NH-, -C(O)-, -O-, or -S-;
L² is a chemical bond, Val-Ala, Ala-Ala, Gly-Glu, Gly-Asp, Gly-Glu-Gly, or Gly-Gly-Phe-Gly;
Tr is a chemical bond, 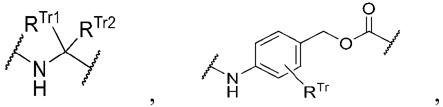 or 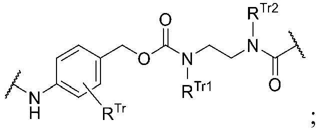 R^{Tr}, R^{Tr1}, and R^{Tr2} are each independently hydrogen or -C₁₋₆ alkyl;
D is a tubulin inhibitor, a topoisomerase inhibitor, a DNA damaging agent, an RNA polymerase inhibitor, an immunomodulator, or an immunostimulant, for example, D is 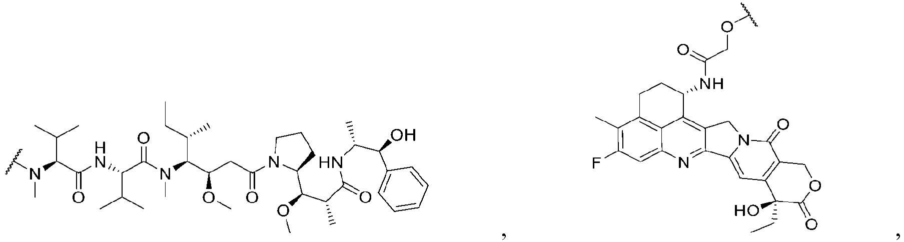 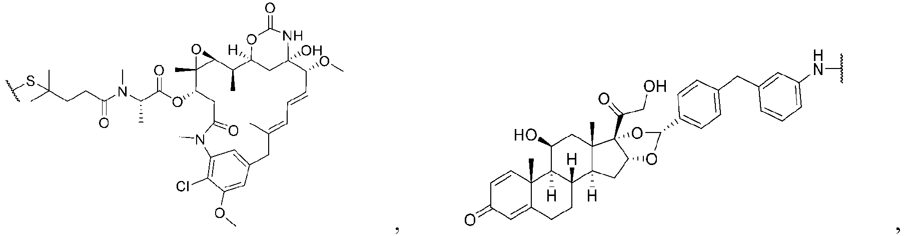 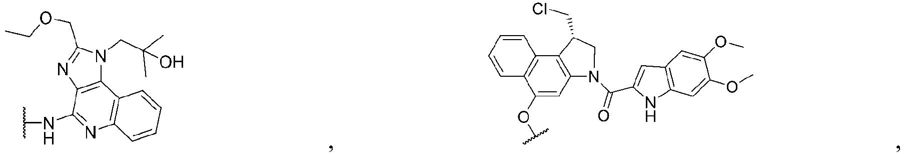 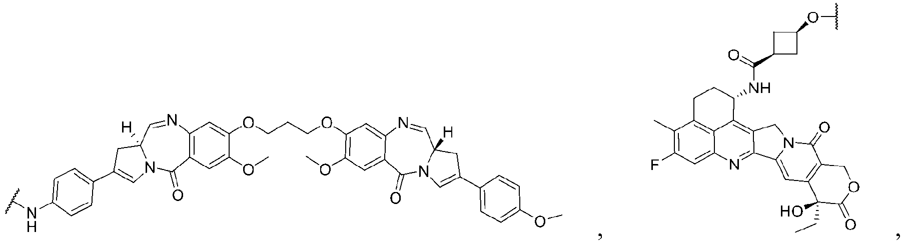 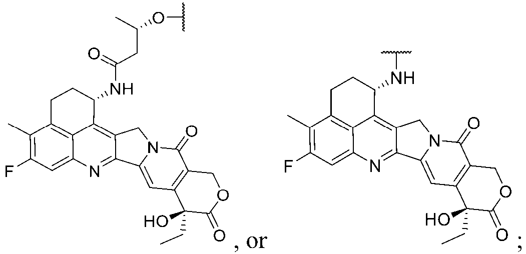
Scheme 2: R¹ is -C₁₋₆ alkyl;
ring G is 5- to 6-membered heteroaryl, wherein the heteroatom of the 5- to 6-membered heteroaryl is N, and the number of heteroatoms is 1, 2, or 3;
ring E is 4- to 6-membered heterocycloalkylene, wherein the heteroatom of the 4- to 6-membered heterocycloalkylene is N, and the number of heteroatoms is 1 or 2;
L¹ is 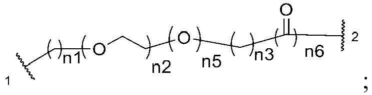 wherein position "1" is connected to ring E, and position "2" is connected to L²;
each of n1 and n2 is 0; n3 is 0, 1, 2, 3, or 4; n5 is 0 or 1; n6 is 1;
L² is Val-Ala, Ala-Ala, Gly-Glu, Gly-Asp, Gly-Glu-Gly, Gly-Gly-Phe-Gly, or Val-Cit; wherein the N-terminus is connected to ring E, and the C-terminus is connected to L²;
Tr is 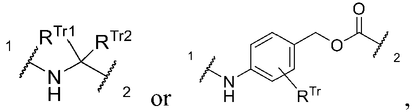 wherein position "1" is connected to L², and position "2" is connected to D; R^{Tr}, R^{Tr1}, and R^{Tr2} are each independently hydrogen or -C₁₋₆ alkyl;
D is 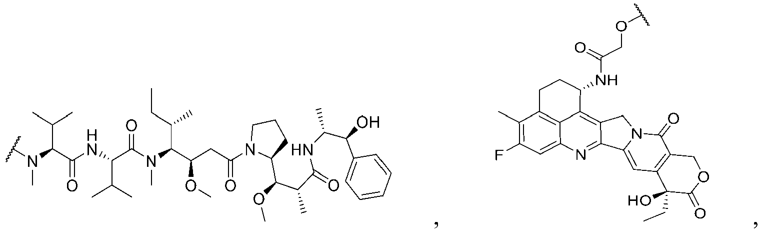 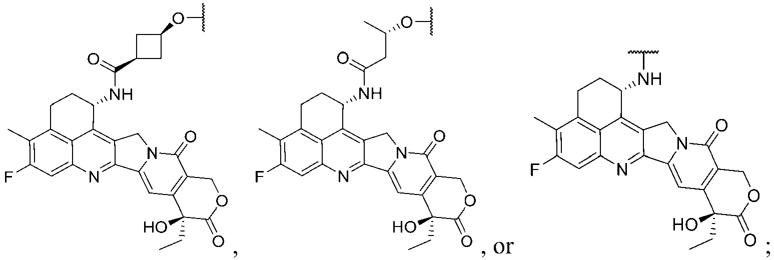
Scheme 3: R¹ is -C₁₋₆ alkyl;
ring G is 5- to 6-membered heteroaryl, wherein the heteroatom of the 5- to 6-membered heteroaryl is N, and the number of heteroatoms is 1, 2, or 3;
ring E is 4- to 6-membered heterocycloalkylene, wherein the heteroatom of the 4- to 6-membered heterocycloalkylene is N, and the number of heteroatoms is 1 or 2;
L¹ is 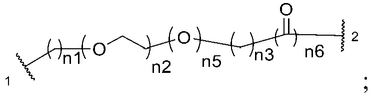 wherein position "1" is connected to ring E, and position "2" is connected to L²;
each of n1 and n2 is 0; n3 is 0, 1, 2, 3, or 4; n5 is 0 or 1; n6 is 1;
L² is Val-Ala, Ala-Ala, Gly-Glu, Gly-Gly-Phe-Gly, or Val-Cit, wherein the N-terminus is connected to ring E, and the C-terminus is connected to L²;
Tr is 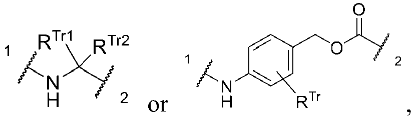 wherein position "1" is connected to L², and position "2" is connected to D; R^{Tr}, R^{Tr1}, and R^{Tr2} are each independently hydrogen or -C₁₋₆ alkyl;
D is 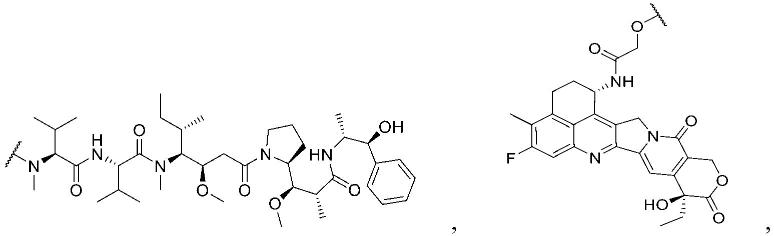 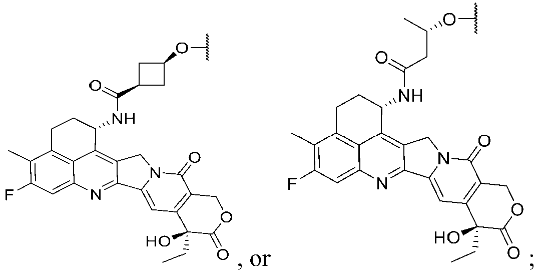
Scheme 4: R¹ is -C₁₋₆ alkyl;
ring G is 5- to 6-membered heteroaryl, wherein the heteroatom of the 5- to 6-membered heteroaryl is N, and the number of heteroatoms is 1, 2, or 3;
ring E is 4- to 6-membered heterocycloalkylene, wherein the heteroatom of the 4- to 6-membered heterocycloalkylene is N, and the number of heteroatoms is 1 or 2;
L¹ is 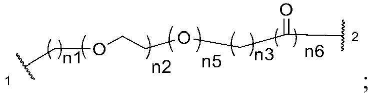 wherein position "1" is connected to ring E, and position "2" is connected to L²;
each of n1 and n2 is 0; n3 is 1, 2, 3, or 4; n5 is 1; n6 is 1;
L² is Gly-Gly-Phe-Gly or Val-Cit; wherein the N-terminus is connected to ring E, and the C-terminus is connected to L²;
Tr is 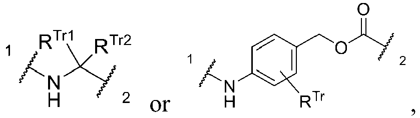 wherein position "1" is connected to L², and position "2" is connected to D; R^{Tr}, R^{Tr1}, and R^{Tr2} are each independently hydrogen or -C₁₋₆ alkyl;
D is 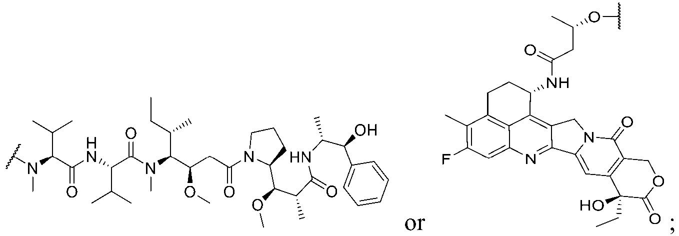
Scheme 5: the compound of formula (III) is a compound of the following formula: 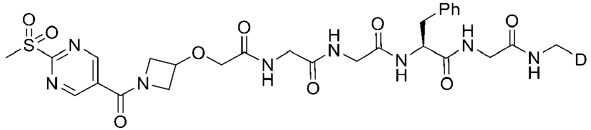 or 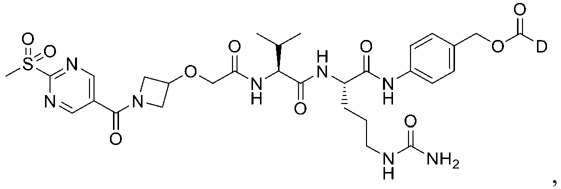
wherein D is as defined in any one of claims 1-5.

7. The compound of formula (III), or the mesomer, racemate, enantiomer, diastereomer, or any mixture thereof, or the pharmaceutically acceptable salt thereof, or the solvate thereof, or the pharmaceutically acceptable salt of the solvate thereof according to any one of claims 1-6, wherein the compound of formula (III) is a compound of formula (IIIa), 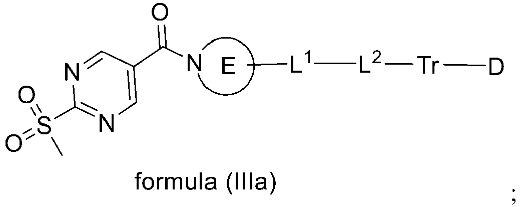 preferably, the compound of formula (III) is any one of the following compounds,
| | |
|---|---|
| C1 | 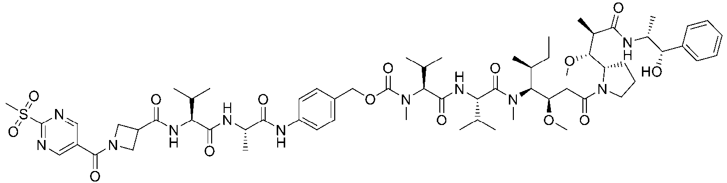 |
| C2 | 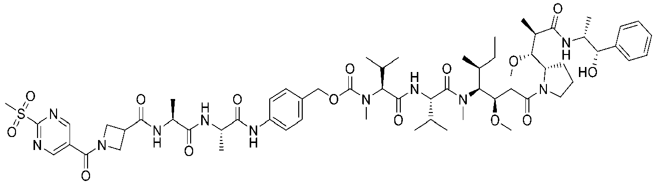 |
| C3 | 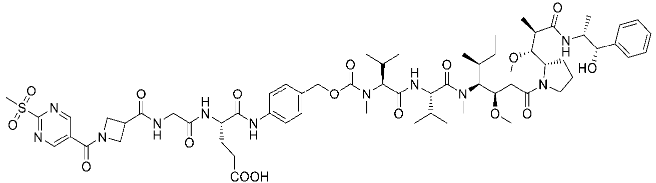 |
| C4 | 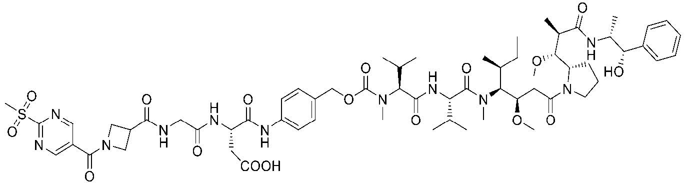 |
| C5 | 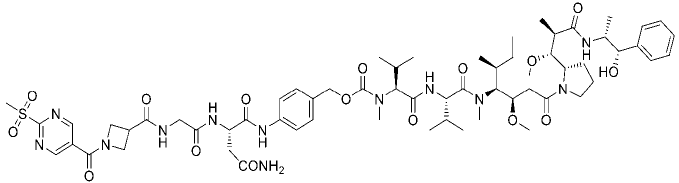 |
| C6 | 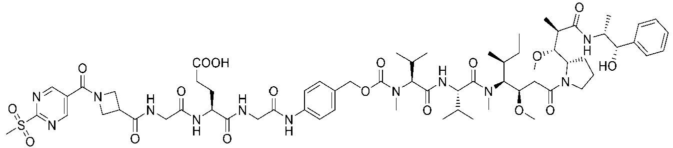 |
| C7 | 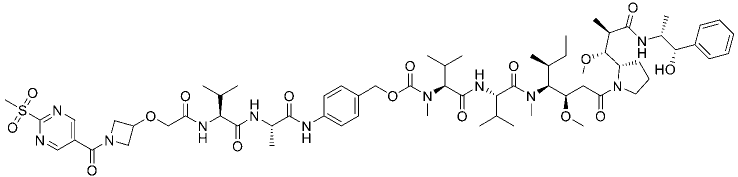 |
| C8 | 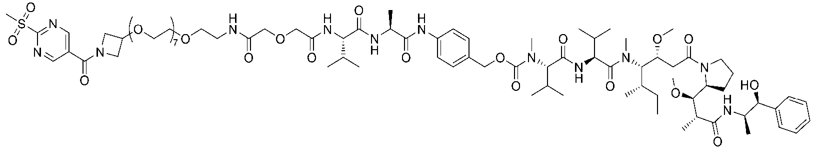 |
| C9 | 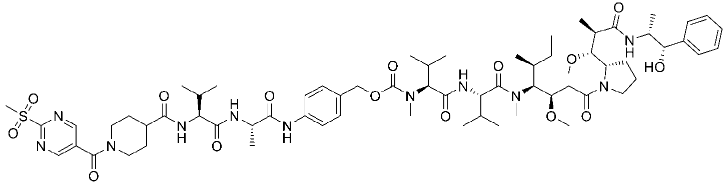 |
| C10 | 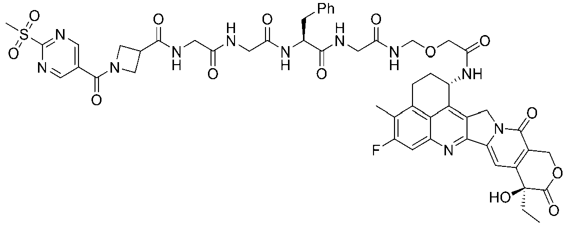 |
| C11 | 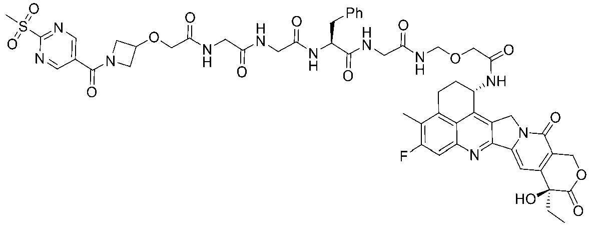 |
| C12 | 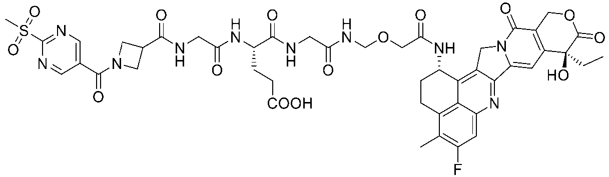 |
| C13 | 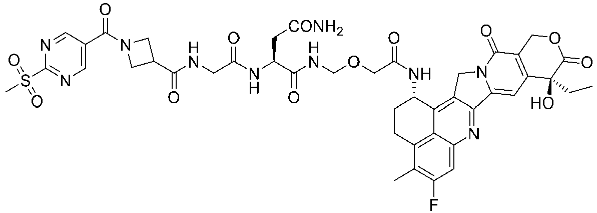 |
| C14 | 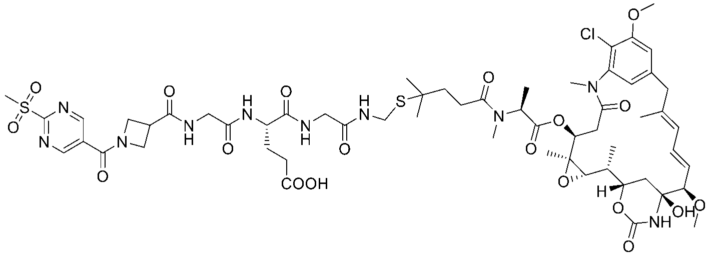 |
| C15 | 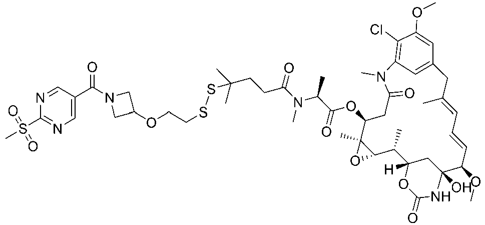 |
| C16 | 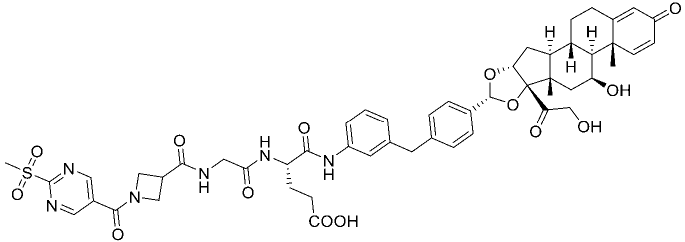 |
| C17 | 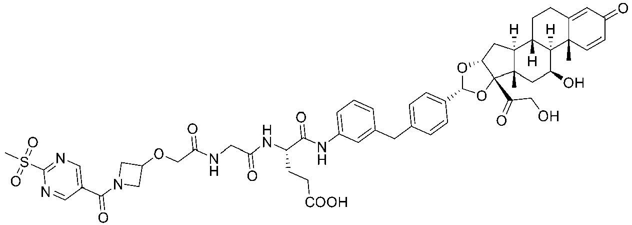 |
| C18 | 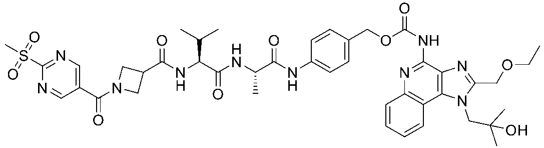 |
| C19 | 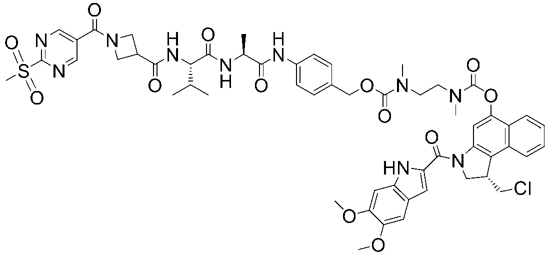 |
| C20 | 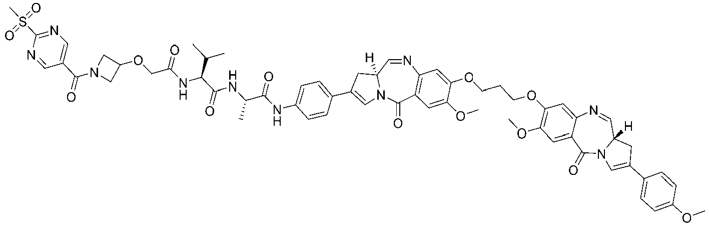 |
| C21 | 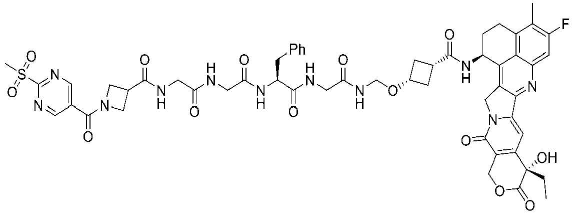 |
| C22 | 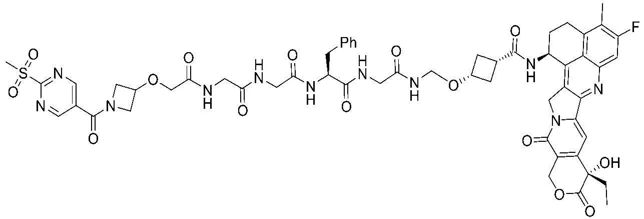 |
| C23 | 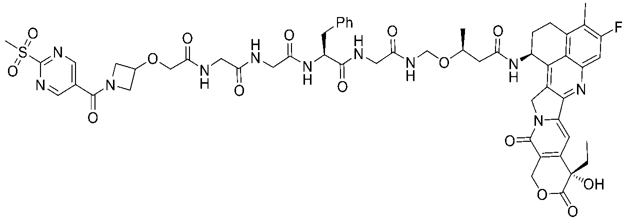 |
| C24 | 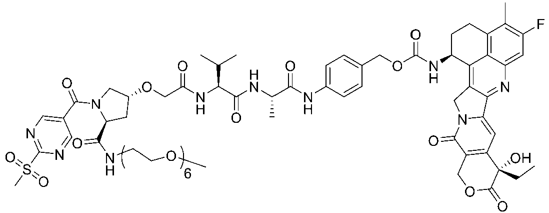 |
| C25 | 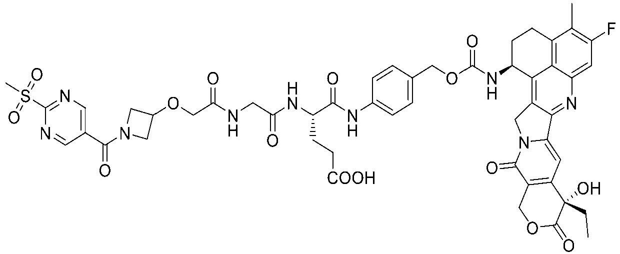 |
| C26 | 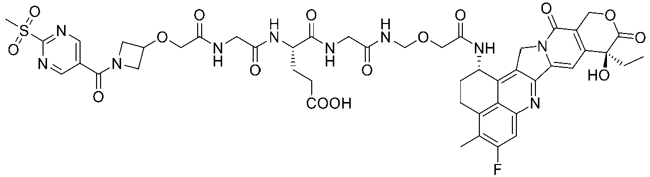 |
| C27 | 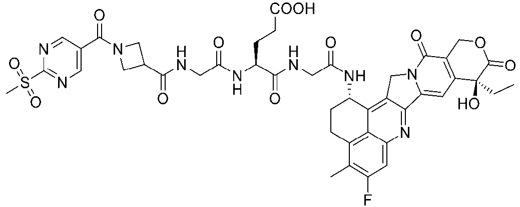 |
| C28 | 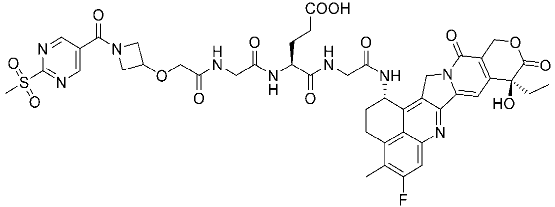 |
| C29 | 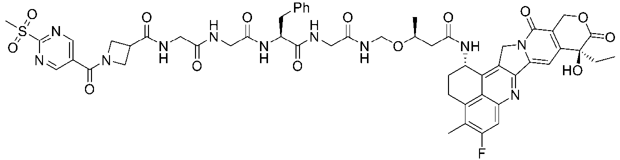 |
| C30 | 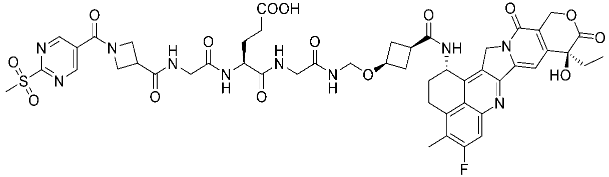 |
| C31 | 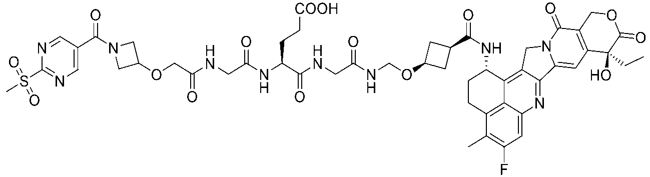 |
| C32 | 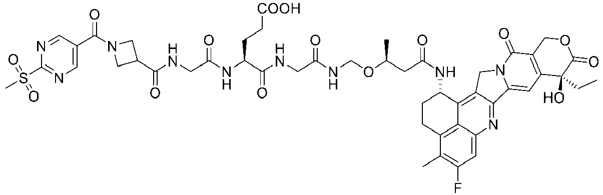 |
| C33 | 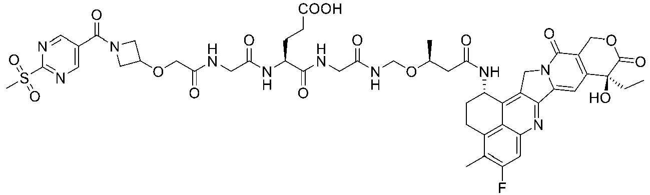 |
| C34 | 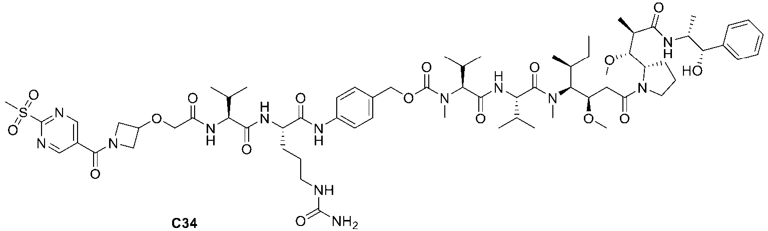 |

8. A compound of formula (III-1), or a mesomer, racemate, enantiomer, diastereomer, or any mixture thereof, or a pharmaceutically acceptable salt thereof, or a solvate thereof, or a pharmaceutically acceptable salt of the solvate thereof: 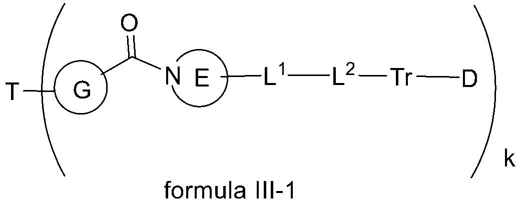 wherein
T is a ligand binding to a target, wherein the T forms a covalent linkage with ring G via a thiol or amino group thereon;
k is a natural number or a decimal from 1 to 16;
the ring G, ring E, L¹, L², Tr, and D are as defined in any one of claims 1-7.

9. The compound of formula (III-1), or the mesomer, racemate, enantiomer, diastereomer, or any mixture thereof, or the pharmaceutically acceptable salt thereof, or the solvate thereof, or the pharmaceutically acceptable salt of the solvate thereof according to claim 8, wherein T is a target-binding polypeptide, an antibody, or an antigen-binding fragment thereof; preferably, T is an antibody or an antigen-binding fragment thereof;
preferably, the compound of formula (III-1) satisfies one or more of the following conditions,
(1) the antibody is a fully human antibody, a humanized antibody, a chimeric antibody, a probody, a bispecific antibody, a multispecific antibody, a monoclonal antibody, or a polyclonal antibody; preferably, the antibody is selected from Scheme 1 or Scheme 2; Scheme 1: the antibody is an anti-HER2 antibody (e.g., Trastuzumab or Pertuzumab) or a variant thereof, a Trop-2 antibody (Sacituzumab, M1, M2, or M3) or a variant thereof, or a TNF-α antibody (Adalimumab); preferably, the antibody is Trastuzumab, Sacituzumab, Adalimumab, or a monoclonal antibody; Scheme 2: the antibody is an anti-HER2 antibody (e.g., Trastuzumab or Pertuzumab) or a variant thereof, an anti-Trop-2 antibody (Sacituzumab, M1, M2, or M3) or a variant thereof, an anti-TNF-α antibody (Adalimumab) or a variant thereof, an anti-GPC-3 antibody (e.g., DB1002 antibody) or a variant thereof, or an anti-ADAM9 antibody (e.g., DB1001 antibody) or a variant thereof; preferably, the antibody is Trastuzumab, Sacituzumab, Adalimumab, DB1001 antibody, or DB 1002 antibody; preferably, the antibody is Trastuzumab, Sacituzumab, DB1001 antibody, or DB1002 antibody; more preferably, the antibody is DB1001 antibody or DB1002 antibody;
(2) the antigen-binding fragment is Fab, Fab', F(ab')₂, Fv, scFv, a diabody, Fd, dAb, VHH, or a complementarity determining region (CDR) fragment;
(3) a T-targeted antigen is Scheme 1 or Scheme 2; Scheme 1: 5T4, AGS-16, ANGPTL4, ApoE, CD19, CTGF, CXCR5, FGF2, MCPT8, MFI2, MS4A7, NCA, Sema5b, SLITRK6, STC2, TGF, 0772P, 5T4, ACTA2, ADGRE1, AG-7, AIF1, AKR1C1, AKR1C2, ASLG659, Axl, B7H3, BAFF-R, BCMA, BMPR1B, BNIP3, C1QA, C1QB, CA6, CADM1, CCD79b, CCL5, CCR5, CCR7, CD11c, CD123, CD138, CD142, CD147, CD166, CD19, CD19,CD22, CD21, CD20, CD205, CD22, CD223, CD228, CD25, CD30, CD33, CD37, CD38, CD40, CD45, CD45(PTPRC), CD46, CD47, CD49D(ITGA4), CD56, CD66e, CD70, CD71, CD72, CD74, CD79a, CD79b, CD80, CDCP1, CDH11, CDllb, CEA, CEACAM5, c-Met, COL6A3, COL7A1, CRIPTO, CSF1R, CTSD, CTSS, CXCL11, CXCL10, DDIT4, DLL3, DLL4, DR5, E16, EFNA4, EGFR, EGFRvIII, EGLN, EGLN3, EMR2, ENPP3, EpCAM, EphA2, EphB2R, ETBR, FcRH2, FcRH1, FGFR2, FGFR3, FLT3, FOLR-α, GD2, GEDA, GPC-1, GPNMB, GPR20, GZMB, HER2, HER3, HLA-DOB, HMOX1, IF16, IFNG, IGF-1R, IGFBP3, IL10RA1, IL-13R, IL-2, IL20Ra, IL-3, IL-4, IL-6, IRTA2, KISS1R, KRT33A, LIV-1, LOX, LRP-1, LRRC15, LUM, LY64, LY6E, Ly86, LYPD3, MDP, MMP10, MMP14, MMP16, MPF, MSG783, MSLN, MUC-1, NaPi2b, Napi3b, Nectin-4, Nectin-4, NOG, P2X5, pCAD, P-Cadherin, PDGFRA, PDK1, PD-L1, PFKFB3, PGF, PGK1, PIK3AP1, PIK3CD, PLOD2, PSCA, PSCAhlg, PSMA, PSMA, PTK7, P-cadherin, RNF43, NaPi2b, ROR1, ROR2, SERPINE1, SLC39A6, SLTRK6, STAT1, STEAP1, STEAP2, TCF4, TENB2, TGFB1, TGFB2, TGFBR1, TNFRSF21, TNFSF9, TNF-α, Trop-2, TrpM4, Tyro7, UPK1B, VEGFA, WNT5A, ADAM9, epidermal growth factor, brevican, mesothelin, sodium phosphate cotransporter 2B, Claudin18.2, endothelin receptor, mucin (such as mucin 1 and mucin 16), guanylate cyclase C, integrin a4p7, integrin a5p6, trophoblast glycoprotein, or tissue factor; Scheme 2: 5T4, AGS-16, ANGPTL4, ApoE, CD19, CTGF, CXCR5, FGF2, MCPT8, MFI2, MS4A7, NCA, Sema5b, SLITRK6, STC2, TGF, 0772P, 5T4, ACTA2, ADGRE1, AG-7, AIF1, AKR1C1, AKR1C2, ASLG659, Axl, B7H3, BAFF-R, BCMA, BMPR1B, BNIP3, C1QA, C1QB, CA6, CADM1, CCD79b, CCL5, CCR5, CCR7, CD11c, CD123, CD138, CD142, CD147, CD166, CD19, CD19,CD22, CD21, CD20, CD205, CD22, CD223, CD228, CD25, CD30, CD33, CD37, CD38, CD40, CD45, CD45(PTPRC), CD46, CD47, CD49D(ITGA4), CD56, CD66e, CD70, CD71, CD72, CD74, CD79a, CD79b, CD80, CDCP1, CDH11, CDllb, CEA, CEACAM5, c-Met, COL6A3, COL7A1, CRIPTO, CSF1R, CTSD, CTSS, CXCL11, CXCL10, DDIT4, DLL3, DLL4, DR5, E16, EFNA4, EGFR, EGFRvIII, EGLN, EGLN3, EMR2, ENPP3, EpCAM, EphA2, EphB2R, ETBR, FcRH2, FcRH1, FGFR2, FGFR3, FLT3, FOLR-α, GD2, GEDA, GPC-1, GPC-3, ADAM9, GPNMB, GPR20, GZMB, HER2, HER3, HLA-DOB, HMOX1, IFI6, IFNG, IGF-1R, IGFBP3, IL10RA1, IL-13R, IL-2, IL20Ra, IL-3, IL-4, IL-6, IRTA2, KISS1R, KRT33A, LIV-1, LOX, LRP-1, LRRC15, LUM, LY64, LY6E, Ly86, LYPD3, MDP, MMP10, MMP14, MMP16, MPF, MSG783, MSLN, MUC-1, NaPi2b, Napi3b, Nectin-4, Nectin-4, NOG, P2X5, pCAD, P-Cadherin, PDGFRA, PDK1, PD-L1, PFKFB3, PGF, PGK1, PIK3AP1, PIK3CD, PLOD2, PSCA, PSCAhlg, PSMA, PSMA, PTK7, P-cadherin, RNF43, NaPi2b, ROR1, ROR2, SERPINE1, SLC39A6, SLTRK6, STAT1, STEAP1, STEAP2, TCF4, TENB2, TGFB1, TGFB2, TGFBR1, TNFRSF21, TNFSF9, TNF-α, Trop-2, TrpM4, Tyro7, UPK1B, VEGFA, WNT5A, ADAM9, epidermal growth factor, brevican, mesothelin, sodium phosphate cotransporter 2B, Claudin18.2, endothelin receptor, mucin (such as mucin 1 and mucin 16), guanylate cyclase C, integrin a4p7, integrin a5p6, trophoblast glycoprotein, or tissue factor;
preferably, the T-targeted antigen is: HER2, HER3, B7H3, TNF-α, TROP2, Claudin18.2, CD30, CD33, CD70, ADAM9, EGFR, GPC-3, or ADAM9; and
(4) k is a natural number or a decimal from 2 to 8, for example, 1.9, 3.6, 3.7, 3.8, 3.9, 4.0, 4.1, 4.3, 4.4, 7.4, 7.5, 7.6, 7.7, 7.8, or 7.9.

10. The compound of formula (III-1), or the mesomer, racemate, enantiomer, diastereomer, or any mixture thereof, or the pharmaceutically acceptable salt thereof, or the solvate thereof, or the pharmaceutically acceptable salt of the solvate thereof according to claim 9, wherein the anti-ADAM9 antibody comprises a heavy chain variable region and a light chain variable region; the heavy chain variable region comprises heavy chain complementarity determining regions HCDR1, HCDR2, and HCDR3, and the light chain variable region comprises light chain complementarity determining regions LCDR1, LCDR2, and LCDR3;
the HCDR1 comprises an amino acid sequence as shown in LYWMX₁, the HCDR2 comprises an amino acid sequence as shown in X₂IIPIFGHTX₃YX₄EKFX₅X₆, the HCDR3 comprises an amino acid sequence as shown in SEQ ID NO: 9, the LCDR1 comprises an amino acid sequence as shown in SEQ ID NO: 10, the LCDR2 comprises an amino acid sequence as shown in SEQ ID NO: 11, and the LCDR3 comprises an amino acid sequence as shown in SEQ ID NO: 12;
wherein X₁ is N, D, H, or E; X₂ is R or D; X₃ is D or K; X₄ is N or E; X₅ is K or R; X₆ is D or N;
preferably, in the compound of formula (III-1), in the HCDR1, X₁ is N or H; in the HCDR2, X₂ is R; X₃ is K; X₄ is N; X₅ is K; X₆ is D or N; for example, in the compound of formula (III-1), X₁ is H, X₂ is R; X₃ is K; X₄ is N; X₅ is K; X₆ is N;
more preferably, in the compound of formula (III-1), the HCDR1 comprises an amino acid sequence as shown in SEQ ID NO: 7, the HCDR2 comprises an amino acid sequence as shown in SEQ ID NO: 8, the HCDR3 comprises an amino acid sequence as shown in SEQ ID NO: 9, the LCDR1 comprises an amino acid sequence as shown in SEQ ID NO: 10, the LCDR2 comprises an amino acid sequence as shown in SEQ ID NO: 11, and the LCDR3 comprises an amino acid sequence as shown in SEQ ID NO: 12; for example, the amino acid sequence of the HCDR1 is as shown in SEQ ID NO: 7, the amino acid sequence of the HCDR2 is as shown in SEQ ID NO: 8, the amino acid sequence of the HCDR3 is as shown in SEQ ID NO: 9, the amino acid sequence of the LCDR1 is as shown in SEQ ID NO: 10, the amino acid sequence of the LCDR2 is as shown in SEQ ID NO: 11, and the amino acid sequence of the LCDR3 is as shown in SEQ ID NO: 12;
further more preferably, in the compound of formula (III-1), the heavy chain variable region and/or the light chain variable region further comprises a framework region, wherein the framework region is a humanized framework region; the heavy chain variable region may comprise an amino acid sequence having at least 90%, at least 95%, or at least 99% sequence identity with SEQ ID NO: 13, and/or, the light chain variable region may comprise an amino acid sequence having at least 90%, at least 95%, or at least 99% sequence identity with SEQ ID NO: 14; the variable region of the amino acid sequence having at least 90%, at least 95%, or at least 99% sequence identity maintains antigen-binding function at least equivalent to that of an original sequence;
further more preferably, in the compound of formula (III-1), the amino acid sequence of the heavy chain variable region is as shown in SEQ ID NO: 13; and/or, the amino acid sequence of the light chain variable region is as shown in SEQ ID NO: 14;
preferably, the antibody or the antigen-binding fragment thereof: is (1) a full-length antibody, Fab, Fab', F(ab')₂, Fv, sdAb, or scFv; and/or, (2) is a monoclonal antibody, bispecific antibody, or multispecific antibody; and/or, (3) the ADAM9 is human or monkey ADAM9;
further more preferably, when the antibody or the antigen-binding fragment thereof is a full-length antibody, the full-length antibody comprises a heavy chain constant region of a heavy chain of a human antibody, preferably a heavy chain constant region of a human antibody IgG1; and/or, the full-length antibody comprises a light chain constant region of a light chain of a human antibody, preferably a light chain constant region of a human antibody κ chain; for example, the heavy chain constant region of the human antibody IgG1 has an amino acid sequence as shown in SEQ ID NO: 15 or having at least 90%, at least 95%, or at least 99% sequence identity with SEQ ID NO: 15; and/or, the light chain constant region of the human antibody κ chain has an amino acid sequence as shown in SEQ ID NO: 16 or having at least 90%, at least 95%, or at least 99% sequence identity with SEQ ID NO: 16; for another example, the amino acid sequence of the heavy chain constant region of the human antibody IgG1 is as shown in SEQ ID NO: 15, and the amino acid sequence of the light chain constant region of the human antibody κ chain is as shown in SEQ ID NO: 16; for yet another example, the heavy chain of the antibody or the antigen-binding fragment thereof comprises an amino acid sequence having at least 90%, at least 95%, or at least 99% sequence identity with SEQ ID NO: 18, and/or, the light chain of the antibody or the antigen-binding fragment thereof comprises an amino acid sequence having at least 90%, at least 95%, or at least 99% sequence identity with SEQ ID NO: 17; the amino acid sequence having at least 90%, at least 95%, or at least 99% sequence identity maintains antigen-binding function at least equivalent to that of the original sequence; for still another example, the amino acid sequence of the heavy chain of the antibody or the antigen-binding fragment thereof is as shown in SEQ ID NO: 18; and/or, the amino acid sequence of the light chain is as shown in SEQ ID NO: 17.

11. The compound of formula (III-1), or the mesomer, racemate, enantiomer, diastereomer, or any mixture thereof, or the pharmaceutically acceptable salt thereof, or the solvate thereof, or the pharmaceutically acceptable salt of the solvate thereof according to any one of claims 8-10, wherein the compound of formula (III-1) is selected from the following Scheme 1, Scheme 2, Scheme 3, or Scheme 4:
Scheme 1: R¹ is -C₁₋₆ alkyl;
ring G is 5- to 6-membered heteroaryl, wherein the heteroatom of the 5- to 6-membered heteroaryl may be N and/or O, and the number of heteroatoms is 1, 2, or 3; for example, ring G is 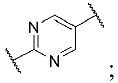
ring E is 4- to 6-membered heterocycloalkylene, wherein the heteroatom of the 4- to 6-membered heterocycloalkylene is N, and the number of heteroatoms is 1 or 2;
the 4- to 6-membered heterocycloalkylene is optionally substituted by one or more - C(O)NH-(CH₂CH₂O)_{y1}CH₃ substituents;
y1 is a natural number from 0 to 30;
L¹ is -(CH₂)ₙ-; wherein n is a natural number from 0 to 50; any -CH₂- moiety in L¹ is independently and optionally replaced by the following structural moieties: -NH-, -C(O)-, -O-, or -S-;
L² is a chemical bond, Val-Ala, Ala-Ala, Gly-Glu, Gly-Asp, Gly-Glu-Gly, or Gly-Gly-Phe-Gly;
Tr is a chemical bond, 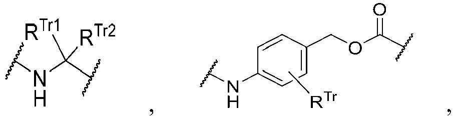 or 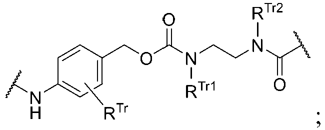
R^{Tr}, R^{Tr1}, and R^{Tr2} are each independently hydrogen or -C₁₋₆ alkyl;
D is a tubulin inhibitor, a topoisomerase inhibitor, a DNA damaging agent, an RNA polymerase inhibitor, an immunomodulator, or an immunostimulant, for example, D is 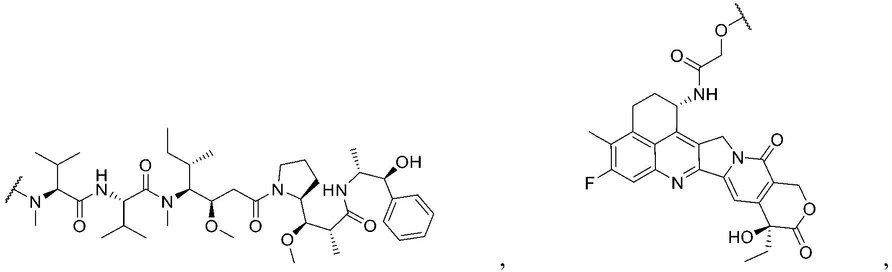 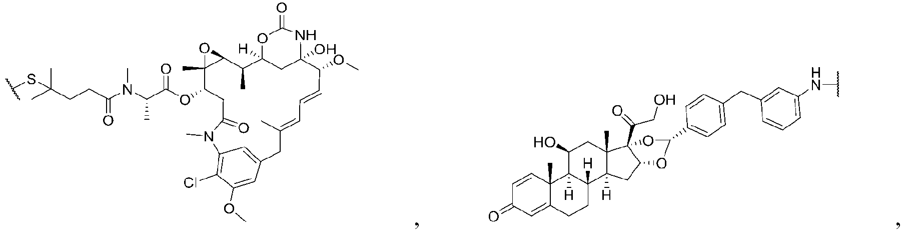 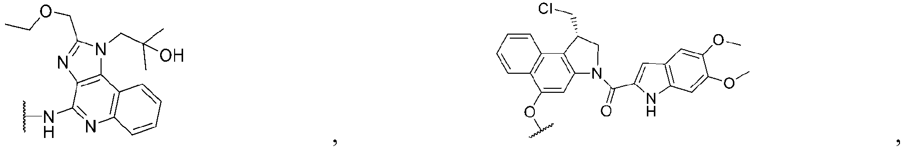 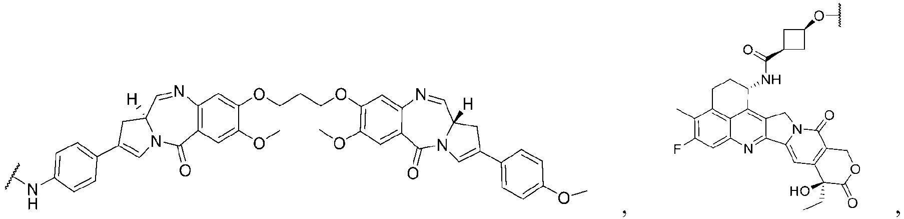 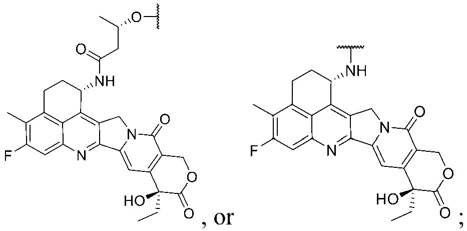
Scheme 2: R¹ is -C₁₋₆ alkyl;
ring G is 5- to 6-membered heteroaryl, wherein the heteroatom of the 5- to 6-membered heteroaryl is N, and the number of heteroatoms is 1, 2, or 3;
ring E is 4- to 6-membered heterocycloalkylene, wherein the heteroatom of the 4- to 6-membered heterocycloalkylene is N, and the number of heteroatoms is 1 or 2;
L¹ is 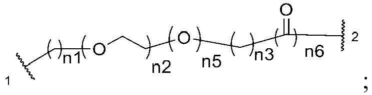 wherein position "1" is connected to ring E, and position "2" is connected to L²;
each of n1 and n2 is 0; n3 is 0, 1, 2, 3, or 4; n5 is 0 or 1; n6 is 1;
L² is Val-Ala, Ala-Ala, Gly-Glu, Gly-Gly-Phe-Gly, or Val-Cit, wherein the N-terminus is connected to ring E, and the C-terminus is connected to L²;
Tr is 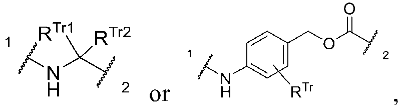 wherein position "1" is connected to L², and position "2" is connected to D; R^{Tr}, R^{Tr1}, and R^{Tr2} are each independently hydrogen or -C₁₋₆ alkyl;
D is 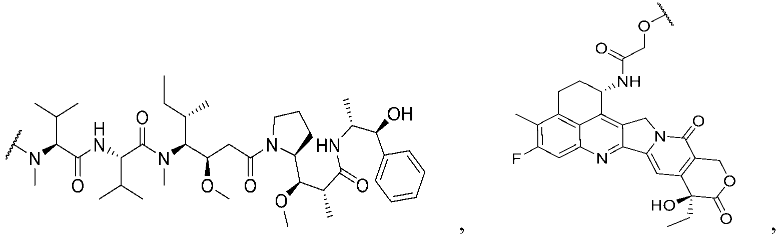 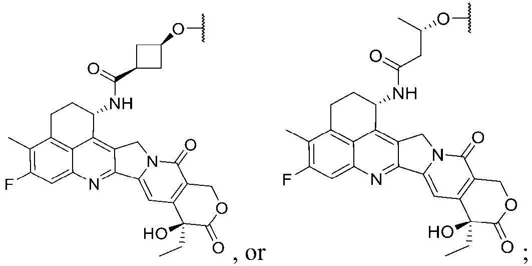
the T is Trastuzumab, Sacituzumab, DB1001 antibody, or DB1002 antibody;
k is a natural number or a decimal from 2 to 8;
Scheme 3: R¹ is -C₁₋₆ alkyl;
ring G is 5- to 6-membered heteroaryl, wherein the heteroatom of the 5- to 6-membered heteroaryl is N, and the number of heteroatoms is 1, 2, or 3;
ring E is 4- to 6-membered heterocycloalkylene, wherein the heteroatom of the 4- to 6-membered heterocycloalkylene is N, and the number of heteroatoms is 1 or 2;
L¹ is 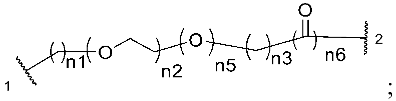 wherein position "1" is connected to ring E, and position "2" is connected to L²;
each of n1 and n2 is 0; n3 is 1, 2, 3, or 4; n5 is 1; n6 is 1;
L² is Gly-Gly-Phe-Gly or Val-Cit; wherein the N-terminus is connected to ring E, and the C-terminus is connected to L²;
Tr is 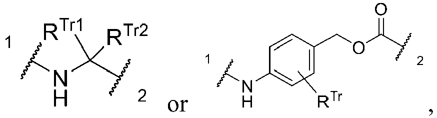 wherein position "1" is connected to L², and position "2" is connected to D; R^{Tr}, R^{Tr1}, and R^{Tr2} are each independently hydrogen or -C₁₋₆ alkyl;
D is 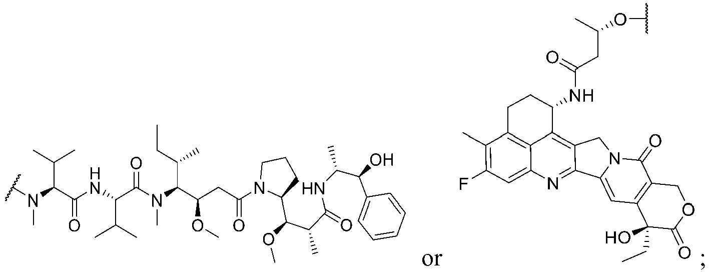
the T is DB 1001 antibody or DB1002 antibody;
k is a natural number or a decimal from 2 to 8;
Scheme 4: the compound of formula (III-1) is a compound of the following formula: 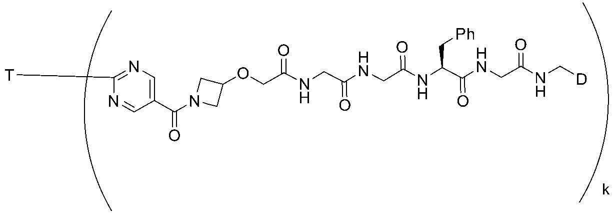 or 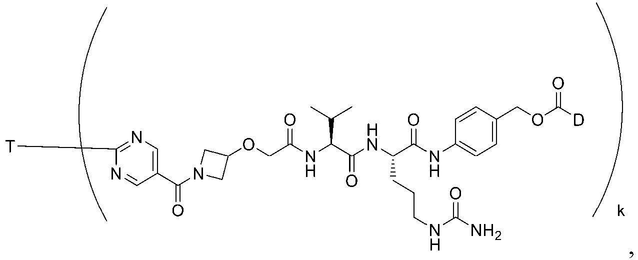
T and k are respectively as defined in any one of claims 8-10; D is as defined in any one of claims 1-7.

12. The compound of formula (III-1), or the mesomer, racemate, enantiomer, diastereomer, or any mixture thereof, or the pharmaceutically acceptable salt thereof, or the solvate thereof, or the pharmaceutically acceptable salt of the solvate thereof according to any one of claims 8-11, wherein the formula (III-1) is a compound of formula (IIIa-1), 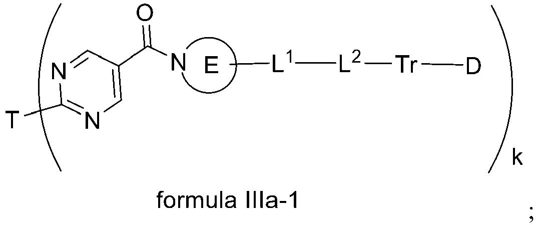 preferably, the compound of formula (III-1) is any one of the following compounds,
| | |
|---|---|
| CC1 | 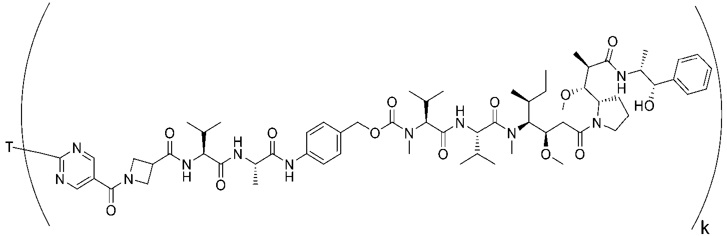 |
| CC2 | 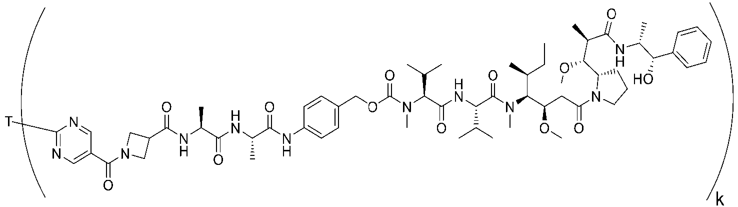 |
| CC3 | 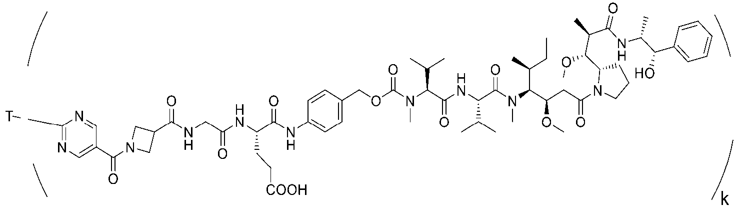 |
| CC4 | 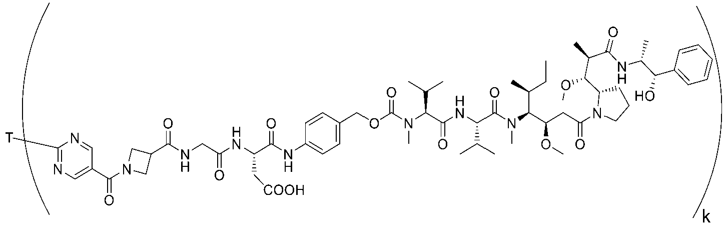 |
| CC5 | 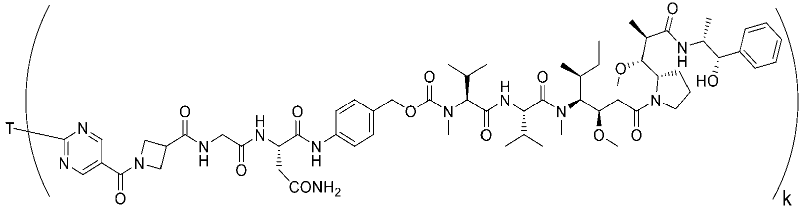 |
| CC6 | 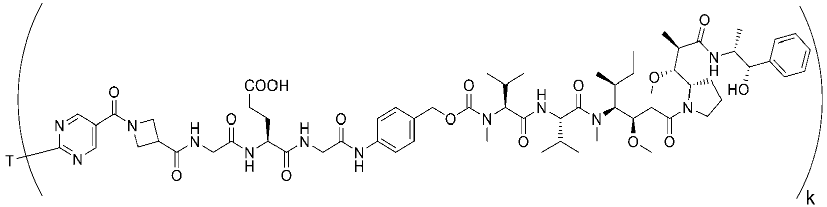 |
| CC7 | 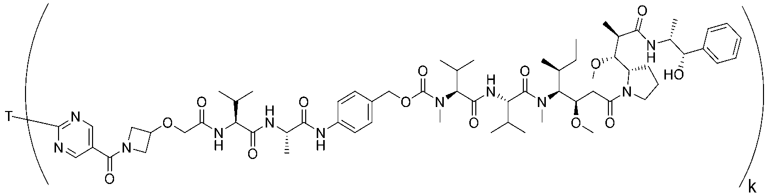 |
| CC8 | 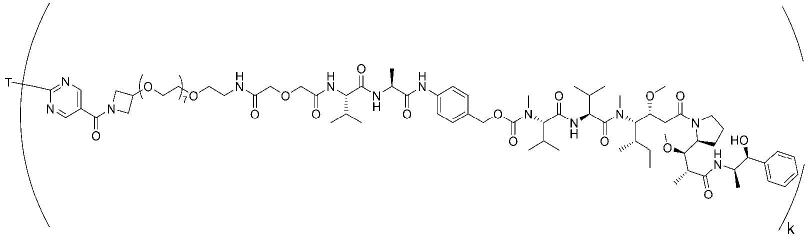 |
| CC9 | 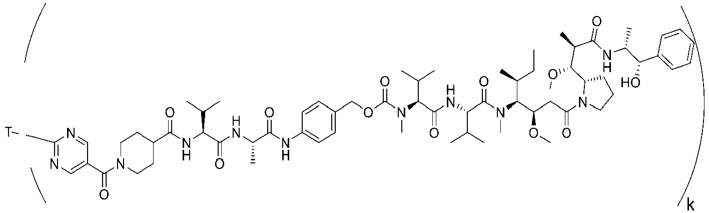 |
| CC10 | 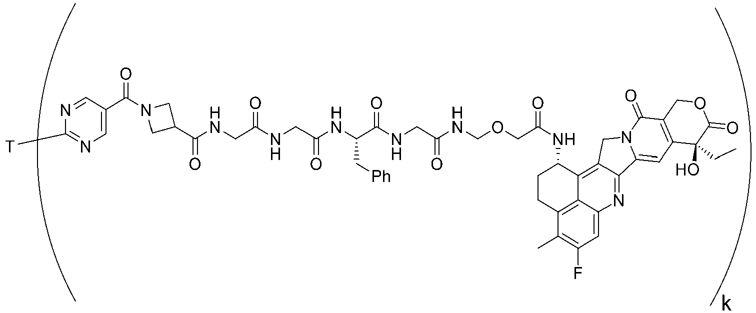 |
| CC11 | 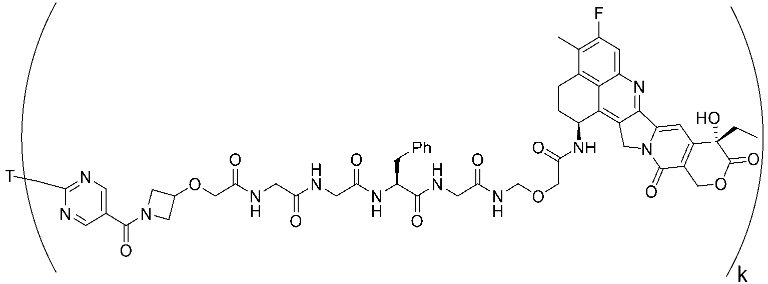 |
| CC12 | 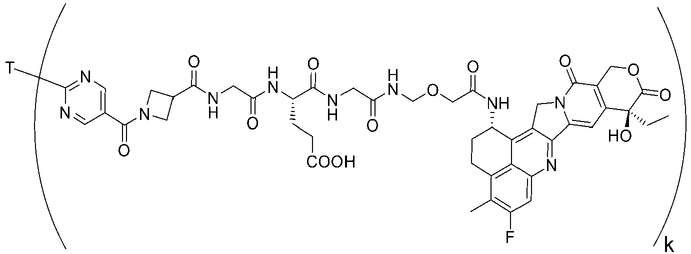 |
| CC13 | 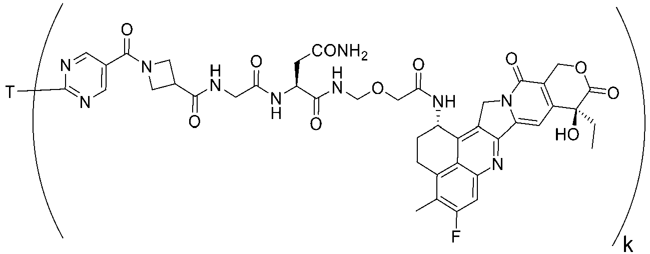 |
| CC14 | 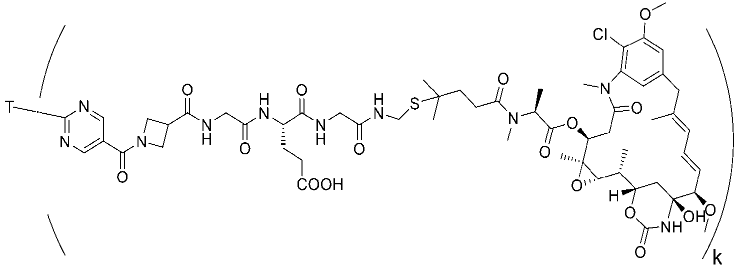 |
| CC15 | 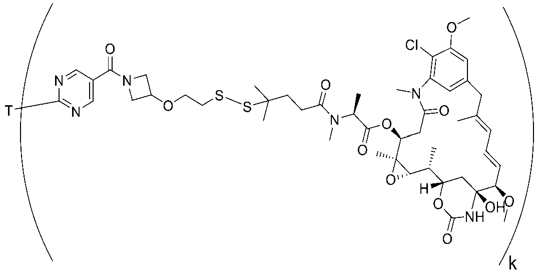 |
| CC16 | 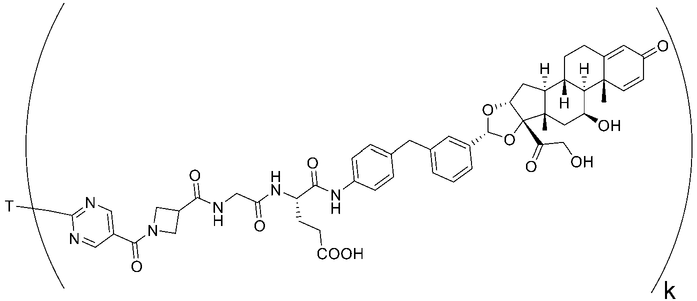 |
| CC17 | 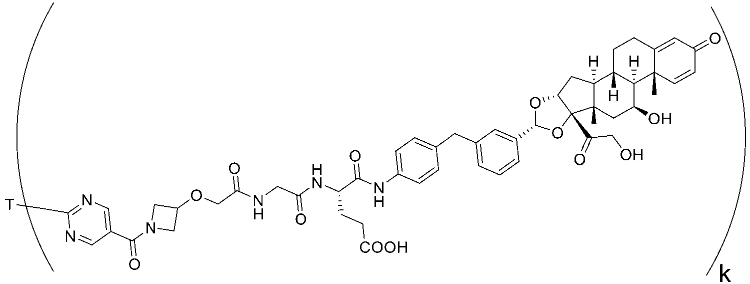 |
| CC19 | 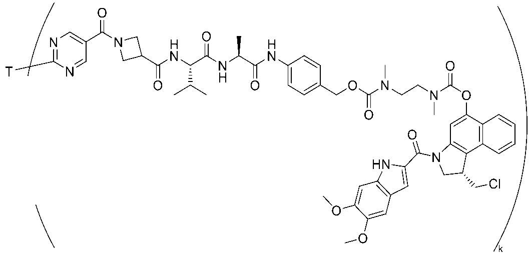 |
| CC20 | 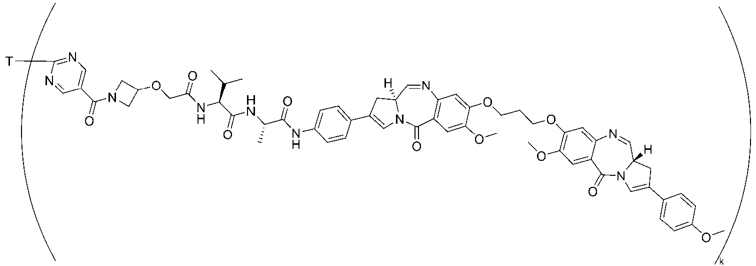 |
| CC22 | 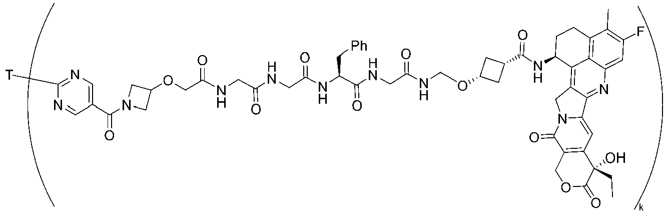 |
| CC23 | 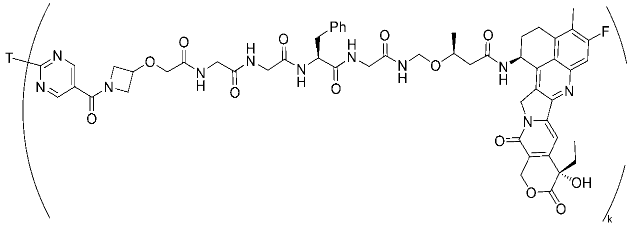 |
| CC24 | 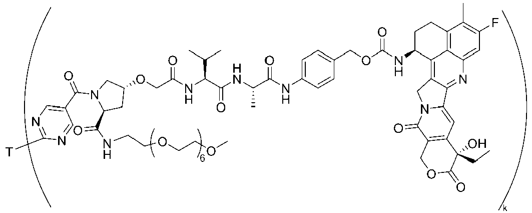 |
| CC25 | 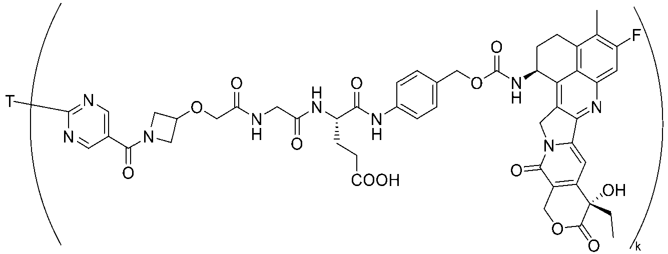 |
| CC26 | 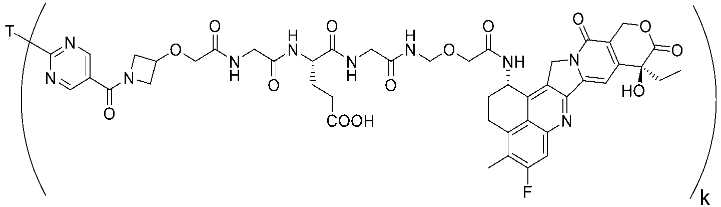 |
| CC27 | 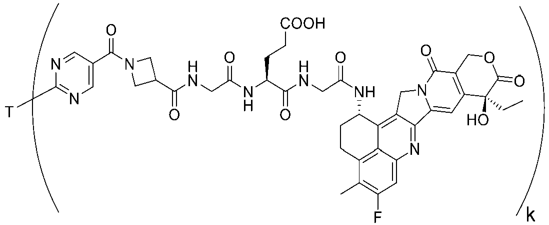 |
| CC28 | 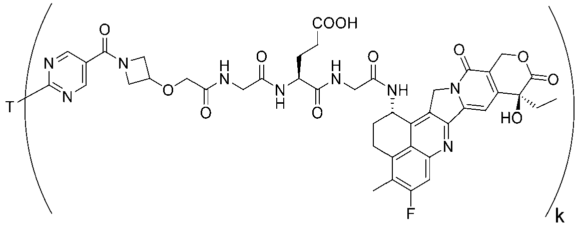 |
| CC29 | 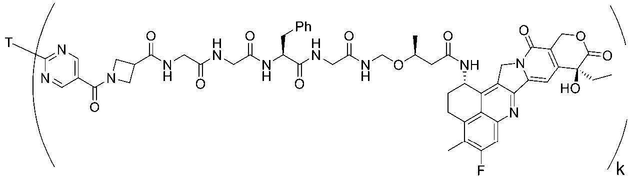 |
| CC30 | 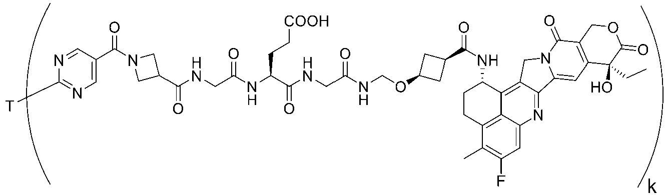 |
| CC31 | 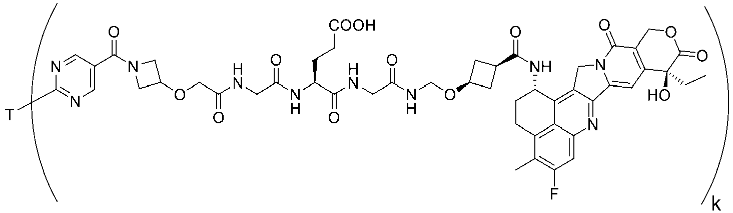 |
| CC32 | 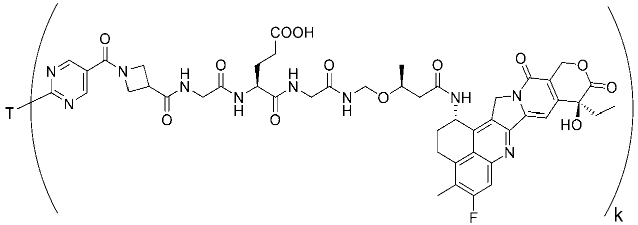 |
| CC33 | 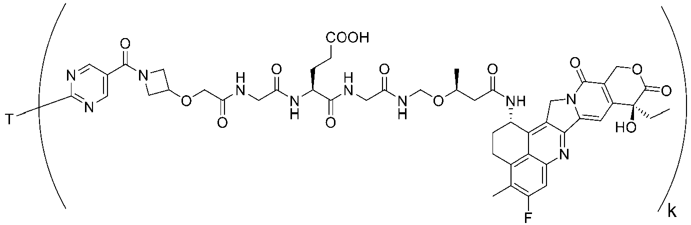 |
preferably, the compound of formula (III-1) is any one of the following compounds,
| | |
|---|---|
| ADC-1C | 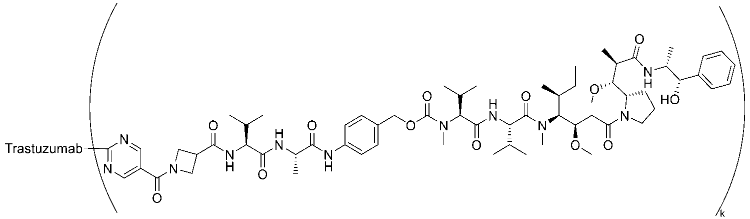 |
| ADC-2C | 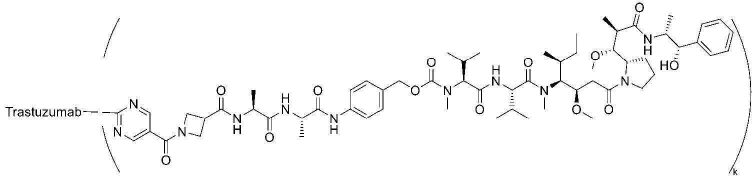 |
| ADC-3C | 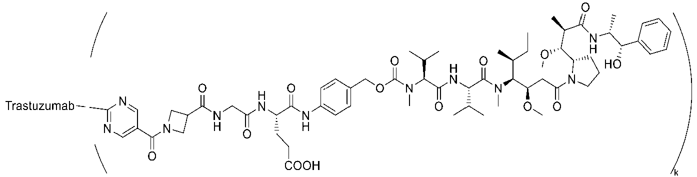 |
| ADC-4C | 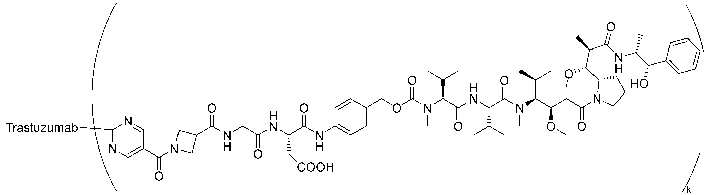 |
| ADC-5C | 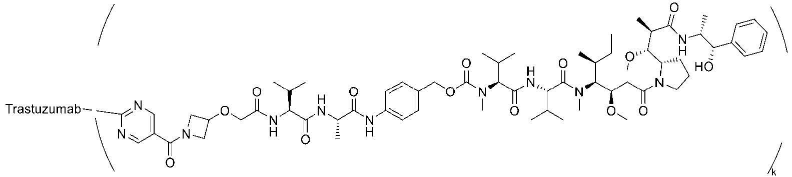 |
| ADC-6C | 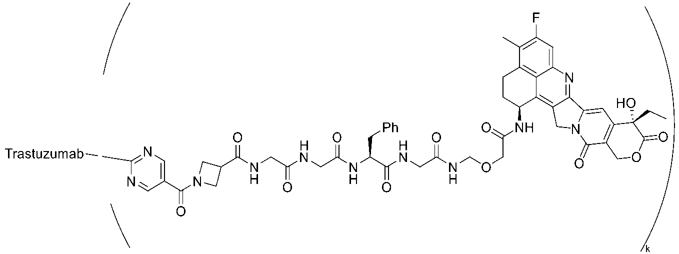 |
| ADC-7C | 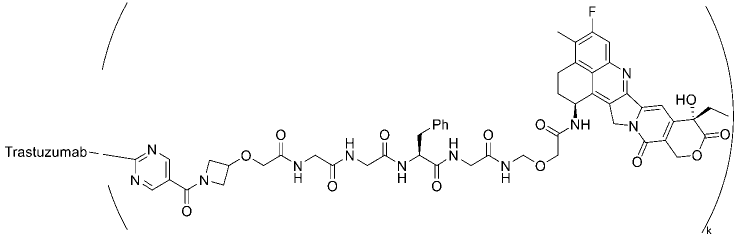 |
| ADC-8C | 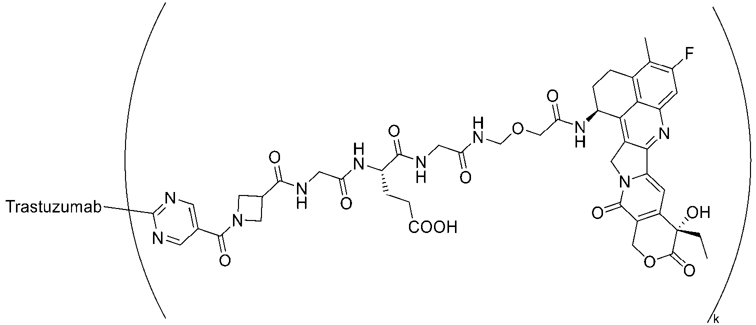 |
| ADC-9C | 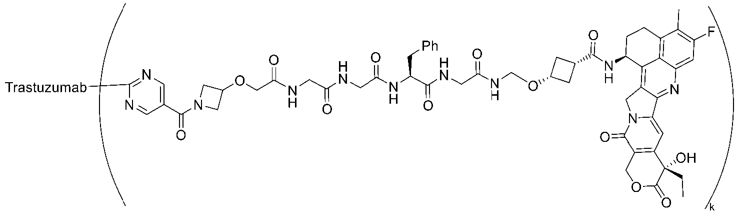 |
| ADC-10C | 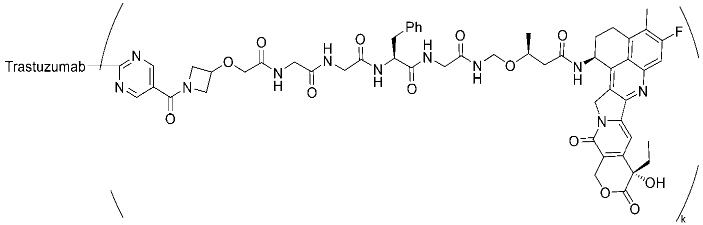 |
| ADC-11C | 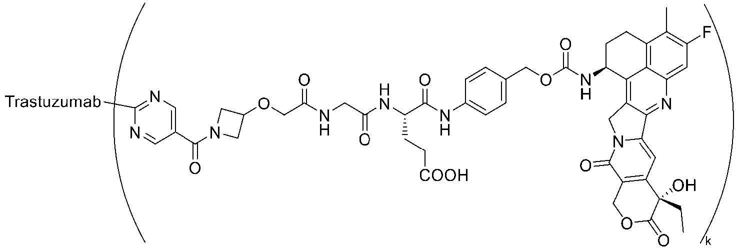 |
| ADC-12C | 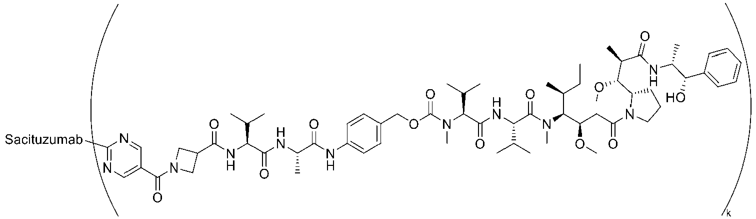 |
| ADC-13C | 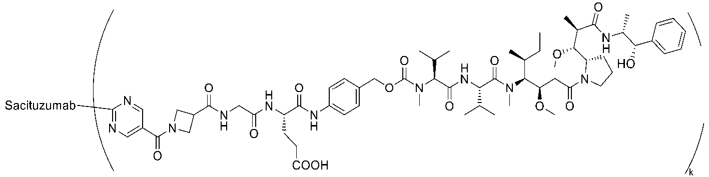 |
| ADC-14C | 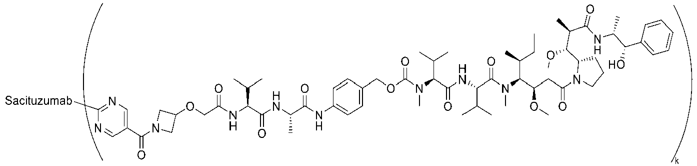 |
| ADC-15C | 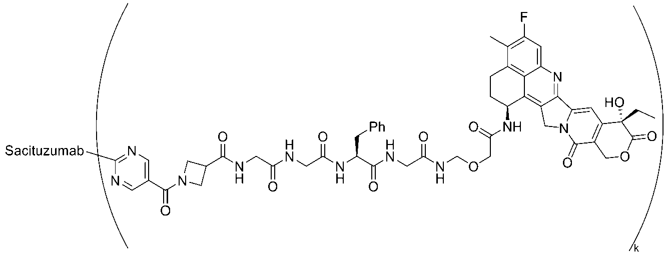 |
| ADC-16C | 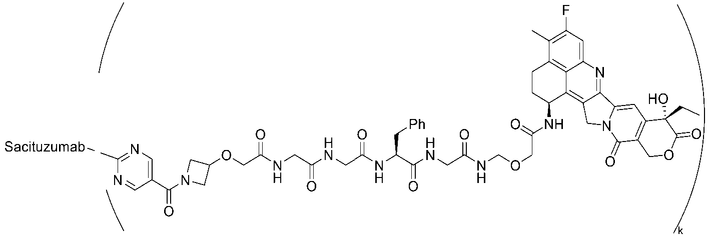 |
| ADC-17C | 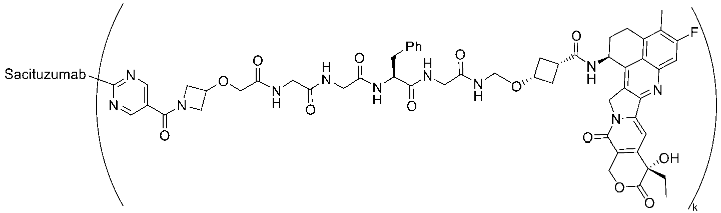 |
| ADC-18C | 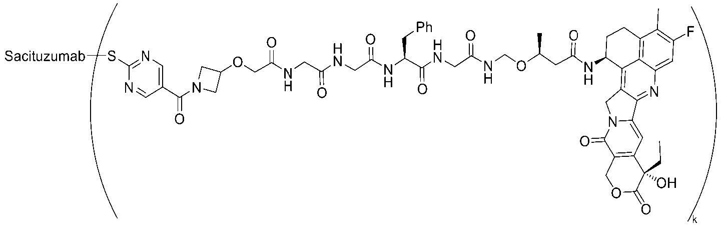 |
| ADC-19C | 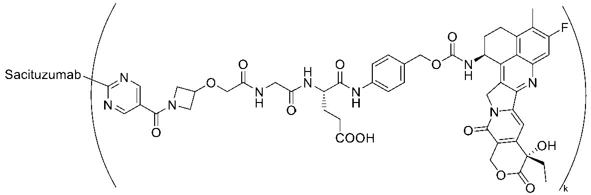 |
| ADC-20C | 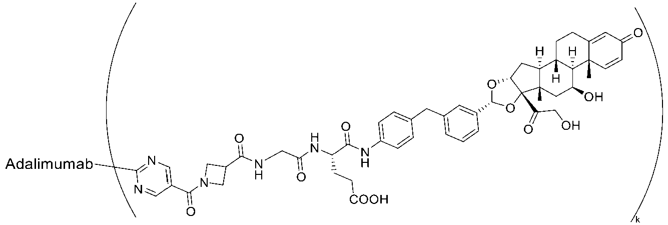 |
| ADC-21C | 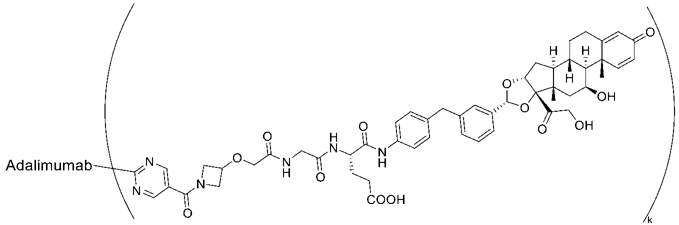 |
| ADC-22C | 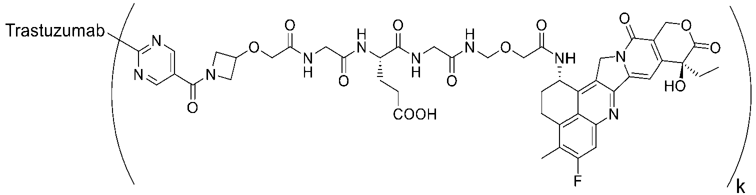 |
| ADC-23C | 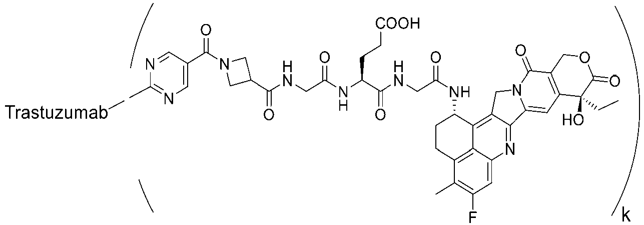 |
| ADC-24C | 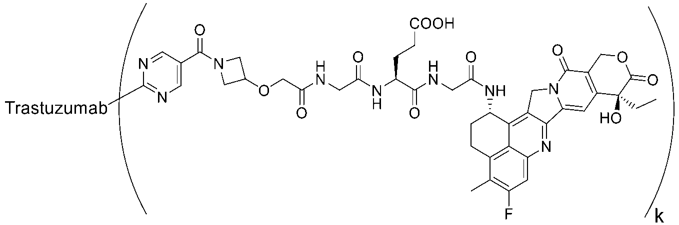 |
| ADC-25C | 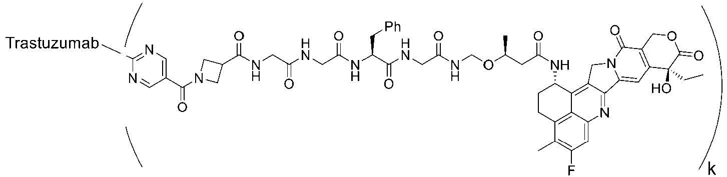 |
| ADC-26C | 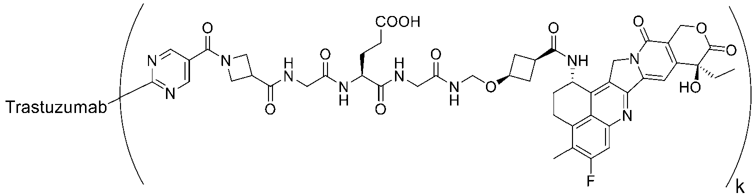 |
| ADC-27C | 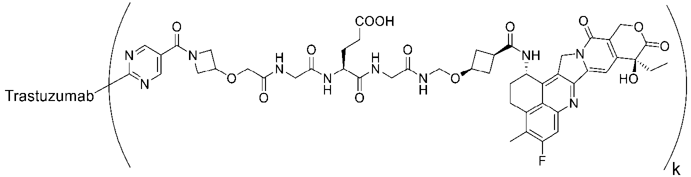 |
| ADC-28C | 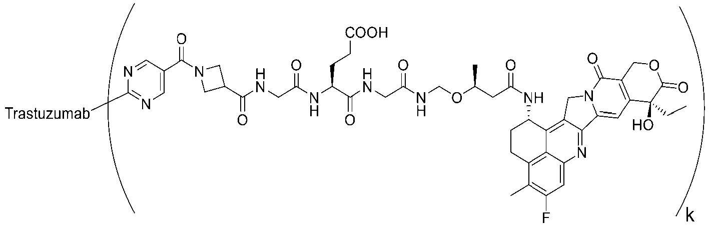 |
| ADC-29C | 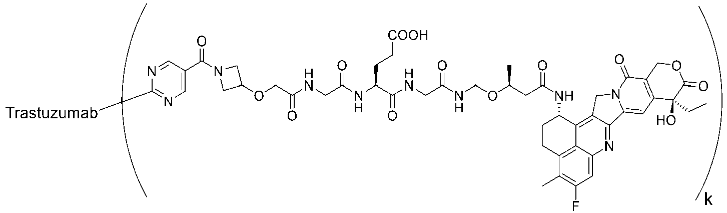 |
| ADC-30C | 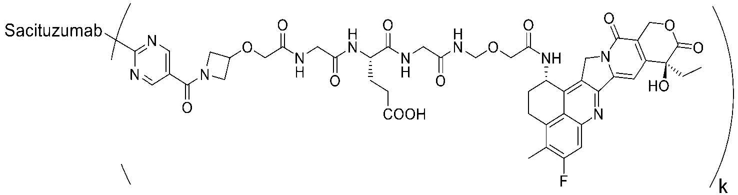 |
| ADC-31C | 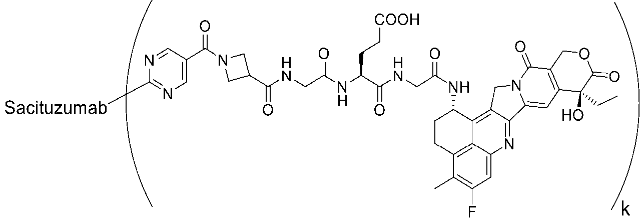 |
| ADC-32C | 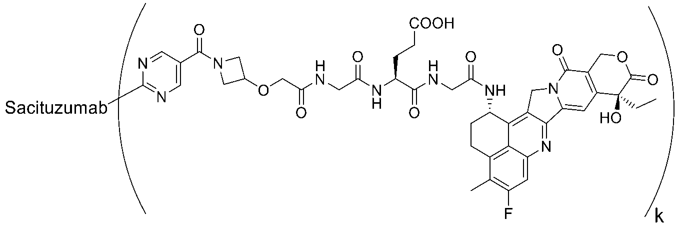 |
| ADC-33C | 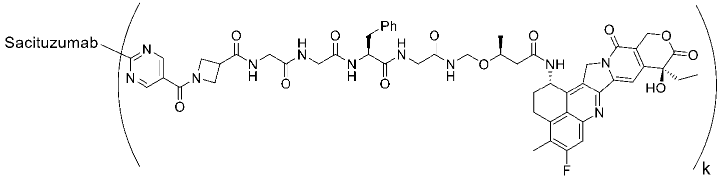 |
| ADC-34C | 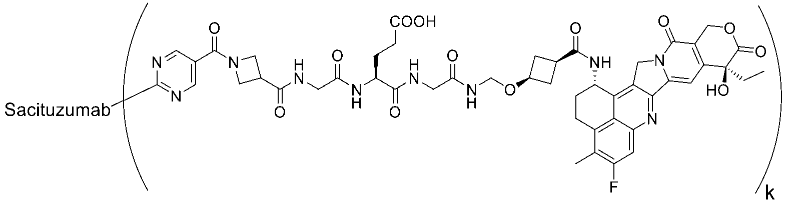 |
| ADC-35C | 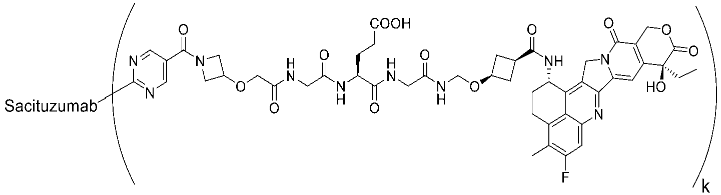 |
| ADC-36C | 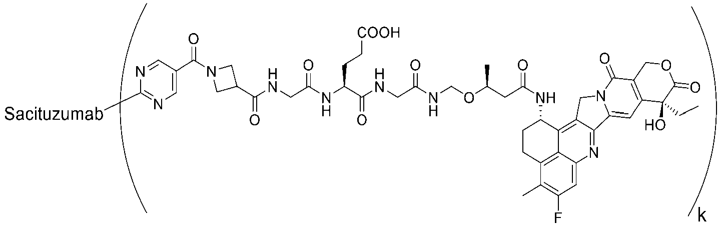 |
| ADC-37C | 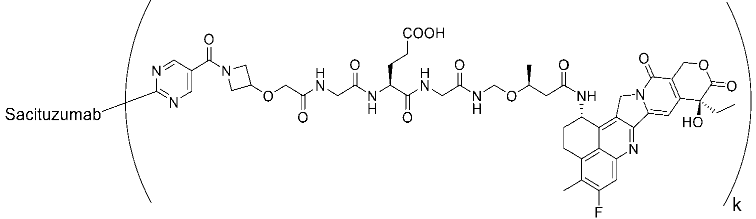 |
; more preferably, the compound of formula (III-1) is any one of the following compounds:
| No. | Structure |
|---|---|
| ADC-1C-1 | 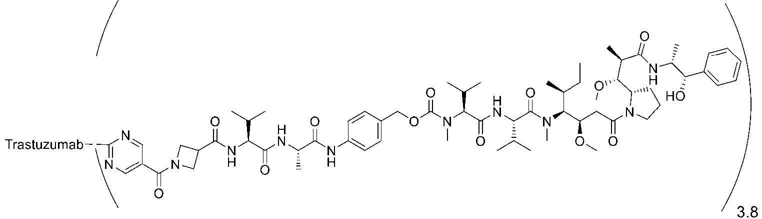 |
| ADC-2C-1 | 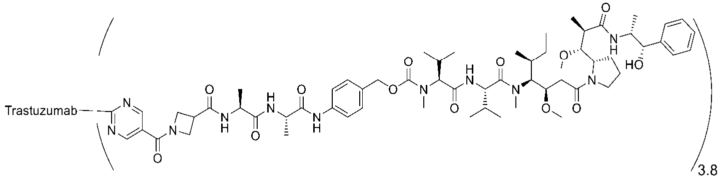 |
| ADC-3C-1 | 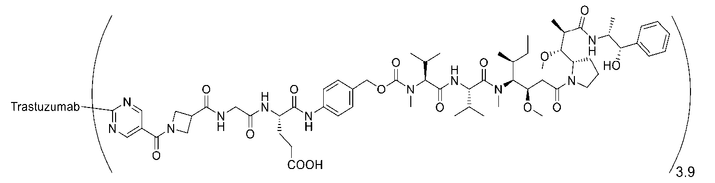 |
| ADC-5C-1 | 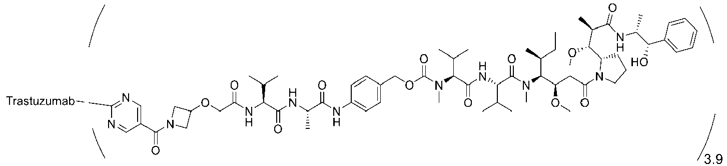 |
| ADC-6C-1 | 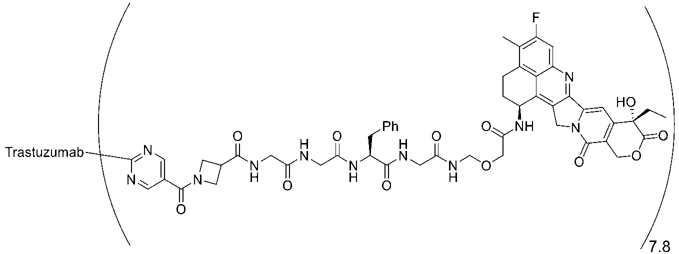 |
| ADC-7C-1 | 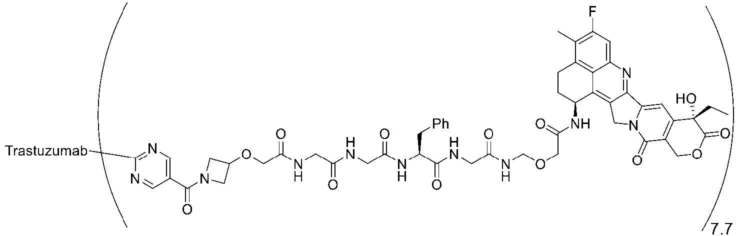 |
| ADC-12C-1 | 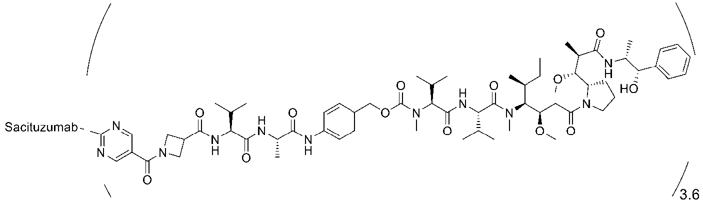 |
| ADC-14C-1 | 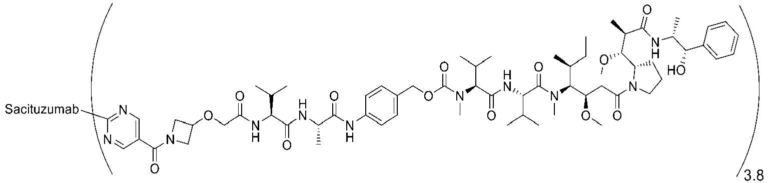 |
| ADC-15C-1 | 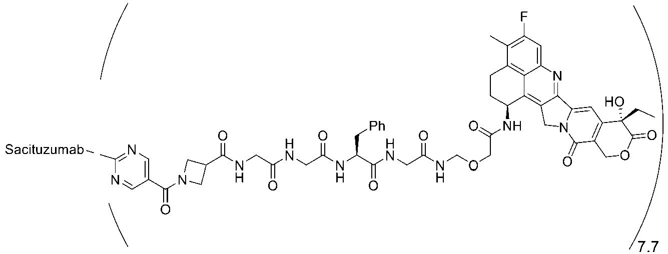 |
| ADC-16C-1 | 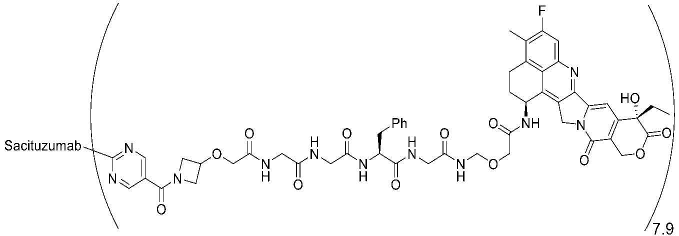 |
| ADC-17C-1 | 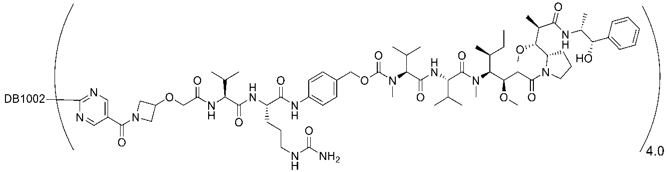 |
| ADC-18C-1 | 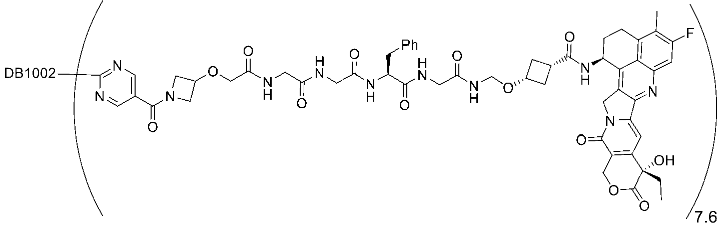 |
| ADC-19C-1 | 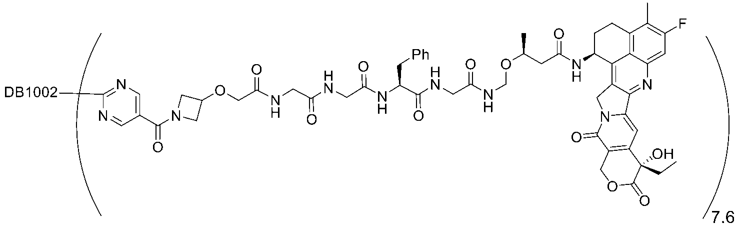 |
| ADC-20C-1 | 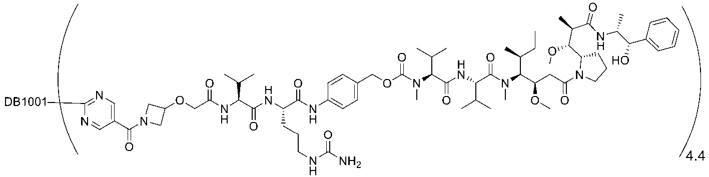 |
| ADC-21C-1 | 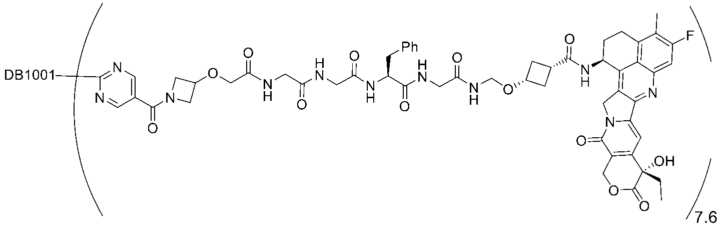 |
| ADC-22C-1 | 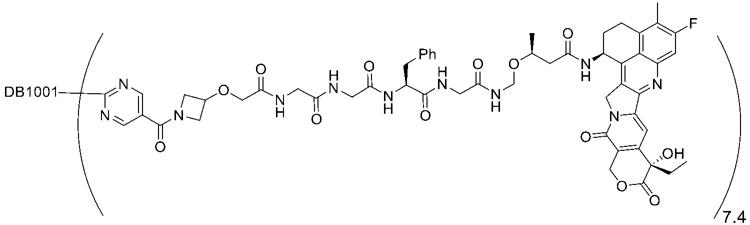 |

13. A compound comprising a structure of formula (III-2), or a mesomer, racemate, enantiomer, diastereomer, or any mixture thereof, or a pharmaceutically acceptable salt thereof, or a solvate thereof, or a pharmaceutically acceptable salt of the solvate thereof: 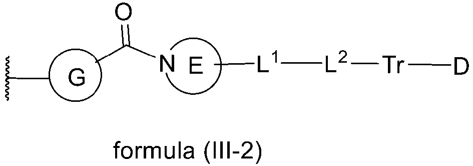 wherein
ring G, ring E, L¹, L², Tr, and D are as defined in any one of claims 1-7.

14. The compound of formula (III-2), or the mesomer, racemate, enantiomer, diastereomer, or any mixture thereof, or the pharmaceutically acceptable salt thereof, or the solvate thereof, or the pharmaceutically acceptable salt of the solvate thereof according to claim 13, wherein
ring G is 5- to 6-membered heteroaryl, wherein the heteroatom of the 5- to 6-membered heteroaryl may be N and/or O, and the number of heteroatoms is 1, 2, or 3; for example, ring G is 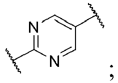
ring E is 4- to 6-membered heterocycloalkylene, wherein the heteroatom of the 4- to 6-membered heterocycloalkylene is N, and the number of heteroatoms is 1 or 2;
the 4- to 6-membered heterocycloalkylene is optionally substituted by one or more - C(O)NH-(CH₂CH₂O)_{y1}CH₃ substituents;
y1 is a natural number from 0 to 30;
L¹ is -(CH₂)ₙ-; wherein n is a natural number from 0 to 50; any -CH₂- moiety in L¹ is independently and optionally replaced by the following structural moieties: -NH-, -C(O)-, -O-, or -S-;
L² is a chemical bond, Val-Ala, Ala-Ala, Gly-Glu, Gly-Asp, Gly-Glu-Gly, or Gly-Gly-Phe-Gly;
Tr is a chemical bond, 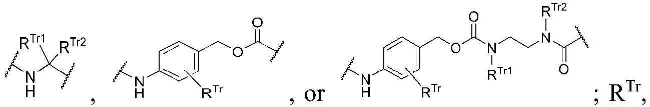 R^{Tr1}, and R^{Tr2} are each independently hydrogen or -C₁₋₆ alkyl;
D is a tubulin inhibitor, a topoisomerase inhibitor, a DNA damaging agent, an RNA polymerase inhibitor, an immunomodulator, or an immunostimulant, for example, D is 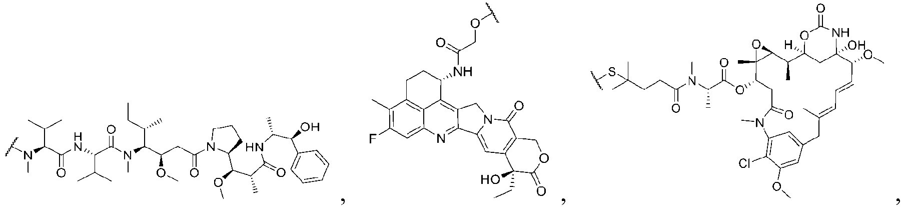 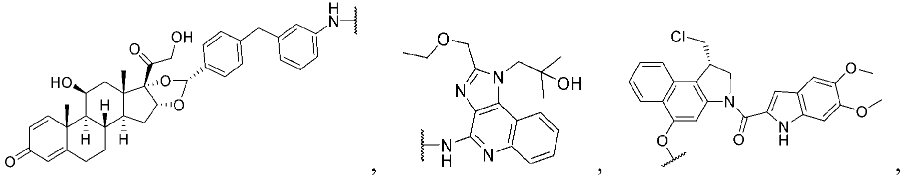 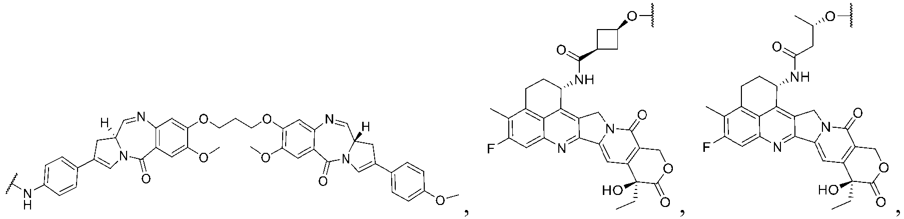
or

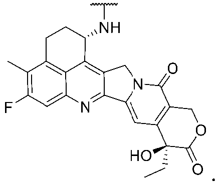

15. The compound comprising formula (III-2), or the mesomer, racemate, enantiomer, diastereomer, or any mixture thereof, or the pharmaceutically acceptable salt thereof, or the solvate thereof, or the pharmaceutically acceptable salt of the solvate thereof according to any one of claim 13 or 14, wherein the formula (III-2) is a compound of formula (IIIa-2),

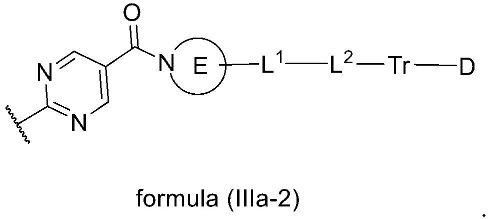

16. A preparation method for the compound of formula (III-1), or the mesomer, racemate, enantiomer, diastereomer, or any mixture thereof, or the pharmaceutically acceptable salt thereof, or the solvate thereof, or the pharmaceutically acceptable salt of the solvate thereof according to any one of claims 8-12, comprising a step of conjugating the compound of formula (III), or the mesomer, racemate, enantiomer, diastereomer, or any mixture thereof, or the pharmaceutically acceptable salt thereof, or the solvate thereof, or the pharmaceutically acceptable salt of the solvate thereof with an active group of T; (for example, the compound of formula (III), or the mesomer, racemate, enantiomer, diastereomer, or any mixture thereof, or the pharmaceutically acceptable salt thereof, or the solvate thereof, or the pharmaceutically acceptable salt of the solvate thereof forms a C-S bond with a thiol group of T).

17. The preparation method for the compound of formula (III-1), or the mesomer, racemate, enantiomer, diastereomer, or any mixture thereof, or the pharmaceutically acceptable salt thereof, or the solvate thereof, or the pharmaceutically acceptable salt of the solvate thereof according to claim 16, wherein the preparation method satisfies one or more of the following conditions,
(1) the preparation method further comprises a step of using a reducing agent (e.g., TCEP) to cleave a disulfide bond of T to obtain a thiol group;
(2) the molar ratio of the T to the compound of formula (III), or the mesomer, racemate, enantiomer, diastereomer, or any mixture thereof, or the pharmaceutically acceptable salt thereof, or the solvate thereof, or the pharmaceutically acceptable salt of the solvate thereof is 1:(1-20);
(3) the conjugating is performed in water and/or an organic solvent; preferably, the organic solvent is selected from one or more of N,N-dimethylformamide, dimethyl sulfoxide, *N-*methylpyrrolidone, nitriles (e.g., acetonitrile), and alcohols (e.g., methanol and ethanol);
(4) a step of purifying the conjugation product is further comprised; preferably, the conjugation product is purified by a chromatography method (e.g., one or more of ion exchange chromatography, hydrophobic chromatography, reverse-phase chromatography, or affinity chromatography).

18. A pharmaceutical composition comprising the compound of formula (III) according to any one of claims 1-7, the compound of formula (III-1) according to any one of claims 8-12, or the compound comprising the structure of formula (III-2) according to any one of claims 13-15, or a mesomer, racemate, enantiomer, diastereomer, or any mixture thereof, or a pharmaceutically acceptable salt thereof, or a solvate thereof, or a pharmaceutically acceptable salt of the solvate thereof, and one or more pharmaceutical excipients.

19. A use of substance H or the pharmaceutical composition according to claim 18 in the manufacture of a medicament for treating a disease associated with abnormal cell activity; wherein the substance H is the compound of formula (III), or the mesomer, racemate, enantiomer, diastereomer, or any mixture thereof, or the pharmaceutically acceptable salt thereof, or the solvate thereof, or the pharmaceutically acceptable salt of the solvate thereof according to any one of claims 1-7; or the substance H is the compound of formula (III-1), or the mesomer, racemate, enantiomer, diastereomer, or any mixture thereof, or the pharmaceutically acceptable salt thereof, or the solvate thereof, or the pharmaceutically acceptable salt of the solvate thereof according to any one of claims 8-12; or the substance H is the compound comprising the structure of formula (III-2), or the mesomer, racemate, enantiomer, diastereomer, or any mixture thereof, or the pharmaceutically acceptable salt thereof, or the solvate thereof, or the pharmaceutically acceptable salt of the solvate thereof according to any one of claims 13-15;
preferably, treating the disease associated with abnormal cell activity is a solid tumor or a non-solid tumor, for example, selected from esophageal cancer (e.g., esophageal adenocarcinoma and esophageal squamous cell carcinoma), brain tumor, lung cancer (e.g., small cell lung cancer and non-small cell lung cancer), squamous cell carcinoma, bladder cancer, gastric cancer, ovarian cancer, peritoneal cancer, pancreatic cancer, breast cancer, head and neck cancer, cervical cancer, endometrial cancer, colorectal cancer, liver cancer, kidney cancer, non-Hodgkin's lymphoma, central nervous system tumor (e.g., neuroglioma, glioblastoma multiforme, glioma, or sarcoma), prostate cancer, or thyroid cancer.

20. A use of substance H or the pharmaceutical composition according to claim 18 in the manufacture of a medicament, wherein the substance H is the compound of formula (III), or the mesomer, racemate, enantiomer, diastereomer, or any mixture thereof, or the pharmaceutically acceptable salt thereof, or the solvate thereof, or the pharmaceutically acceptable salt of the solvate thereof according to any one of claims 1-7; or the substance H is the compound of formula (III-1), or the mesomer, racemate, enantiomer, diastereomer, or any mixture thereof, or the pharmaceutically acceptable salt thereof, or the solvate thereof, or the pharmaceutically acceptable salt of the solvate thereof according to any one of claims 8-12; or the substance H is the compound comprising the structure of formula (III-2), or the mesomer, racemate, enantiomer, diastereomer, or any mixture thereof, or the pharmaceutically acceptable salt thereof, or the solvate thereof, or the pharmaceutically acceptable salt of the solvate thereof according to any one of claims 13-15;
the medicament is used for preventing or treating cancer, wherein the cancer disease may be a solid tumor or a non-solid tumor, for example, selected from esophageal cancer (e.g., esophageal adenocarcinoma and esophageal squamous cell carcinoma), brain tumor, lung cancer (e.g., small cell lung cancer and non-small cell lung cancer), squamous cell carcinoma, bladder cancer, gastric cancer, ovarian cancer, peritoneal cancer, pancreatic cancer, breast cancer, head and neck cancer, cervical cancer, endometrial cancer, colorectal cancer, liver cancer, kidney cancer, non-Hodgkin's lymphoma, central nervous system tumor (e.g., neuroglioma, glioblastoma multiforme, glioma, or sarcoma), prostate cancer, or thyroid cancer;
alternatively, the medicament is used for treating a disease associated with a target of T; T is as defined in any one of claims 8-12.

21. A method for treating a disease associated with abnormal cell activity (e.g., a cancer disease), comprising administering to an individual in need thereof an effective amount of substance H or the pharmaceutical composition according to claim 18, wherein the substance H is the compound of formula (III), or the mesomer, racemate, enantiomer, diastereomer, or any mixture thereof, or the pharmaceutically acceptable salt thereof, or the solvate thereof, or the pharmaceutically acceptable salt of the solvate thereof according to any one of claims 1-7; or the substance H is the compound of formula (III-1), or the mesomer, racemate, enantiomer, diastereomer, or any mixture thereof, or the pharmaceutically acceptable salt thereof, or the solvate thereof, or the pharmaceutically acceptable salt of the solvate thereof according to any one of claims 8-12; or the substance H is the compound comprising the structure of formula (III-2), or the mesomer, racemate, enantiomer, diastereomer, or any mixture thereof, or the pharmaceutically acceptable salt thereof, or the solvate thereof, or the pharmaceutically acceptable salt of the solvate thereof according to any one of claims 13-15.
